# EUROPÄISCHE PATENTSCHRIFT

(11) **EP 2 406 216 B1**
(45) Veröffentlichungstag und Bekanntmachung des Hinweises auf die Patenterteilung: **19.04.2017**
(21) Anmeldenummer: 10708924.5
(22) Anmeldetag: 05.03.2010
(51) Int. Cl.: C07D 209/54, C07D 307/94, C07D 487/04, C07D 493/10, A01N 43/12, A01N 43/16

(54) **HALOGENALKYLMETHYLENOXY-PHENYL-SUBSTITUIERTE KETOENOLE**
HALOGENALKYLMETHYLENOXY-PHENYL-SUBSTITUTED KETOENOLS
KÉTOÉNOLES SUBSTITUÉS PAR HALOGENALKYLMÉTHYLENOXY-PHÉNYL

(30) Priorität: 11.03.2009 EP 09154888
(43) Veröffentlichungstag der Anmeldung: 18.01.2012
(60) Teilanmeldung: 16193742.0
(73) Patentinhaber: Bayer Intellectual Property GmbH, 40789 Monheim am Rhein (DE)
(72) Erfinder: FISCHER, Reiner, 40789 Monheim (DE); BRETSCHNEIDER, Thomas, 53797 Lohmar (DE); LEHR, Stefan, 65835 Liederbach (DE); FRANKEN, Eva-Maria, 51381 Leverkusen (DE); MALSAM, Olga, 51503 Rösrath (DE); VOERSTE, Arnd, 50674 Köln (DE); GÖRGENS, Ulrich, 40882 Ratingen (DE); DITTGEN, Jan, 60316 Frankfurt (DE); FEUCHT, Dieter, 65760 Eschborn (DE); HÄUSER-HAHN, Isolde, 51375 Leverkusen (DE); ROSINGER, Christopher Hugh, 65719 Hofheim (DE); ANGERMANN, Alfred, 65830 Kriftel (DE)
(74) Vertreter: BIP Patents
(86) Internationale Anmeldenummer: PCT/EP2010/001392
(87) Internationale Veröffentlichungsnummer: WO 2010/102758

(56) Entgegenhaltungen:
- EP-A2- 0 415 211
- EP-A2- 0 442 077
- EP-A2- 0 502 492
- WO-A1-02/24704
- WO-A1-2008/110307
- WO-A1-2008/121065
- WO-A1-2008/121066
- DATABASE REGISTRY [Online] CHEMICAL ABSTRACTS SERVICE, COLUMBUS, OHIO, US; 31. Oktober 2008 (2008-10-31), XP002586767 Database accession no. 1069123-08-5

## Beschreibung

Die vorliegende Erfindung betrifft neue Halogenalkylmethylenoxy-phenyl-substituierte Ketoenole, mehrere Verfahren zu ihrer Herstellung und ihre Verwendung als Schädlingsbekämpfungsmittel und/oder Herbizide. Auch Gegenstand der Erfindung sind selektiv herbizide Mittel, die Halogenalkylmethylenoxy-phenyl-substituierte Ketoenole einerseits und eine die Kulturpflanzen-verträglichkeit verbessernde Verbindung andererseits enthalten.

Die vorliegende Erfindung betrifft weiterhin die Steigerung der Wirkung von Pflanzenschutzmitteln enthaltend insbesondere Halogenalkylmethylenoxy-phenyl-substituierte Ketoenole, durch die Zugabe von Ammonium- oder Phosphoniumsalzen und gegebenenfalls Penetrationsförderern, die entsprechenden Mittel, Verfahren zur ihrer Herstellung und ihre Anwendung im Pflanzenschutz als Insektizide und/oder Akarizide und/oder zur Verhinderung von unerwünschtem Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 95/01 971, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869, WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049569, WO 05/066125, WO 05/092897, WO 06/000355, WO 06/029799, WO 06/056281, WO 06/056282, WO 06/089633, WO 07/048545, DEA 102 00505 9892, WO 07/073856, WO 07/096058, WO 07/121868, WO 07/140881, WO 08/067873, WO 08/067910, WO 08/067911, WO 08/138551, WO 09/015801, WO 09/039975, WO 09/049851, WO 09/115262, EP-Anmeldenummer 08170489). Außerdem sind ketalsubstituierte 1-H-Arylpyrrolidin-2,4-dione aus WO 99/16748 und (spiro)-ketalsubstituierte N-Alkoxy-alkoxysubstituierte Aryl-pyrrolidindione aus JP-A-14 205 984 und Ito M. et al.. Bioscience, Biotechnology and Biochemistry 67, 1230-1238, (2003) bekannt. Der Zusatz von Safenern zu Ketoenolen ist ebenfalls prinzipiell aus der WO 03/013249 bekannt. Außerdem sind aus WO 06/024411 herbizide Mittel enthaltend Ketoenole bekannt.

Es ist bekannt, dass bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften bekannt aus: EP-A-528 156, EP-A-647 637, WO 95/26 954, WO 96/20 196, WO 96/25 395, WO 96/35 664, WO 97//01 535, WO 97/02 243, WO 97/36 868, WO 98//05 638, WO 98/06 721, WO 99/16 748, WO 98/25 928, WO 99/43 649, WO 99/48 869, WO 99/55 673, WO 01/23354, WO 01/74 770, WO 01/17 972, WO 04/024 688, WO 04/080 962, WO 04/111 042, WO 05/092 897, WO 06/000 355, WO 06/029 799, WO 07/048545, WO 07/073856, WO 07/096058, WO 07/121868, WO 07/140881, WO 08/067911, WO 08/083950, WO 09/015801, WO 09/039975.

Auch 3-Aryl-Δ³-dihydrothiphen-on-Derivate sind bekannt aus WO 95/26 345, 96/25 395, WO 97/01 535, WO 97/02 243, WO 97/36 868, WO 98/05638, WO 98/25928, WO 99/16748, WO 99/43649, WO 99/48869, WO 99/55673, WO 01/ 17972, WO 01/23354, WO 01/74770, WO 03/013249, WO 04/080 962, WO 04/111 042, WO 05/092897, WO 06/029799 und WO 07/096058.

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenylpyron-Derivate mit herbiziden, akariziden und insektiziden Eigenschaften sind in EP-A-588 137, WO 96/25 395, WO 96/35 664, WO 97/01 535, WO 97/02 243, WO 97/16 436, WO 97/19 941, WO 97/36 868, WO 98/05638, WO 99/43649, WO 99/48869, WO 99/55673, WO 01/17972, WO 01/74770, WO 03/013249, WO 04/080 962, WO 04/111 042, WO 05/092897, WO 06/029799 und WO 07/096058 beschrieben. Weiterhin sind isomere Pyran-3.5-dione in WO 08/071405 und WO 09/074314 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/2 5395, WO 96/35 664, WO 97/01 535, WO 97/02 243, WO 97/02 243, WO 97/36 868, WO 99/43649, WO 99/48869, WO 99/55673, WO 01/17972, WO 01/74770, WO 03/013249, WO 04/080 962, WO 04/111 042, WO 05/092897, WO 06/029799 und WO 07/096058 beschrieben.

Es ist bekannt, dass bestimmte substituierte 2-Arylcyclopentandione, herbizide, insektizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO99/48869, WO 99/55673, WO 01/17972, WO 01/74770, WO 03/062244, WO 04/080962, WO04/111042, WO05/092897, WO06/029799, WO07/080066, WO07/096058, WO 09/019005, WO 09/019015 und EP-Anmeldenummer 08166352). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-A 2 361 084 bekannt, mit Angabe von herbiziden und akariziden Wirkungen.

Es ist bekannt, dass bestimmte substituierte 2-Arylcyclohexandione herbizide, insektizide und akarizide Eigenschaften besitzen (US-4 175 135, 4 256 657, 4 256 658, 4 256 659, 4 257 858, 4 283 348, 4 303 669, 4 351 666, 4 409 153, 4 436 666, 4 526 723, 4 613 617, 4 659 372, DE-A 2 813 341, sowie Wheeler, T.N., J. Org. Chem. 44, 4906 (1979)), WO 99/43649, WO 99/48869, WO 99/55673, WO 01/17972, WO 01/74770, WO 03/013249, WO 04/080 962, WO 04/111 042, WO 05/092897, WO 06/029799, WO 07/096058, WO 08/071405,WO 08/110307, WO 08/110308 und WO 08/145336.

Es ist bekannt, dass bestimmte substituierte 4-Aryl-pyrazolidin-3,5-dione akarizide, insektizide und herbizide Eigenschaften besitzen (vgl. z.B. WO 92/16 510, EP-A-508 126, WO 96/11 574, WO 96/21 652, WO 99/47525, WO 01/17 351, WO 01/17 352, WO 01/17 353, WO 01/17 972, WO 01/17 973, WO 03/028 466, WO 03/062 244, WO 04/080 962, WO 04/111 042, WO 05/005428, WO 05/016873, WO 05/092897, WO 06/029799 und WO 07/096058).

Es ist bekannt, dass bestimmte Tetrahydropyridone herbizide Eigenschaften besitzen (JP 0832530). Außerdem sind spezielle 4-Hydroxytetrahydropyridone mit akariziden, insektiziden und herbiziden Eigenschaften bekannt (JP 11152273). Weiterhin bekannt wurden 4-Hydroxytetrahydropyridone als Schädlingsbekämpfungsmittel und Herbizide in WO 01/79204 und WO 07/096058.

Es ist bekannt, dass bestimmte 5,6-Dihydropyron-Derivate als Proteaseinhibitoren antivirale Eigenschaften haben (WO 95/14012). Weiterhin ist das 4-Phenyl-6-(2-phenethyl)-5,6-dihydropyron aus der Synthese von Kawalakton-Derivaten bekannt (Kappe et al., Arch. Pharm. 309, 558-564 (1976)). Außerdem sind 5,6-Dihydropyron-Derivate als Zwischenprodukte bekannt (White, J.D., Brenner, J.B., Deinsdale, M. J., J. Amer. Chem. Soc. 93, 281 - 282 (1971)). 3-Phenyl-5,6-dihydropyron-Derivate mit Anwendungen im Pflanzenschutz sind in WO 01/98288 und WO 07/09658 beschrieben.

4-Phenylsubstituierte [1.2]-Oxazin-3,5-dione sind als Herbizide erstmalig in WO 01/17972 beschrieben. Weiterhin wurden 4-Acyl-substituierte [1.2]-Oxazin-3,5-dione als Pestizide vor allem aber als Herbizide und Wachstumsregulatoren beschrieben z.B. in EP-A-39 48 89; WO 92/07837, US 5,728,831 sowie als Herbizide und Schädlingsbekämpfungsmittel in WO03/048138.

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I) gefunden 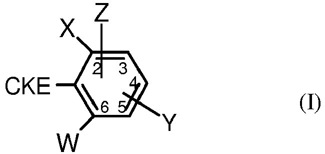 in welcher
- W: für Wasserstoff, Alkyl, Halogen, Halogenalkyl, Alkoxy oder Halogenalkoxy steht,
- X: für Alkyl, Alkenyl, Alkinyl, Halogen, Alkoxy, Halogenalkyl, Halogenalkoxy oder Cyano steht,
- Y: für Wasserstoff, Alkyl, Alkoxy oder Halogen steht,
- Z: für eine Gruppe 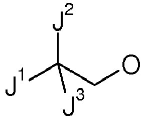
steht, in welcher J¹ und J² jeweils unabhängig voneinander für Wasserstoff oder Halogen stehen und J³ für Halogen oder eine Halogenalkylgruppe steht,
- CKE: für eine der Gruppen 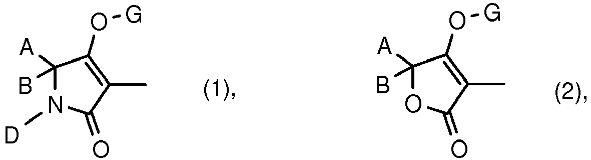 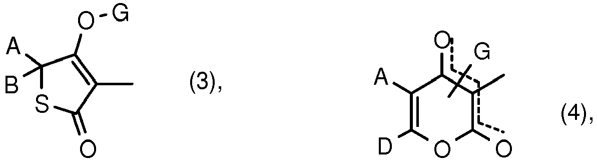 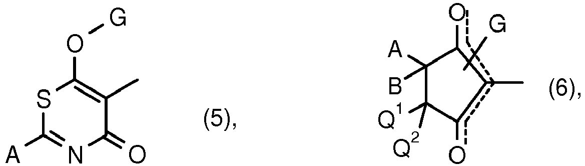 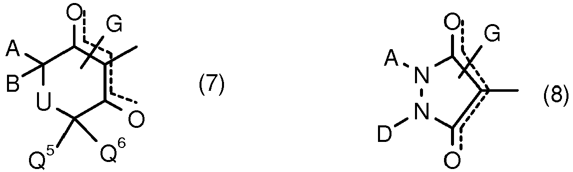 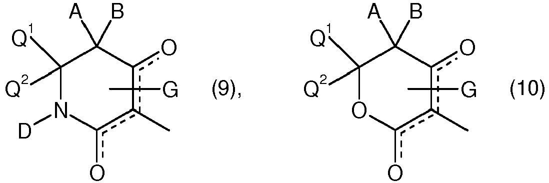
oder 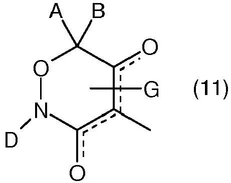 steht, worin
- U: für -S-, -S(O)-, -S(O)₂-, -O-, 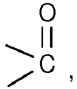
eine S=N-, S(O)=N- oder 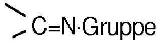 oder für gegebenenfalls durch Q³ und Q⁴ substituiertes C₁-C₄-Alkylen steht, welches gegebenenfalls durch Sauerstoff unterbrochen sein kann,
- A: für Wasserstoff, für jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
- B: für Wasserstoff, Alkyl oder Alkoxyalkyl steht, oder
- A und B: gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
- D: für Wasserstoff oder einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome ersetzt sind, für jeweils gegebenenfalls substituiertes Arylalkyl, Aryl, Hetarylalkyl oder Hetaryl steht oder
- A und D: gemeinsam mit den Atomen an die sie gebunden sind für einen gesättigten oder ungesättigten und gegebenenfalls mindestens ein (im Falle CKE=8 und 11 ein weiteres) Heteroatom enthaltenden, im A,D-Teil unsubstituierten oder substituierten Cyclus stehen, oder
- A und Q¹: gemeinsam für jeweils gegebenenfalls substituiertes Alkandiyl oder Alkendiyl stehen welche gegebenenfalls durch mindestens ein Heteroatom,
eine 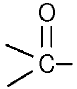 oder substituierte 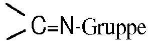 unterbrochen sein können, oder,
- B und Q²: gemeinsam mit den Atomen, an die sie gebunden sind, für einen gesättigten oder ungesättigten und gegebenenfalls mindestens ein Heteroatom enthaltenden, im B, Q²-Teil unsubstituierten oder substituierten Cyclus stehen, oder
- D und Q¹: gemeinsam mit den Atomen, an die sie gebunden sind, für einen gesättigten oder ungesättigten und gegebenenfalls mindestens ein Heteroatom enthaltenden, im D, Q¹-Teil unsubstituierten oder substituierten Cyclus stehen,
- Q¹: für Wasserstoff, Alkyl, Alkoxyalkyl, gegebenenfalls substituiertes Cycloalkyl, worin gegebenenfalls eine Methylengruppe durch Sauerstoff oder Schwefel ersetzt ist oder für gegebenenfalls substituiertes Phenyl steht,
- Q², Q⁴, Q⁵ und Q⁶: unabhängig voneinander für Wasserstoff oder Alkyl stehen,
- Q³: für Wasserstoff, für jeweils gegebenenfalls substituiertes Alkyl, Alkoxy, Alkylthio, Alkoxyalkyl, Alkylthioalkyl, gegebenenfalls substituiertes Cycloalkyl, worin gegebenenfalls eine oder zwei Methylengruppen durch Sauerstoff oder Schwefel ersetzt sind oder für gegebenenfalls substituiertes Phenyl steht, oder
- Q¹ und Q²: gemeinsam mit dem Kohlenstoffatom, an das sie gebunden sind, für einen gegebenenfalls ein Heteroatom enthaltenden unsubstituierten oder substituierten Cyclus stehen, oder
- Q³ und Q⁴: gemeinsam mit dem Kohlenstoffatom, an das sie gebunden sind, für einen gesättigten oder ungesättigten, gegebenenfalls mindestens ein Heteroatom enthaltenden unsubstituierten oder substituierten Cyclus stehen, oder
- A und Q³: gemeinsam mit dem Kohlenstoffatom, an das sie gebunden sind, für einen gesättigten oder ungesättigten, gegebenenfalls mindestens ein Heteroatom enthaltenden unsubstituierten oder substituierten Cyclus stehen, oder
- A und Q⁵: gemeinsam mit dem Kohlenstoffatom, an das sie gebunden sind, für einen gesättigten oder ungesättigten, gegebenenfalls mindestens ein Heteroatom enthaltenden unsubstituierten oder substituierten Cyclus stehen,
- G: für Wasserstoff (a) oder für eine der Gruppen 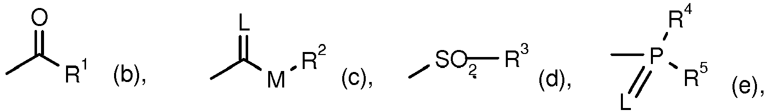
E (f) oder 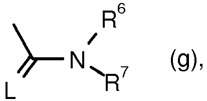 steht, worin
- E: für ein Metallionäquivalent oder ein Ammoniumion steht,
- L: für Sauerstoff oder Schwefel steht,
- M: für Sauerstoff oder Schwefel steht,
- R¹: für jeweils gegebenenfalls durch Halogen substituiertes Alkyl, Alkenyl, Alkoxyalkyl, Alkylthioalkyl, Polyalkoxyalkyl oder für gegebenenfalls durch Halogen, Alkyl oder Alkoxy substituiertes Cycloalkyl, das durch mindestens ein Heteroatom unterbrochen sein kann, für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, für jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Cyclus stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische sind in erfindungsgemäßen Mitteln einsetzbar und lassen sich in ihrer Wirkung durch erfindungsgemäße Ammonium- oder Phosphoniumsalze steigern. Im folgenden wird der Einfachheit halber stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (11) der Gruppe CKE ergeben sich folgende hauptsächliche Strukturen (I-1) bis (I-11): 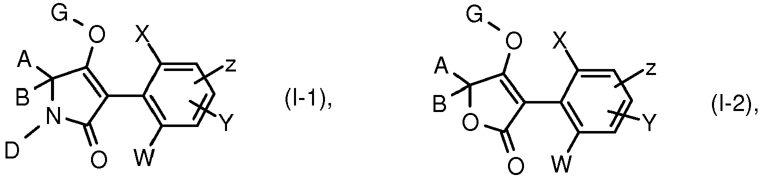 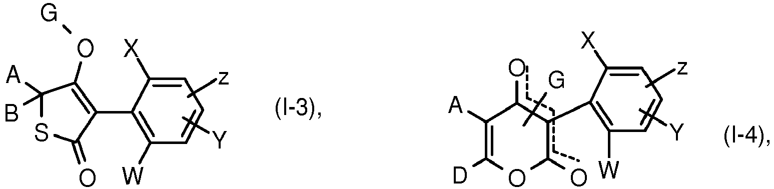 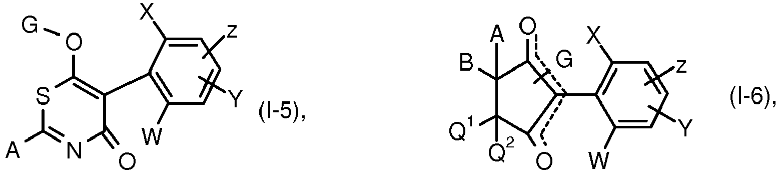 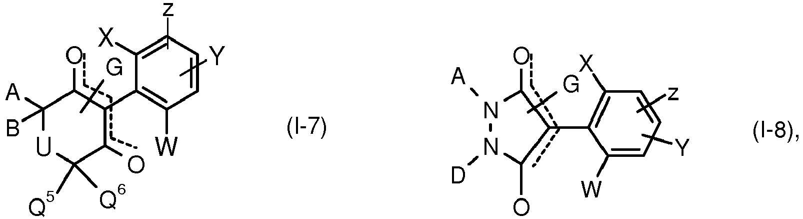 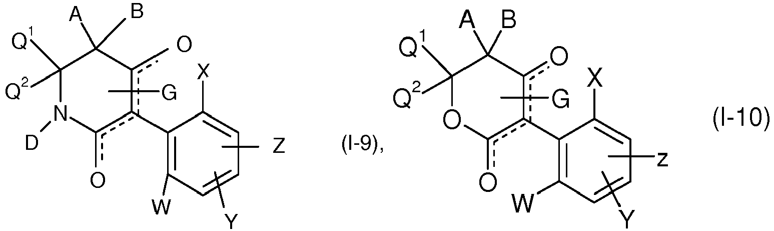 und 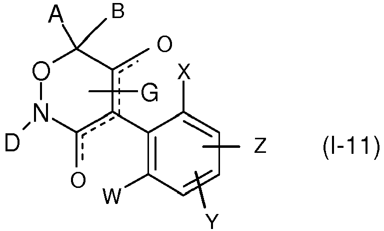 worin
A, B, D, G, Q¹, Q², Q⁵, Q⁶ U, W, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn CKE für die Gruppe (1) steht, 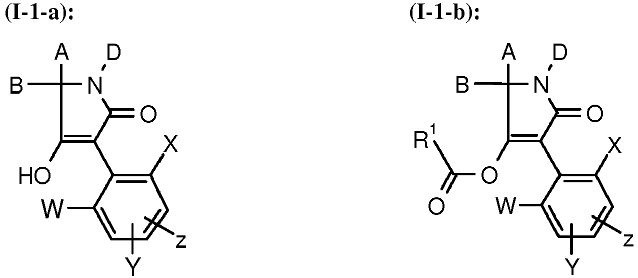 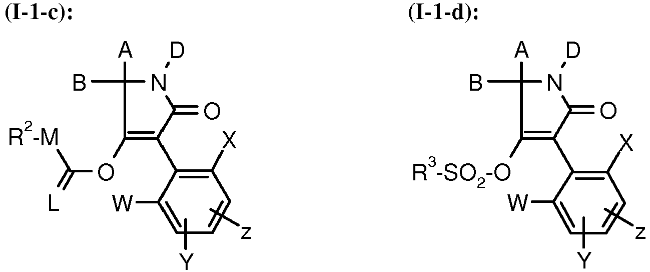 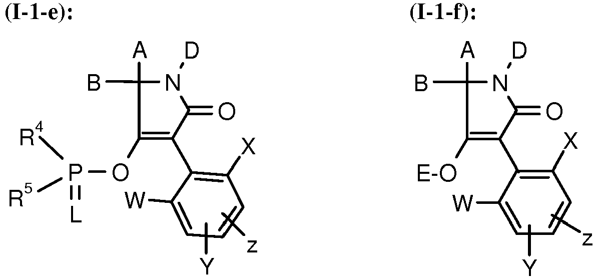 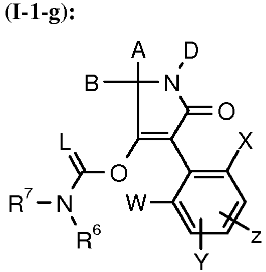 worin
A, B, D, E, L, M, W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn CKE für die Gruppe (2) steht, 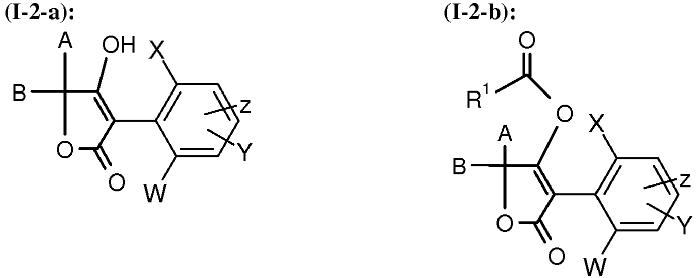 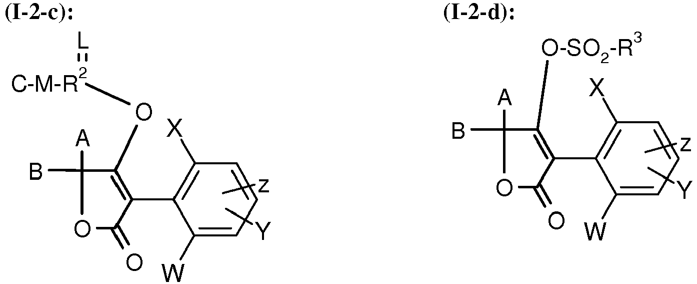 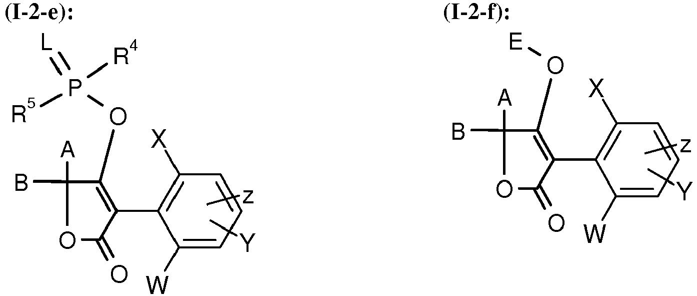 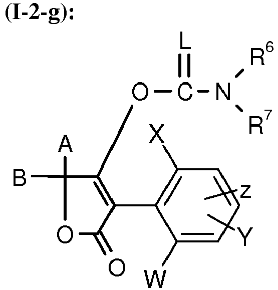 worin
A, B, E, L, M, W, X, Y, Z, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn CKE für die Gruppe (3) steht, 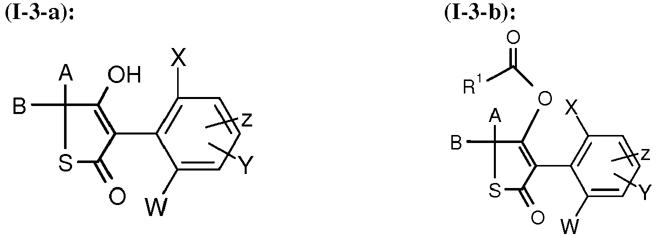 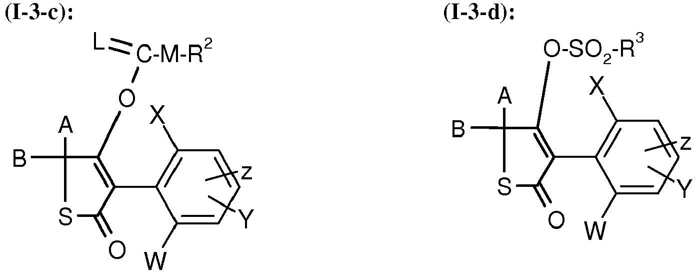 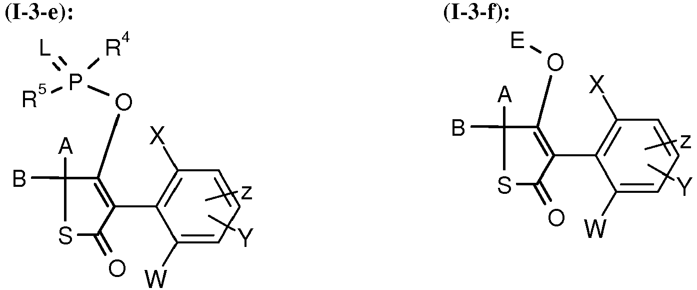 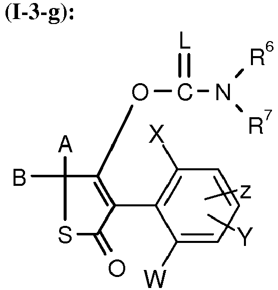 worin
A, B, E, L, M, W, X, Y, Z, R¹, R², R³, R⁴, R⁵, R⁶ und R⁷ die oben angegebenen Bedeutung besitzen.

Die Verbindungen der Formel (I-4) können in Abhängigkeit von der Stellung des Substituenten G in den zwei isomeren Formen der Formeln (I-4-A) und (I-4-B) vorliegen, 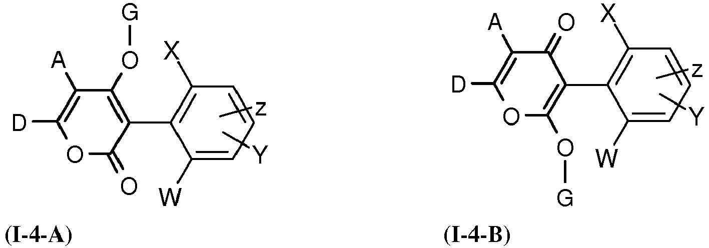 was durch die gestrichelte Linie in der Formel (I-4) zum Ausdruck gebracht werden soll.

Die Verbindungen der Formeln (I-4-A) und (I-4-B) können sowohl als Gemische als auch in Form ihrer reinen Isomeren vorliegen. Gemische der Verbindungen der Formeln (I-4-A) und (I-4-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-4-a) bis (I-4-g), wenn CKE für die Gruppe (4) steht, 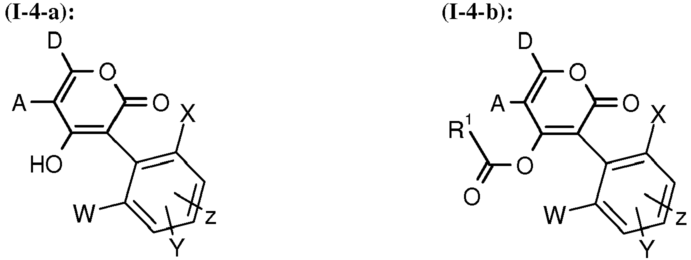 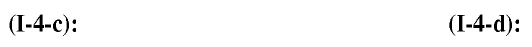 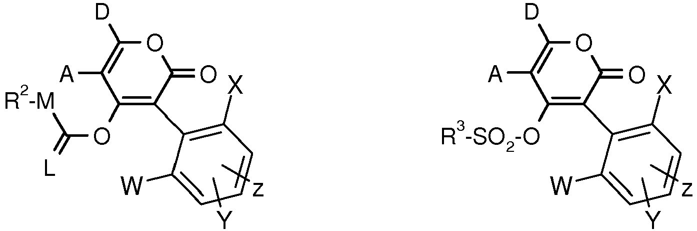 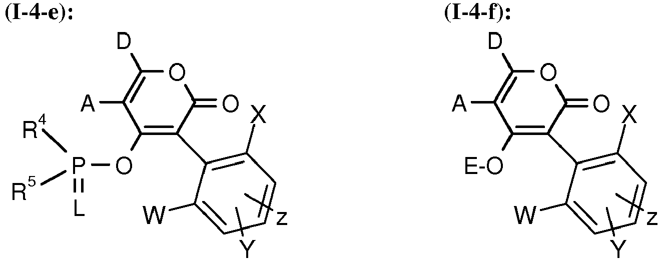 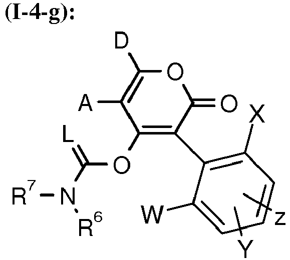 worin
A, D, E, L, M, W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-5-a) bis (I-5-g), wenn CKE für die Gruppe (5) steht, 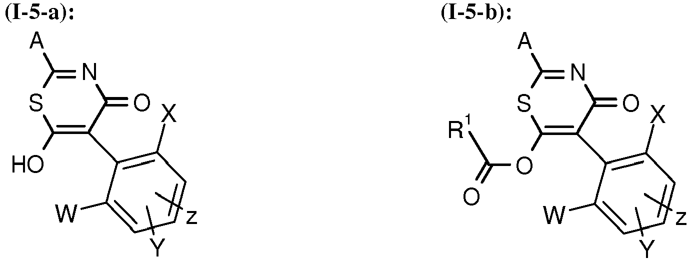 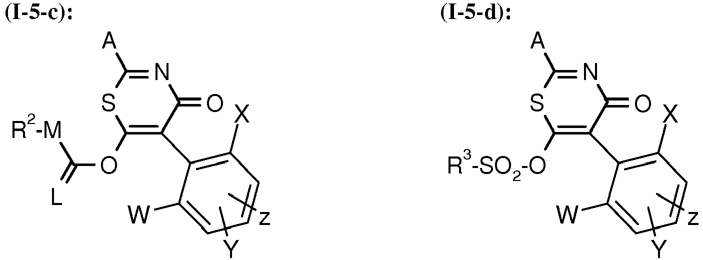 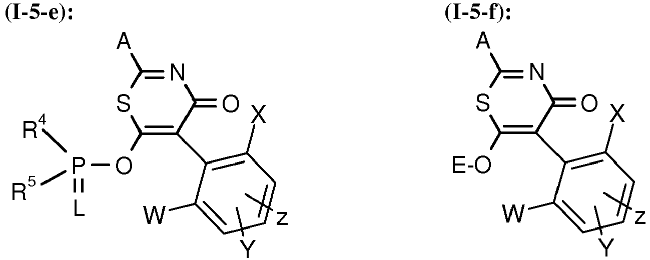 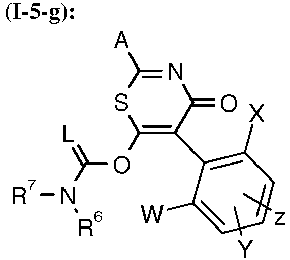 worin
A, E, L, M, W, X, Y, Z, R¹, R², R³, R⁴, R⁵, R⁶ und R⁷ die oben angegebenen Bedeutungen besitzen.

Die Verbindungen der Formel (I-6) können in Abhängigkeit von der Stellung des Substituenten G in den zwei isomeren Formen der Formeln (I-6-A) und (I-6-B) vorliegen, 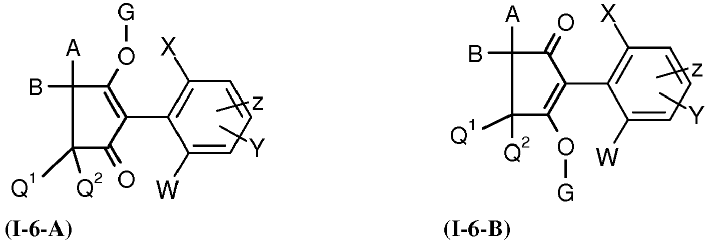 was durch die gestrichelte Linie in der Formel (I-6) zum Ausdruck gebracht werden soll.

Die Verbindungen der Formeln (I-6-A) und (I-6-B) können sowohl als Gemische als auch in Form ihrer reinen Isomeren vorliegen. Gemische der Verbindungen der Formeln (I-6-A) und (I-6-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächlichen Strukturen (I-6-a) bis (I-6-g), wenn CKE für die Gruppe (6) steht, 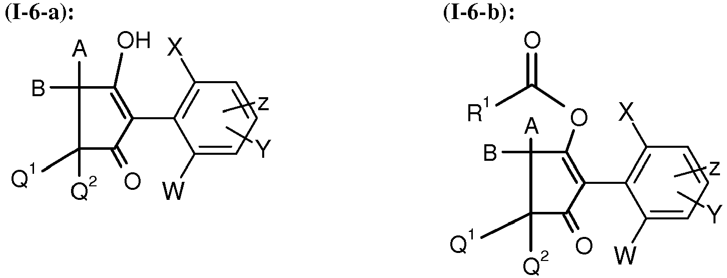 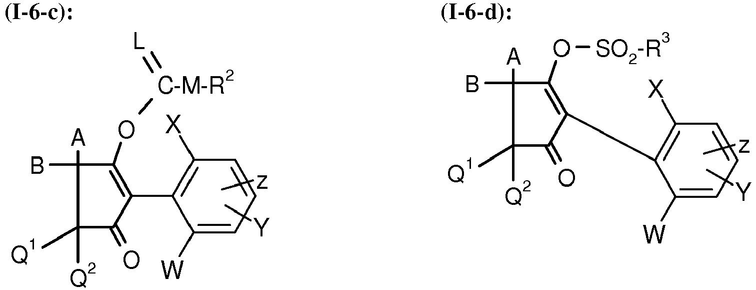 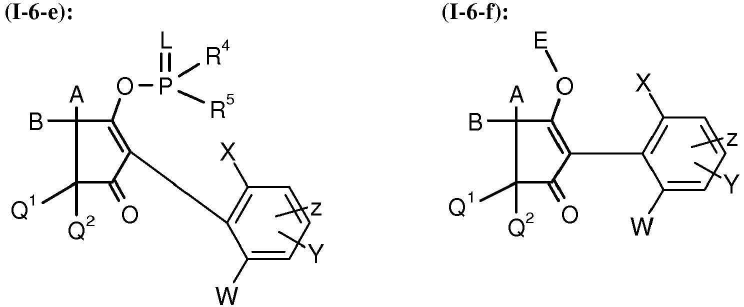 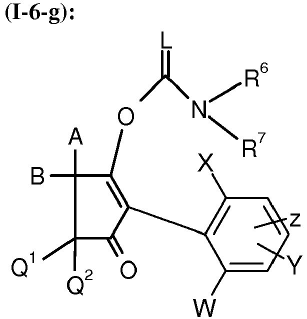 worin
A, B, Q¹, Q², E, L, M, W, X, Y, Z, R¹, R², R³, R⁴, R⁵, R⁶ und R⁷ die oben angegebenen Bedeutungen besitzen.

Die Verbindungen der Formel (I-7) können in Abhängigkeit von der Stellung des Substituenten G in den zwei isomeren Formen der Formeln (I-7-A) bzw. (I-7-B) vorliegen, was durch die gestrichelte Linie in der Formel (I-7) zum Ausdruck gebracht werden soll:

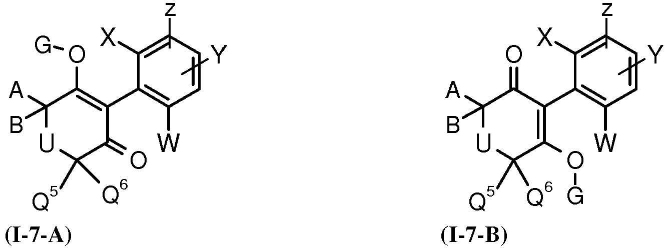

Die Verbindungen der Formeln (I-7-A) bzw. (I-7-B) können sowohl als Gemische als auch in Form ihrer reinen Isomeren vorliegen. Gemische der Verbindungen der Formeln (I-7-A) und (I-7-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt ein, dass die betreffende Verbindung gegebenenfalls als Isomerengemisch oder in der jeweils anderen isomeren Form vorliegen kann.

Unter Einbeziehung der verschiedenen Bedeutungen (a), (b), (c), (d), (e), (f) und (g) der Gruppe G ergeben sich folgende hauptsächlichen Strukturen (I-7-a) bis (I-7-g), wenn CKE für die Gruppe (7) steht, 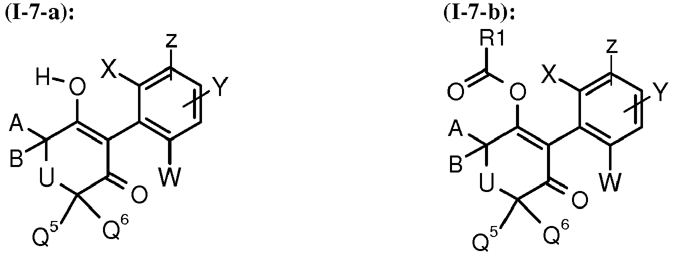 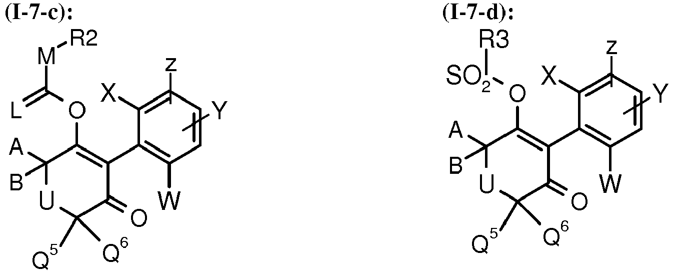 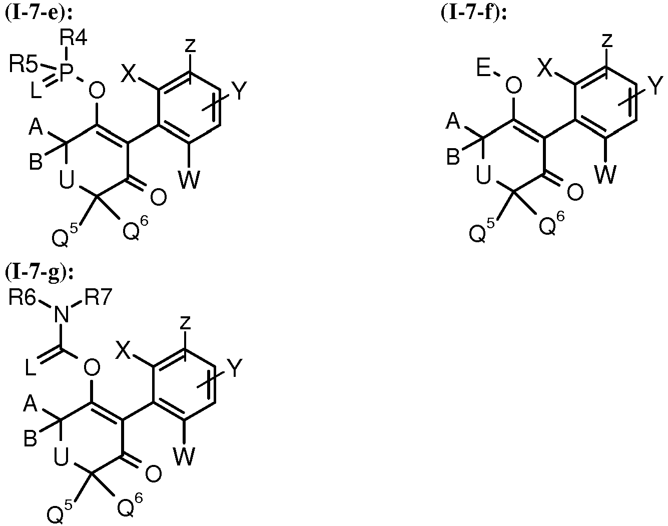 worin
A, B, E, L, M, Q⁵, Q⁶, U, W, X, Y, Z, R¹, R², R³, R⁴, R⁵, R⁶ und R⁷ die oben angegebenen Bedeutungen besitzen.

Die Verbindungen der Formel (I-8) können in Abhängigkeit von der Stellung des Substituenten G in den zwei isomeren Formeln (I-8-A) und (I-8-B) vorliegen, 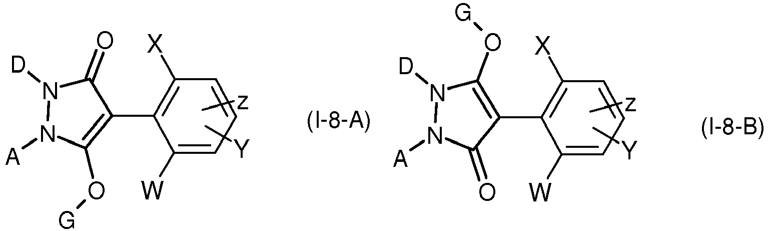 was durch die gestrichelte Linie in der Formel (I-8) zum Ausdruck gebracht werden soll.

Die Verbindungen der Formeln (I-8-A) und (I-8-B) können sowohl als Gemische als auch in Form ihrer reinen Isomeren vorliegen. Gemische der Verbindungen der Formel (I-8-A) und (I-8-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-8-a) (bis (I-8-g), wenn CKE für die Gruppe (8) steht, 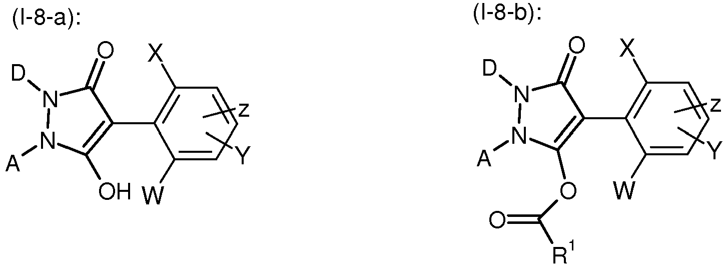 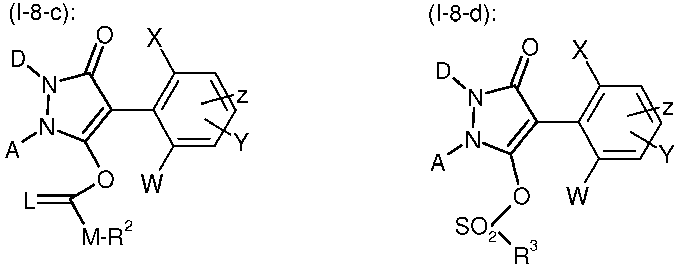 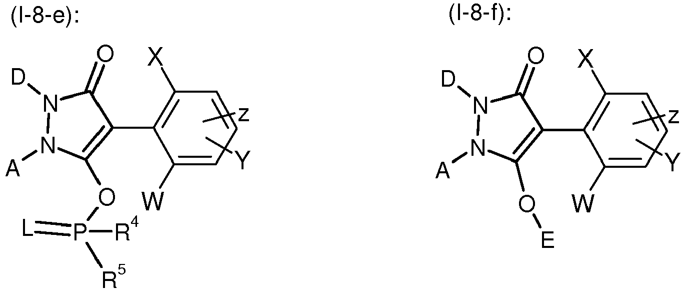 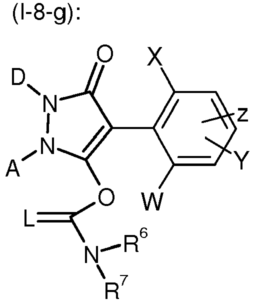 worin
A, D, E, L, M, W, X, Y, Z, R¹, R², R³, R⁴, R⁵, R⁶ und R⁷ die oben angegebenen Bedeutungen besitzen.

Die Verbindungen der Formel (I-9) können in Abhängigkeit von der Stellung des Substituenten G in den zwei isomeren Formen der Formeln (I-9-A) und (I-9-B) vorliegen, was durch die gestrichelte Linie in der Formel (I-9) zum Ausdruck gebracht werden soll.

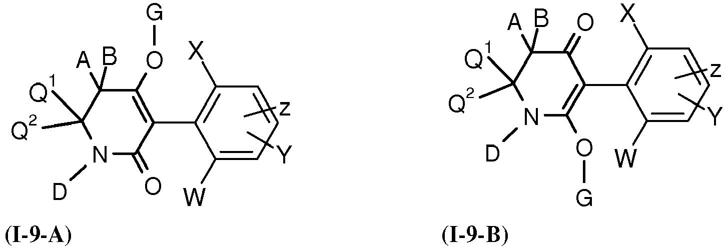

Die Verbindungen der Formeln (I-9-A) und (I-9-B) können sowohl als Gemische als auch in Form ihrer reinen Isomeren vorliegen. Gemische der Verbindungen der Formeln (I-9-A) und (I-9-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-9-a) bis (I-9-g), wenn CKE für die Gruppe (9) steht, 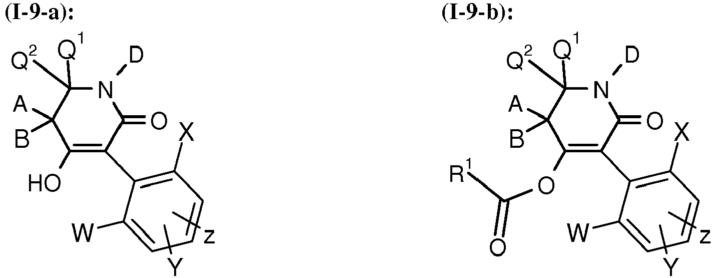 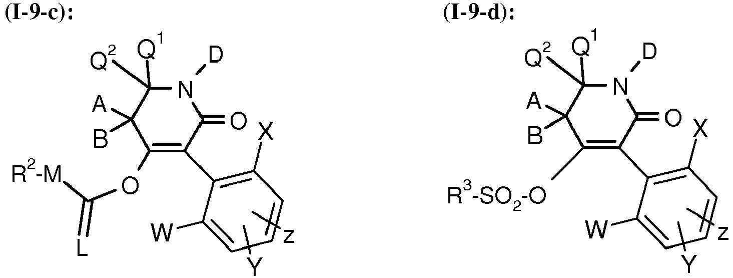 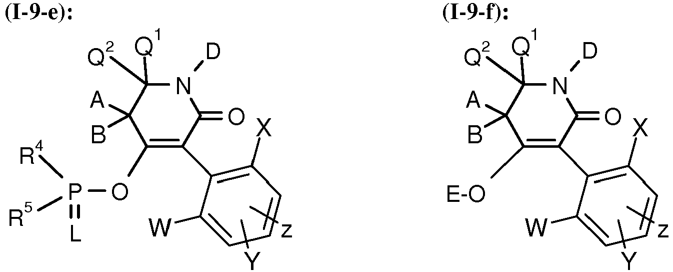 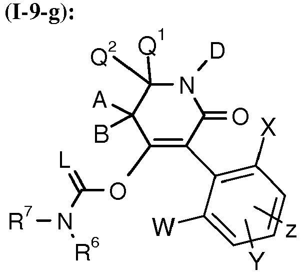 worin
A, B, D, E, L, M, Q¹, Q², W, X, Y, Z, R¹, R², R³, R⁴, R⁵, R⁶ und R⁷ die oben angegebenen Bedeutungen besitzen.

Die Verbindungen der Formel (I-10) können in Abhängigkeit von der Stellung des Substituenten G in den zwei isomeren Formen der Formeln (I-10-A) und (I-10-B) vorliegen, 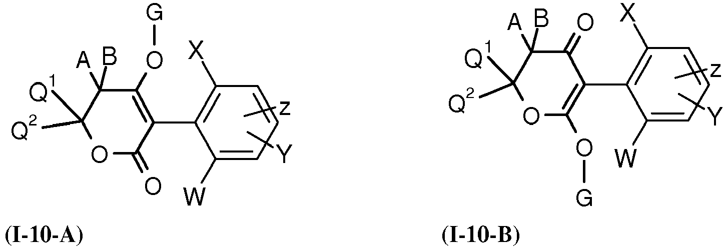 was durch die gestrichelte Linie in der Formel (I-10) zum Ausdruck gebracht werden soll.

Die Verbindungen der Formeln (I-10-A) und (I-10-B) können sowohl als Gemische als auch in Form ihrer reinen Isomeren vorliegen. Gemische der Verbindungen der Formeln (I-10-A) und (I-10-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-10-a) bis (I-10-g), wenn CKE für die Gruppe (10) steht, 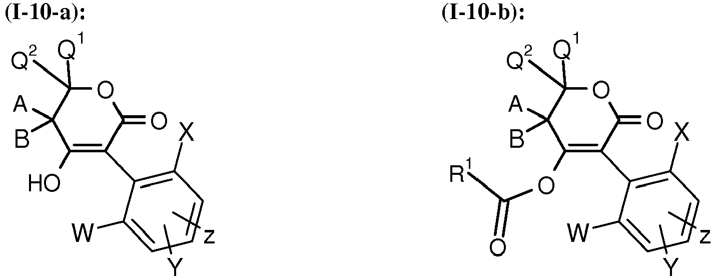 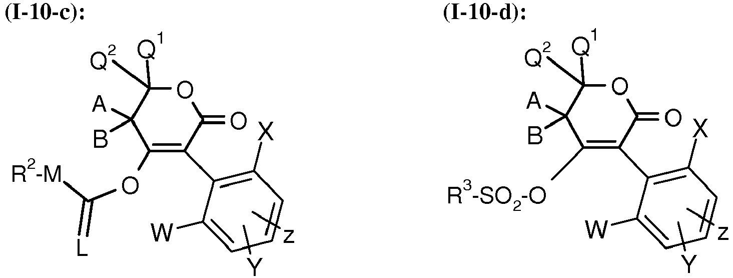 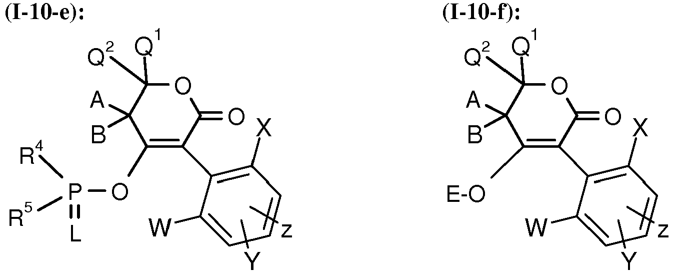 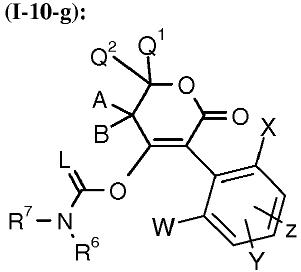 worin
A, B, E, L, M, Q¹, Q², W, X, Y, Z, R¹, R², R³, R⁴, R⁵, R⁶ und R⁷ die oben angegebenen Bedeutungen besitzen.

Die Verbindungen der Formel (I-11) können in Abhängigkeit von der Stellung des Substituenten G in den zwei isomeren Formen der Formeln (I-11-A) und (I-11-B) vorliegen, was durch die gestrichelte Linie in der Formel (I-11) zum Ausdruck gebracht werden soll.

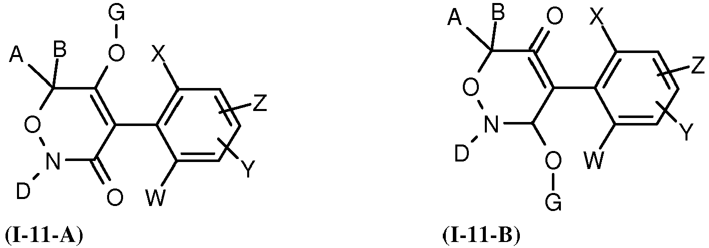

Die Verbindungen der Formeln (I-11-A) und (I-11-B) können sowohl als Gemische als auch in Form ihrer reinen Isomeren vorliegen. Gemische der Verbindungen der Formeln (I-11-A) und (I-11-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-11-a) bis (I-11-g), wenn CKE für die Gruppe (11) steht, 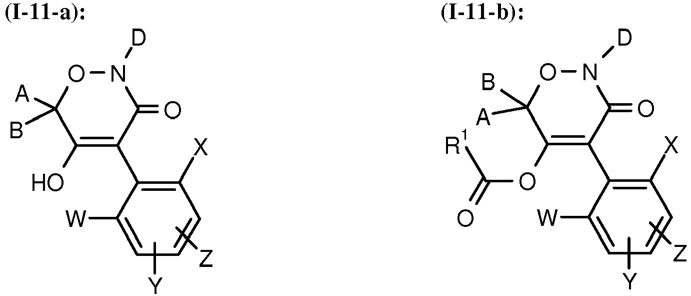 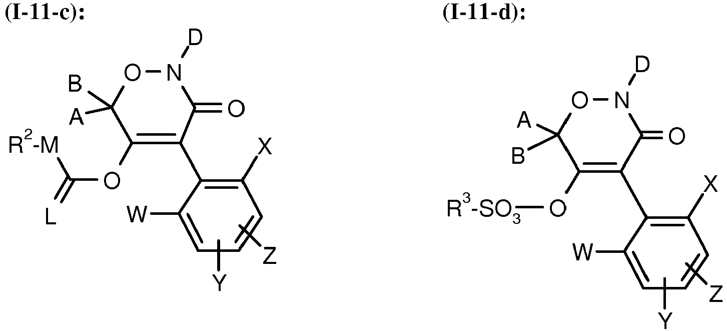 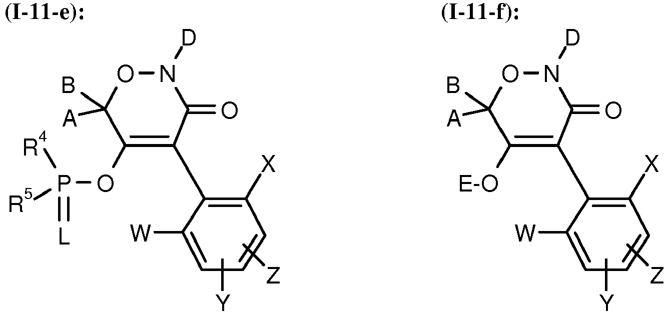 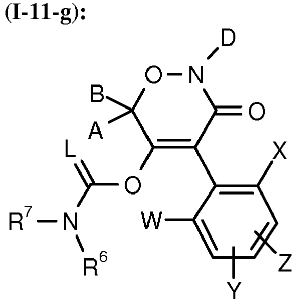 worin
A, B, D, E, L, M, W, X, Y, Z, R¹, R², R³, R⁴, R⁵, R⁶ und R⁷ die oben angegebenen Bedeutungen besitzen.

Weiterhin wurde gefunden, dass man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-1-a) 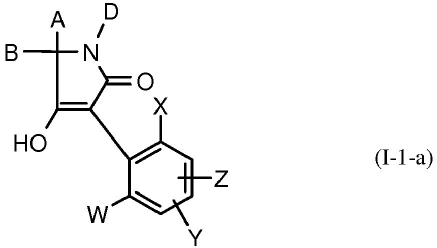 in welcher
   A, B, D, W, X, Y und Z die oben angegebenen Bedeutungen haben, wenn man
      N-Acylaminosäureester der Formel (II) 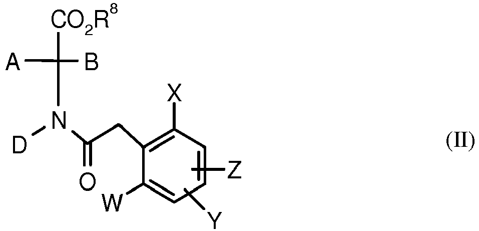
   in welcher
   A, B, D, W, X, Y und Z die oben angegebenen Bedeutungen haben,
      und
   R⁸ für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man substituierte 3-Phenyl-4-hydroxy-Δ³-dihydrofuran-on-Derivate der Formel (I-2-a) 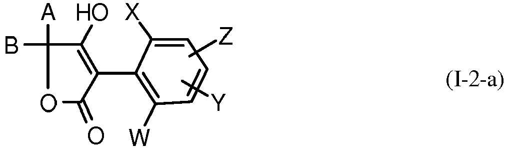 in welcher
   A, B, W, X, Y und Z die oben angegebenen Bedeutungen haben,
      erhält, wenn man
      Carbonsäureester der Formel (III) 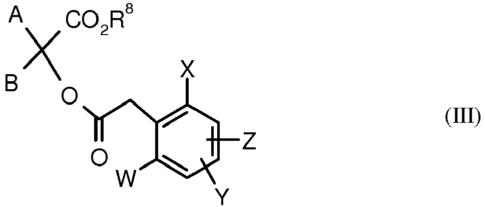
   in welcher
   A, B, W, X, Y, Z und R⁸ die oben angegebenen Bedeutungen haben,
      in Gegenwart eines Verdünnungsmittels und in Gegenwart einer Base intramolekular kondensiert.
(C) Weiterhin wurde gefunden, dass man substituierte 3-Phenyl-4-hydroxy-Δ³-dihydrothio-phenon-Derivate der Formel (I-3-a) 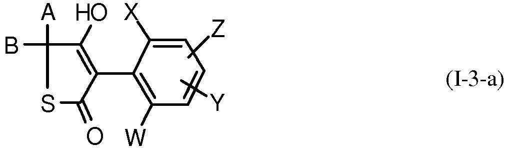 in welcher
   A, B, W, X, Y und Z die oben angegebenen Bedeutungen haben,
      erhält, wenn man
      ß-Ketocarbonsäureester der Formel (IV) 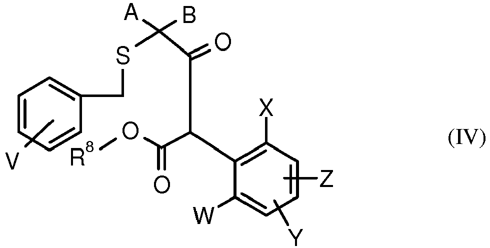
   in welcher
   A, B, W, X, Y, Z und R⁸ die oben angegebenen Bedeutungen haben und
   V für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(D) Weiterhin wurde gefunden, dass man die neuen substituierten 3-Phenylpyron-Derivate der Formel (I-4-a) 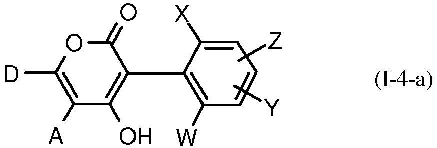 in welcher
   A, D, W, X, Y und Z die oben angegebenen Bedeutungen haben,
      erhält, wenn man
      Carbonylverbindungen der Formel (V) 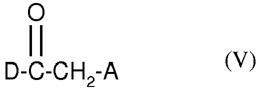
   in welcher
   - A und D: die oben angegebenen Bedeutungen haben,
   oder deren Silylenolether der Formel (Va) 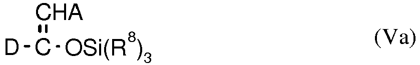 in welcher
   A, D und R⁸ die oben angegebene Bedeutung haben,
   mit Ketensäurehalogeniden der Formel (VI) 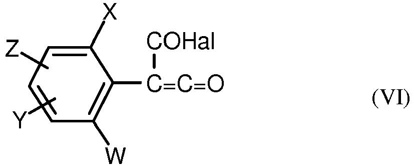 in welcher
   W, X, Y und Z die oben angegebenen Bedeutungen haben und
   Hal für Halogen (vorzugsweise für Chlor oder Brom) steht,
   gegebenenfalls in Gegenwart eines Verdünnungsmittels und gegebenenfalls in Gegenwart eines Säureakzeptors umsetzt.
   Weiterhin wurde gefunden,
(E) dass man die neuen substituierten Phenyl-1,3-thiazin-Derivate der Formel (I-5-a) 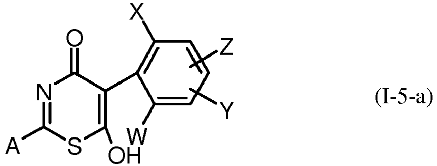 in welcher
   A,W, X, Y und Z die oben angegebene Bedeutung haben,
   erhält, wenn man Thioamide der Formel (VII) 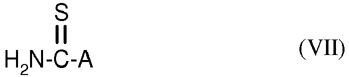 in welcher
   - A: die oben angegebene Bedeutung hat,
   mit Ketensäurehalogeniden der Formel (VI) 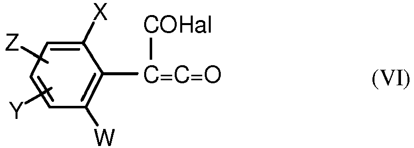 in welcher
   Hal, W, X, Y und Z die oben angegebenen Bedeutungen haben,
   gegebenenfalls in Gegenwart eines Verdünnungsmittels und gegebenenfalls in Gegenwart eines Säureakzeptors umsetzt.
   Weiterhin wurde gefunden,
(F) dass man Verbindungen der Formel (I-6-a) 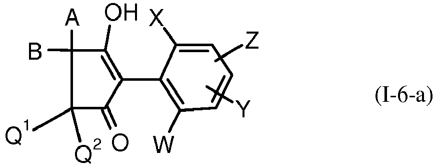 in welcher
   A, B, Q¹, Q², W, X, Y und Z die oben angegebene Bedeutung haben,
   erhält, wenn man Ketocarbonsäureester der Formel (VIII) 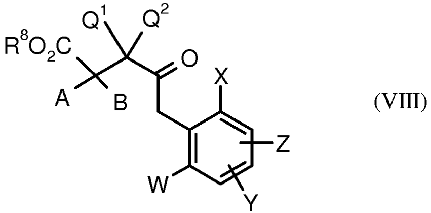 in welcher
   A, B, Q¹, Q², W, X, Y und Z die oben angegebene Bedeutung haben, und
   R⁸ für Alkyl (insbesondere C₁-C₈-Alkyl) steht,
   gegebenenfalls in Gegenwart eines Verdünnungsmittels und in Gegenwart einer Base intramolekular cyclisiert.
   Außerdem wurde gefunden,
(G) dass man Verbindungen der Formel (I-7-a) 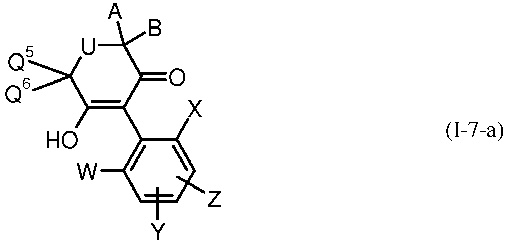 in welcher
   A, B, Q⁵, Q⁶, U, W, X, Y und Z die oben angegebene Bedeutung haben,
   erhält, wenn man
   6-Aryl-5-keto-hexansäureester der Formel (IX) 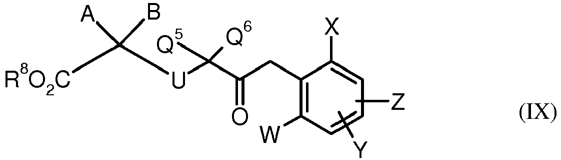 in welcher
   A, B, Q⁵, Q⁶, U, W, X, Y und Z die oben angegebene Bedeutung haben
      und
   R⁸ für Alkyl (bevorzugt C₁-C₆-Alkyl) steht,
   in Gegenwart eines Verdünnungsmittels und in Gegenwart einer Base intramolekular kondensiert.
(H) Weiterhin wurde gefunden, dass man die Verbindungen der Formel (I-8-a) 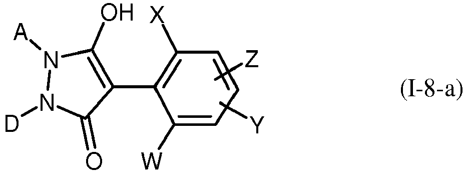 in welcher
   A, D, W, X, Y und Z die oben angegebenen Bedeutungen haben,
   erhält, wenn man
   Verbindungen der Formel (X) 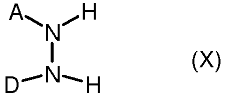 in welcher
   - A und D: die oben angegebene Bedeutung haben,

   α) mit Verbindungen der Formel (VI) 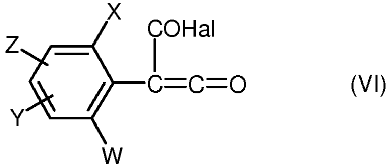 in welcher
      Hal, W, X, Y und Z die oben angegebenen Bedeutungen haben,
      gegebenenfalls in Gegenwart eines Verdünnungsmittels und gegebenenfalls in Gegenwart eines Säureakzeptors umsetzt, oder
   ß) mit Verbindungen der Formel (XI) 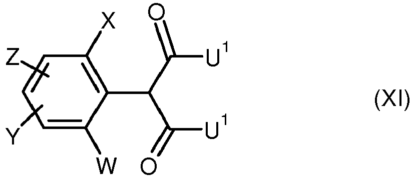 in welcher
      W, X, Y und Z die oben angegebene Bedeutung haben,
      und U¹ für NH₂ oder O-R⁸ steht, wobei R⁸ die oben genannte Bedeutung hat,
      gegebenenfalls in Gegenwart eines Verdünnungsmittels und gegebenenfalls in Gegenwart einer Base umsetzt, oder
   γ) mit Verbindungen der Formel (XII) 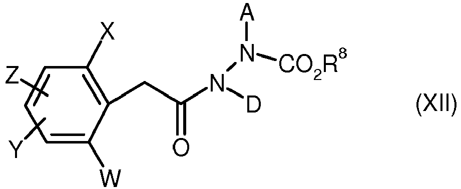 in welcher
      A, D, W, X, Y, Z und R⁸ die oben angegebene Bedeutung haben, gegebenenfalls in Gegenwart eines Verdünnungsmittels und gegebenenfalls in Gegenwart einer Base umsetzt.

Außerdem wurde gefunden, dass man die neuen Verbindungen der Formel (I-9-a) nach einem der im folgenden beschriebenen Verfahren erhält:
(I) Man erhält substituierte Tetrahydropyridin-2,4-dione bzw. deren Enole der Formel (I-9-a) 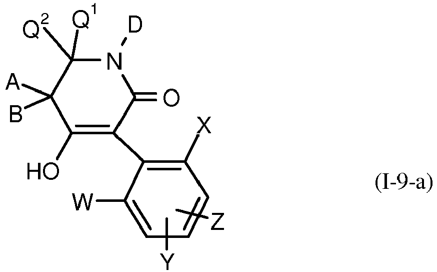 in welcher
   A, B, D, Q¹, Q², W, X, Y und Z die oben angegebenen Bedeutungen haben,
   wenn man
   N-Acylaminosäureester der Formel (XIII) 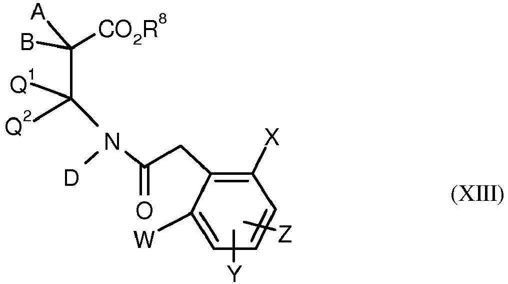 in welcher
   A, B, D, Q¹, Q², W, X, Y und Z die oben angegebenen Bedeutungen haben,
      und
   R⁸ für Alkyl (bevorzugt C₁-C₆-Alkyl) steht, in Gegenwart eines Verdünnungsmittels und in Gegenwart einer Base intramolekular kondensiert.
   Weiterhin wurde gefunden,
(J) dass man substituierte 5,6-Dihydropyrone der Formel (I-10-a) erhält 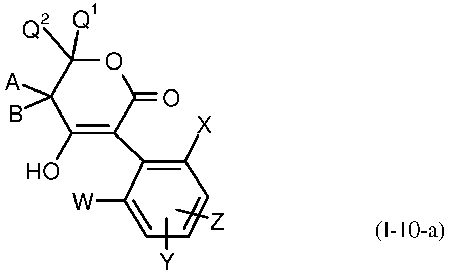 in welcher
   A, B, Q¹, Q², W, X, Y und Z die oben angegebene Bedeutungen haben,
      wenn man
      O-Acylhydroxycarbonsäureester der Formel (XIV) 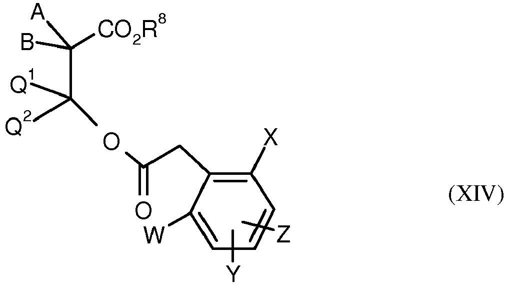
   in welcher
   A, B, Q¹, Q², W, X, Y und Z die oben angegebenen Bedeutungen haben,
      und
   R⁸ für Alkyl (bevorzugt C₁-C₆-Alkyl) steht,
   gegebenenfalls in Gegenwart eines Verdünnungsmittels und gegebenenfalls in Gegenwart einer Base umsetzt.

Weiterhin wurde gefunden, dass man die neuen Verbindungen der Formel (I-11-a) nach einem der im folgenden beschriebenen Verfahren erhält:
(K) Man erhält substituierte Oxazin-3,5-dione bzw. deren Enole der Formel (I-11-a) 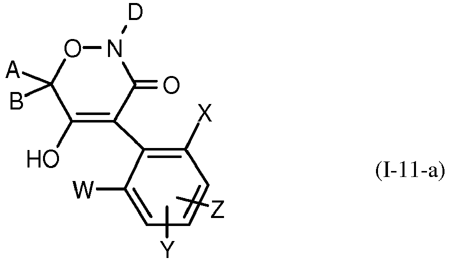 in welcher
   A, B, D, W, X, Y und Z die oben angegebenen Bedeutungen haben,
      wenn man
      N-Acylaminosäureester der Formel (XV) 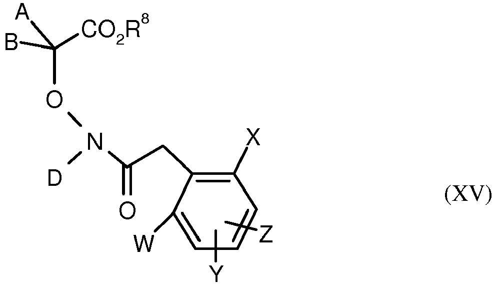
   in welcher
   A, B, D, W, X, Y und Z die oben angegebenen Bedeutungen haben,
      und
   R⁸ für Alkyl (bevorzugt C₁-C₆-Alkyl) steht,
   in Gegenwart eines Verdünnungsmittels und in Gegenwart einer Base intramolekular kondensiert.
   Außerdem wurde gefunden
(L) dass man die Verbindungen der oben gezeigten Formeln (I-1-b) bis (I-11-b), in welchen A, B, D, Q¹, Q², Q⁵, Q⁶, R¹, U, W, X, Y und Z die oben angegebenen Bedeutungen haben, erhält, wenn man Verbindungen der oben gezeigten Formeln (I-1-a) bis (I-11-a), in welchen A, B, D, Q¹, Q², Q⁵, Q⁶ U, W, X, Y und Z die oben angegebenen Bedeutungen haben, jeweils
   (α) mit Säurehalogeniden der Formel (XVI) 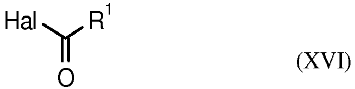 in welcher
      - R¹: die oben angegebene Bedeutung hat und
      - Hal: für Halogen (insbesondere Chlor oder Brom) steht
      oder
   (β) mit Carbonsäureanhydriden der Formel (XVII)

      R¹-CO-O-CO-R¹ (XVII)
   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(M) dass man die Verbindungen der oben gezeigten Formeln (I-1-c) bis (I-11-c), in welchen A, B, D, Q¹, Q², Q⁵, Q⁶, R², M, U, W, X, Y und Z die oben angegebenen Bedeutungen haben und L für Sauerstoff steht, erhält, wenn man Verbindungen der oben gezeigten Formeln (I-1-a) bis (I-11-a), in welchen A, B, D, Q¹, Q², Q⁵, Q⁶, U W, X, Y und Z die oben angegebenen Bedeutungen haben, jeweils
   mit Chlorameisensäureestern oder Chlorameisensäurethiolestern der Formel (XVIII)

   R²-M-CO-Cl (XVIII)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(N) dass man Verbindungen der oben gezeigten Formeln (I-1-c) bis (I-11-c), in welchen A, B, D, Q¹, Q², Q⁵, Q⁶, R², M, U, W, X, Y und Z die oben angegebenen Bedeutungen haben und L für Schwefel steht, erhält, wenn man Verbindungen der oben gezeigten Formeln (I-1-a) bis (I-11-a), in welchen A, B, D, Q¹, Q², Q⁵, Q⁶, U W, X, Y und Z die oben angegebenen Bedeutungen haben, jeweils
   mit Chlormonothioameisensäureestern oder Chlordithioameisensäureestern der Formel (XIX) 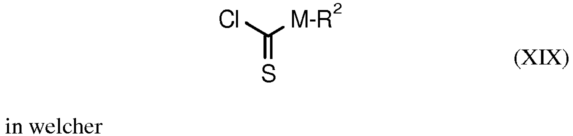
   - M und R²: die oben angegebenen Bedeutungen haben,
   gegebenenfalls in Gegenwart eines Verdünnungsmittels und gegebenenfalls in Gegenwart eines Säurebindemittels umsetzt
   und
(O) dass man Verbindungen der oben gezeigten Formeln (I-1-d) bis (I-11-d), in welchen A, B, D, Q¹, Q², Q⁵, Q⁶, R³, U, W, X, Y und Z die oben angegebenen Bedeutungen haben, erhält, wenn man Verbindungen der oben gezeigten Formeln (I-1-a) bis (I-11-a), in welchen A, B, D, Q¹, Q², Q⁵, Q⁶, U W, X, Y und Z die oben angegebenen Bedeutungen haben, jeweils
   mit Sulfonsäurechloriden der Formel (XX)

   R³-SO₂-Cl (XX)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(P) dass man Verbindungen der oben gezeigten Formeln (I-1-e) bis (I-11-e), in welchen A, B, D, L, Q¹, Q², Q⁵, Q⁶, R⁴, R⁵, U, W, X, Y und Z die oben angegebenen Bedeutungen haben, erhält, wenn man Verbindungen der oben gezeigten Formeln (I-1-a) bis (I-11-a), in welchen A, B, D, Q¹, Q², Q⁵, Q⁶, U, W, X, Y und Z die oben angegebenen Bedeutungen haben, jeweils
   mit Phosphorverbindungen der Formel (XXI) 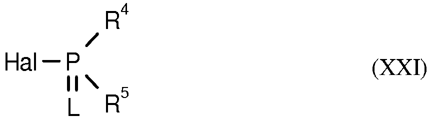 in welcher
   L, R⁴ und R⁵ die oben angegebenen Bedeutungen haben und
   Hal für Halogen (insbesondere Chlor oder Brom) steht,
   gegebenenfalls in Gegenwart eines Verdünnungsmittels und gegebenenfalls in Gegenwart eines Säurebindemittels umsetzt,
(Q) dass man Verbindungen der oben gezeigten Formeln (I-1-f) bis (I-11-f), in welchen A, B, D, E, Q¹, Q², Q⁵, Q⁶, U, W, X, Y und Z die oben angegebenen Bedeutungen haben, erhält, wenn man Verbindungen der Formeln (I-1-a) bis (I-11-a), in welchen A, B, D, Q¹, Q² Q⁵, Q⁶, U, W, X, Y und Z die oben angegebenen Bedeutungen haben, jeweils
   mit Metallverbindungen oder Aminen der Formeln (XXII) oder (XXIII)

   Me(OR¹⁰)ₜ (XXII)

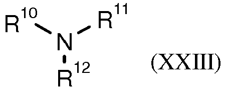

   in welchen
   - Me: für ein ein- oder zweiwertiges Metall (bevorzugt ein Alkali- oder Erdalkalimetall wie Lithium, Natrium, Kalium, Magnesium oder Calcium), oder für ein Ammoniumion

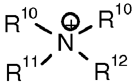

   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(R) dass man Verbindungen der oben gezeigten Formeln (I-1-g) bis (I-11-g), in welchen A, B, D, L, Q¹, Q², Q⁵, Q⁶, R⁶, R⁷, U, W, X, Y und Z die oben angegebenen Bedeutungen haben, erhält, wenn man Verbindungen der oben gezeigten Formeln (I-1-a) bis (I-11-a), in welchen A, B, D, Q¹, Q², Q⁵, Q⁶ U, W, X, Y und Z die oben angegebenen Bedeutungen haben, jeweils
   (α) mit Isocyanaten oder Isothiocyanaten der Formel (XXIV)

      R⁶-N=C=L (XXIV)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   (β) mit Carbamidsäurechloriden oder Thiocarbamidsäurechloriden der Formel (XXV) 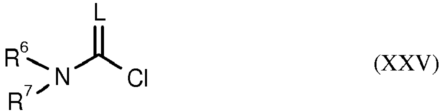 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt,
(S) dass man Verbindungen der oben gezeigten Formeln (I-1-a) bis (I-11-g), in welchen A, B, D, G, Q¹, Q², Q⁵, Q⁶, U, W, X, Y und Z die oben angegebene Bedeutung haben, erhält, wenn man in Verbindungen der Formeln (I-1') bis (I-11'), in welchen A, B, D, G, Q¹, Q², Q⁵, Q⁶, U, W, X, und Y die oben genannte Bedeutung haben und Z' bevorzugt für Brom oder Iod steht 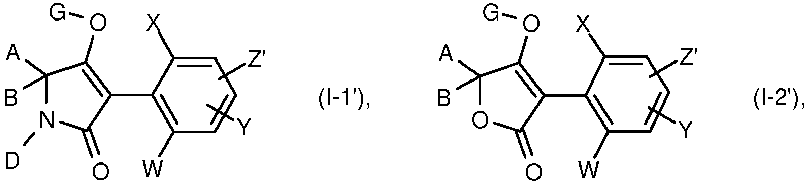 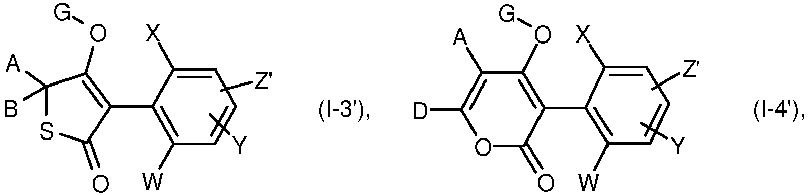 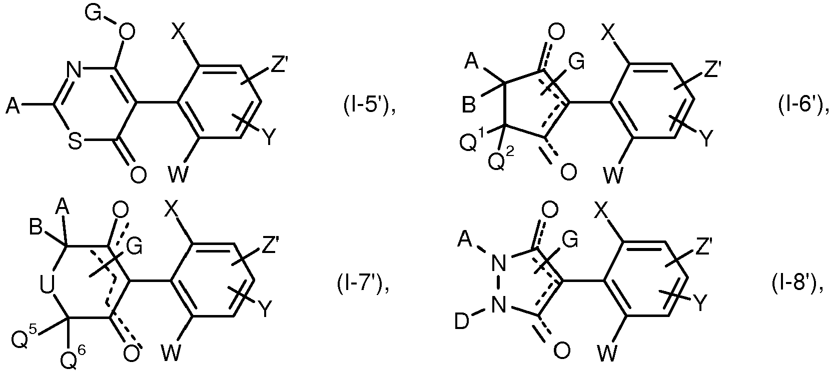 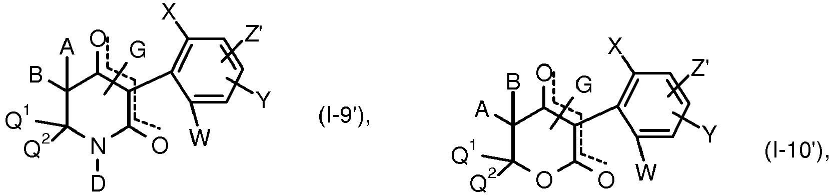 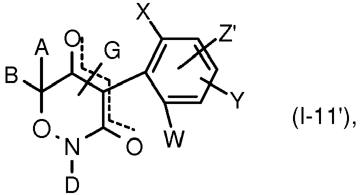
mit halogenierten Alkoholen, z.B. Trifluorethanol der Formel (XXVI) XXV)

Z-OH (XXVI)

in Gegenwart eines Lösungsmittels in Gegenwart eines Kupfersalzes (z. B. Cu-I-J) und in Gegenwart einer Base (z.B. Kalium-tert.-butylat, Natriumhydrid) das Brom- oder Jodatom austauscht.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Herbizide aufweisen.

Überraschenderweise wurde nun auch gefunden, dass bestimmte Halogenalkylmethylenoxy-phenyl-substituierte Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Raps, Soja und Reis, verwendet werden können.

Gegenstand der Erfindung sind auch selektiv-herbizide Mittel enthaltend einen wirksamen Gehalt an einer Wirkstoffkombination umfassend als Komponenten
a') mindestens eine Verbindung der Formel (I), in welcher CKE, W, X, Y und Z die oben angegebene Bedeutung haben
   und
(b') zumindest eine die Kulturpflanzen-Verträglichkeit verbessernde Verbindung (Safener). Die Safener sind vorzugsweise ausgewählt aus der Gruppe bestehend aus:
   S1) Verbindungen der Formel (S1), 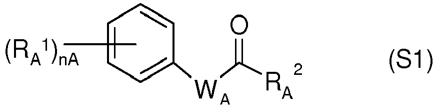 wobei die Symbole und Indizes folgende Bedeutungen haben:
      - n_{A}: ist eine natürliche Zahl von 0 bis 5, vorzugsweise 0 bis 3;
      - R_{A}¹: ist Halogen, (C₁-C₄)Alkyl, (C₁-C₄)Alkoxy, Nitro oder (C₁-C₄)Haloalkyl;
      - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴),

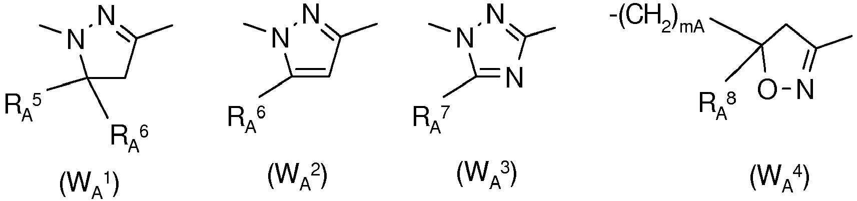

      - m_{A}: ist 0 oder 1;
      - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
      - R_{A}³: ist Wasserstoff oder ein unsubstituierter oder substituierter aliphatischer Kohlenwasserstoffrest, vorzugsweise mit insgesamt 1 bis 18 C-Atomen;

      - R_{A}⁴: ist Wasserstoff, (C₁-C₆)Alkyl, (C₁-C₆)Alkoxy oder substituiertes oder unsubstituiertes Phenyl;
      - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
      - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
      vorzugsweise:
      a) Verbindungen vom Typ der Dichlorphenylpyrazolin-3-carbonsäure (S1^{a}), vorzugsweise Verbindungen wie 1-(2,4-Dichlorphenyl)-5-ethoxycarbonyl)-5-methyl-2-pyrazolin-3-carbonsäure, 1-(2,4-Dichlorphenyl)-5-ethoxycarbonyl)-5-methyl-2-pyrazolin-3-carbonsäureethylester (S1-1) ("Mefenpyrdiethyl"), und verwandte Verbindungen, wie sie in der WO-A-91/07874 beschrieben sind;
      b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
      c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
      d) Verbindungen vom Typ der Triazolcarbonsäuren (S1^{d}), vorzugsweise Verbindungen wie Fenchlorazol(-ethylester), d.h. 1-(2,4-Dichlorphenyl)-5-richlormethyl-(1H)-1,2,4-triazol-3-carbonsäureethylester (S1-7), und verwandte Verbindungen wie sie in EP-A-174 562 und EP-A-346 620 beschrieben sind;
      e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
   S2) Chinolinderivate der Formel (S2), 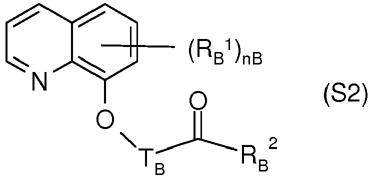 wobei die Symbole und Indizes folgende Bedeutungen haben:
      - R_{B}¹: ist Halogen, (C₁-C₄)Alkyl, (C₁-C₄)Alkoxy, Nitro oder (C₁-C₄)Haloalkyl;
      - n_{B}: ist eine natürliche Zahl von 0 bis 5, vorzugsweise 0 bis 3;
      - R_{B}²: ist OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
      - R_{B}³: ist Wasserstoff oder ein unsubstituierter oder substituierter aliphatischer Kohlenwasserstoffrest, vorzugsweise mit insgesamt 1 bis 18 C-Atomen;
      - R_{B}⁴: ist Wasserstoff, (C₁-C₆)Alkyl, (C₁-C₆)Alkoxy oder substituiertes oder unsubstituiertes Phenyl;
      - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
      vorzugsweise:
      a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
      b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
   S3) Verbindungen der Formel (S3) 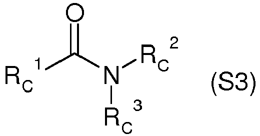 wobei die Symbole und Indizes folgende Bedeutungen haben:
      - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
      - R_{C}², R_{C}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
      vorzugsweise:
      Wirkstoffe vom Typ der Dichloracetamide, die häufig als Vorauflaufsafener (bodenwirksame Safener) angewendet werden, wie z. B.
      "Dichlormid" (N,N-Diallyl-2,2-dichloracetamid) (S3-1),
      "R-29148" (3-Dichloracetyl-2,2,5-trimethyl-1,3-oxazolidin) der Firma Stauffer (S3-2),
      "R-28725" (3-Dichloracetyl-2,2,-dimethyl-1,3-oxazolidin) der Firma Stauffer (S3-3),
      "Benoxacor" (4-Dichloracetyl-3,4-dihydro-3-methyl-2H-1,4-benzoxazin) (S3-4),
      "PPG-1292" (N-Allyl-N-[(1,3-dioxolan-2-yl)-methyl]-dichloracetamid) der Firma PPG Industries (S3-5),
      "DKA-24" (N-Allyl-N-[(allylaminocarbonyl)methyl]-dichloracetamid) der Firma Sagro-Chem (S3-6),
      "AD-67" oder "MON 4660" (3-Dichloracetyl-1-oxa-3-aza-spiro[4,5]decan) der Firma Nitrokemia bzw. Monsanto (S3-7),
      "TI-35" (1-Dichloracetyl-azepan) der Firma TRI-Chemical RT (S3-8),
      "Diclonon" (Dicyclonon) oder "BAS145138" oder "LAB145138" (S3-9) (3-Dichloracetyl-2,5,5-trimethyl-1,3-diazabicyclo[4.3.0]nonan) der Firma BASF,
      "Furilazol" oder "MON 13900" ((RS)-3-Dichloracetyl-5-(2-furyl)-2,2-dimethyl-oxazolidin) (S3-10); sowie dessen (R)-Isomer (S3-11).
   S4) N-Acylsulfonamide der Formel (S4) und ihre Salze, 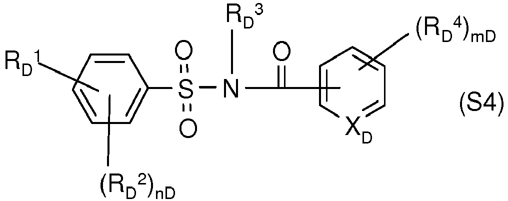 worin die Symbole und Indizes folgende Bedeutungen haben:
      - X_{D}: ist CH oder N;
      - R_{D}¹: ist CO-NR_{D}⁵R_{D}⁶ oder NHCO-R_{D}⁷;
      - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
      - R_{D}³: ist Wasserstoff, (C₁-C₄)Alkyl, (C₂-C₄)Alkenyl oder (C₂-C₄)Alkinyl;
      - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
      - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
      - R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
      - R_{D}⁵ und R_{D}⁶: gemeinsam mit dem sie tragenden Stickstoffatom einen Pyrrolidinyl- oder Piperidinyl-Rest bilden;
      - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
      - n_{D}: ist 0, 1 oder 2;
      - m_{D}: ist 1 oder 2;
      - v_{D}: ist 0, 1, 2 oder 3;
      davon bevorzugt sind Verbindungen von Typ der N-Acylsulfonamide, z.B. der nachfolgenden Formel (S4^{a}), die z. B. bekannt sind aus WO-A-97/45016 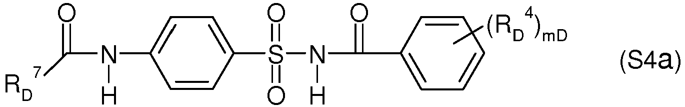 worin
      - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
      - R_{D}⁴: Halogen, (C₁-C₄)Alkyl, (C₁-C₄)Alkoxy, CF₃;
      - m_{D}: 1 oder 2;
      - v_{D}: ist 0, 1, 2 oder 3 bedeutet;
      sowie
      Acylsulfamoylbenzoesäureamide, z.B. der nachfolgenden Formel (S4^{b}), die z.B. bekannt sind aus WO-A-99/16744, 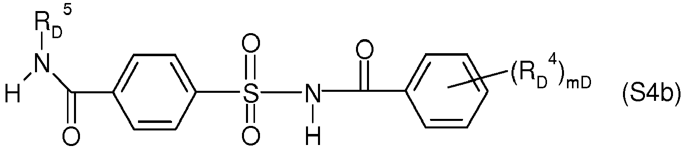 z.B. solche worin
      - R_{D}⁵ =: Cyclopropyl und (R_{D}⁴) = 2-OMe ist ("Cyprosulfamide", S4-1),
      - R_{D}⁵ =: Cyclopropyl und (R_{D}⁴) = 5-Cl-2-OMe ist (S4-2),
      - R_{D}⁵ =: Ethyl und (R_{D}⁴) = 2-OMe ist (S4-3),
      - R_{D}⁵ =: Isopropyl und (R_{D}⁴) = 5-Cl-2-OMe ist (S4-4) und
      - R_{D}⁵ =: Isopropyl und (R_{D}⁴) = 2-OMe ist (S4-5)
      sowie
      Verbindungen vom Typ der N-Acylsulfamoylphenylharnstoffe der Formel (S4^{c}), die z.B. bekannt sind aus der EP-A-365484, 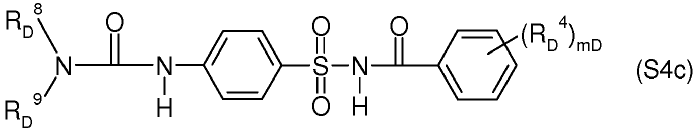 worin
      - R_{D}⁸ und R_{D}⁹: unabhängig voneinander Wasserstoff, (C₁-C₈)Alkyl, (C₃-C₈)Cycloalkyl, (C₃-C₆)Alkenyl, (C₃-C₆)Alkinyl,
      - R_{D}⁴: Halogen, (C₁-C₄)Alkyl, (C₁-C₄)Alkoxy, CF₃
      - m_{D}: 1 oder 2 bedeutet;
      beispielsweise
      1-[4-(N-2-Methoxybenzoylsulfamoyl)phenyl]-3-methylharnstoff, 1-[4-(N-2-Methoxybenzoylsulfamoyl)phenyl]-3,3-dimethylharnstoff, 1-[4-(N-4,5-Dimethylbenzoylsulfamoyl)phenyl]-3-methylharnstoff.
   S5) Wirkstoffe aus der Klasse der Hydroxyaromaten und der aromatisch-aliphatischen Carbonsäurederivate (S5), z.B.
      3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure, wie sie in der WO-A-2004/084631, WO-A-2005/015994, WO-A-2005/016001 beschrieben sind.
   S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-[2-(Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on, wie sie in der WO-A-2005/112630 beschrieben sind.
   S7) Verbindungen der Formel (S7),wie sie in der WO-A-1998/38856 beschrieben sind 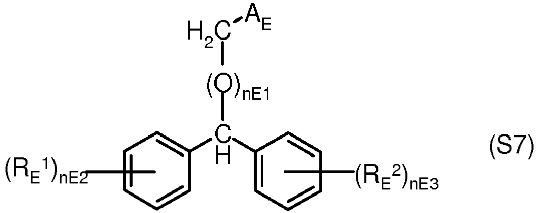 worin die Symbole und Indizes folgende Bedeutungen haben:
      - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
      - A_{E}: ist COOR_{E}³ oder COSR_{E}⁴
      - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
      - n_{E}¹: ist 0 oder 1
      - n_{E}², n_{E}³: sind unabhängig voneinander 0, 1 oder 2,
      vorzugsweise:
      Diphenylmethoxyessigsäure,
      Diphenylmethoxyessigsäureethylester,
      Diphenylmethoxyessigsäuremethylester (CAS-Reg.Nr. 41858-19-9) (S7-1).
   S8) Verbindungen der Formel (S8),wie sie in der WO-A-98/27049 beschrieben sind 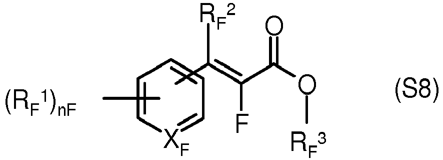 worin
      - X_{F}: CH oder N,
      - n_{F}: für den Fall, dass X_{F}=N ist, eine ganze Zahl von 0 bis 4 und
      für den Fall, dass X_{F}=CH ist, eine ganze Zahl von 0 bis 5 ,
      - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
      - R_{F}²: Wasserstoff oder (C₁-C₄)Alkyl
      - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
      vorzugsweise Verbindungen worin
      - X_{F}: CH,
      - n_{F}: eine ganze Zahl von 0 bis 2 ,
      - R_{F}¹: Halogen, (C₁-C₄)Alkyl, (C₁-C₄)Haloalkyl, (C₁-C₄)Alkoxy, (C₁-C₄)Haloalkoxy,
      - R_{F}²: Wasserstoff oder (C₁-C₄)Alkyl,
      - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
   S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
   S10) Verbindungen der Formeln (S10^{a}) oder (S10^{b})
      wie sie in der WO-A-2007/023719 und WO-A-2007/023764 beschrieben sind 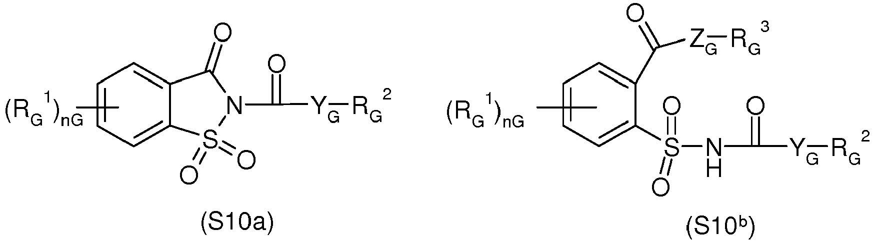 worin
      - R_{G}¹: Halogen, (C₁-C₄)Alkyl, Methoxy, Nitro, Cyano, CF₃, OCF₃
      - Y_{G,} Z_{G}: unabhängig voneinander O oder S,
      - n_{G}: eine ganze Zahl von 0 bis 4,
      - R_{G}²: (C₁-C₁₆)Alkyl, (C₂-C₆)Alkenyl, (C₃-C₆)Cycloalkyl, Aryl; Benzyl, Halogenbenzyl,
      - R_{G}³: Wasserstoff oder (C₁-C₆)Alkyl bedeutet.
   S11) Wirkstoffe vom Typ der Oxyimino-Verbindungen (S11), die als Saatbeizmittel bekannt sind, wie z. B. "Oxabetrinil" ((Z)-1,3-Dioxolan--ylmethoxyimino-(phenyl)acetonitril) (S11-1), das als Saatbeiz-Safener für Hirse gegen Schäden von Metolachlor bekannt ist,
      "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
      "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
   S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
   S13) Eine oder mehrere Verbindungen aus Gruppe (S13):
      "Naphthalic anhydrid" (1,8-Naphthalindicarbonsäureanhydrid) (S13-1), das als Saatbeiz-Safener für Mais gegen Schäden von Thiocarbamatherbiziden bekannt ist,
      "Fenclorim" (4,6-Dichlor-2-phenylpyrimidin) (S13-2), das als Safener für Pretilachlor in gesätem Reis bekannt ist,
      "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
      "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
      "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
      "MG-838" (CAS-Reg.Nr. 133993-74-5) (2-propenyl 1-oxa-4-azaspiro[4.5]decane-4-carbodithioate) (S 13-6) der Firma Nitrokemia,
      "Disulfoton" (O,O-Diethyl S-2-ethylthioethyl phosphordithioat) (S13-7),
      "Dietholate" (O,O-Diethyl-O-phenylphosphorotioat) (S13-8),
      "Mephenate" (4-Chlorphenyl-methylcarbamat) (S13-9).
   S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (*S*-1-Methyl-1-phenylethyl-piperidin-1-carbothioat), das als Safener für Reis gegen Schäden des Herbizids Molinate bekannt ist,
      "Daimuron" oder "SK 23" (1-(1-Methyl-1-phenylethyl)-3-p-tolyl-harnstoff), das als Safener für Reis gegen Schäden des Herbizids Imazosulfuron bekannt ist,
      "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
      "Methoxyphenon" oder "NK 049" (3,3'-Dimethyl-4-methoxy-benzophenon), das als Safener für Reis gegen Schäden einiger Herbizide bekannt ist,
      "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
   S15) Wirkstoffe, die vorrangig als Herbizide eingesetzt werden, jedoch auch Safenerwirkung auf Kulturpflanzen aufweisen, z.B.
      (2,4-Dichlorphenoxy)essigsäure (2,4-D),
      (4-Chlorphenoxy)essigsäure,
      (R,S)-2-(4-Chlor-o-tolyloxy)propionsäure (Mecoprop),
      4-(2,4-Dichlorphenoxy)buttersäure (2,4-DB),
      (4-Chlor-o-tolyloxy)essigsäure (MCPA),
      4-(4-Chlor-o-tolyloxy)buttersäure,
      4-(4-Chlorphenoxy)buttersäure,
      3,6-Dichlor-2-methoxybenzoesäure (Dicamba),
      1-(Ethoxycarbonyl)ethyl-3,6-dichlor-2-methoxybenzoat (Lactidichlor-ethyl).

Als die die Kulturpflanzen-Verträglichkeit verbessernde Verbindung [Komponente (b')] sind Cloquintocet-mexyl, Fenchlorazol-ethylester, Isoxadifen-ethyl, Mefenpyr-diethyl, Fenclorim, Cumyluron, S4-1 und S4-5 am meisten bevorzugt, wobei Mefenpyr-diethyl besonders hervorgehoben ist. Cyprosulfamide (S4-1) ist ebenfalls hervorgehoben.

Es wurde nun überraschend gefunden, dass die oben definierten Wirkstoffkombinationen aus Verbindungen der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von Verbindungen der Formel (I) auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
- W: steht bevorzugt für Wasserstoff, C₁-C₆-Alkyl, Halogen, C₁-C₆-Alkoxy, C₁-C₄-Halogenalkyl oder C₁-C₄-Halogenalkoxy,
- X: steht bevorzugt für Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy oder Cyano,
- Y: steht bevorzugt für Wasserstoff, Halogen, C₁-C₆-Alkyl, oder C₁-C₆-Alkoxy,
- Z: steht bevorzugt für eine Gruppe 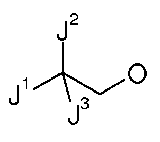
in welcher J¹ und J² bevorzugt jeweils unabhängig voneinander für Wasserstoff, Fluor oder Chlor stehen und J³ bevozugt für Halogen oder C₁-C₄-Halogenalkyl steht,
- CKE: steht bevorzugt für eine der Gruppen 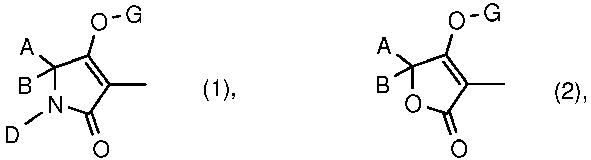 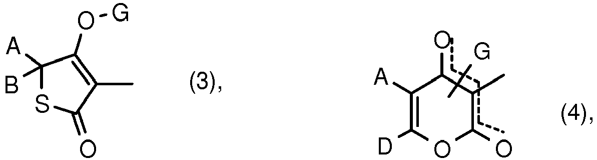 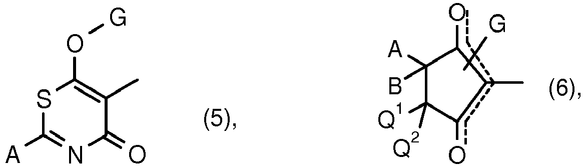 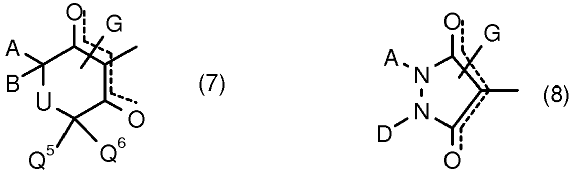 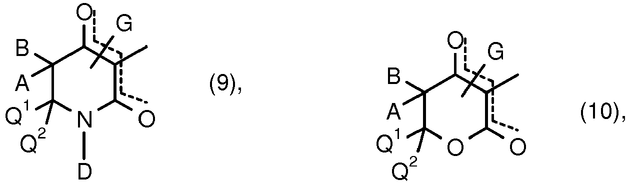
oder 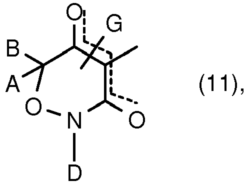
- U: steht bevorzugt für -S-, -S(O)-, -S(O)₂-, -O-, 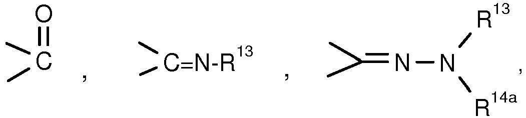
S=N-R¹³, S(O)=N-R¹³ oder 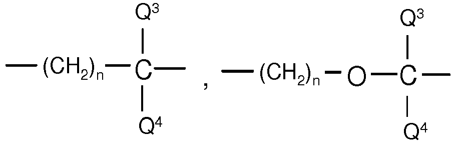 in welcher n für die Zahl 0, 1 oder 2 steht,
- A: steht bevorzugt für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Hetaryl mit 5 bis 6 Ringatomen (beispielsweise Furanyl, Pyridyl, Imidazolyl, Triazolyl, Pyrazolyl, Pyrimidyl, Thiazolyl oder Thienyl), Phenyl-C₁-C₆-alkyl oder Naphthyl-C₁-C₆-alkyl,
- B: steht bevorzugt für Wasserstoff, C₁-C₁₂-Alkyl oder C₁-C₈-Alkoxy-C₁-C₆-alkyl oder
- A, B: und das Kohlenstoffatom an das sie gebunden sind, stehen bevorzugt für gesättigtes C₃-C₁₀-Cycloalkyl oder ungesättigtes C₅-C₁₀-Cycloalkyl, worin gegebenenfalls ein Ringglied durch Stickstoff, Sauerstoff oder Schwefel ersetzt ist und welche gegebenenfalls einfach oder zweifach durch C₁-C₈-Alkyl, C₁-C₈-Alkoxy, C₃-C₈-Alkenyloxy, C₁-C₆-Alkoxy-C₁-C₆-Alkyl, C₃-C₆-Cycloalkyl-C₁-C₂-alkoxy, C₃-C₁₀-Cycloalkyl, C₁-C₈-Halogenalkyl, C₂-C₆-Halogenalkoxy, C₁-C₆-Alkoxy-C₁-C₄-alkoxy substituiert sind, wobei die zuvor genannten Reste auch als N-Substituenten in Frage kommen, oder
- A, B: und das Kohlenstoffatom, an das sie gebunden sind, stehen bevorzugt für C₃-C₆-Cycloalkyl, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl- oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, stehen bevorzugt für C₃-C₈-Cycloalkyl oder C₅-C₈-Cycloalkenyl, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
- D: steht bevorzugt für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₁-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl), Phenyl-C₁-C₆-alkyl oder Hetaryl-C₁-C₆-alkyl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl) oder
- A und D: stehen gemeinsam bevorzugt für jeweils gegebenenfalls substituiertes C₃-C₆-Alkandiyl oder C₃-C₆-Alkendiyl, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder Schwefel enthalten kann, oder worin gegebenenfalls eine der folgenden Gruppen 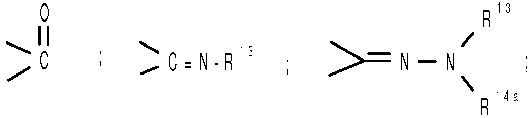 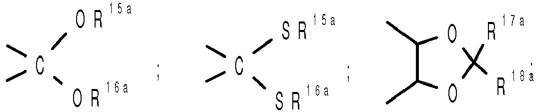 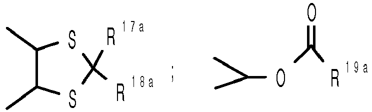
oder 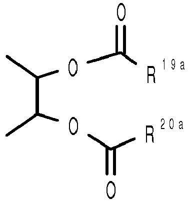 enthalten ist, oder
- A und Q¹: stehen bevorzugt gemeinsam mit den Kohlenstoffatomen, an die sie gebunden sind, für jeweils gegebenenfalls einfach oder zweifach, gleich oder verschieden durch Halogen, Hydroxy, durch jeweils gegebenenfalls einfach bis dreifach, gleich oder verschieden durch Halogen substituiertes C₁-C₁₀-Alkyl, C₁-C₈-Alkenyl, C₁-C₆-Alkoxy, C₁-C₆-Alkylthio, C₃-C₇-Cycloalkyl oder durch jeweils gegebenenfalls einfach bis dreifach, gleich oder verschieden durch Halogen, C₁-C₆-Alkyl oder C₁-C₆-Alkoxy substituiertes Benzyloxy oder Phenyl substituiertes C₃-C₆-Alkandiyl oder C₄-C₆-Alkendiyl, welches außerdem gegebenenfalls eine der nachstehenden Gruppen 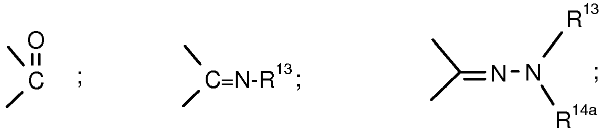 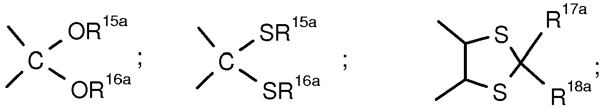 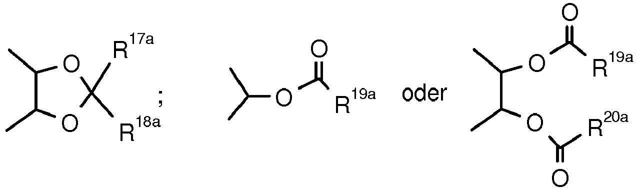
enthält oder durch eine C₁-C₂-Alkandiylgruppe oder durch ein Sauerstoffatom überbrückt ist oder
- B und Q²: stehen gemeinsam bevorzugt für gegebenenfalls durch C₁-C₂-Alkyl substituiertes C₁-C₃-Alkandiyl, welches gegebenenfalls durch Sauerstoff unterbrochen sein kann, oder
- D und Q¹: stehen gemeinsam bevorzugt für gegebenenfalls einfach oder zweifach, gleich oder verschieden durch C₁-C₄-Alkyl, C₁-C₄-Alkoxy substituiertes C₃-C₆-Alkandiyl, oder
- Q¹: steht bevorzugt für Wasserstoff, C₁-C₆-Alkyl, C₁-C₆-Alkoxy-C₁-C₂-alkyl, gegebenenfalls durch Fluor, Chlor, C₁-C₄-Alkyl, C₁-C₂-Halogenalkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl,
- Q², Q⁴**,** Q⁵ und Q⁶: stehen unabhängig voneinander bevorzugt für Wasserstoff oder C₁-C₄-Alkyl,
- Q³: steht bevorzugt für Wasserstoff, C₁-C₆-Alkyl, C₁-C₆-Alkoxy, C₁-C₆-Alkylthio, C₁-C₆-Alkoxy-C₁-C₂-alkyl, C₁-C₆-Alkylthio-C₁-C₂-alkyl, gegebenenfalls durch C₁-C₄-Alkyl oder C₁-C₄-Alkoxy substituiertes C₃-C₈-Cycloalkyl, worin gegebenenfalls eine oder zwei Methylengruppen durch Sauerstoff oder Schwefel ersetzt sind oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl, oder
- Q¹ und Q²: stehen bevorzugt mit dem Kohlenstoffatom, an das sie gebunden sind, für gegebenenfalls durch C₁-C₆-Alkyl, C₁-C₆-Alkoxy oder C₁-C₂-Halogenalkyl substituierten C₃-C₇-Ring, in welchem gegebenenfalls ein Ringglied durch Sauerstoff oder Schwefel ersetzt ist,
- Q³ und Q⁴: stehen bevorzugt gemeinsam mit dem Kohlenstoffatom, an das sie gebunden sind, für einen gegebenenfalls durch C₁-C₄-Alkyl, C₁-C₄-Alkoxy oder C₁-C₂-Halogenalkyl substituierten gesättigten oder ungesättigten C₃-C₇-Ring, in welchem gegebenenfalls ein oder zwei Ringglieder durch Sauerstoff oder Schwefel ersetzt sind,
- A und Q³: stehen bevorzugt gemeinsam mit den Kohlenstoffatomen, an die sie gebunden sind, für einen gesättigten oder ungesättigten, gegebenenfalls durch C₁-C₄-Alkyl, C₁-C₄-Alkoxy oder C₁-C₂-Halogenalkyl substituierten C₃-C₇-Ring, in welchem gegebenenfalls ein oder zwei Ringglieder durch Sauerstoff oder Schwefel ersetzt sind,
- A und Q⁵: stehen bevorzugt gemeinsam mit den Kohlenstoffatomen, an die sie gebunden sind, für einen gesättigten oder ungesättigten, gegebenenfalls durch C₁-C₄-Alkyl, C₁-C₄-Alkoxy oder C₁-C₂-Halogenalkyl substituierten C₃-C₇-Ring, in welchem gegebenenfalls ein Ringglied durch Sauerstoff oder Schwefel ersetzt ist,
- G: steht bevorzugt für Wasserstoff (a) oder für eine der Gruppen 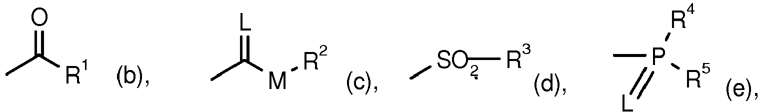 E (f) oder 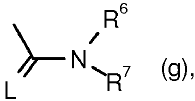 insbesondere für (a), (b), (c) oder (g) in welchen
E für ein Metallionäquivalent oder ein Ammoniumion steht,
L für Sauerstoff oder Schwefel steht und
M für Sauerstoff oder Schwefel steht.
- R¹: steht bevorzugt für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl (beispielsweise Pyrazolyl, Thiazolyl, Pyridyl, Pyrimidyl, Furanyl oder Thienyl),
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl (beispielsweise Pyridyloxy-C₁-C₆-alkyl, Pyrimidyloxy-C₁-C₆-alkyl oder Thiazolyloxy-C₁-C₆-alkyl),
- R²: steht bevorzugt für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
- R³: steht bevorzugt für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
- R⁴ und R⁵: stehen bevorzugt unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio,
- R⁶ und R⁷: stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest, in welchem gegebenenfalls ein Kohlenstoffatom durch Sauerstoff oder Schwefel ersetzt ist,
- R¹³: steht bevorzugt für Wasserstoff, für jeweils gegebenenfalls durch Halogen substituiertes C₁-C₈-Alkyl oder C₁-C₈-Alkoxy (nur im Fall der C=N-R¹³-Gruppe), für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl, Hetaryl-C₁-C₄-alkyl, oder nur im Fall der C=N-R¹³-Gruppe für Phenyl-C₁-C₄-alkoxy oder Hetaryl-C₁-C₄-alkoxy,
- R^{14a}: steht bevorzugt für Wasserstoff oder C₁-C₈-Alkyl oder
- R¹³ und R^{14a}: stehen gemeinsam bevorzugt für gegebenenfalls durch C₁-C₄-Alkyl substituiertes C₄-C₆-Alkandiyl, welches gegebenenfalls durch Sauerstoff oder Schwefel unterbrochen sein kann,
- R^{15a} und R^{16a}: sind gleich oder verschieden und stehen bevorzugt für C₁-C₆-Alkyl oder
- R^{15a} und R^{16a}: stehen gemeinsam bevorzugt für einen C₂-C₄-Alkandiylrest oder C₄-Alkandiylrest, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist,
- R^{17a} und R^{18a}: stehen unabhängig voneinander bevorzugt für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder
- R^{17a} und R^{18a}: stehen gemeinsam mit dem Kohlenstoffatom, an das sie gebunden sind, bevorzugt für eine Carbonylgruppe oder für gegebenenfalls durch Halogen, C₁-C₄-Alkyl oder C₁-C₄-Alkoxy substituiertes C₅-C₇-Cycloalkyl, in dem gegebenenfalls eine Methylengruppe durch Sauerstoff oder Schwefel ersetzt ist,
- R^{19a} und R^{20a}: stehen unabhängig voneinander bevorzugt für C₁-C₁₀-Alkyl, C₂-C₁₀-Alkenyl, C₁-C₁₀-Alkoxy, C₁-C₁₀-Alkylamino, C₃-C₁₀-Alkenylaniino, Di-(C₁-C₁₀-alkyl)amino oder Di-(C₃-C₁₀-alkenyl)amino.

In den als bevorzugt genannten Restedefinitionen steht Halogen für Fluor, Chlor, Brom und Jod, insbesondere für Fluor, Chlor und Brom.
- W: steht besonders bevorzugt für Wasserstoff, Fluor, Chlor, Brom, C₁-C₄-Alkyl, C₁-C₄-Alkoxy, C₁-C₂-Halogenalkyl oder C₁-C₂-Halogenalkoxy,
- X: steht besonders bevorzugt für Chlor, Brom, Iod, C₁-C₄-Alkyl, C₂-C₄-Alkenyl, C₂-C₄-Alkinyl, C₁-C₄-Alkoxy, C₁-C₂-Halogenalkyl, C₁-C₂-Halogenalkoxy oder Cyano,
- Y: steht besonders bevorzugt für Wasserstoff, Methyl, Ethyl, Fluor, Chlor, Brom, Iod, Methoxy oder Ethoxy,
- Z: steht besonders bevorzugt für die Gruppe 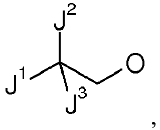
in welcher J¹ und J² besonders bevorzugt jeweils unabhängig voneinander für Wasserstoff, Fluor oder Chlor stehen und J³ für Fluor, Chlor Trichlormethyl, Difluormethyl, Difluorchlormethyl, Dichlorfluormethyl oder Trifluormethyl steht,
CKE steht besonders bevorzugt für eine der Gruppen 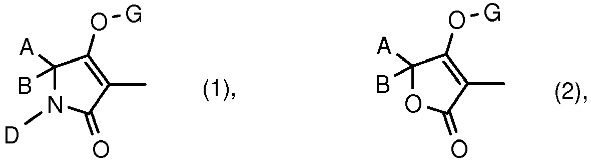 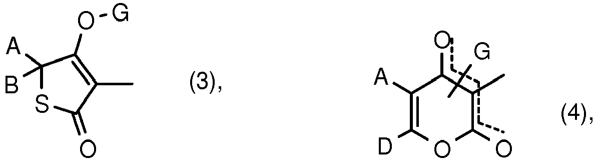 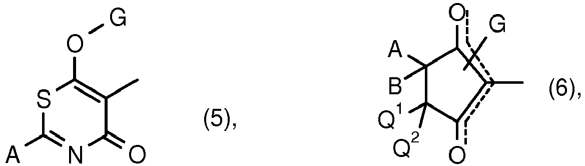 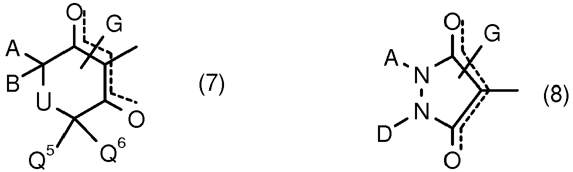 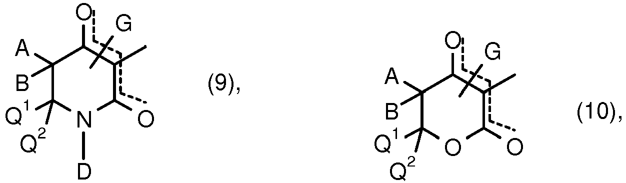 oder 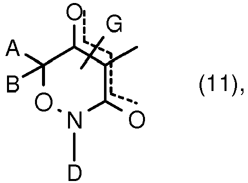
- U: steht besonders bevorzugt für -CH₂-, -CH₂-CH₂-, -O- oder

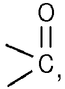

- A: steht besonders bevorzugt für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes und gegebenenfalls durch ein Sauerstoffatom unterbrochenes C₃-C₆-Cycloalkyl oder (jedoch nicht im Fall der Verbindungen der Formeln (I-3), (I-4), (I-6), (I-7), (I-9), (I-10) und (I-11)) jeweils gegebenenfalls einfach bis zweifach durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl, Pyridyl oder Benzyl,
- B: steht besonders bevorzugt für Wasserstoff, C₁-C₄-Alkyl oder C₁-C₂-Alkoxyl-C₁-C₂-alkyl oder
- A, B: und das Kohlenstoffatom an das sie gebunden sind, stehen besonders bevorzugt für gesättigtes oder ungesättigtes C₃-C₇-Cycloalkyl, worin gegebenenfalls ein Ringglied durch Stickstoff, Sauerstoff oder Schwefel ersetzt ist und welches gegebenenfalls einfach bis zweifach durch C₁-C₆-Alkyl, C₁-C₄-Alkoxy-C₁-C₂-alkyl, Trifluormethyl, C₁-C₆-Alkoxy, C₃-C₆-Alkenyloxy, Trifluorethoxy, C₁-C₃-Alkoxy-C₁-C₃-alkoxy oder C₃-C₆-Cycloalkylmethoxy substituiert ist, wobei die zuvor genannten Reste auch als N-Substituenten in Frage kommen, mit der Maßgabe, dass dann Q³ besonders bevorzugt für Wasserstoff oder Methyl steht oder
- A, B: und das Kohlenstoffatom, an das sie gebunden sind, stehen besonders bevorzugt für C₅-C₆-Cycloalkyl, welches durch eine gegebenenfalls ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet mit der Maßgabe, dass dann Q³ besonders bevorzugt für Wasserstoff oder Methyl steht, oder
- A, B: und das Kohlenstoffatom, an das sie gebunden sind, stehen besonders bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen, mit der Maßgabe, dass dann Q³ besonders bevorzugt für Wasserstoff oder Methyl steht,
- D: steht besonders bevorzugt für Wasserstoff, für jeweils gegebenenfalls einfach bis dreifach durch Fluor substituiertes C₁-C₆-Alkyl, C₃-C₆-Alkenyl, C₁-C₄-Alkoxy-C₁-C₃-alkyl, für gegebenenfalls einfach bis zweifach durch C₁-C₄-Alkyl, C₁-C₄-Alkoxy oder C₁-C₂-Halogenalkyl substituiertes C₃-C₆-Cycloalkyl, in welchem gegebenenfalls eine Methylengruppe durch Sauerstoff ersetzt ist oder (nur im Fall der Verbindungen der Formeln (I-4)) für jeweils gegebenenfalls einfach bis zweifach durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl oder Pyridyl, oder
- A und D: stehen gemeinsam besonders bevorzugt für gegebenenfalls einfach bis zweifach substituiertes C₃-C₅-Alkandiyl, in welchem eine Methylengruppe durch eine Carbonylgruppe (nicht jedoch im Fall der Verbindungen der Formel (I-11)), Sauerstoff oder Schwefel ersetzt sein kann, wobei als Substituenten C₁-C₂-Alkyl oder C₁-C₂-Alkoxy in Frage kommen oder
- A und D: stehen (im Fall der Verbindungen der Formel (I-1)) gemeinsam mit den Atomen, an die sie gebunden sind, für eine der Gruppen AD-1 bis AD-10: 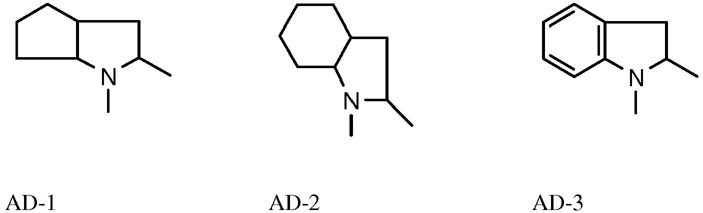 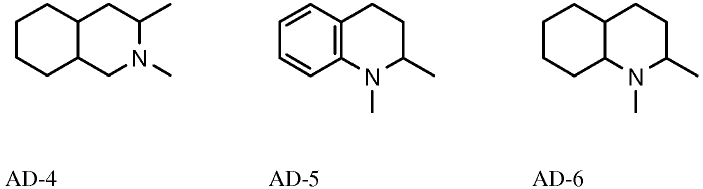 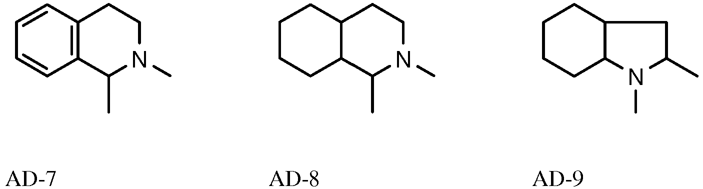 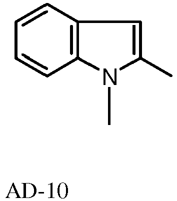
oder

A und D stehen gemeinsam besonders bevorzugt für C₃-C₅-Alkandiyl, welches gegebenenfalls durch eine mit zwei nicht direkt benachbarten Sauerstoffatomen enthaltende gegebenenfalls einfach bis vierfach durch C₁-C₄-Alkyl oder C₁-C₃-Alkoxy-C₁-C₂-Alkyl substituierte Alkylendioxyl-Gruppe substituiert ist, wobei ein weiterer 5- oder 6-Ring gebildet wird, oder
- A und Q¹: stehen gemeinsam besonders bevorzugt für gegebenenfalls einfach oder zweifach, gleich oder verschieden durch C₁-C₂-Alkyl oder C₁-C₂-Alkoxy substituiertes C₃-C₄-Alkandiyl, welches gegebenenfalls die nachfolgende Gruppe enthält: 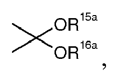
in welcher
- R^{15a} und R^{16a}: gleich oder verschieden besonders bevorzugt für Methyl oder Ethyl stehen, oder
- R^{15a} und R^{16a}: gemeinsam besonders bevorzugt für einen C₂-C₄-Alkandiyl- oder C₄-Alkendiylrest stehen, der gegebenenfalls durch Methyl oder Ethyl substituiert ist, oder
- B und Q²: stehen gemeinsam besonders bevorzugt für -CH₂-, -CH₂-CH₂- -CH₂-CH₂-CH₂-, oder -CH₂-O-CH₂-, oder
- D und Q¹: stehen gemeinsam besonders bevorzugt für C₃-C₄-Alkandiyl, oder
- Q¹: steht besonders bevorzugt für Wasserstoff, C₁-C₄-Alkyl, C₁-C₄-Alkoxy-C₁-C₂-alkyl, oder gegebenenfalls durch Methyl oder Methoxy substituiertes C₃-C₆-Cycloalkyl, worin gegebenenfalls eine Methylengruppe durch Sauerstoff ersetzt ist,
- Q²: steht besonders bevorzugt für Wasserstoff, Methyl oder Ethyl,
- Q⁴, Q⁵ und Q⁶: stehen besonders bevorzugt unabhängig voneinander für Wasserstoff oder C₁-C₃-Alkyl,
- Q³: steht besonders bevorzugt für Wasserstoff, C₁-C₄-Alkyl, C₁-C₄-Alkoxy, oder für gegebenenfalls einfach bis zweifach durch Methyl oder Methoxy substituiertes gegebenenfalls durch ein Sauerstoffatom unterbrochenes C₃-C₆-Cycloalkyl, oder
- Q¹ und Q²: stehen besonders bevorzugt mit dem Kohlenstoff, an das sie gebunden sind, für gegebenenfalls durch Methyl oder Methoxy substituiertes C₃-C₆-Cycloalkyl, worin gegebenenfalls eine Methylengruppe durch Sauerstoff ersetzt ist, mit der Maßgabe, dass dann A und B besonders bevorzugt unabhängig voneinander für Wasserstoff oder Methyl stehen, oder
- Q³ und Q⁴: stehen besonders bevorzugt gemeinsam mit dem Kohlenstoff, an das sie gebunden sind, für einen gegebenenfalls durch C₁-C₂-Alkyl oder C₁-C₂-Alkoxy substituierten gesättigten C₅-C₆-Ring, in welchem gegebenenfalls ein oder zwei Ringglieder durch Sauerstoff oder Schwefel ersetzt sind, mit der Maßgabe, dass dann A besonders bevorzugt für Wasserstoff oder Methyl steht, oder
- A und Q³: stehen besonders bevorzugt gemeinsam mit dem Kohlenstoff, an das sie gebunden sind, für einen gegebenenfalls durch C₁-C₂-Alkyl oder C₁-C₂-Alkoxy substituierten gesättigten C₅-C₆-Ring, in welchem gegebenenfalls ein Ringglied durch Sauerstoff oder Schwefel ersetzt ist, mit der Maßgabe, dass dann B, Q⁴, Q⁵ und Q⁶ besonders bevorzugt unabhängig voneinander für Wasserstoff oder Methyl stehen, oder
- A und Q⁵: stehen besonders bevorzugt gemeinsam mit den Kohlenstoffatomen, an die sie gebunden sind, für einen gegebenenfalls durch C₁-C₂-Alkyl oder C₁-C₂-Alkoxy substituierten gesättigten oder ungesättigten C₅-C₆-Ring, mit der Maßgabe, dass dann B, Q³, Q⁴ und Q6 besonders bevorzugt unabhängig voneinenander für Wasserstoff oder Methyl stehen,
- G: steht besonders bevorzugt für Wasserstoff (a) oder für eine der Gruppen 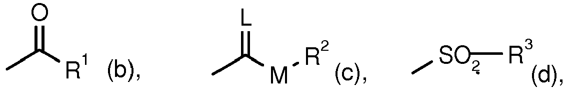 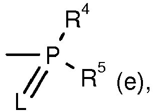 E (f) oder 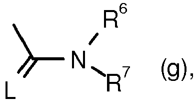 insbesondere für (a), (b) oder (c), in welchen
E für ein Metallionäquivalent oder ein Ammoniumion steht,
L für Sauerstoff oder Schwefel steht und
M für Sauerstoff oder Schwefel steht,
- R¹: steht besonders bevorzugt für jeweils gegebenenfalls einfach bis dreifach durch Fluor oder Chlor substituiertes C₁-C₈-Alkyl, C₂-C₈-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenenfalls ein oder zwei nicht direkt benachbarte Ringglieder durch Sauerstoff ersetzt sind,
für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl,
- R²: steht besonders bevorzugt für jeweils gegebenenfalls einfach bis dreifach durch Fluor substituiertes C₁-C₈-Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl,
- R³: steht besonders bevorzugt für gegebenenfalls einfach bis dreifach durch Fluor substituiertes C₁-C₆-Alkyl oder für gegebenenfalls einfach durch Fluor, Chlor, Brom, C₁-C₄-Alkyl, C₁-C₄-Alkoxy, Trifluormethyl, Trifluormethoxy, Cyano oder Nitro substituiertes Phenyl,
- R⁴: steht besonders bevorzugt für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder Trifluormethyl substituiertes Phenyl, Phenoxy oder Phenylthio,
- R⁵: steht besonders bevorzugt für C₁-C₆-Alkoxy oder C₁-C₆-Alkylthio,
- R⁶: steht besonders bevorzugt für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phenyl, für gegebenenfalls einfach durch Fluor, Chlor, Brom, C₁-C₄-Alkyl, Trifluormethyl oder C₁-C₄-Alkoxy substituiertes Benzyl,
- R⁷: steht besonders bevorzugt für C₁-C₆-Alkyl, C₃-C₆-Alkenyl oder C₁-C₆-Alkoxy-C₁-C₄-alkyl,
- R⁶ und R⁷: stehen besonders bevorzugt zusammen für einen gegebenenfalls durch Methyl oder Ethyl substituierten C₄-C₅-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- W: steht ganz besonders bevorzugt für Wasserstoff, Chlor, Methyl oder Ethyl,
- X: steht ganz besonders bevorzugt für Chlor, Methyl, Ethyl, Methoxy oder Ethoxy,
- Y: steht ganz besonders bevorzugt für Wasserstoff, Methyl oder Chlor,
- Z: steht ganz besonders bevorzugt für die Gruppe 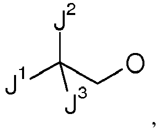
in welcher J¹ und J² ganz besonders bevorzugt jeweils unabhängig für Wasserstoff oder Fluor stehen und J³ für Fluor, Chlor oder Trifluormethyl steht,
- CKE: steht ganz besonders bevorzugt für eine der Gruppen 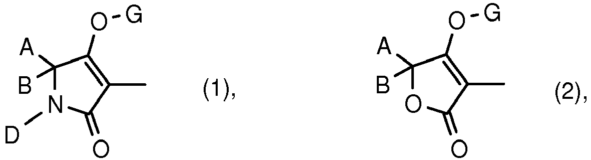 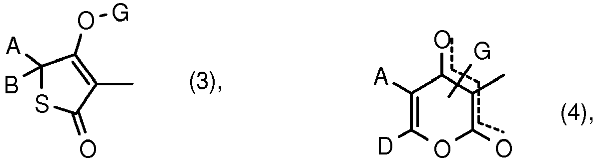 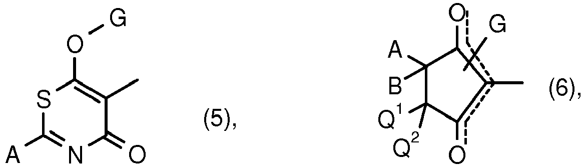 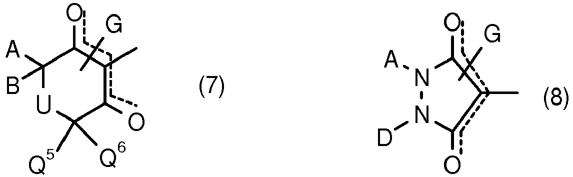 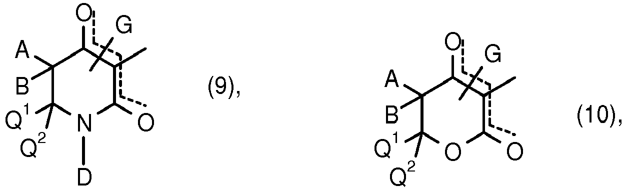
oder 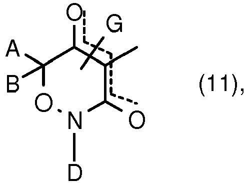
- U: steht ganz besonders bevorzugt für -CH₂-, -CH₂-CH₂-, -O- oder

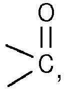

- A: steht ganz besonders bevorzugt für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl und im Fall der Verbindungen der Formel (I-5) für gegebenenfalls einfach bis zweifach durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl,
- B: steht ganz besonders bevorzugt für Wasserstoff, Methyl oder Ethyl oder
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Stickstoff, Sauerstoff oder Schwefel ersetzt ist und welches gegebenenfalls einfach oder zweifach durch Methyl, Ethyl, Methoxymethyl, Ethoxymethyl, Methoxyethyl, Ethoxyethyl, Trifluormethyl, Methoxy, Ethoxy, Propoxy, Butoxy, Methoxyethoxy, Ethoxyethoxy, Allyloxy, Trifluorethoxy oder Cyclopropylmethoxy substituiert ist, wobei die zuvor genannten Reste auch als N-Substituenten in Frage kommen, mit der Maßgabe, dass dann Q³ ganz besonders bevorzugt für Wasserstoff steht oder
- A, B: und das Kohlenstoffatom, an das sie gebunden sind, stehen ganz besonders bevorzugt für C₆-Cycloalkyl, welches gegebenenfalls durch eine gegebenenfalls durch ein Sauerstoffatom unterbrochene Alkylidendiyl-Gruppe oder durch eine mit zwei nicht direkt benachbarten Sauerstoffatomen enthaltende Alkylendioxy-Gruppe substituiert ist, wobei ein weiter 5- oder 6-Ring gebildet wird (der einfach oder zweifach durch Methyl substituiert sein kann) mit der Maßgabe, dass dann Q³ ganz besonders bevorzugt für Wasserstoff steht oder
- A, B: und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl oder Butadiendiyl stehen, mit der Maßgabe, dass dann Q³ ganz besonders bevorzugt für Wasserstoff steht,
- D: steht ganz besonders bevorzugt für Wasserstoff, für jeweils gegebenenfalls einfach bis dreifach durch Fluor substituiertes C₁-C₄-Alkyl, C₃-C₄-Alkenyl, C₁-C₄-Alkoxy-C₁-C₃-alkyl, für Cyclopropyl, Cyclopentyl oder Cyclohexyl oder (im Fall der Verbindungen der Formeln (I-4)) für jeweils gegebenenfalls einfach durch Fluor, Chlor, Methyl, Ethyl, n-Propyl, iso-Propyl, Methoxy, Ethoxy oder Trifluormethyl substituiertes Phenyl oder Pyridyl,
oder
- A und D: stehen gemeinsam ganz besonders bevorzugt für gegebenenfalls einfach durch Methyl oder Methoxy substituiertes C₃-C₅-Alkandiyl, worin gegebenenfalls ein Kohlenstoffatom durch eine Carbonylgruppe (nicht jedoch im Fall der Verbindung der Formel (I-11)), Sauerstoff oder Schwefel ersetzt ist oder für die Gruppe AD-1 steht, oder

A und D stehen gemeinsam ganz besonders bevorzugt für C₃-C₅-Alkandiyl, welches gegebenenfalls durch eine mit zwei nicht direkt benachbarten Sauerstoffatomen enthaltende gegebenenfalls einfach bis zweifach durch C₁-C₂-Alkyl substituierte Alkylendioxyl-Gruppe substituiert ist, wobei ein weiterer 5- Ring gebildet wird, oder
- A und Q¹: stehen gemeinsam ganz besonders bevorzugt für gegebenenfalls einfach oder zweifach durch Methyl oder Methoxy substituiertes C₃-C₄-Alkandiyl, welches gegebenenfalls die nachfolgende Gruppe enthält: 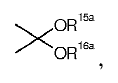
in welcher R^{15a} und R^{16a} gemeinsam ganz besonders bevorzugt für einen C₂-C₄-Alkandiyl- oder C₄-Alkendiylrest stehen, oder
- B und Q²: stehen gemeinsam ganz besonders bevorzugt für -CH₂-CH₂-CH₂-, oder -CH₂-O-CH₂-, oder
- D und Q¹: stehen gemeinsam ganz besonders bevorzugt für C₃-C₄-Alkandiyl oder
- Q¹: steht ganz besonders bevorzugt für Wasserstoff, Methyl, Ethyl, Propyl, iso-Propyl, Cyclopropyl, Cyclopentyl oder Cyclohexyl,
- Q²: steht ganz besonders bevorzugt für Wasserstoff, Methyl oder Ethyl,
- Q⁴, Q⁵ und Q⁶: stehen ganz besonders bevorzugt unabhängig voneinander für Wasserstoff oder Methyl,
- Q³: steht ganz besonders bevorzugt für Wasserstoff, Methyl, Ethyl, Propyl, Methoxy oder Ethoxy, oder für gegebenenfalls einfach durch Methyl oder Methoxy substituiertes gegebenenfalls durch ein Sauerstoffatom unterbrochenes C₃-C₆-Cycloalkyl, oder
- Q¹ und Q²: stehen ganz besonders bevorzugt mit dem Kohlenstoffatom, an das sie gebunden sind, für gegebenenfalls durch Methyl oder Methoxy substituiertes C₅-C₆-Cycloalkyl worin gegebenenfalls eine Methylengruppe durch Sauerstoff ersetzt ist, mit der Maßgabe, dass A und B für Wasserstoff stehen, oder
- Q³ und Q⁴: stehen ganz besonders bevorzugt gemeinsam mit dem Kohlenstoff, an den sie gebunden sind, für einen gegebenenfalls einfach durch Methyl oder Methoxy substituierten gegebenenfalls durch ein Sauerstoffatom unterbrochenen, gesättigten C₅-C₆-Ring, mit der Maßgabe, dass dann A, B, Q⁵ und Q⁶ ganz besonders bevorzugt für Wasserstoff stehen,
- G: steht ganz besonders bevorzugt für Wasserstoff (a) oder für eine der Gruppen 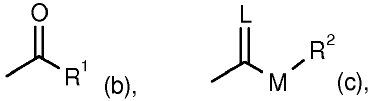 -SO₂-R³ (d) oder E (f), in welchen
L für Sauerstoff oder Schwefel steht,
M für Sauerstoff oder Schwefel steht und
E für ein Metallionäquivalent oder ein Ammoniumion steht,
- R¹: steht ganz besonders bevorzugt für jeweils gegebenenfalls einfach durch Chlor substituiertes C₁-C₆-Alkyl, C₂-C₆-Alkenyl, C₁-C₂-Alkoxy-C₁-alkyl, C₁-C₂-Alkylthio-C₁-alkyl oder jeweils gegebenenfalls einfach durch Fluor, Chlor, Methyl oder Methoxy substituiertes Cyclopropyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
- R²: steht ganz besonders bevorzugt für jeweils gegebenenfalls einfach durch Fluor substituiertes C₁-C₈-Alkyl, C₂-C₆-Alkenyl oder C₁-C₄-Alkoxy-C₂-C₃-alkyl, Phenyl oder Benzyl,
- R³: steht ganz besonders bevorzugt für C₁-C₈-Alkyl.
- W: steht insbesondere bevorzugt für Wasserstoff, Methyl oder Ethyl,
- X: steht insbesondere bevorzugt für Chlor, Methyl oder Ethyl,
- Y: steht insbesondere bevorzugt für Wasserstoff,
- Z: steht insbesondere bevorzugt für OCH₂-CF₃ in der 3-Position,
- Z: steht insbesondere bevorzugt auch für OCH₂-CF₃ in der 4-Position,
- Z: steht insbesondere bevorzugt ebenso für OCH₂-CF₃ in der 5-Position,
- CKE: steht insbesondere bevorzugt für eine der Gruppen

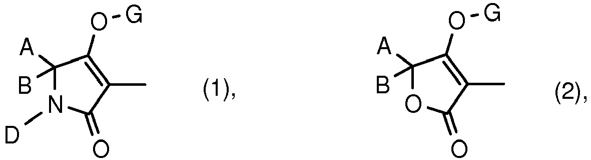

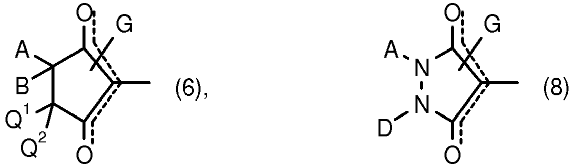

- A: steht insbesondere bevorzugt für Methyl oder Ethyl,
- B: steht insbesondere bevorzugt für Wasserstoff oder Methyl,
- A, B: und das Kohlenstoffatom an das sie gebunden sind, stehen insbesondere bevorzugt für gesättigtes C₅-C₆-Cycloalkyl, in welchem gegebenenfalls ein Ringglied durch Sauerstoff ersetzt ist und welches gegebenenfalls einfach oder zweifach durch Methyl, Ethyl, Methoxymethyl, Methoxy, Ethoxy, Propoxy, Butoxy, Trifluorethoxy substituiert ist, oder
- A, B: und das Kohlenstoffatom, an das sie gebunden sind, stehen ganz besonders bevorzugt für C₆-Cycloalkyl, welches gegebenenfalls durch eine mit zwei nicht direkt benachbarten Sauerstoffatomen enthaltende Alkylendioxyl-Gruppe substituiert ist, wobei ein weiterer 5- oder 6-Ring gebildet wird, der einfach oder zweifach durch Methyl substituiert sein kann,
- D: steht insbesondere bevorzugt für Wasserstoff, oder
- A und D: stehen gemeinsam insbesondere bevorzugt für C₃-C₅-Alkandiyl, worin gegebenenfalls ein Kohlenstoffatom durch Sauerstoff ersetzt ist oder

A und D stehen gemeinsam insbesondere bevorzugt für C₃-C₅-Alkandiyl, welches gegebenenfalls durch eine mit zwei nicht direkt benachbarten Sauerstoffatomen enthaltende gegebenenfalls einfach bis zweifach durch Methyl substituierte Alkylendioxyl-Gruppe substituiert ist, wobei ein weiterer 5- Ring gebildet wird, (hervorgehoben stehen A und D gemeinsam für C₃-C₅-Alkandiyl, welches gegebenenfalls durch eine mit zwei nicht direkt benachbarten Sauerstoffatomen enthaltende Alkylendioxy-Gruppe substituiert ist, wobei ein weiterer 5- Ring gebildet wird), oder
- A und Q¹: stehen gemeinsam insbesondere bevorzugt für C₃-C₄-Alkandiyl,
- Q²: steht insbesondere bevorzugt für Wasserstoff,
- G: steht insbesondere bevorzugt für Wasserstoff (a) oder für eine der Gruppen 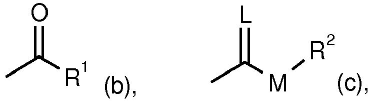
in welchen
- L: für Sauerstoff steht,
- M: für Sauerstoff steht,
- R¹: steht insbesondere bevorzugt für C₁-C₆-Alkyl,
- R²: steht insbesondere bevorzugt für C₁-C₆-Alkyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Hervorgehoben sind Verbindungen der Formel (I) in welchen G für Wasserstoff steht.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im Einzelnen seien außer bei den Beispielen genannten Verbindungen die folgenden Verbindungen mit Z = OCH₂-CF₃ genannt:

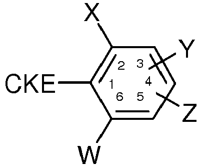

**Tabelle 1**

| **X** | **W** | **Y** | **Z** |
|---|---|---|---|
| CH₃ | H | H | 4 |
| CH₃ | H | H | 5 |
| Cl | H | H | 4 |
| Cl | H | H | 5 |
| OCH₃ | H | H | 4 |
| OCH₃ | H | H | 5 |
| C₂H₅ | H | H | 4 |
| C₂H₅ | H | H | 5 |
| CH₃ | CH₃ | H | 4 |
| CH₃ | CH₃ | H | 5 |
| C₂H₅ | CH₃ | H | 4 |
| C₂H₅ | C₂H₅ | H | 4 |
| CH₃ | Cl | H | 4 |
| C₂H₅ | Cl | H | 4 |

Als erfindungsgemäße Wirkstoffe kommen insbesondere bevorzugt Verbindungen aus den in Tabelle 1 genannten Restekombinationen für W, X, Y und Z mit den in Tabellen 2a und 2b genannten Restekombinationen für A, B und D in Frage.

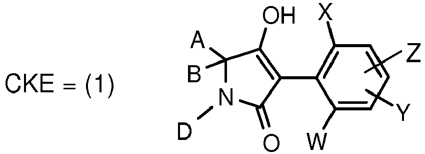

**Tabelle 2a**

| **A** | **B** | **D** |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 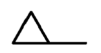 | CH₃ | H |
| 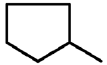 | CH₃ | H |
| 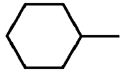 | CH₃ | H |
| H₃CO-CH₂- | CH₃ | H |
| H₅C₂O-CH₂- | CH₃ | H |
| H₃CO-(CH₂)₂- | CH₃ | H |
| H₅C₂O-(CH₂)₂- | CH₃ | H |
| 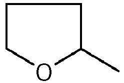 | CH₃ | H |
| 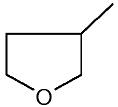 | CH₃ | H |
| -(CH₂)₂- | | H |
| -(CH₂)₄- | | H |
| -(CH₂)₅- | | H |
| -(CH₂)₆- | | H |
| -(CH₂)₇- | | H |
| 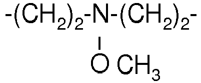 | | H |
| 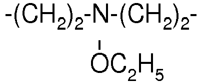 | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -CH₂-CHOCH₃-(CH₂)₂- | | H |
| -CH₂-CHOC₂H₅-(CH₂)₂- | | H |
| -CH₂-CHOC₃H₇-(CH₂)₂- | | H |
| -CH₂-CHOC₄H₉-(CH₂)₂- | | H |
| -CH₂-CHO(CH₂)₂OCH₃-(CH₂)₂- | | H |
| 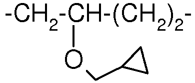 | | H |
| -CH₂-CHOCH₃-(CH₂)₃- | | H |
| -CH₂-CHOC₂H₅-(CH₂)₃- | | H |
| -CH₂-CHOC₃H₇-(CH₂)₃- | | H |
| -CH₂-CHOC₄H₉-(CH₂)₃- | | H |
| -CH₂-CHO(CH₂)₂OCH₃-(CH₂)₃- | | H |
| 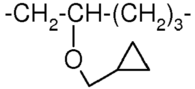 | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHO-CH₂CF₃-(CH₂)₂- | | H |
| -(CH₂)₂-C(CH₃)₂-(CH₂)₂- | | H |
| -CH₂-(CHCH₃)₂-(CH₂)₂- | | H |
| 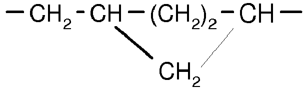 | | H |
| 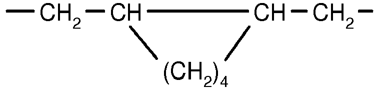 | | H |
| 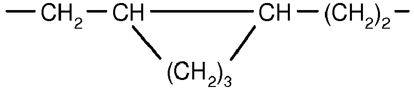 | | H |
| 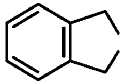 | | H |
| 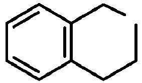 | | H |
| 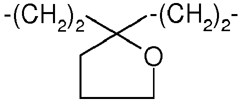 | | H |
| 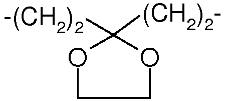 | | H |
| 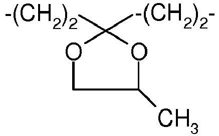 | | H |
| 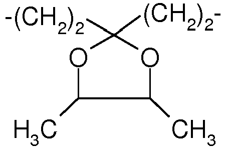 | | H |
| 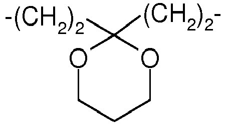 | | H |
| 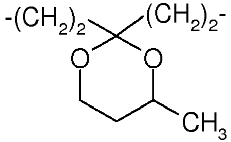 | | H |
| 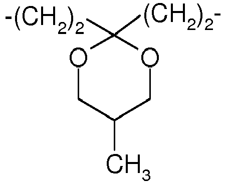 | | H |
| 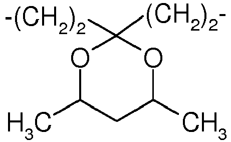 | | H |
| 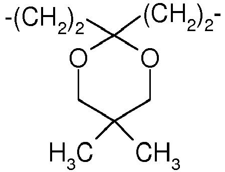 | | H |
| 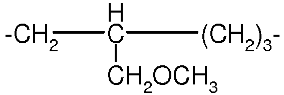 | | H |
| 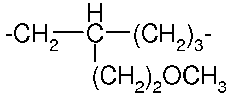 | | H |
| 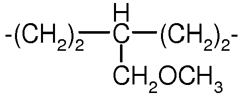 | | H |
| 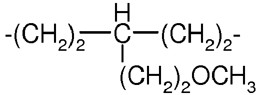 | | H |
| 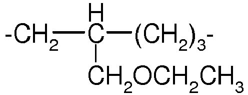 | | H |
| 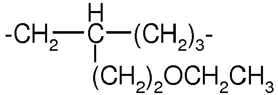 | | H |
| 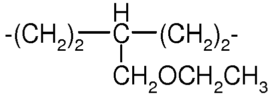 | | H |
| 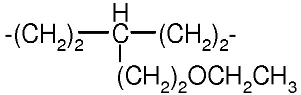 | | H |

**Tabelle 2b**

| **A** | **D** | **B** |
|---|---|---|
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-CH(OCH₃)-CH₂- | | H |
| -CH₂-CH=CH-CH₂- | | H |
| 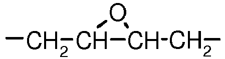 | | H |
| -CH₂-S-CH₂- | | H |
| -CH₂-S-(CH₂)₂- | | H |
| -(CH₂)₂-S-CH₂- | | H |
| 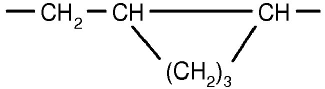 | | H |
| H | CH₃ | H |
| H | C₂H₅ | H |
| H | C₃H₇ | H |
| H | i-C₃H₇ | H |
| H | 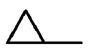 | H |
| H | 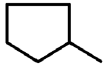 | H |
| H | 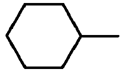 | H |
| CH₃ | CH₃ | H |
| CH₃ | C₂H₅ | H |
| CH₃ | C₃H₇ | H |
| CH₃ | i-C₃H₇ | H |
| CH₃ | 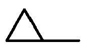 | H |
| CH₃ | 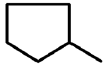 | H |
| CH₃ | 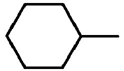 | H |
| C₂H₅ | CH₃ | H |
| C₂H₅ | C₂H₅ | H |
| H | H₃CO-(CH₂)₂- | H |
| H | H₅C₂O-(CH₂)₂- | H |
| H | H₃CO-CH₂-CH(CH₃)- | H |
| H | H₃CO-CHCH₃-CH₂- | H |
| CH₃ | H₃CO-(CH₂)₂- | H |
| CH₃ | H₅C₂O-(CH₂)₂- | H |
| CH₃ | H₃CO-CH₂-CH(CH₃)- | H |
| CH₃ | H₃CO-CHCH₃-CH₂- | H |

Als Wirkstoffe hervorgehoben werden insbesonders bevorzugte Verbindungen mit den in Tabelle 1 genannten Restekombinationen für W, X, Y und Z und den in den Tabellen 2a und 2b für A, B und D genannten Restekombinationen.

Als erfindungsgemäße Wirkstoffe kommen weiterhin insbesonders bevorzugt Verbindungen aus mit den in Tabelle 1 genannten Restekombinationen für W, X, Y und Z mit den in Tabelle 3 genannten Restekombinationen für A und B in Frage.

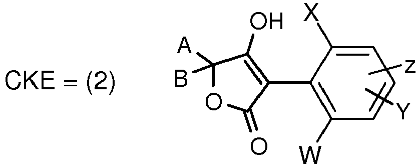

**Tabelle 3**

| **A** | **B** |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 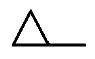 | CH₃ |
| 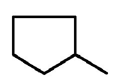 | CH₃ |
| 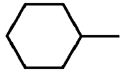 | CH₃ |
| H₃CO-CH₂- | CH₃ |
| H₅C₂O-CH₂- | CH₃ |
| H₃CO-(CH₂)₂- | CH₃ |
| H₅C₂O-(CH₂)₂- | CH₃ |
| 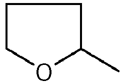 | CH₃ |
| 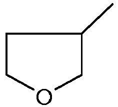 | CH₃ |
| -(CH₂)₂- | |
| -(CH₂)₄- | |
| -(CH₂)₅- | |
| -(CH₂)₅- | |
| -(CH₂)₇- | |
| 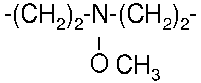 | |
| 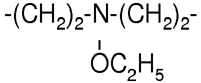 | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -CH₂-CHOCH₃-(CH₂)₃- | |
| -CH₂-CHOC₂H₅-(CH₂)₃- | |
| -CH₂-CHOC₃H₇-(CH₂)₃- | |
| -CH₂-CHOC₄H₉-(CH₂)₃- | |
| -CH₂-CHO(CH₂)₂OCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHO-CH₂CF₃-(CH₂)₂- | |
| -(CH₂)₂-C(CH₃)₂-(CH₂)₂- | |
| -CH₂-(CHCH₃)₂-(CH₂)₂- | |
| 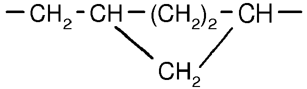 | |
| 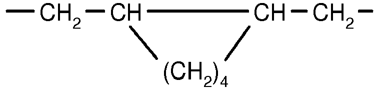 | |
| 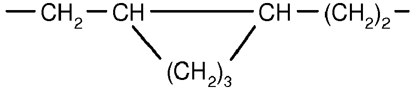 | |
| 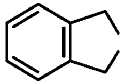 | |
| 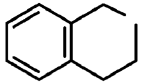 | |
| 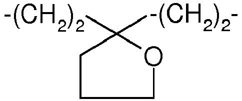 | |
| 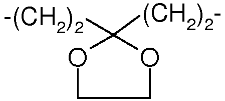 | |
| 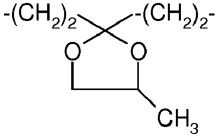 | |
| 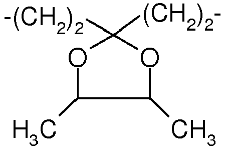 | |
| 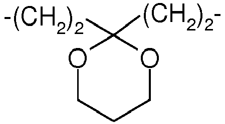 | |
| 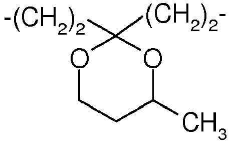 | |
| 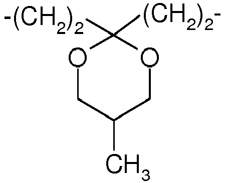 | |
| 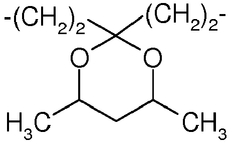 | |
| 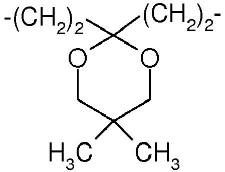 | |
| 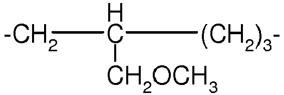 | |
| 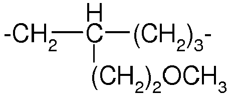 | |
| 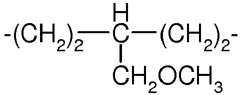 | |
| 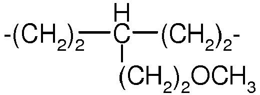 | |
| 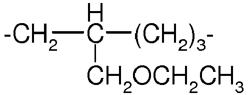 | |
| 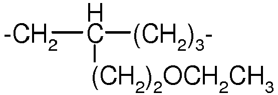 | |
| 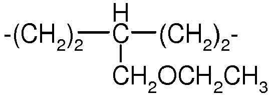 | |
| 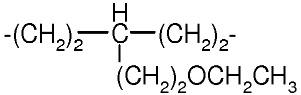 | |

Als Wirkstoffe hervorgehoben werden insbesondere bevorzugte Verbindungen mit den in Tabelle 1 genannten Restekombinationen für W, X, Y und Z und den in Tabelle 3 für A und B genannten Restekombinationen.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und für phenylsubstituierte Cyclische Ketoenole beschrieben (US 6 645 914, EP-A2 0 036 106, WO 07/068427). Eine entsprechende Wirkungssteigerung bei Insektiziden wurde bereits durch WO 07/068428 beschrieben.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun ebenfalls überraschend gefunden, dass sich die Wirkung von Insektiziden und/oder Akariziden und/oder Herbiziden aus der Klasse der Halogenalkylmethylenoxy-phenyl-substituierte Ketoenole der Formel (I) durch den Zusatz von Ammonium- oder Phosphoniumsalzen zur Anwendungslösung oder durch den Einbau dieser Salze in eine Formulierung enthaltend der Halogenalkylmethylenoxy-phenyl-substituierte Ketoenole der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die herbizid und/oder insektizid und/oder akarizid wirksame der Halogenalkylmethylenoxy-phenyl-substituierte Ketoenole der Formel (I) als Wirkstoff enthalten. Gegenstand der Erfindung sind ebenfalls Mittel, die herbizid und/oder akarizid und/oder insektizid wirksame Halogenalkylmethylenoxy-phenyl-substituierte Ketoenole der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Verbindungen der Formel (I) besitzen eine breite insektizide und/oder akarizide und/oder herbizide Wirkung, die Wirkung und/oder Pflanzenverträglichkeit lässt im Einzelnen aber zu wünschen übrig.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Fettsäure-Biosynthese-Inhibitoren steigern, werden durch Formel (III') definiert 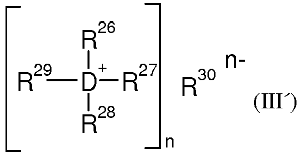 in welcher
- D: für Stickstoff oder Phosphor steht,
- D: bevorzugt für Stickstoff steht,
- R²⁶, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R²⁶, R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
R²⁶, R²⁷, R²⁸ und R²⁹ besonders bevorzugt unabhängig voneinander für Wasserstoff, Methyl, Ethyl, n-Propyl, i-Propyl, n-Butyl, i-Butyl, s-Butyl oder t-Butyl stehen,
- R²⁶, R²⁷, R²⁸ und R²⁹: ganz besonders bevorzugt für Wasserstoff stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R³⁰: besonders bevorzugt für Laktat, Sulfat, Nitrat, Thiosulfat, Thiocyanat, Oxalat oder Formiat steht,
- R³⁰: ganz besonders bevorzugt für Sulfat steht.

Erfindungsgemäß hervorgehobene Kombinationen von Wirkstoff, Salz und Penetrationsförderer sind in folgender Tabelle aufgeführt. "Penetrationsförderer gemäß Test" bedeutet dabei, dass jede Verbindung geeignet ist, die in dem Test für die Kutikelpenetration (Baur et al., 1997, Pesticide Science 51, 131-152) als Penetrationsförderer wirkt.

Die Ammonium- und Phosphoniumsalze der Formel (III') können in einem breiten Konzentrationsbereich zur Steigerung der Wirkung von Pflanzenschutzmitteln enthaltend Ketoenole eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid und/oder akarizid und/oder herbizid wirksame Halogenalkylmethylenoxy-phenyl-substituierte Ketoenole der Formel (I) als Wirkstoff enthalten. Gegenstand der Erfindung sind ebenfalls Mittel, die herbizid und/oder akarizid und/oder insektizid wirksame Halogenalkylmethylenoxy-phenyl-substituierte Ketoenole der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässrigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)ᵥ-R' (IV')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,

- PO: für 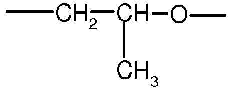 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,

- PO: für 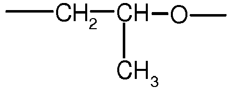 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
- R und R': die oben angegebenen Bedeutungen haben,
- EO: für -CH₂-CH₂-O- steht,

- BO: für 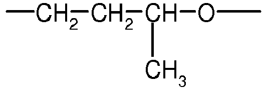 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
- R und R': die oben angegebenen Bedeutungen haben,
- BO: für 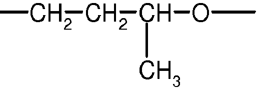 steht,
- EO: für -CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV-c) sei 2-Ethyl-hexyl-alkoxylat der Formel 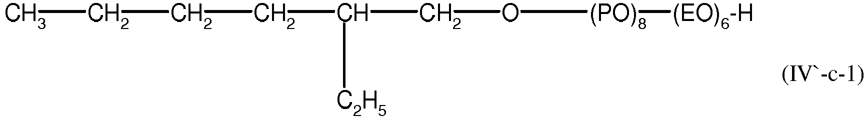 in welcher
- EO: für -CH₂-CH₂-O- steht,
- PO: für 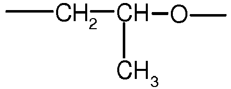 steht und
die Zahlen 8 und 6 Durchschnittswerte darstellen, genannt.

Als Beispiel für ein Alkanol-Alkoxylat der Formel (IV-d) sei die Formel

CH₃-(CH₂)₁₀-O-(-EO-)₆-(-BO-)₂-CH₃ (IV'-d-1)

in welcher
- EO: für -CH₂-CH₂-O- steht,
- BO: für 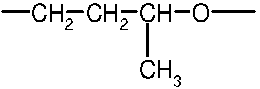 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Verwendet man beispielsweise gemäß Verfahren (A) N-(2,6-Dimethyl-4-trifluorethoxy-phenylacetyl)-1-amino-cyclohexan-carbonsäureethylester als Ausgangsstoff, so kann der Verlauf des erfindungsgemäßen Verfahrens durch folgendes Reaktionsschema wiedergegeben werden:

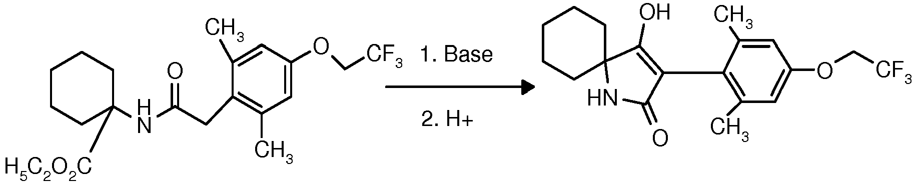

Verwendet man beispielsweise gemäß Verfahren (B) O-(2,6-Dimethyl-4-trifluorethoxy-phenylacetyl)-2-hydroxyisobuttersäureethylester, so kann der Verlauf des erfindungsgemäßen Verfahrens durch folgendes Reaktionsschema wiedergegeben werden:

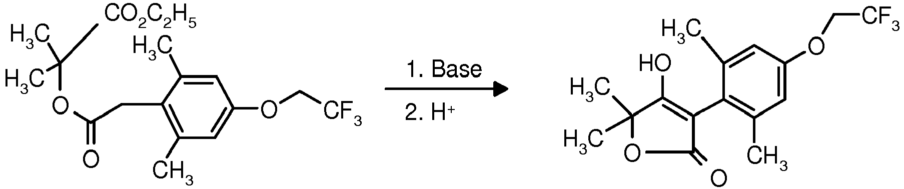

Verwendet man beispielsweise gemäß Verfahren (C) 2-(2,6-Dimethyl-4-trifluorethoxy-phenyl)-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden: 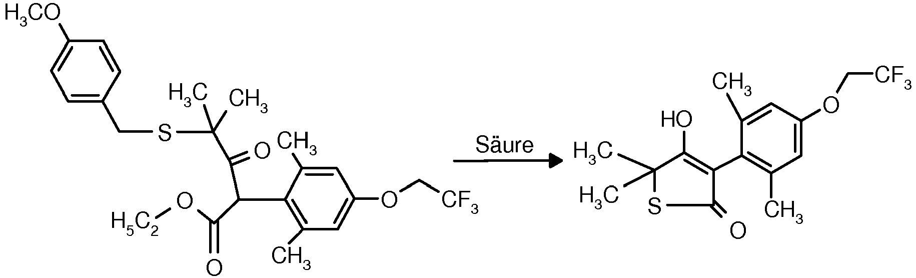 Verwendet man beispielsweise gemäß Verfahren (D) (Chlorcarbonyl)-2-[(2,6-Dimethyl-4-trifluorethoxy-phenyl)-keten und Aceton als Ausgangsverbindungen, so kann der Verlauf des erfindungsgemäßen Verfahrens durch das folgende Reaktionsschema wiedergegeben werden:

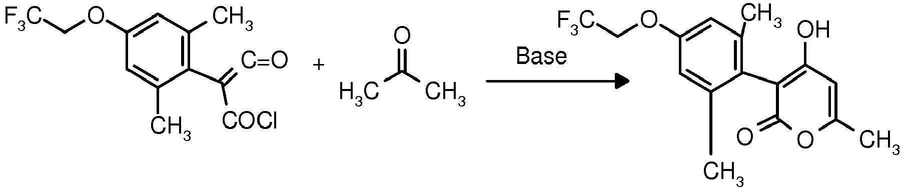

Verwendet man beispielsweise gemäß Verfahren (E) (Chlorcarbonyl)-2-(2,6-Dimethyl-4-trifluorethoxy-phenyl)-keten und Thiobenzamid als Ausgangsverbindungen, so kann der Verlauf des erfindungsgemäßen Verfahrens durch das folgende Reaktionsschema wiedergegeben werden:

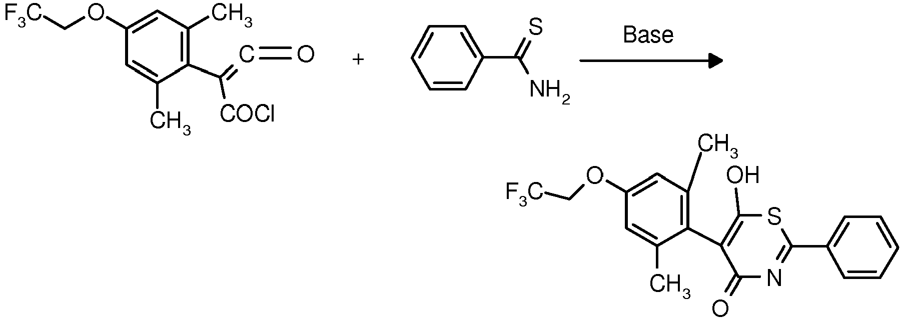

Verwendet man beispielsweise gemäß Verfahren (F) 5-(2,6-Dimethyl-4-trifluorethoxy-phenyl)-2,3-trimethylen-4-oxo-valeriansäureethylester, so kann der Verlauf des erfindungsgemäßen Verfahrens durch folgendes Reaktionsschema wiedergegeben werden:

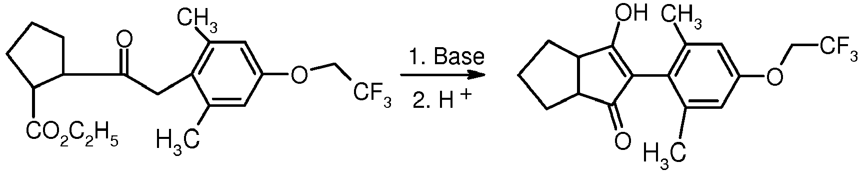

Verwendet man beispielsweise gemäß Verfahren (G) 6-[(2,6-Dimethyl-4-trifluorethoxy-)-phenyl]-2-dimethyl-5-oxo-hexansäure-ethylester, so kann der Verlauf des erfindungsgemäßen Verfahrens durch folgendes Reaktionsschema wiedergegeben werden:

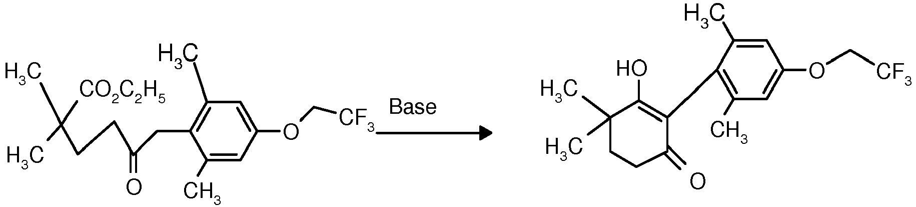

Verwendet man beispielsweise gemäß Verfahren (Hα) Hexahydropyridazin und (Chlorcarbonyl)-2-[(2,6-dimethyl-4-trifluorethoxy-)-phenyl]-keten als Ausgangsverbindungen, so kann der Reaktionsverlauf des erfindungsgemäßen Verfahrens durch folgendes Reaktionsschema wiedergegeben werden:

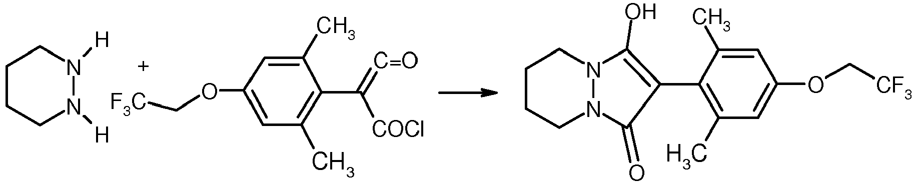

Verwendet man beispielsweise gemäß Verfahren (Hβ) Hexahydropyridazin und 2-(2,6-Dimethyl-4-trifluorethoxy-)-phenylmalonsäuredimethylester als Ausgangsprodukte, so kann der Verlauf des erfindungsgemäßen Verfahrens durch folgendes Reaktionsschema wiedergegeben werden:

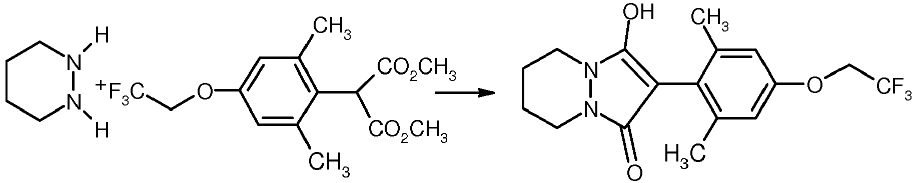

Verwendet man beispielsweise gemäß Verfahren (Hγ) 1-Ethoxycarbonyl-2-[(2,6-dimethyl-4-trifluorethoxy)-phenylacetyl]-hexahydropyridazin als Ausgangsprodukt, so kann der Reaktionsverlauf durch folgendes Schema wiedergegeben werden:

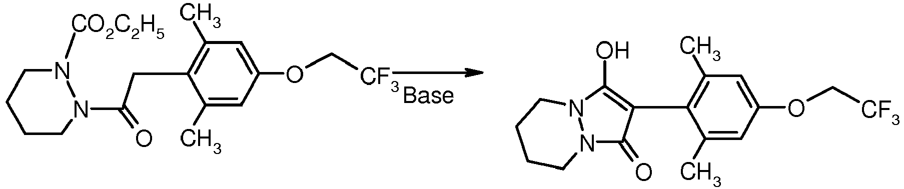

Verwendet man beispielsweise gemäß Verfahren (I) N-(2,6-Dimethyl-4-trifluorethoxy-phenylacetyl)-1-aminomethyl-cyclohexan-carbonsäureethylester als Ausgangsstoff, so kann der Verlauf des erfindungsgemäßen Verfahrens durch folgendes Reaktionsschema wiedergegeben werden:

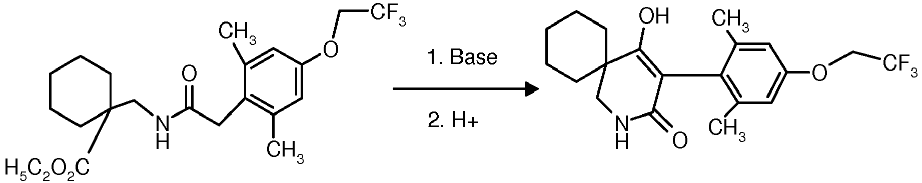

Verwendet man beispielsweise gemäß Verfahren (J) O-(2,6-Dimethyl-4-trifluorethoxy-phenylacetyl)-3-hydroxy-2,2-dimethyl-propion-säureethylester, so kann der Verlauf des erfindungsgemäßen Verfahrens durch folgendes Reaktionsschema wiedergegeben werden:

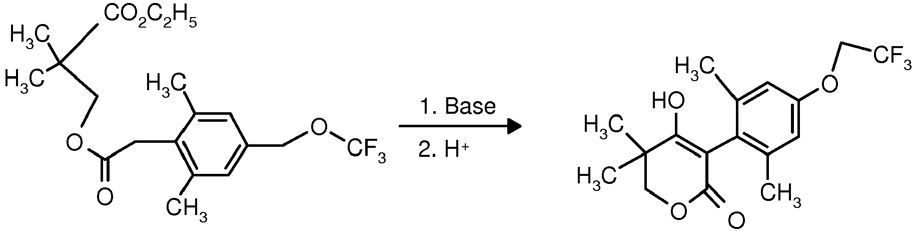

Verwendet man gemäß Verfahren (K) N-Methyl-N-[(2,6-Dimethyl-4-trifluorethoxy)-phenyl-acetyl]-1-amino-oxy-cyclopentan-carbonsäureethylester als Ausgangsstoff, so kann der Verlauf des erfindungsgemäßen Verfahrens durch folgendes Reaktionsschema wiedergegeben werden:

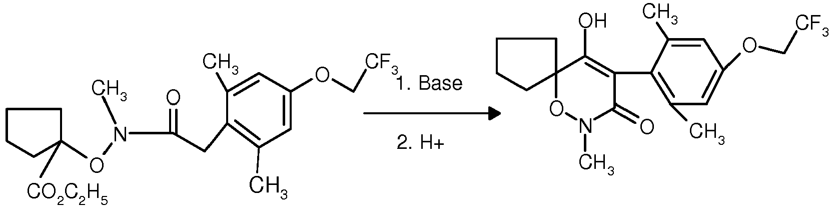

Verwendet man beispielsweise gemäß Verfahren (Lα) 3-(2-Methyl-4-trifluorethoxy-6-ethyl-phenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

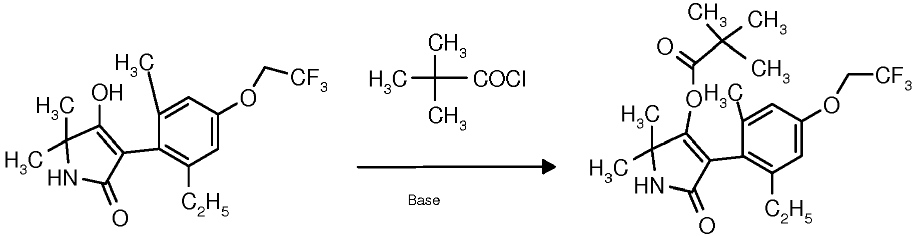

Verwendet man beispielsweise gemäß Verfahren (Lß) 3-(2,6-Dimethyl-4-trifluorethoxy-phenyl)-5,5-dimethylpyrrolidin-2,4-dion und Acetanhydrid als Ausgangsverbindungen, so kann der Verlauf des erfindungsgemäßen Verfahrens durch folgendes Reaktionsschema wiedergegeben werden:

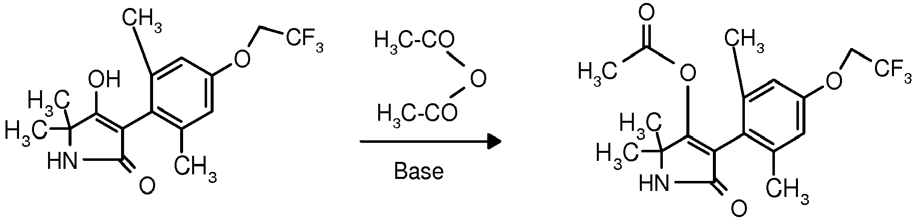

Verwendet man beispielsweise gemäß Verfahren (M) 8-[(2,6-Dimethyl-4-trifluorethoxy)-phenyl]-1-aza-bicyclo-(4,3,0^{1,6})-nonan-7,9-dion und Chlorameisensäureethylester als Ausgangsverbindungen, so kann der Verlauf des erfindungsgemäßen Verfahrens durch folgendes Reaktionsschema wiedergegeben werden:

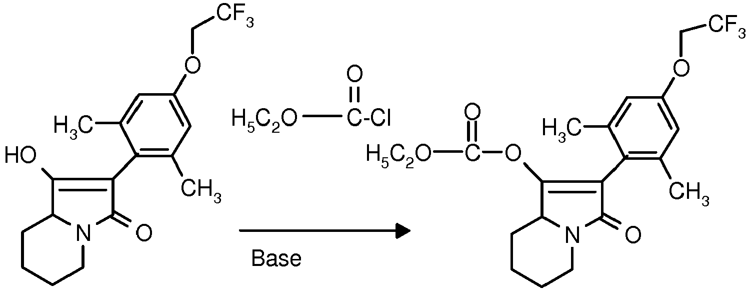

Verwendet man beispielsweise gemäß Verfahren (N), 3-(2,6-Dimethyl-4-trifluorethoxy-phenyl)-4-hydroxy-5-methyl-6-(phenyl)-pyron und Chlormonothioameisensäuremethylester als Ausgangsprodukte, so kann der Reaktionsverlauf folgendermaßen wiedergegeben werden:

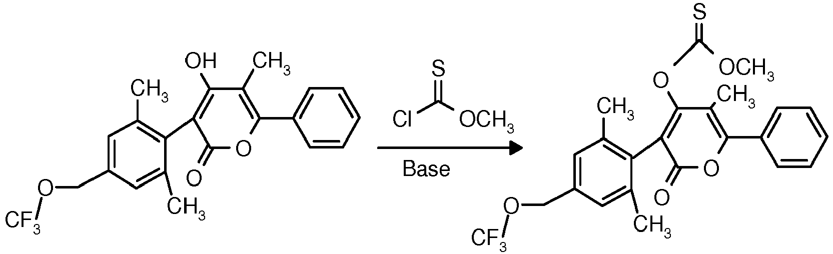

Verwendet man beispielsweise gemäß Verfahren (O) 3-(2,6-Dimethyl-4-trifluorethoxy-phenyl)-5,5-pentamethylen-pyrrolidin-2,4-dion und Methansulfonsäurechlorid als Ausgangsprodukt, so kann der Reaktionsverlauf durch folgendes Reaktionsschema wiedergegeben werden:

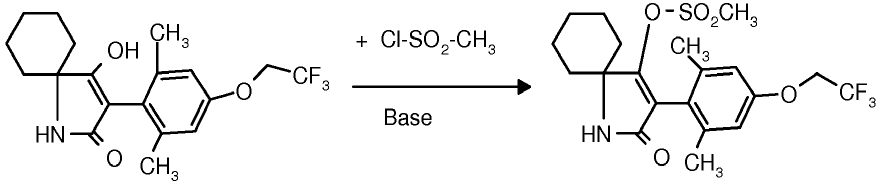

Verwendet man beispielsweise gemäß Verfahren (P) 3-(2,6-Dimethyl-4-trifluorethoxy-phenyl)-4-hydroxy-5,5-dimethyl-pyrrolidin-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

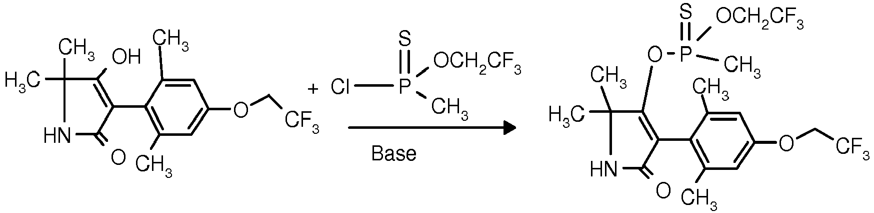

Verwendet man beispielsweise gemäß Verfahren (Q) 3-(2-Ethyl-4-trifluorethoxy-6-methyl-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

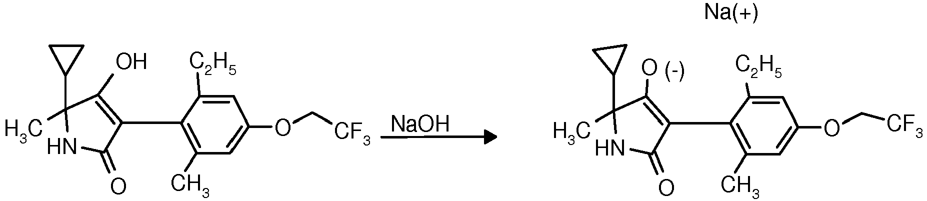

Verwendet man beispielsweise gemäß Verfahren (R) Variante α 3-(2,6-Dimethyl-4-trifluorethoxyphenyl)-4-hydroxy-5,5-tetramethylen-Δ³-dihydro-furan-2-on und Ethylisocyanat als Ausgangsprodukte, so kann der Reaktionsverlauf durch folgendes Reaktionsschema wiedergegeben werden:

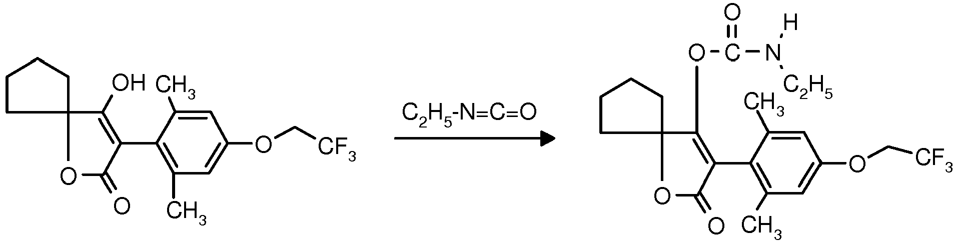

Verwendet man beispielsweise gemäß Verfahren (R) Variante ß 3-(2-Methyl-4-trifluorethoxy-6-ethyl-phenyl)-5-methyl-pyrrolidin-2,4-dion und Dimethylcarbamidsäurechlorid als Ausgangsprodukte, so kann der Reaktionsverlauf durch folgendes Schema wiedergegeben werden:

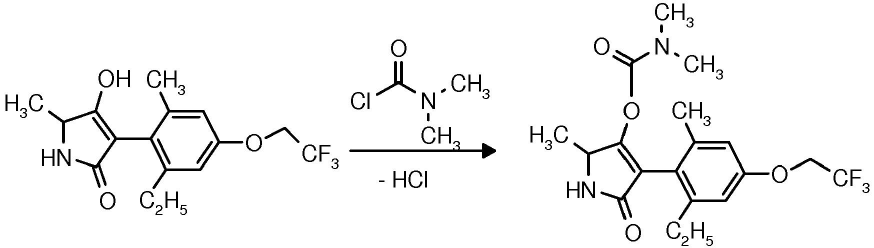

Verwendet man beispielsweise gemäß Verfahren (S) 3-(4-Brom-2,6-dimethyl-phenyl)-5,5-dimethyl-pyrrolidin-2,4-dion und Trifluorethanol als Ausgangsstoffe, so kann der Reaktionsverlauf durch folgendes Schema wiedergegeben werden:

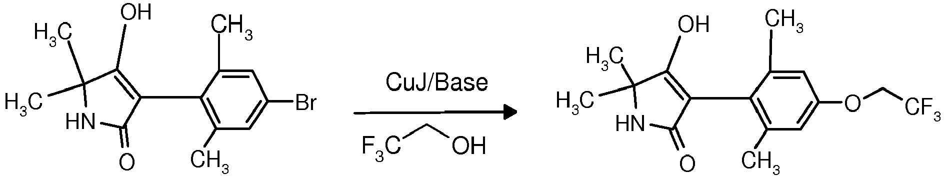

Die beim erfindungsgemäßen Verfahren (a) als Ausgangsstoffe benötigten Verbindungen der Formel (II) 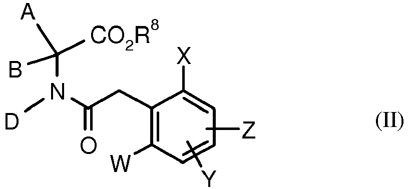 in welcher
A, B, D, W X, Y, Z und R⁸ die oben angegebenen Bedeutungen haben,
sind neu.

Man erhält die Acylaminosäureester der Formel (II) beispielsweise, wenn man Aminosäurederivate der Formel (XXVII) 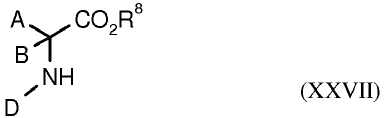 in welcher
A, B, R⁸ und D die oben angegebenen Bedeutungen haben,
mit substituierten Phenylessigsäurederivaten der Formel (XXVIII) 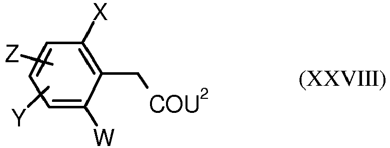
in welcher
W, X, Y und Z die oben angegebenen Bedeutungen haben und
U² für eine durch Carbonsäureaktivierungsreagenzien wie Carbonyldiimidazol, Carbonyldiimide (wie z.B. Dicyclohexylcarbondiimid), Phosphorylierungsreagenzen (wie z.B. POCl₃, BOP-Cl), Halogenierungsmittel z.B. Thionylchlorid, Oxalylchlorid, Phosgen oder Chlorameisensäsureester eingeführte Abgangsgruppe steht,
acyliert (Chem. Reviews 52, 237-416 (1953); Bhattacharya, Indian J. Chem. 6, 341-5, 1968) oder wenn man Acylaminosäuren der Formel (XXIX) 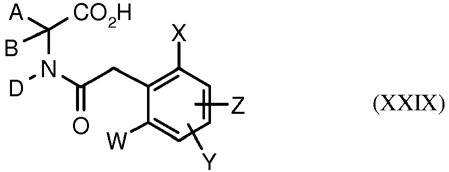 in welcher
A, B, D, W, X, Y und Z die oben angegebenen Bedeutungen haben, verestert (Chem. Ind. (London) 1568 (1968)).

Die Verbindungen der Formel (XXIX) 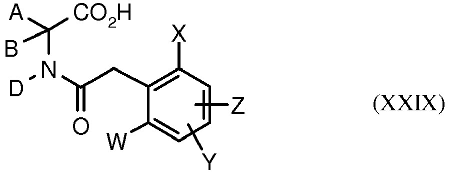 in welcher A, B, D, W, X,Y und Z die oben angegebenen Bedeutungen haben,
sind neu.

Man erhält die Verbindungen der Formel (XXIX), wenn man Aminosäuren der Formel (XXX) 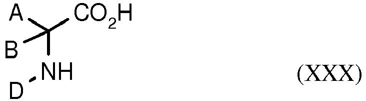 in welcher
A, B und D die oben angegebenen Bedeutungen haben,
mit substituierten Phenylessigsäurederivaten der Formel (XXVIII) 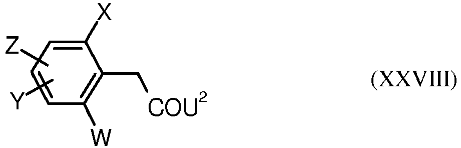 in welcher
W, X, Y und Z die oben angegebenen Bedeutungen haben und
U² die oben angegebene Bedeutung hat,
beispielsweise nach Schotten-Baumann acyliert (Organikum, VEB Deutscher Verlag der Wissenschaften, Berlin 1977, S. 505).

Die Verbindungen der Formel (XXVIII) sind neu. Sie lassen sich nach im Prinzip bekannten Verfahren darstellen (s. z.B. H. Henecka, Houben-Weyl, Methoden der Organischen Chemie, Bd. 8, S. 467-469 (1952)) WO 97/02243, WO 99/43699 oder werden mit den oben angegebenen Reagenzien in situ erzeugt.

Man erhält die Verbindungen der Formel (XXVIII) beispielsweise, indem man substituierte Phenylessigsäuren der Formel (XXXI) 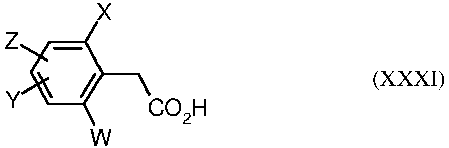 in welcher
W, X, Y und Z die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid), Phosphonylierungsreagenzien wie (z.B. POCl₃, BOP-Cl), Carbonyldiimidazol, Carbonyldiimide (z.B. Dicyclohexylcarbonyldiimid) gegebenenfalls in Gegenwart eines Verdünnungsmittels (z.B. gegebenenfalls chlorierten aliphatischen oder aromatischen Kohlenwasserstoffen wie Toluol oder Methylenchlorid oder Ethern, z.B. Tetrahydrofuran, Dioxan, Methyl-tert.-butylether) bei Temperaturen von -20°C bis 150°C, bevorzugt von -10°C bis 100°C, umsetzt.

Die Verbindungen der Formel (XXVII) und (XXX) sind teilweise aus der eingangs zitierten Patentliteratur bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXX), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als ß bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 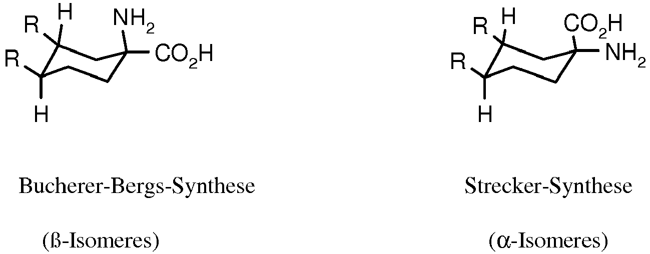 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 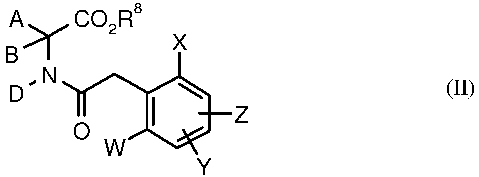 in welcher
A, B, D, W, X, Y, Z und R⁸ die oben angegebenen Bedeutungen haben,
herstellen, wenn man Aminonitrile der Formel (XXXII) 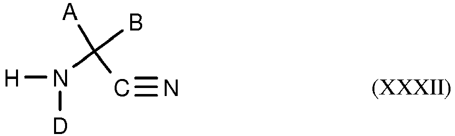 in welcher
- A, B und D: die oben angegebenen Bedeutungen haben,
mit substituierten Phenylessigsäurederivaten der Formel (XXVIII) 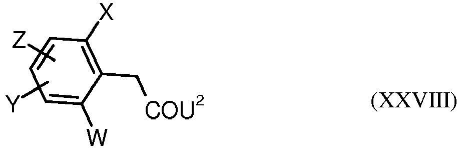 in welcher
W, X, Y, Z und U² die oben angegebenen Bedeutungen haben,
zu Verbindungen der Formel (XXXIII) 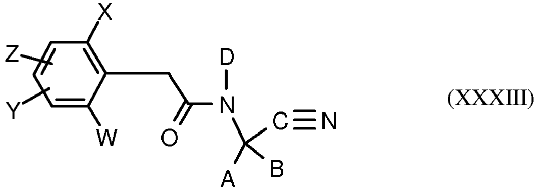
in welcher
A, B, D, W, X, Y und Z die oben angegebenen Bedeutungen haben,
umsetzt,
und diese anschließend einer sauren Alkoholyse unterwirft.

Die Verbindungen der Formel (XXXIII) sind ebenfalls neu.

Die bei dem erfindungsgemäßen Verfahren (B) als Ausgangstoffe benötigten Verbindungen der Formel (III) 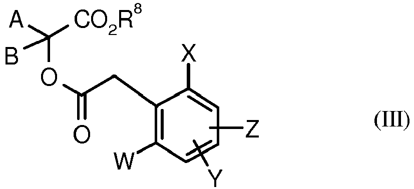 in welcher
A, B, W, X, Y, Z und R⁸ die oben angegebenen Bedeutungen haben,
sind neu.

Sie lassen sich nach im Prinzip bekannten Methoden herstellen.

So erhält man die Verbindungen der Formel (III) beispielsweise, wenn man 2-Hydroxycarbonsäureester der Formel (XXXIV-A) 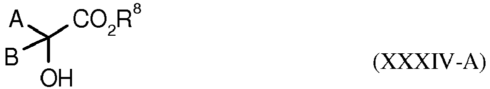 in welcher
- A, B und R⁸: die oben angegebenen Bedeutungen haben,
mit substituierten Phenylessigsäurederivaten der Formel (XXVIII) 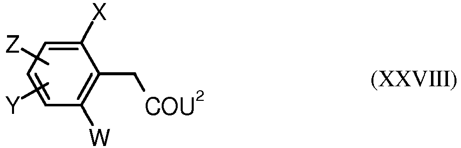 in welcher
W, X, Y, Z und U² die oben angegebenen Bedeutungen haben,
acyliert (Chem. Reviews 52, 237-416 (1953)).

Weiterhin erhält man Verbindungen der Formel (III), wenn man
substituierte Phenylessigsäuren der Formel (XXXI) 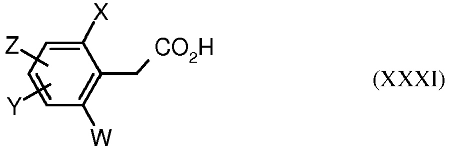 in welcher
W, X, Y und Z die oben angegebenen Bedeutungen haben,
mit α-Halogencarbonsäureestern der Formel (XXXIV-B) 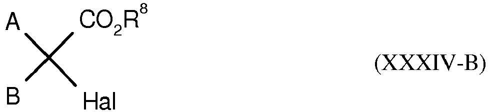 in welcher
- A, B und R⁸: die oben angegebenen Bedeutungen haben und
- Hal: für Chlor oder Brom steht,
alkyliert.

Die Verbindungen der Formel (XXXIV-A) sind teilweise käuflich oder aus den eingangs erwähnten Offenbarungen bekannt.

Die Verbindungen der Formel (XXXIV-B) sind käuflich.

Die Verbindungen der Formel (XXXI) sind neu, ausgenommen die Verbindung der Formel

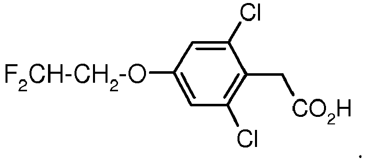

Beispielsweise erhält man die Verbindungen der Formel (XXXI), 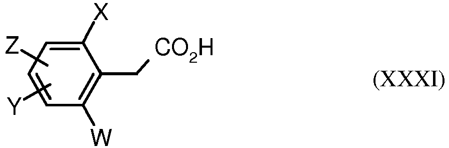 in welcher
W, X, Y und Z die oben angegebenen Bedeutungen haben,
wenn man Phenylessigsäureester der Formel (XXXV) 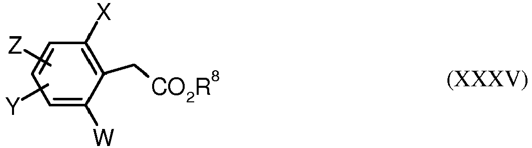 in welcher
W, X, Y, Z und R⁸ die oben angegebene Bedeutung haben,
in Gegenwart von Säuren oder Basen, in Gegenwart eines Lösungsmittels unter allgemein bekannten Standardbedingungen verseift.

Die Verbindungen der Formel (XXXV) sind neu.

Die Verbindungen der Formel (XXXV) 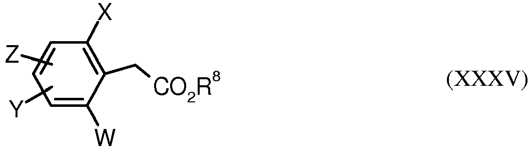 in welcher
W, X, Y, Z und R⁸ die oben angegebene Bedeutung haben,
erhält man nach dem in den Beispielen beschriebenen Verfahren analog dem Verfahren (S), wenn man Phenylessigsäureester der Formel (XXXV-a) 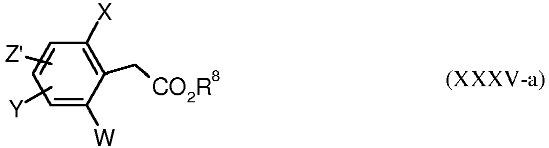 in welcher
R⁸, W, X und Y die oben angegebene Bedeutung haben
und Z' für Brom oder Iod steht,
in Gegenwart von Halogenalkylalkoholen z.B. Trifluorethanol in Gegenwart einer Base und gegebenenfalls in Gegenwart eines Kupfersalzes (bevorzugt Cu-I-J) umsetzt.

Die Phenylessigsäureester der Formel (XXXV-a) sind prinzipiell beispielsweise aus den Offenlegungen WO 96/35 664, WO 97/02243, WO 97/01535, WO 98/05638 und DE-A-10 301 804 bekannt und lassen sich nach den dort beschriebenen Verfahren herstellen.

Die bei dem obigen Verfahren (C) als Ausgangsstoffe benötigten Verbindungen der Formel (IV) 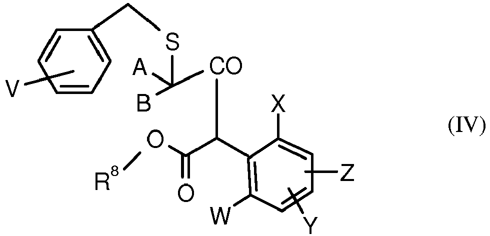 in welcher
A, B, V, W, X, Y, Z und R⁸ die oben angegebenen Bedeutungen haben,
sind neu.

Sie lassen sich nach im Prinzip bekannten Methoden herstellen.

Man erhält die Verbindungen der Formel (IV) beispielsweise, wenn man substituierte Phenylessigsäureester der Formel (XXXV) 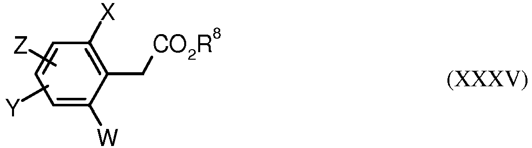 in welcher
- W, X, Y, Z und R⁸: die oben angegebenen Bedeutungen haben,
mit 2-Benzylthio-carbonsäurehalogeniden der Formel (XXXVI) 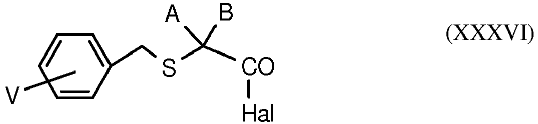 in welcher
- A, B und V: die oben angegebenen Bedeutungen haben und
- Hal: für Halogen (insbesondere Chlor oder Brom) steht,

in Gegenwart von starken Basen acyliert (siehe z.B. M.S. Chambers, E.J. Thomas, D.J. Williams, J. Chem. Soc. Chem. Commun., (1987), 1228).

Die Benzylthio-carbonsäurehalogenide der Formel (XXXVI) sind teilweise bekannt und/oder lassen sich nach bekannten Verfahren herstellen (J. Antibiotics (1983), 26, 1589).

Die bei den obigen Verfahren (D), (E) und (H-α) als Ausgangsstoffe benötigten Halogencarbonylketene der Formel (VI) sind neu. Sie lassen sich nach im Prinzip bekannten Methoden herstellen (vgl. beispielsweise Org. Prep. Proced. Int., 7, (4), 155-158, 1975 und DE 1 945 703). So erhält man z.B. die Verbindungen der Formel (VI) 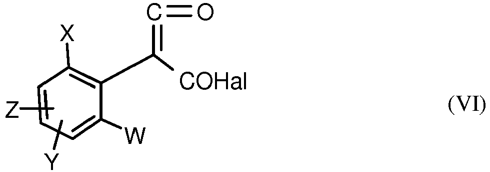 in welcher
W, X, Y und Z die oben angegebenen Bedeutungen haben und
Hal für Chlor oder Brom steht,
wenn man
substituierte Phenylmalonsäuren der Formel (XXXVII) 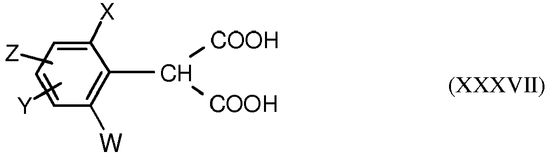 in welcher
W, X, Y und Z die oben angegebenen Bedeutungen haben,
mit Säurehalogeniden, wie beispielsweise Thionylchlorid, Phosphor(V)chlorid, Phosphor(III)-chlorid, Oxalylchlorid, Phosgen oder Thionylbromid gegebenenfalls in Gegenwart von Katalysatoren, wie beispielsweise Dimethylformamid, Methyl-Stearylformamid oder Triphenylphosphin und gegebenenfalls in Gegenwart von Basen wie z.B. Pyridin oder Triethylamin, umsetzt.

Die substituierten Phenylmalonsäuren der Formel (XXXVII) sind neu. Sie lassen sich in einfacher Weise nach bekannten Verfahren herstellen (vgl. z.B. Organikum, VEB Deutscher Verlag der Wissenschaften, Berlin 1977, S. 517 ff, EP-A-528 156, WO 96/35 664, WO 97/02 243, WO 97/01535, WO 97/36868 und WO 98/05638).

So erhält man Phenylmalonsäuren der Formel (XXXVII) 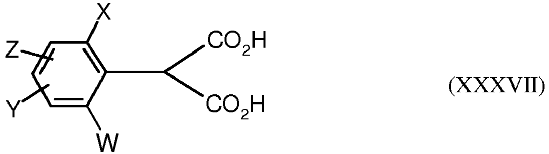 in welcher
W, X, Y und Z die oben angegebenen Bedeutungen haben,
wenn man Phenylmalonsäureester der Formel (XI) 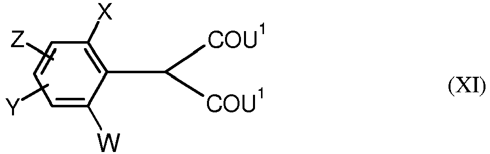 in welcher
W, X, Y und Z die oben angegebene Bedeutung haben,
und U¹ für OR⁸ steht, wobei R⁸ die oben angegebene Bedeutung hat,
zunächst in Gegenwart einer Base und einem Lösungsmittel verseift und anschließend vorsichtig ansäuert (s. beispielsweise EP-A-528 156, WO 96/35 664, WO 97/02 243).

Die Malonsäureester der Formel (XI) 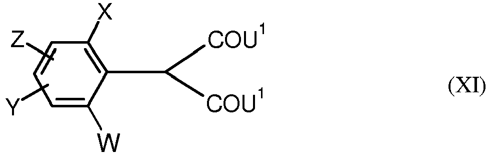 in welcher
W, X, Y und Z die oben angegebene Bedeutung haben,
und U¹ für OR⁸ steht,
wobei R⁸ die oben angegebene Bedeutung hat
sind neu.

Sie lassen sich nach allgemein bekannten Methoden der Organischen Chemie darstellen (vgl. z.B. Tetrahedron Lett. 27, 2763 (1986), Organikum VEB Deutscher Verlag der Wissenschaften, Berlin 1977, S. 587 ff., WO 96/35664, WO 97/02243, WO 97/01535, WO 97/36868, WO 98/05638 und WO 99/47525).

Die für das erfindungsgemäße Verfahren (D) als Ausgangsstoffe benötigten Carbonylverbindungen der Formel (V) 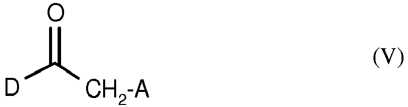 in welcher
- A und D: die oben angegebenen Bedeutungen haben,
oder deren Silylenolether der Formel (Va) 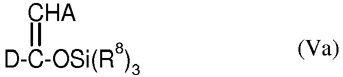 in welcher
- A, D und R⁸: die oben angegebenen Bedeutungen haben,
sind käufliche, allgemeine bekannte oder nach bekannten Verfahren zugängliche Verbindungen.

Die prinzipielle Herstellung der zur Durchführung des erfindungsgemäßen Verfahrens (E) als Ausgangsstoffe benötigten Ketensäurechloride der Formel (VI) wurden bereits im Zusammenhang bei dem Verfahren D beschrieben. Die zur Durchführung des erfindungsgemäßen Verfahrens (E) benötigten Thioamide der Formel (VII) 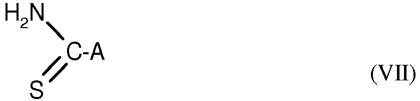 in welcher
- A: die oben angegebene Bedeutung hat,
sind allgemein in der Organischen Chemie bekannte Verbindungen.

Die bei dem obigen Verfahren (F) als Ausgangsstoffe benötigten Verbindungen der Formel (VIII) 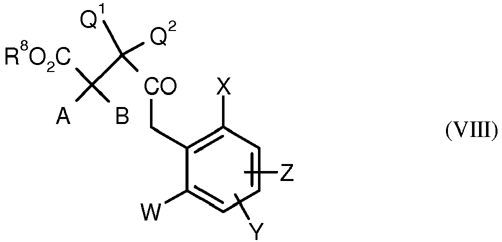 in welcher
A, B, Q¹, Q², W, X, Y, Z und R⁸ die oben angegebene Bedeutung haben,
sind neu.

Sie lassen sich nach im Prinzip bekannten Methoden herstellen.

Man erhält die 5-Aryl-4-ketocarbonsäureester der Formel (VIII) beispielsweise, wenn man 5-Aryl-4-ketocarbonsäuren der Formel (XXXVIII) 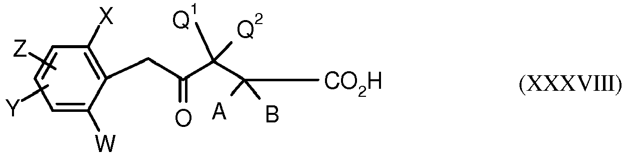 in welcher
- W, X, Y, Z, A, B, Q¹ und Q²: die oben angegebene Bedeutung haben,
verestert (vgl. z.B. Organikum, 15. Auflage, Berlin, 1977, Seite 499) oder alkyliert (siehe Herstellungsbeispiel).

Die 5-Aryl-4-ketocarbonsäuren der Formel (XXXVIII) 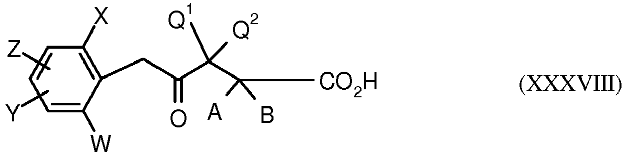 in welcher
A, B, Q¹, Q², W, X, Y und Z die oben angegebene Bedeutung haben,
sind neu, lassen sich aber nach im Prinzip bekannten Methoden herstellen (WO 96/01 798, WO 97/14667, WO 98/39281).

Man erhält die 5-Aryl-4-ketocarbonsäuren der Formel (XXXVIII) beispielsweise, wenn man 2-Phenyl-3-oxo-adipinsäureester der Formel (XXXIX) 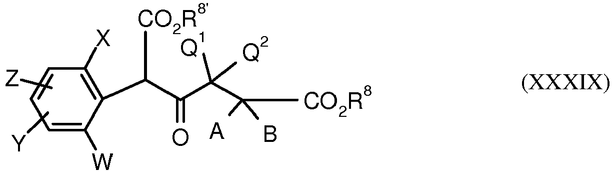 in welcher
A, B, Q¹, Q², W, X, Y und Z die oben angegebene Bedeutung haben und
R⁸ und R^{8'} für Alkyl (insbesondere C₁-C₈-Alkyl) stehen und bei Einsatz der Verbindung der Formel (XLI-a) R⁸ für Wasserstoff steht,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521, WO 96/01798, WO 97/14667, WO 98/39281).

Die Verbindungen der Formel (XXXIX) 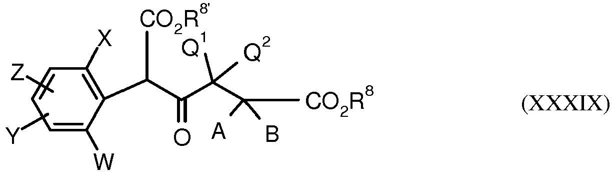 in welcher
A, B, Q¹, Q², W, X, Y, Z, R⁸ und R^{8'} die oben angegebene Bedeutung haben und bei Einsatz der Verbindung der Formel (XLI-a) R⁸ für Wasserstoff steht
sind neu.

Man erhält die Verbindungen der Formel (XXXIX) beispielsweise,
wenn man Dicarbonsäurehalbesterchloride der Formel (XL), 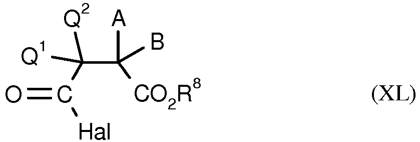 in welcher
- A, B, Q¹, Q² und R⁸: die oben angegebene Bedeutung haben und
- Hal: für Chlor oder Brom steht,
oder Carbonsäureanhydride der Formel (XLI-a) 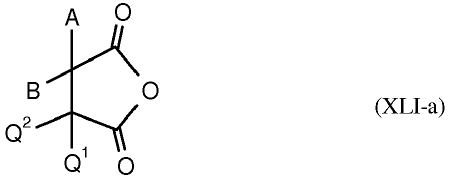 in welcher
A, B, Q¹ und Q² die oben angegebene Bedeutung haben,
mit einem Phenylessigsäureester der Formel (XXXV) 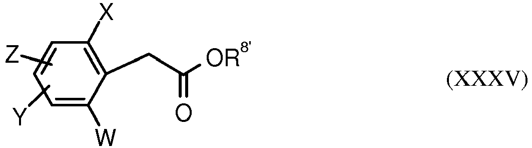 in welcher
W, X, Y, Z und R^{8'} die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XL) und (XLI-a) sind teilweise bekannte Verbindungen der Organischen Chemie und/oder lassen sich nach im Prinzip bekannten Methoden in einfacher Weise herstellen.

Die bei dem obigen Verfahren (G) als Ausgangsstoffe benötigten Verbindungen der Formel (IX) 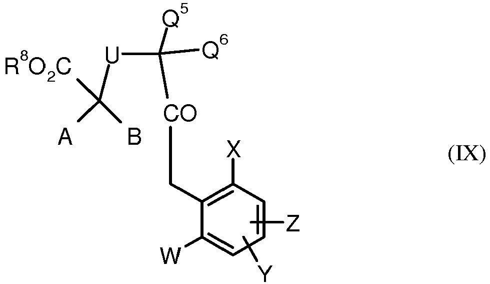 in welcher
A, B, Q⁵, Q⁶, U, W, X, Y, Z und R⁸ die oben angegebene Bedeutung haben,
sind neu.

Sie lassen sich nach im Prinzip bekannten Methoden herstellen.

Man erhält die 6-Aryl-5-ketocarbonsäureester der Formel (IX) beispielsweise, wenn man 6-Aryl-5-ketocarbonsäuren der Formel (XLII) 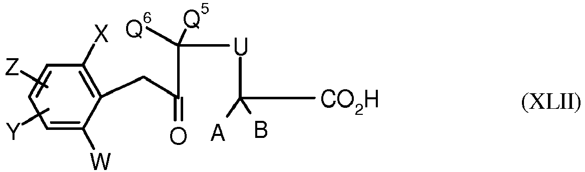 in welcher
A, B, Q⁵, Q⁶, U, W, X, Y und Z die oben angegebene Bedeutung haben,
verestert, (vgl. z.B. Organikum, 15. Auflage, Berlin, 1977, Seite 499, WO 99/43649, WO 99/48869).

Die 6-Aryl-5-ketocarbonsäuren der Formel (XLII) 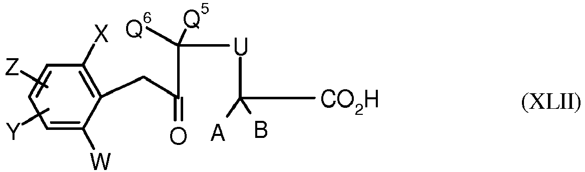 in welcher
A, B, Q⁵, Q⁶, U, W, X, Y und Z die oben angegebene Bedeutung haben,
sind neu. Sie lassen sich nach im Prinzip bekannten Methoden herstellen (WO 99/43649, WO 99/48869), beispielsweise wenn man
substituierte 2-Phenyl-3-oxo-heptandisäureester der Formel (XLIII) 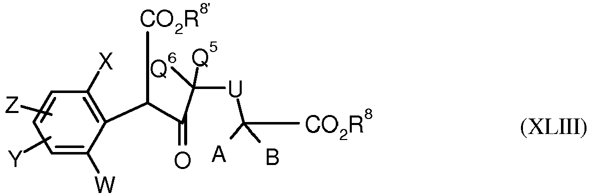 in welcher
A, B, Q⁵, Q⁶, U, W, X und Z die oben angegebene Bedeutung haben und
R⁸ und R^{8'} für Alkyl (bevorzugt C₁-C₆-Alkyl), stehen, und
bei Einsatz der Verbindung der Formel (XLI-b) R⁸ für Wasserstoff steht,
gegebenenfalls in Gegenwart eines Verdünnungsmittels und gegebenenfalls in Gegenwart einer Base oder Säure verseift und decarboxyliert (vgl. z.B. Organikum, 15. Auflage, Berlin, 1977, Seite 519 bis 521, WO 99/43649, WO 99/48869).

Die Verbindungen der Formel (XLIII) 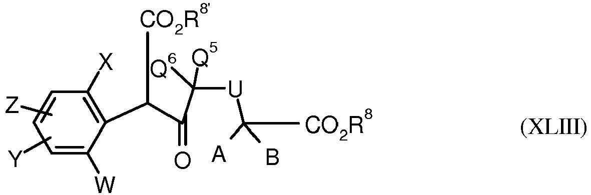 in welcher
A, B, Q⁵, Q⁶, U, W, X, Y, Z, R⁸ und R^{8'} die oben angegebene Bedeutung haben,
sind neu und erhältlich,
wenn man Dicarbonsäureester der Formel (XLIV), 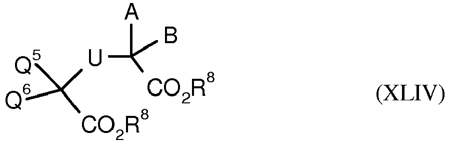 in welcher
A, B, Q⁵, Q⁶, U und R⁸ die oben angegebene Bedeutung haben,
oder Carbonsäureanhydride der Formel (XLI-b) 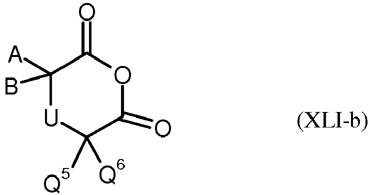 in welcher A, B, Q⁵, Q⁶ und U die oben angegebene Bedeutung haben mit einem substituierten Phenylessigsäureester der Formel (XXXV) 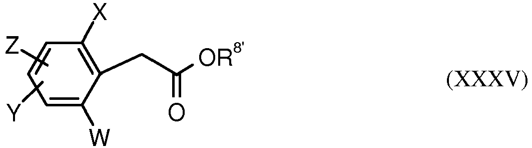 in welcher
W, X, Y, Z und R^{8'} die oben angegebene Bedeutung haben,
in Gegenwart eines Verdünnungsmittels und in Gegenwart einer Base kondensiert.

Die Verbindungen der Formel (XLIV) sind teilweise bekannt und/oder lassen sich nach bekannten Verfahren darstellen.

Die für das erfindungsgemäße Verfahren (H-α) und (H-ß) als Ausgangsstoffe benötigten Hydrazine der Formel (X)

A-NH-NH-D (X)

in welcher
A und D die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Ferri, Seite 212, 513; Georg Thieme Verlag Stuttgart, 1978; Liebigs Ann. Chem. 443, 242 (1925); Chem. Ber. 98, 2551 (1965), EP-A-508 126, WO 92/16510, WO 99/47 525, WO 01/17 972).

Die für das erfindungsgemäße Verfahren (H-γ) benötigten Verbindungen der Formel (XII) 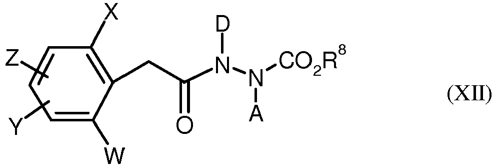 in welcher
A, D, W, X, Y, Z und R⁸ die oben angegebene Bedeutung haben,
sind neu.

Man erhält die Acylcarbazate der Formel (XII) beispielsweise, wenn man Carbazate der Formel (XLV) 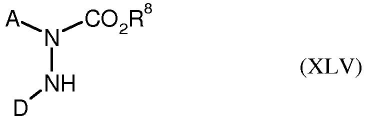 in welcher
A, R⁸ und D die oben angegebenen Bedeutungen haben,
mit substituierten Phenylessigsäurederivaten der Formel (XXVIII) 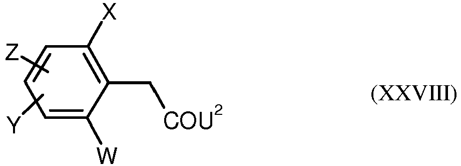 in welcher
W, X, Y, Z, und U² die oben angegebenen Bedeutungen haben
acyliert (Chem. Reviews 52, 237-416 (1953); Bhattacharya, Indian J. Chem. 6, 341-5, 1968).

Die Carbazate der Formel (XLV) sind teilweise käufliche und teilweise bekannte Verbindungen oder lassen sich nach im Prinzip bekannten Verfahren der organischen Chemie herstellen.

Die Verbindungen der Formel (XXVIII) wurden bereits bei den Vorstufen für das Verfahren (A) und (B) beschrieben.

Die beim erfindungsgemäßen Verfahren (I) als Ausgangsstoffe benötigten Verbindungen der Formel (XIII) 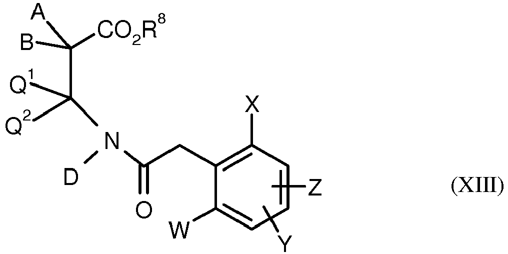 in welcher
A, B, D, Q¹, Q², W, X, Y, Z und R⁸ die oben angegebenen Bedeutungen haben, sind neu.

Man erhält die Acylaminosäureester der Formel (XIII) beispielsweise, wenn man Aminosäurederivate der Formel (XLVI) 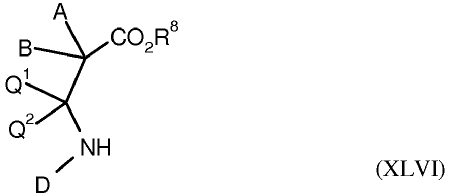 in welcher
A, B, Q¹, Q², R⁸ und D die oben angegebenen Bedeutungen haben,
mit substituierten Hetarylessigsäurederivaten der Formel (XXVIII) 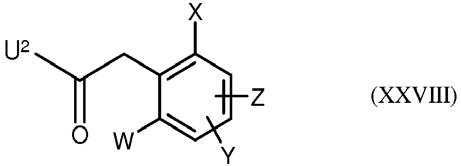 in welcher
W, X, Y, Z und U² die oben angegebene Bedeutung haben
acyliert (Chem. Reviews 52, 237-416 (1953); Bhattacharya, Indian J. Chem. 6, 341-5, 1968) oder wenn man Acylaminosäuren der Formel (XLVII) 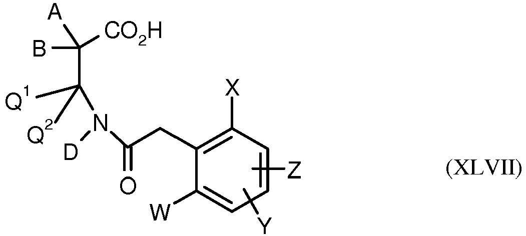 in welcher
A, B, D, Q¹, Q², W, X, Y und Z die oben angegebenen Bedeutungen haben,
verestert (Chem. Ind. (London) 1568 (1968)).

Die Verbindungen der Formel (XLVII) 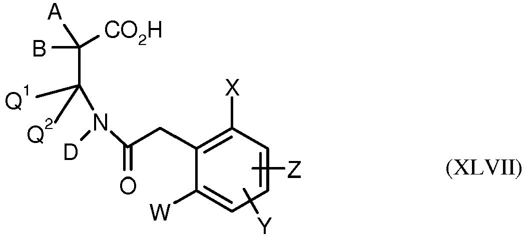 in welcher
A, B, D, Q¹, Q², W, X, Y und Z die oben angegebenen Bedeutungen haben, sind neu.

Man erhält die Verbindungen der Formel (XLVII), wenn man β-Aminosäuren der Formel (XLVIII) 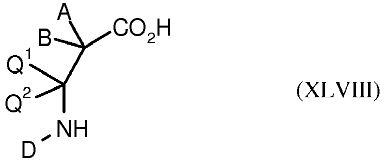 in welcher
A, B, Q¹, Q² und D die oben angegebenen Bedeutungen haben,
mit substituierten Phenylessigsäurederivaten der Formel (XXVIII) 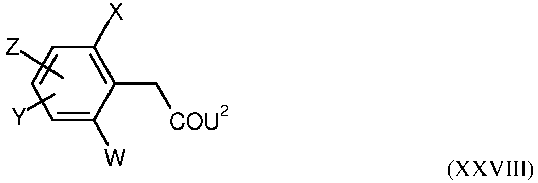 in welcher
W, X, Y, Z und U² die oben angegebenen Bedeutungen haben
beispielsweise nach Schotten-Baumann acyliert (Organikum, VEB Deutscher Verlag der Wissenschaften, Berlin 1977, S. 505).

Die Verbindungen der Formel (XLVI) und (XLVIII) sind teilweise aus WO 01/79204 bekannt oder lassen sich nach dem dort genannten im Prinzip bekannten Verfahren herstellen.

Die beim erfindungsgemäßen Verfahren (J) als Ausgangsstoffe benötigten Verbindungen der Formel (XIV) 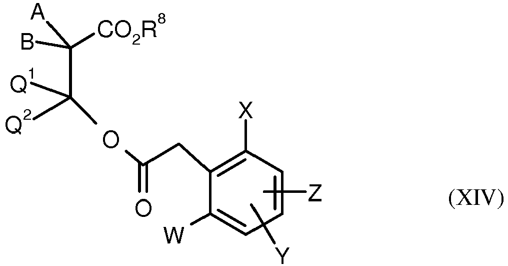 in welcher
A, B, Q¹, Q², W, X, Y, Z und R⁸ die oben angegebenen Bedeutungen haben,
sind neu.

Man erhält die Acylhydroxycarbonsäureester der Formel (XIV) beispielsweise, wenn man Hydroxycarbonsäureester der Formel (XLIX) 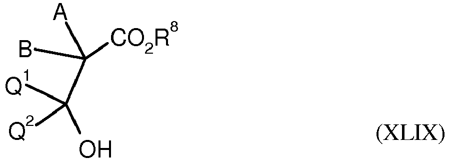 in welcher
A, B, Q¹, Q² und R⁸ die oben angegebenen Bedeutungen haben,
mit substituierten Phenylessigsäurederivaten der Formel (XXVIII) 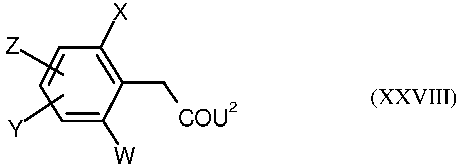 in welcher
W, X, Y, Z und U² die oben angegebenen Bedeutungen haben
acyliert (s. Herstellungsbeispiele der Formel (II)).

Die Verbindungen der Formel (XLIX) sind teilweise aus WO 01/98288 bekannt oder lassen sich nach im Prinzip bekannten Verfahren z.B. durch Reformatskij-Synthese herstellen (Organikum, VEB Deutscher Verlag der Wissenschaften, Berlin 1990, 18. Aufl., S. 501 ff.)

Die beim erfindungsgemäßen Verfahren (K) als Ausgangsstoffe benötigten Verbindungen der Formel (XV) 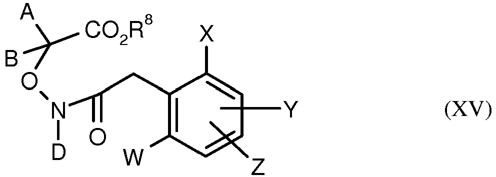 in welcher
A, B, D, W, X, Y, Z und R⁸ die oben angegebenen Bedeutungen haben,
sind neu.

Man erhält die Acylhydroxylaminosäureester der Formel (XV) beispielsweise, wenn man Aminosäurederivate der Formel (L) 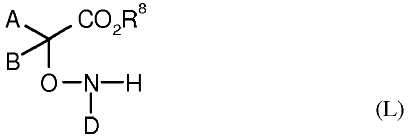 in welcher
A, B, R⁸ und D die oben angegebenen Bedeutungen haben,
mit substituierten Phenylessigsäurederivaten der Formel (XXVIII) 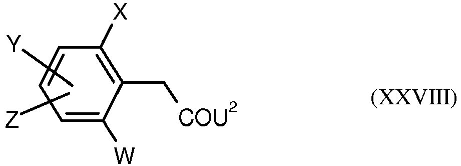 in welcher
W, X, Y, Z und U² die oben angegebenen Bedeutungen haben
acyliert (Chem. Reviews 52, 237-416 (1953); Bhattacharya, Indian J. Chem. 6, 341-5, 1968).

Die zur Herstellung von Verbindungen der Formel (II) als Ausgangsstoffe benötigten Hydroxylaminosäureester der Formel (L) 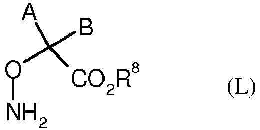 in welcher
- A, B, und R⁸: die oben angegebene Bedeutung haben sind teilweise neu und lassen sich nach bekannten Verfahren herstellen (N.A. Porter et. al. J. Org. Chem. 63 5547 (1998), WO 03/048138).

So erhält man beispielsweise Hydroxylaminosäureester der Formel (L) 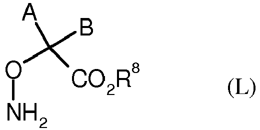 in welcher
A, B und R⁸ die oben angegebene Bedeutung haben, wenn man N-Hydroxyphthalimid der Formel (LI) 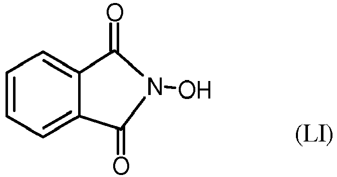 mit Halogenalkylester der Formel (LII) 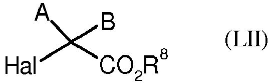 in welcher
A, B und R⁸ die oben angegebenen Bedeutung haben und
Hal für Chlor, Brom oder Jod, bevorzugt für Brom steht
   zu O-Alkoxyphthalimiden der Formel (LIII), 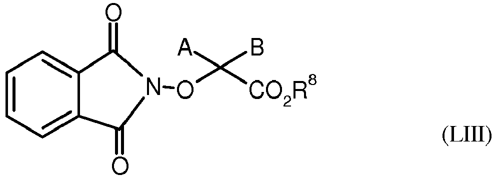
in welcher
A, B und R⁸ die oben angegebene Bedeutung haben,
umsetzt und daraus anschließend die Verbindungen der Formel (L) beispielsweise durch Hydrazinolyse freisetzt.

Die Verbindungen der Formeln (LII) und (LI) sind ebenfalls bekannt und lassen sich nach bekannten Verfahren herstellen (N.A. Porter et. al. J. Org. Chem. 63, 5547-5554, 1998).

Weiterhin erhält man beispielsweise Acylhydroxylaminosäureester der Formel (XV) 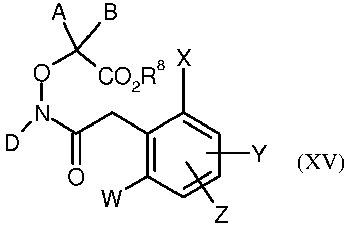 in welcher
A, B, D, W, X, Y, Z und R⁸ die oben angegebene Bedeutung haben
D jedoch bevorzugt ungleich Wasserstoff steht,
wenn man beispielsweise Phenylessigsäurederivate der Formel (XXVIII) 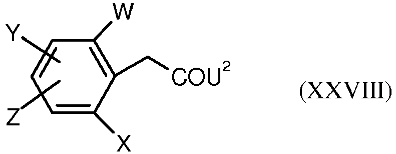 in welcher
W, X, Y, Z und U² die oben angegebene Bedeutung haben
mit Hydroxylaminen der Formel (LIV) 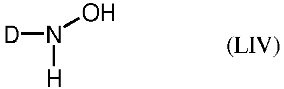 in welcher
- D: die oben angegebene Bedeutung hat, jedoch bevorzugt ungleich Wasserstoff steht
zu Verbindungen der Formel (LV) 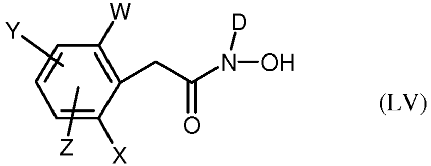 in welcher
D, W, X, Y und Z die oben angegebene Bedeutung haben,
umsetzt und diese mit Halogenalkylester der Formel (LII), 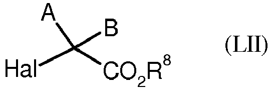 in welcher
- A, B und R⁸: die oben angegebene Bedeutung haben
und
- Hal: für Chlor, Brom und Jod, bevorzugt für Brom steht,
zu Verbindungen der Formel (XV) alkyliert (E.K. Ryo et. al., Bull. Korean Chem. Soc. 20 965 (1999)).

Die Verbindungen der Formel (LIV) sind teilweise käuflich, teilweise bekannt und lassen sich nach bekannten Verfahren herstellen.

Außerdem erhält man Verbindungen der Formel (XV), worin D ungleich Wasserstoff ist, wenn man Verbindungen der Formel (XV-a) 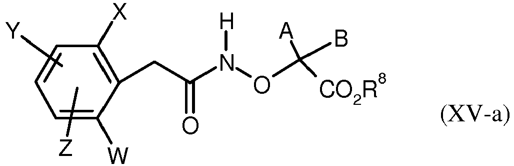 in welcher
A, B, W, X, Y, Z und R⁸ die oben angegebene Bedeutung haben
mit Verbindungen der Formel (LVI)

D-LG (LVI)

in welcher
- D: die oben angegebene Bedeutung hat, jedoch ungleich Wasserstoff steht
und
- LG: für eine Fluchtgruppe steht wie beispielsweise Chlor, Brom, Jod, Mesylat, Tosylat oder Triflat steht
zu Verbindungen der Formel (XV) alkyliert (WO 03/048138).

Die Verbindungen der Formel (LVI) sind teilweise käuflich, teilweise bekannt und nach bekannten Verfahren herstellbar.

Die Verbindungen der Formeln (LIII) und (LV) sind bekannt und entsprechend der eingangs zitierten Literatur herstellbar.

Die zur Durchführung der erfindungsgemäßen Verfahren (L), (M), (N), (O), (P), (Q), (R) und (S) außerdem als Ausgangsstoffe benötigten Säurehalogenide der Formel (XVI), Carbonsäureanhydride der Formel (XVII), Chlorameisensäureester oder Chlorameisensäurethioester der Formel (XVIII), Chlormonothioameisensäureester oder Chlordithioameisensäureester der Formel (XIX), Sulfonsäurechloride der Formel (XX), Phosphorverbindungen der Formel (XXI) und Metallhydroxide, Metallalkoxide oder Amine der Formel (XXII) und (XXIII) und Isocyanate der Formel (XXIV) und Carbamidsäurechloride der Formel (XXV) sowie Halogenalkanole der Formel (XXVI) sind allgemein bekannte Verbindungen der Organischen bzw. Anorganischen Chemie.

Die Verbindungen der Formeln (V), (VII), (X), (XXVII), (XXX), (XXXII), (XXXIV-A), (XXXIV-B), (XXXVI), (XL), (XLI-a), (XLI-b), (XLIV), (XLV), (XLVI), (XLVIII), (XLIX), (LI), (LII), (LIV) und (LVI) sind darüber hinaus aus den eingangs zitierten Patentanmeldungen bekannt und/oder lassen sich nach den dort angegebenen Methoden herstellen.

Die Verbindungen der Formeln (I-1' - I-11') lassen sich analog den beschriebenen Verfahren A bis R herstellen und sind teilweise neu. Die Verbindungen der Formel (I-1'-a) sind neu und lassen sich gemäß Verfahren A herstellen. Die zur Herstellung der Verbindungen der Formel (I-1'-a) benötigten Phenylessigsäuren der Formel (XXXI') 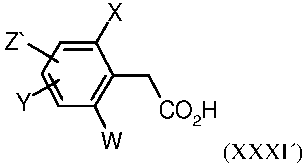 in welcher W, X, Y und Z' die oben angegebenen Bedeutungen haben sind teilweise neu. Neu sind Verbindungen der Formel (XXXI') in welcher Z' in der 3-Position steht und Y für Wasserstoff steht.

Das Verfahren (A) ist dadurch gekennzeichnet, dass man Verbindungen der Formel (II), in welcher A, B, D, W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass Verbindungen der Formel (III), in welcher A, B, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (B) wird im Allgemeinen unter Normaldruck durchgeführt.

Bei der Durchführung des erfindungsgemäßen Verfahrens (B) setzt man die Reaktionskomponenten der Formel (III) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (C) ist dadurch gekennzeichnet, dass man Verbindungen der Formel (IV) in welcher A, B, V, W, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (C) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (C) wird im Allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure als Lösungsmittel oder als Katalysator zu verwenden.

Das erfindungsgemäße Verfahren (D) ist dadurch gekennzeichnet, dass man Carbonylverbindungen der Formel (V) oder deren Enolether der Formel (V-a) mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (D) alle inerten organischen Solventien eingesetzt werden. Vorzugsweise verwendbar sind gegebenenfalls halogenierte Kohlenwasserstoffe, wie Toluol, Xylol, Mesitylen, Chlorbenzol und Dichlorbenzol, ferner Ether, wie Dibutylether, Glykoldimethylether Diglykoldimethylether und Diphenylether, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante (D) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante (D) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (D) wird zweckmäßigerweise unter Normaldruck durchgeführt.

Bei der Durchführung des erfindungsgemäßen Verfahrens (D) setzt man die Reaktionskomponenten der Formeln (V) und (VI), in welchen A, D, W, X, Y und Z die oben angegebenen Bedeutungen haben und Hal für Halogen steht, und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das erfindungsgemäße Verfahren (E) ist dadurch gekennzeichnet, dass man Thioamide der Formel (VII) mit Ketensäurehalogeniden der Formel (VI) in Gegenwart eines Verdünnungsmittels und gegebenenfalls in Gegenwart eines Säureakzeptors umsetzt.

Als Verdünnungsmittel können bei der erfindungsgemäßen Verfahrensvariante (E) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (E) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (E) wird zweckmäßigerweise unter Normaldruck durchgeführt.

Bei der Durchführung des erfindungsgemäßen Verfahrens (E) setzt man die Reaktionskomponenten der Formeln (VII) und (VI), in welchen A, W, X, Y und Z die oben angegebenen Bedeutungen haben und Hal für Halogen steht und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das Verfahren (F) ist dadurch gekennzeichnet, dass man Verbindungen der Formel (VIII), in welcher A, B, Q¹, Q², W, X, Y, Z und R⁸ die oben angegebene Bedeutung haben, in Gegenwart einer Base einer intramolekularen Kondensation unterwirft.

Als Verdünnungsmittel können bei dem erfindungsgemäßen Verfahren (F) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Collidin, Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (F) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (F) innerhalb eines größeren Bereiches variiert werden. Im Allgemeinen arbeitet man bei Temperaturen zwischen -75°C und 250°C, vorzugsweise zwischen -50°C und 150°C.

Das erfindungsgemäße Verfahren (F) wird im Allgemeinen unter Normaldruck durchgeführt.

Bei der Durchführung des erfindungsgemäßen Verfahrens (F) setzt man die Reaktionskomponenten der Formel (VIII) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (G) ist dadurch gekennzeichnet, dass man Verbindungen der Formel (IX), in welcher A, B, Q⁵, Q⁶, U, W, X, Y, Z und R⁸ die oben angegebene Bedeutung haben, in Gegenwart von Basen einer intramolekularen Kondensation unterwirft.

Als Verdünnungsmittel können bei dem erfindungsgemäßen Verfahren (G) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (G) alle üblichen Protonenakzeptoren eingesetzt werden.

Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (G) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (G) wird im Allgemeinen unter Normaldruck durchgeführt.

Bei der Durchführung des erfindungsgemäßen Verfahrens (G) setzt man die Reaktionskomponenten der Formel (IX) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das erfindungsgemäße Verfahren (H-α) ist dadurch gekennzeichnet, dass man Hydrazine der Formel (X) oder Salze dieser Verbindungen mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (H-α) alle inerten organischen Solventien eingesetzt werden. Vorzugsweise verwendbar sind gegebenenfalls chlorierte Kohlenwasserstoffe, wie beispielsweise Mesitylen, Chlorbenzol und Dichlorbenzol, Toluol, Xylol, ferner Ether, wie Dibutylether, Glykoldimethylether, Diglykoldimethylether und Diphenylethan, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante (H-α) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante (H-α) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (H-α) wird zweckmäßigerweise unter Normaldruck durchgeführt.

Bei der Durchführung des erfindungsgemäßen Verfahrens (H-α) setzt man die Reaktionskomponenten der Formeln (VI) und (X), in welchen A, D, W, X, Y und Z die oben angegebenen Bedeutungen haben und Hal für Halogen steht, und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das Verfahren (H-ß) ist dadurch gekennzeichnet, dass man Hydrazine der Formel (X) oder Salze dieser Verbindung, in welcher A und D die oben angegebenen Bedeutungen haben, mit Malonestern oder Malonsäureamiden der Formel (XI), in welcher U¹, W, X, Y, Z und R⁸ die oben angegebene Bedeutung haben, in Gegenwart einer Base einer Kondensation unterwirft.

Als Verdünnungsmittel können bei dem erfindungsgemäßen Verfahren (H-ß) alle inerten organischen Solventien eingesetzt werden. Vorzugsweise verwendbar sind gegebenenfalls halogenierte Kohlenwasserstoffe, wie Toluol, Xylol, Mesitylen, Chlorbenzol und Dichlorbenzol, ferner Ether, wie Dibutylether, Tetrahydrofuran, Dioxan, Diphenylether, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-ß) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Verwendbar sind auch tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (H-ß) innerhalb eines größeren Bereiches variiert werden. Im Allgemeinen arbeitet man bei Temperaturen zwischen 0°C und 280°C, vorzugsweise zwischen 50°C und 180°C.

Das erfindungsgemäße Verfahren (H-ß) wird im Allgemeinen unter Normaldruck durchgeführt.

Bei der Durchführung des erfindungsgemäßen Verfahrens (H-ß) setzt man die Reaktionskomponenten der Formeln (XI) und (X) im Allgemeinen in etwa äquimolaren Mengen ein. Es ist jedoch auch möglich, die eine oder andere Komponente in einem größeren Überschuss (bis zu 3 mol) zu verwenden.

Das Verfahren (H-γ) ist dadurch gekennzeichnet, dass man Verbindungen der Formel (XII), in welcher A, D, W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (H-γ) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-γ) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (H-γ) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (H-γ) wird im Allgemeinen unter Normaldruck durchgeführt.

Bei der Durchführung des erfindungsgemäßen Verfahrens (H-γ) setzt man die Reaktionskomponenten der Formel (XII) und die deprotonierenden Basen im Allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (I) ist dadurch gekennzeichnet, dass man Verbindungen der Formel (XIII), in welcher A, B, D, Q¹, Q², W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (I) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (I) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (I) innerhalb eines größeren Bereiches variiert werden. Im allgemeinen arbeitet man bei Temperaturen zwischen -80°C und 180°C, vorzugsweise zwischen -50°C und 120°C.

Das erfindungsgemäße Verfahren (I) wird im allgemeinen unter Normaldruck durchgeführt.

Bei der Durchführung des erfindungsgemäßen Verfahrens (I) setzt man die Reaktionskomponenten der Formel (XIII) und die deprotonierenden Basen im allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (J) ist dadurch gekennzeichnet, dass man Verbindungen der Formel (XIV), in welcher A, B, Q¹, Q², W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (J) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, iso-Propanol, Butanol, iso-Butanol und tert-Butanol.

Als Base (Deprotonierungsmittel) können bei der Durchführung des erfindungsgemäßen Verfahrens (J) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert-butylat einsetzbar.

Die Reaktionstemperaturen können bei der Durchführung des erfindungsgemäßen Verfahrens (J) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (J) wird im allgemeinen unter Normaldruck durchgeführt.

Bei der Durchführung des erfindungsgemäßen Verfahrens (J) setzt man die Reaktionskomponenten der Formel (XIV) und die deprotonierenden Basen im allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (K) ist dadurch gekennzeichnet, dass man Verbindungen der Formel (XV), in welcher A, B, D, W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (K) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (K) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (K) innerhalb eines größeren Bereiches variiert werden. Im Allgemeinen arbeitet man bei Temperaturen zwischen -78°C und 250°C, vorzugsweise zwischen 0°C und 150°C.

Das erfindungsgemäße Verfahren (K) wird im Allgemeinen unter Normaldruck durchgeführt.

Bei der Durchführung des erfindungsgemäßen Verfahrens (K) setzt man die Reaktionskomponenten der Formel (XV) und die deprotonierenden Basen im Allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (L-α) ist dadurch gekennzeichnet, dass man Verbindungen der Formeln (I-1-a) bis (I-11-a) jeweils mit Carbonsäurehalogeniden der Formel (XVI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (L-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (L-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (L-α) innerhalb eines größeren Bereiches variiert werden. Im Allgemeinen arbeitet man bei Temperaturen zwischen-20°C und +150°C, vorzugsweise zwischen 0°C und 100°C.

Bei der Durchführung des erfindungsgemäßen Verfahrens (L-α) werden die Ausgangsstoffe der Formeln (I-1-a) bis (I-11-a) und das Carbonsäurehalogenid der Formel (XVI) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (L-ß) ist dadurch gekennzeichnet, dass man Verbindungen der Formeln (I-1-a) bis (I-11-a) mit Carbonsäureanhydriden der Formel (XVII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (L-ß) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (L-ß) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (L-ß) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (L-ß) werden die Ausgangsstoffe der Formeln (I-1-a) bis (I-11-a) und das Carbonsäureanhydrid der Formel (XVII) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (M) ist dadurch gekennzeichnet, dass man Verbindungen der Formeln (I-1-a) bis (I-11-a) jeweils mit Chlorameisensäureestern oder Chlorameisensäurethiolestern der Formel (XVIII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (M) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (M) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (M) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (M) wird im Allgemeinen unter Normaldruck durchgeführt.

Bei der Durchführung des erfindungsgemäßen Verfahrens (M) werden die Ausgangsstoffe der Formeln (I-1-a) bis (I-11-a) und der entsprechende Chlorameisensäureester bzw. Chlorameisensäurethiolester der Formel (XVIII) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (N) ist dadurch gekennzeichnet, dass man Verbindungen der Formeln (I-1-a) bis (I-11-a) jeweils mit Verbindungen der Formel (XIX) in Gegenwart eines Verdünnungsmittels und gegebenenfalls in Gegenwart eines Säurebindemittels umsetzt.

Beim Herstellungsverfahren (N) setzt man pro Mol Ausgangsverbindung der Formeln (I-1-a) bis (I-11-a) ca. 1 Mol Chlormonothioameisensäureester bzw. Chlordithioameisensäureester der Formel (XIX) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-11-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (O) ist dadurch gekennzeichnet, dass man Verbindungen der Formeln (I-1-a) bis (I-11-a) jeweils mit Sulfonsäurechloriden der Formel (XX) gegebenenfalls in Gegenwart eines Verdünnungsmittels und gegebenenfalls in Gegenwart eines Säurebindemittels umsetzt.

Beim Herstellungsverfahren (O) setzt man pro Mol Ausgangsverbindung der Formel (I-1-a bis I-11-a) ca. 1 Mol Sulfonsäurechlorid der Formel (XX) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-11-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (P) ist dadurch gekennzeichnet, dass man Verbindungen der Formeln (I-1-a) bis (I-11-a) jeweils mit Phosphorverbindungen der Formel (XXI) gegebenenfalls in Gegenwart eines Verdünnungsmittels und gegebenenfalls in Gegenwart eines Säurebindemittels umsetzt.

Beim Herstellungsverfahren (P) setzt man zum Erhalt von Verbindungen der Formeln (I-1-e) bis (I-11-e) auf 1 Mol der Verbindungen (I-1-a) bis (I-11-a), 1 bis 2, vorzugsweise 1 bis 1,3 Mol der Phosphorverbindung der Formel (XXI) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (Q) ist dadurch gekennzeichnet, dass man Verbindungen der Formeln (I-1-a) bis (I-11-a) mit Metallhydroxiden bzw. Metallalkoxiden der Formel (XXII) oder Aminen der Formel (XXII), gegebenenfalls in Gegenwart eines Verdünnungsmittels, umsetzt.

Als Verdünnungsmittel können bei dem erfindungsgemäßen Verfahren (Q) vorzugsweise Ether wie Tetrahydrofuran, Dioxan, Diethylether oder aber Alkohole wie Methanol, Ethanol, Iso-propanol, aber auch Wasser eingesetzt werden.

Das erfindungsgemäße Verfahren (Q) wird im Allgemeinen unter Normaldruck durchgeführt.

Die Reaktionstemperaturen liegen im Allgemeinen zwischen -20°C und 100°C, vorzugsweise zwischen 0°C und 50°C.

Das erfindungsgemäße Verfahren (R) ist dadurch gekennzeichnet, dass man Verbindungen der Formeln (I-1-a) bis (I-11-a) jeweils mit (R-α) Verbindungen der Formel (XXIV) gegebenenfalls in Gegenwart eines Verdünnungsmittels und gegebenenfalls in Gegenwart eines Katalysators oder (R-ß) mit Verbindungen der Formel (XXV) gegebenenfalls in Gegenwart eines Verdünnungsmittels und gegebenenfalls in Gegenwart eines Säurebindemittels umsetzt.

Bei Herstellungsverfahren (R-α) setzt man pro Mol Ausgangsverbindung der Formeln (I-1-a) bis (I-11-a) ca. 1 Mol Isocyanat der Formel (XXIV) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (R-ß) setzt man pro Mol Ausgangsverbindung der Formeln (I-1-a) bis (I-11-a) ca. 1 Mol Carbamidsäurechlorid der Formel (XXV) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-11-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das Verfahren (S) ist dadurch gekennzeichnet, dass man Verbindungen der Formeln (I-1') bis (I-1'), in welchen A, B, D, G, Q¹, Q², U, Q⁵, Q⁶, W, X und Y die oben angegebenen Bedeutungen haben und Z' bevorzugt für Brom oder Jod steht, mit Alkoholen der Formel ZOH, in welcher Z die oben angegebene Bedeutung hat, in Gegenwart einer Base und eines Cu-(I)-Salzes (z.B. CuBr oder CuJ) umsetzt.

Als Verdünnungsmittel können bei dem erfindungsgemäßen Verfahren (S) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Collidin, Dimethylsulfoxid, Sulfolan, Dimethylformamid, Dimethylacetamid und N-Methyl-pyrrolidon, Ester wie Methylacetat, Ethylacetat, Propylacetat sowie Alkohole der Formel WOH wie z.B. Methanol, Ethanol, Propanol, Iso-Propanol, Butanol und Iso-Butanol.

Als Base (Deprotonierungsmittel) können bei der Durchführung des erfindungsgemäßen Verfahrens (S) alle üblichen Protonenakzeptoren eingesetzt werden. Vorzugsweise verwendbar sind Alkalimetalle wie Natrium oder Kalium. Ferner sind Alkalimetall- und Erdalkalimetallamide und -hydride, wie Natriumamid, Natriumhydrid und Calciumhydrid, und bevorzugt auch Alkalimetallalkoholate, wie Natriummethylat, Natriumethylat, Natriumisopropylat, Natrium-tert.-butylat und Kalium-tert.-butylat einsetzbar.

Die Reaktionstemperatur kann bei der Durchführung des erfindungsgemäßen Verfahrens (S) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (S) wird im Allgemeinen unter Normaldruck durchgeführt.

Bei der Durchführung des erfindungsgemäßen Verfahrens (S) setzt man die Reaktionskomponente der Formel (I-1') bis (I-11') im Allgemeinen mit Überschüssen der Alkohole ZOH und der Basen bis zu 20 Mol, bevorzugt 3 bis 5 Mol um. Die Kupfer-I-Salze werden in der Regel katalytisch eingesetzt; 0,001 bis 0,5 Mol, bevorzugt 0,01 bis 0,2 Mol. Es ist jedoch auch möglich diese äquimolar einzusetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus dem Stamm Mollusca z.B. aus der Klasse der Lamellibranchiata z.B. Dreissena spp.

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus dem Stamm Arthropoda z.B. aus der Ordnung der Isopoda z.B. Armadillidium vulgare, Oniscus asellus, Porcellio scaber.

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Centruroides spp., Chorioptes spp., Dermanyssus gallinae, Dermatophagoides pteronyssius, Dermatophagoides farinae, Dermacentor spp., Eotetranychus spp., Epitrimerus pyri, Eutetranychus spp., Eriophyes spp., Halotydeus destructor, Hemitarsonemus spp., Hyalomma spp., Ixodes spp., Latrodectus spp., Loxosceles spp., Metatetranychus spp., Nuphersa spp., Oligonychus spp., Ornithodorus spp., Ornithonyssus spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vaejovis spp., Vasates lycopersici.

Aus der Ordnung der Symphyla z.B. Scutigerella spp..

Aus der Ordnung der Chilopoda z.B. Geophilus spp., Scutigera spp..

Aus der Ordnung der Collembola z.B. Onychiurus armatus.

Aus der Ordnung der Diplopoda z.B. Blaniulus guttulatus.

Aus der Ordnung der Zygentoma z.B. Lepisma saccharina, Thermobia domestica.

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta spp., Pulex irritans, Schistocerca gregaria, Supella longipalpa.

Aus der Ordnung der Isoptera z.B. Coptotermes spp., Cornitermes cumulans, Cryptotermes spp., Incisitermes spp., Microtermes obesi, Odontotermes spp., Reticulitermes spp.,

Aus der Ordnung der Heteroptera z.B. Anasa tristis, Antestiopsis spp., Boisea spp., Blissus spp., Calocoris spp., Campylomma livida, Cavelerius spp., Cimex lectularius, Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Anoplura (Phthiraptera) z.B. Damalinia spp., Haematopinus spp., Linognathus spp., Pediculus spp., Ptirus pubis, Trichodectes spp..

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Coleoptera z.B. Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Prostephanus truncatus, Psylliodes spp., Ptinus spp., Rhizobius ventralis, Rhizopertha dominica, Sitophilus spp., Sphenophorus spp., Stegobium paniceum, Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Hymenoptera z.B. Acromyrmex spp., Athalia spp., Atta spp., Diprion spp., Hoplocampa spp., Lasius spp., Monomorium pharaonis, Solenopsis invicta, Tapinoma spp., Vespa spp..

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichophaga tapetzella, Trichoplusia spp., Tuta absoluta, Virachola spp..

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Chrysops spp., Cochliomyia spp., Contarinia spp., Cordylobia anthropophaga, Culex spp., Culicoides spp., Culiseta spp., Cuterebra spp., Dacus oleae, Dasyneura spp., Delia spp., Dermatobia hominis, Drosophila spp., Echinocnemus spp., Fannia spp., Gasterophilus spp., Glossina spp., Haematopota spp., Hydrellia spp., Hylemyia spp., Hyppobosca spp., Hypoderma spp., Liriomyza spp.. Lucilia spp., Lutzomia spp., Mansonia spp., Musca spp., Nezara spp., Oestrus spp., Oscinella frit, Pegomyia spp., Phlebotomus spp., Phorbia spp., Phormia spp., Prodiplosis spp., Psila rosae, Rhagoletis spp., Sarcophaga spp., Simulium spp, Stomoxys spp., Tabanus spp., Tannia spp., Tetanops spp., Tipula spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Siphonaptera z.B. Ceratophyllus spp., Ctenocephalides spp., Tunga penetrans, Xenopsylla cheopis.

Aus dem Stämmen der Plathelminthen und Nematoden als Tierparasiten z.B. aus der Klasse der Helminthen z.B. Ancylostoma duodenale, Ancylostoma ceylanicum, Acylostoma braziliensis, Ancylostoma spp., Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Aus dem Stamm der Nematoden als Pflanzenschädlinge z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Aus dem Subphylum der Protozoa z.B. Eimeria.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile, gemischt oder verbunden sind. Der feste oder flüssige Trägerstoff ist im Allgemeinen inert und sollte in der Landwirtschaft verwendbar sein.

### Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propeller Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Verbindungen der Formel (I) (Wirkstoffe) weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Mit den erfindungsgemäßen Wirkstoffen können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten, Nematoden oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen. Die Wirkstoffe können auch in transgenen Pflanzen, die sich durch höhere Erträge , z. B. durch eine verbesserte Photosyntheseleistung oder verbesserte Nährstoffaufnahme auszeichnen, eingesetzt werden.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant). Des weiteren wurden transgene Pflanzen beschrieben, die gegen synthetische Auxine (z. B 2,4 D) HRAC mode of action Class O und Aryloxy-phenoxy Propionate (fops, HRAC, Class A) resistent sind (DHT, Dow Agroscience Herbicide Tolerance Trait)
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- Gentechnisch veränderte Pflanzen, die neue Insektenresistenzen, z. B. basierend auf der Expression von Toxinen aus Photorhabdus, Xenorhabdus Symbionten aus entomopathogenen Nematoden und Toxinen aus Spinnen, Skorpionen, Ameisen, parasitischen Wespen aufweisen.
- gentechnisch veränderte Kulturpflanzen mit neuen Inhalts- oder Sekundärstoffen z. B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- Transgene Kulturpflanzen, die sich durch erhöhte Toleranzen gegen abiotische und biotische Stressoren auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetyl CoA Carboxylasen, Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der FOPs, Sulfonylharnstoffe, der Glyphosate, Glufosinate oder Benzoylisoxazole und analogen Wirkstoffe, oder gegen beliebige Kombinationen dieser Wirkstoffe, resistent sind.
- Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum™ GAT™ (Glyphosate ALS Tolerant) eingesetzt werden. Des weiteren und besonders bevorzugt können die erfindungsgemäßen Verbindungen in transgenen Pflanzen, die gegen synthetische Auxine (z. B 2,4 D) mit "HRAC mode of action Class O" und Aryloxy-phenoxy Propionate (fops) mit "HRAC mode of action Class A" resistent sind (z. B. DHT, Dow Agroscience Herbicide Tolerance Trait) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Das erfindungsgemäße Behandlungsverfahren wird vorzugsweise auf genetisch modifizierten Organismen, wie beispielsweise Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp*., die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere herbizidresistente Pflanzen sind Pflanzen, die gegenüber ACCase-Hemmern tolerant gemacht worden sind.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus*, das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfleld® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die Bezeichnung "Wirkstoffe" bzw. "Verbindungen" schließt immer auch die hier genannten Wirkstoffkombinationen mit ein.

### Herstellungsbeispiele:

### Beispiel (I-1-a-1):

Verfahren A

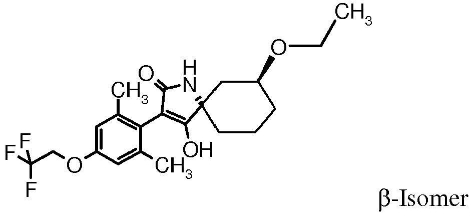

0.41 g (0.58 mmol) der Verbindung gemäß Beispiel II-1-a-1 werden in einer Lösung von 2 ml DMF zu einer Lösung von 5 ml DMF und 164 mg (2.5 eq) Kalium-t-butylat innerhalb von 30 min bei Raumtemperatur zugetropft und für 18 h bei dieser Temperatur gerührt. Man stellt den Ansatz mit 1N Salzsäure auf pH = 1 und filtriert den erhaltenen Rückstand ab. Nach säulenchromatographischer Reinigung (RP-Silicagel Gradient Acetonitril / Wasser) erhält man das erfindungsgemäße Produkt (I-1-a-1) = 200 mg (38 % d. Theorie).
¹H-NMR (400 MHz, d₆-DMSO): δ = 6.75 (s, 2H, Ar-H), 4.70 (q, 2H, CH₂-CF₃), 3.57 (m, 1H, CH-OCH₂), 3,45 (m, 2H, OCH₂CH₃), 2.08 (d, 3H, Ar CH₃), 1.98 (m), 1.70 (m), 1.29 (m, zusammen 8 H, Cyclohexyl), 1.10 (t, 3H, CH₃) ppm.

### Beispiel I-1-a-2:

Verfahren S

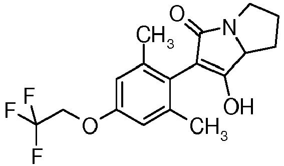

0.5 g (1.55 mmol) der Verbindung (I-1-a-1') (generisch bekannt aus WO 97/02243) werden mit 0.348 g (2 eq) Kalium-t-butylat versetzt und in 5ml DMA gelöst (Lösung 1). Weiterhin werden 0.296 g (1 eq) Kupfer-(I)-iodid und 1,35 g (6.7 eq) 2,2,2-Trifluorethanol in 5 ml DMA (Dimethylacetamid) unter Inertgas suspendiert und mit 1.17 g (6.7 eq) Kalium-t-butylat versetzt. Nach Beendigung der exothermen Reaktion wird mit Lösung 1 versetzt und unter Mikrowellenbestrahlung bei 145 °C für 2 h gerührt. Der Reaktionsansatz wird unter reduziertem Druck vom Lösungsmittel befreit, mit 200 ml Wasser versetzt, der zurückbleibende Rückstand wird abgetrennt und verworfen. Die wässrige Phase wird mit In Salzsäure auf pH 1 gestellt und und der entstandene Rückstand abfiltriert. Nach säulenchromatographischer Reinigung (RP-Silicagel, Gradient Wasser / Acetonitril) erhält man 0.13 g = 24 % d. Theorie an erfindungsgemäßer Verbindung I-1-a-2 mit einem Fp. von 202- 205 °C.

In Analogie zu Beispiel (I-1-a-1) und Beispiel (I-1-a-2) sowie gemäß den allgemeinen Angaben zur Herstellung erhält man folgende Verbindungen der Formel (I-1-a):

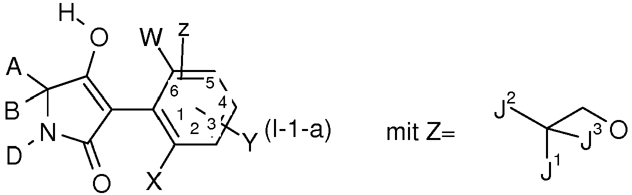

| Bsp-Nr. | W | X | Y | Z | J¹ | J² | J³ | D | A | B | Analytik | Isomer |
|---|---|---|---|---|---|---|---|---|---|---|---|---|
| I-1-a-3 | CH₃ | C₂H₅ | H | 4- | F | F | F | H | -(CH₂)₂-CH(OCH₃)-(CH₂)₂- | | 1H-NMR (400 MHz, CDCl₃): 4.34 (q, 2H, CH₂-CF₃), 3.23 (m, 1H, CH-OCH₃) | cis |
| I-1-a-4 | C₂H₅ | C₂H₅ | H | 4- | F | F | F | H | -(CH₂)₂-CH(OCH₃)-(CH₂)₂- | | 1H-NMR (400 MHz, CDCl₃): 4.47 (q, 2H, CH₂-CF₃), 3.29 (m, 1H, CH-OCH₃) | cis |
| I-1-a-5 | CH₃ | C₂H₅ | H | 4- | F | F | F | H | -CH₂-CH(OCH₂CH₃)-(CH₂)₃- | | 1H-NMR (400 MHz, CDCl₃): 4.32 (q, 2H, CH₂-CF₃), 3.86 (m, 1H, CH-OCH₂) | trans |
| I-1-a-6 | CH₃ | C₂H₅ | H | 4- | F | F | F | H | -(CH₂)₂-C(-OCH₂-CH₂O-)-(CH₂)₂- | | 1H-NMR (400 MHz, CDCl₃): 4.34 (q, 2H, CH₂-CF₃), 3.96 (m, 4H, OCH₂CH₂O) | |
| I-1-a-7 | CH₃ | C₂H₅ | H | 4- | F | F | F | H | -(CH₂)₂-C(-OCH₂-CH (CH₃) -O-)-CH₂)₂- | | 1H-NMR (400 MHz, CDCl₃): 4.33 (q, 2H, CH₂-CF₃), 1.27 (dd, 3H, OCHCH₃) | |
| I-1-a-8 | CH₃ | C₂H₅ | H | 4- | F | F | F | H | -(CH₂)₂-CH(CH₃)-(CH₂)₂- | | 1H-NMR (400 MHz, CDCl₃): 4.34 (q, 2H, CH₂-CF₃), 0.97 (d, 3H, CH-CH₃) | |
| I-1-a-9 | C₂H₅ | C₂H₅ | H | 4- | F | F | F | H | CH₃ | CH₃ | 1H-NMR (400 MHz, d₆-DMSO): 3.43 (m, 4H, CH₂-O und CH₂-CF₃) | |
| I-1-a-10 | CH₃ | C₂H₅ | H | 4- | F | F | F | H | -(CH₂)₂-C(-O-CH(CH₃) -CH₂-CH(CH₃) -O-)-(CH₂)₂- | | 1H-NMR (400 MHz, CDCl₃): 4.33 (q, 2H, CH₂-CF₃), 4.08 und 3.93 (je m, je 1H, OCHCH₃) | |
| I-1-a-11 | CH₃ | C₂H₅ | H | 4- | F | F | F | H | C₂H₅ | CH₃ | 1H-NMR (400 MHz, CDCl₃): 4.34 (q, 2H, CH₂-CF₃), 0.90 (t, 3H CH₂-CH₃) | |
| I-1-a-12 | CH₃ | C₂H₅ | H | 4- | F | F | F | H | -CH₂-CH(CH₂-OCH₃)-(CH₂)₃- | | 1H-NMR (400 MHz, CDCl₃): 4.34 (q, 2H, CH₂-CF₃), 3.26 (m, CH₂-O-CH₃) | trans:cis ca 4:1 |
| I-1-a-13 | CH₃ | C₂H₅ | H | 4- | F | F | F | H | -CH₂-CH(OCH₃)-(CH₂)₃- | | 1H-NMR (400 MHz, CDCl₃): 4.33 (q, 2H, CH₂-CF₃), 3.73 (m, CH-O-CH₃) | trans |
| I-1-a-14 | CH₃ | C₂H₅ | H | 4- | F | F | F | H | -CH₂-CH(OC₃H₇)-(CH₂)₃- | | 1H-NMR (400 MHz, CDCl₃): 4.34 (q, 2H, CH₂-CF₃), 3.86 (m, CH-O-CH₂) | trans |
| I-1-a-15 | CH₃ | C₂H₅ | H | 4- | F | F | F | H | -CH₂-CH(OC₄H₉)-(CH₂)₃- | | 1H-NMR (400 MHz, CDCl₃): 4.34 (q, 2H, CH₂-CF₃), 3.86 (m, CH-O-CH₂) | trans |
| I-1-a-16 | CH₃ | C₂H₅ | H | 4- | F | F | F | H | CH₃ | CH₃ | 1H-NMR (400 MHz, CDCl₃): 4.34 (q, 2H, CH₂-CF₃), 1.45 (d, 6H, C(CH₃)₂) | |
| I-1-a-17 | CH₃ | CH₃ | H | 4- | F | F | F | H | -(CH₂)₂-CH(CH₂-OCH₃)-(CH₂)₂- | | 1H-NMR (400 MHz, d₆-DMSO): 4.66 (q, 2H, CH₂-CF₃), 3.14 (d, CH₂-O-CH₃) | β |
| I-1-a-18 | CH₃ | CH₃ | H | 4- | F | F | F | H | -(CH₂)₂-CH(CH₃)-(CH₂)₂- | | 1H-NMR (400 MHz, d₆-DMSO): 4.68 (q, 2H, CH₂-CF₃), 0.91 (d, CH-CH₃) | β |
| I-1-a-19 | CH₃ | CH₃ | H | 4- | F | F | F | H | -CH₂-CH(OC₃H₇)-(CH₂)₃- | | 1H-NMR (400 MHz, d₆-DMSO): 4.65 (q, 2H, CH₂-CF₃), 3.33 (m, CH-O-CH₂) | trans |
| I-1-a-20 | CH₃ | CH₃ | H | 4- | F | F | F | -(CH₂)₄- | | H | 1H-NMR (400 MHz, d₆-DMSO): 4.68 (q, 2H, CH₂-CF₃), 4.05 (m, CH-N) | |
| I-1-a-21 | CH₃ | C₂H₅ | H | 4- | F | F | F | -(CH₂)₃- | | H | 1H-NMR (400 MHz, d₆-DMSO): 4.68 (q, 2H, CH₂-CF₃), 4.15 (m, CH-N) | |
| I-1-a-22 | CH₃ | C₂H₅ | H | 4- | F | F | F | -(CH₂)₄- | | H | 1H-NMR (400 MHz, d₆-DMSO): 4.68 (q, 2H, CH₂-CF₃), 4.05 (m, CH-N) | |
| I-1-a-23 | H | C₂H₅ | H | 4- | F | F | F | H | -(CH₂)₂-CHOCH₃-(CH₂)₂- | | 1H-NMR (300 MHz, CDCl₃): 4.34 (q, 2H, O-CH₂-CF₃), 3.16 (m, 1 H, CH-O-CH₃) | β |
| I-1-a-24 | C₂H₅ | Cl | H | 4- | F | F | F | H | -CH₂-CH(OC₄H₉)-(CH₂)₃- | | | trans |
| I-1-a-25 | CH₃ | CH₃ | H | 4- | F | F | F | H | -(CH₂)₂-CH(OCH₃)-(CH₂)₂- | | 1H-NMR (400 MHz, d₆-DMSO): 1.43-1.60 (m, 4H, CH₂), 1.88-2.00 (m, 4H, CH₂), 2.06 (s, 6H, 2xAr-CH₃), 3.27 (s, 3H, OCH₃), 4.61-4.66 (q, 2H, OCH₂CF₃), 6.73 (s, 2H, ArH) | cis |
| I-1-a-26 | CH₃ | CH₃ | H | 4- | F | F | F | H | -(CH₂)₂-O-(CH₂)₂- | | 1H-NMR (400 MHz, d₆-DMSO): 1.27-1.30 (2m, 2H, CH₂), 2.07 (s, 6H, 2xAr-CH₃), 3.67-3.73 (zt, 2H, OCH₂), 3.82-3.87 (m, 2H, OCH₂), 4.62-4.69 (q, 2H, OCH₂CF₃), 6.74 (s, 2H, ArH) | - |
| I-1-a-27 | H | CH₃ | H | 5- | F | F | F | H | -(CH₂)₂-CH(OCH₃)-(CH₂)₂- | | 1H-NMR (400 MHz, d₆-DMSO): 1.97 (s, 3H, Ar-CH₃), 3.27 (s, 3H, OCH₃), 4.59-4.66 (m, 2H, CH₂CF₃), 6.75 (d, 1H, ArH), 6.86-6.89 (m, 1H, ArH), 7.13 (d, 1H, ArH) | cis |
| I-1-a-28 | CH₃ | CH₃ | H | 3- | F | F | F | H | -(CH₂)₂-CH(OC₂H₅)-(CH₂)₂- | | 1H-NMR (400 MHz, d₆-DMSO): 1.11 (t, 3H, CH₃-CH₂O), 1.97, 2.03 (2s, je 3H, ArCH₃), 3.47-3.52 (q, 2H, O-CH₂CH₃), 4.58-4.65 (q, 2H, O-CH₂CF₃), 6.92 (d, 1H, ArH), 6.99 (d, 1H, ArH) | cis/trans ca. 10 :1 |
| I-1-a-29 | CH₃ | CH₃ | H | 3- | F | F | F | H | -(CH₂)₂-CH(OCH₃)-(CH₂)₂- | | 1H-NMR (400 MHz, d₆-DMSO): 1.97, 2.03 (2s, je 3H, Ar-CH₃), 3.27 (s, 3H, OCH₃), 4.58-4.65 (m, 2H, O-CH₂CF₃), 6.90 (d, 1H, ArH), 6.99 (d, 1H, ArH) | cis |

| Bsp-Nr. | W | X | Y | Z | J¹ | J² | J³ | D | A | B | F.p. °C/Analytik | Isomer |
|---|---|---|---|---|---|---|---|---|---|---|---|---|
| I-1-a-30 | H | CH₃ | H | 5- | F | F | F | H | -(CH₂)₂-O-(CH₂)₂- | | 1H-NMR (400 MHz, d₆-DMSO): 3.59-3.69 (m, 2H, OCH₂), 3.81-3.85 (m, 2H, OCH₂), 4.54-4.61 (m, 2H, O-CH₂CF₃), 6.74-6.76 (m, 1H, ArH), 6.82-6.83 (d, 1H, ArH), 7.04-7.06 (d, 1H, ArH) | - |
| I-1-a-31 | CH₃ | CH₃ | H | 3- | F | F | F | H | -(CH₂)₂-O-(CH₂)₂- | | 1H-NMR (400 MHz, d₆-DMSO): 1.28-1.33 (m, 2H, CH₂-CH₂-O), 1.98, 2.04 (2s, je 3H, ArCH₃), 3.67-3.74 (m, 2H, O-CH₂), 3.84-3.88 (m, 2H, O-CH₂), 4.59-4.65 (m, 2H, O-CH₂CF₃) | - |
| I-1-a-32 | CH₃ | CH₃ | H | 4- | F | F | F | H | 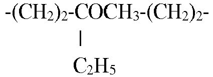 | | 269 | Gemisch |
| I-1-a-33 | CH₃ | CH₃ | H | 4- | F | F | F | H | 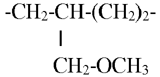 | | 190-193 | cis |
| I-1-a-34 | CH₃ | CH₃ | H | 4- | F | F | F | H | 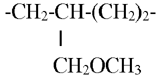 | | 94-100 | trans |
| I-1-a-35 | CH₃ | C₂H₅ | H | 4- | F | F | F | H | 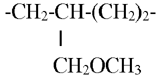 | | d) | trans |
| I-1-a-36 | CH₃ | CH₃ | H | 4- | F | F | F | H | 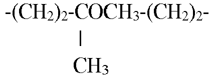 | | a) | trans |
| I-1-a-37 | CH₃ | C₂H₅ | H | 4- | F | F | F | H | 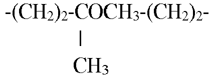 | | 196 | cis |
| I-1-a-38 | CH₃ | C₂H₅ | H | 4- | F | F | F | H | 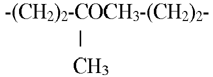 | | 189 | trans |
| I-1-a-39 | CH₃ | CH₃ | H | 4- | F | F | F | H | 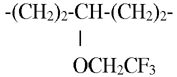 | | 258-264 | Gemisch β |
| I-1-a-40 | CH₃ | C₂H₅ | H | 4- | F | F | F | H | 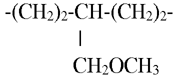 | | 236 | Gemisch β |
| I-1-a-41 | CH₃ | C₂H₅ | H | 4- | F | F | F | H | 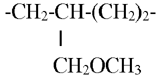 | | e) | cis |
| I-1-a-42 | H | CH₃ | H | 4- | F | F | F | H | -(CH₂)₂-CHOCH₃-(CH₂)₂ | | 101-102 | cis |
| I-1-a-43 | CH₃ | CH₃ | H | 3- | F | F | F | H | 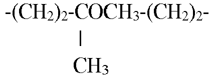 | | 139 | trans |
| I-1-a-44 | H | CH₃ | H | 5- | F | F | F | H | 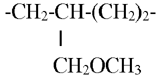 | | b) | cis |
| I-1-a-45 | H | CH₃ | H | 5- | F | F | F | H | 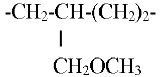 | | c) | trans |
| I-1-a-46 | CH₃ | C₂H₅ | H | 4- | F | F | F | -CH₂-C(-O-CH₂-CH₂-O-)-CH₂- | | H | 201-204 | |

a) ¹H-NMR (400 MHz, d₆-DMSO): δ = 1.09 (s,3H,CH₃), 1.12-1.15 (dm,2H,CH₂), 1.61-1.69 (tm, 2H,CH₂), 1.74-1.78 (dm,2H,CH₃), 2.06 (s,6H,ArCH₃), 3.11 (s,3H,OCH₃), 4.61-4.68 (q, 2H,OCH₂CF₃), 6.73 (s,2H,ArH), 7.81 (br,1H,NH) ppm
b) ¹H-NMR (600 MHz, d₆-DMSO): δ = 1.12 (t,3H,CH₂CH₃), 1.43-1.64 (3m,3H), 2.21-2.25 (m,1H), 2.34-2.36 (cm,1H), 3.32-3.45 (2m,4H,OCH₂-CH₃,O-CH₂), 4.66-4.70 (q,2H,OCH₂CF₃), 6.75 (d,1H,ArH), 6.88-6.90 (m,1H,ArH), 7.14-7.15 (d,1H,ArH), 7.79 (sbr,1H,NH)
c) ¹H-NMR (400 MHz, d₆-DMSO): δ = 1.11 (t,3H,CH₂-CH₃), 1.47-1.49 (m,1H), 1.59-1.68 (m,1H), 2.09 (s,3H,Ar-CH₃), 3.32-3.36 (cm,2H,OCH₂), 3.41-3.47 (q,2H,OCH₂-CH₃), 4.59-4.66 (q,2H,O-CH₂-CF₃), 6.75 (d,1H,ArH), 6.86-6.89 (m,1H,ArH), 7.12-7.14 (d,1H,ArH), 7.69 (s,br,1H,NH)
d) ¹H-NMR (400 MHz, CDCl₃): δ = 3.42 (dd,2H,OCH₃), 4.32 (m,2H,OCH₂CF₃)
e) ¹H-NMR (400 MHz, CDCl₃): δ = 3.52 (dd,2H,OCH₃), 4.32 (m,2H,OCH₂CF₃)

### Beispiel (I-1-b-1):

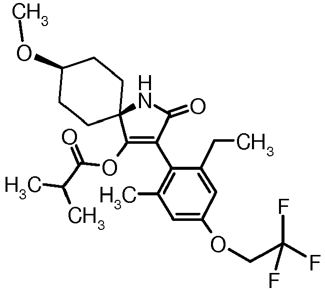

0.15 g (0.36 mmol) der Verbindung gemäß Beispiel (I-1-a-3) werden mit 0.44 g (1.2 eq) Triethylamin sowie 1.5 mg DMAP in 8ml EtOAc¹⁾ vorgelegt und 10min bei 50°C gerührt, Anschließend tropft man 0.043 g (1.1 eq) Isobuttersäurechlorid in 2ml EtOAc¹⁾ über 20min zu und läßt anschließend 6h bei 50°C und anschließend über Nacht bei RT rühren. Man versetzt mit 10ml Natriumhydrogencarbonat-Lösung, trennt die organische Phase ab, reextrahiert die wässrige Phase mit 20 ml EtOAc¹⁾, trocknet die vereingten org. Phasen mit Natriumsulfat. Der nach dem Einengen verbleibende Rückstand wird in einer Mischung von EtOAc¹⁾ und n-Heptan aufgenommen und erneut abfiltriert. Man erhält so 0.07 g an erfindungsgemäßer Verbindung (I-1-b-1) = 40 % d. Theorie.
¹H-NMR (400 MHz, CDCl₃): δ = 6.61 (pseudo d, 2H, Aryl-H), 6.37 (s, 1H, NH), 4.35 (q, 2H, CH₂-CF₃), 3.37 (s, 3H, OCH₃), 3.24 (m, 1H, CH-OCH₃), 2.51 (m, 3H, CH₂-Ar und CH(CH₃)₂), 2.20. (s, 3H, ArylCH₃), 2.19, 1.79, 1.38 (je m, zusammen 8 H Cyclohexyl), 1.13 (t, 3H, Aryl CH₂CH₃), 1.00 (dd, 6H, (CH₃)₂)ppm.

### ¹⁾Essigsäureethylester

In Analogie zu Beispiel (I-1-b-1) erhält man Beispiel (I-1-b-2) 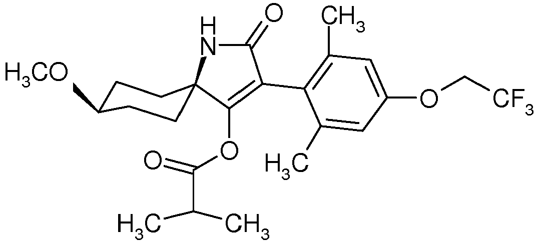 Fp. 198-199°C

### Beispiel (I-1-c-1):

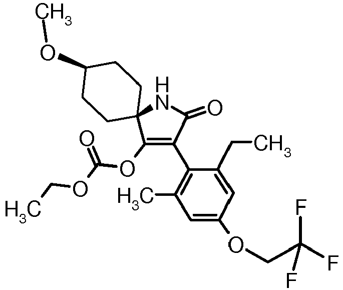

0.15 g (0.36 mmol) der Verbindung gemäß Beispiel (I-1-a-3) werden mit 0.44 g (1.2 eq) Triethylamin in 8ml Dichlormethan vorgelegt und 5 min bei RT gerührt. Anschließend tropft man 0.043 g (1.1 eq) Chlorameisensäureethylester über 20min zu und läßt anschließend über Nacht bei RT rühren. Man versetzt mit 5ml 10% Natriumcarbonat-Lsg, trennt die organische Phase ab und trocknet. Der nach dem Einengen verbleibende Rückstand wurde säulenchromatographisch (Silicagel, Gradient EtOAc¹⁾/n-Heptan) gereinigt. Man erhält so 0.12 g = 68 % d. Theorie an erfindungsgemäßer Verbindung (I-1-c-1).
¹H-NMR (400 MHz, CDCl₃): δ = 6.66 (pseudo d, 2H, Aryl-H), 6.37 (s, 1H, NH), 4.34 (q, 2H, CH₂-CF₃), 4.01 (q, 2H, OCH₂CH₃), 3.40 (s, 3H, OCH₃), 3.25 (m, 1H, CH-OCH₃), 2.49 (m, 2H, CH₂-Ar), 2.21 (s, 3H, ArylCH₃), 2.22, 1.96, 1.75, 1.40 (je m, zusammen 8 H Cyclohexyl), 1.13 (m, 6H, Aryl CH₂CH₃ und OCH₂CH₃) ppm.

In Analogie zu Beispiel (I-1-c-1) sowie gemäß den allgemeinen Angaben zur Herstellung erhält man folgende Verbindungen der Formel (I-1-c):

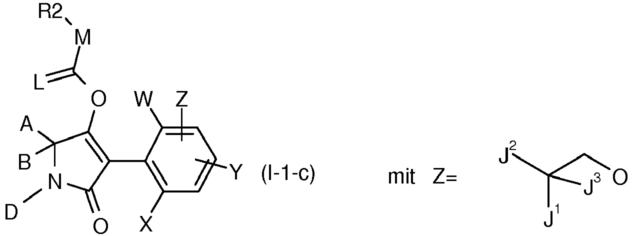

| Bsp-Nr. | W | X | Y | Z | J¹ | J² | J³ | D | A | B | L | M | R² | Analytik | Isomer |
|---|---|---|---|---|---|---|---|---|---|---|---|---|---|---|---|
| I-1-c-2 | CH₃ | CH₃ | H | 4- | F | F | F | H | -CH₂-CH(OC₂H₅)-(CH₂)₃- | | O | O | C₂H₅ | ¹H-NMR (400 MHz, CDCl₃): 4.34 (q, 2H, CH₂-CF₃), 4.01 (q, 2H, OCH₂), 3.42 (m, 3H, CH-OCH₂) | trans |
| I-1-c-3 | CH₃ | CH₃ | H | 4- | F | F | F | H | -CH₂-CH(OC₄H₉)-(CH₂)₃- | | O | O | C₂H₅ | | trans |
| I-1-c-4 | C₂H₅ | C₂H₅ | H | 4- | F | F | F | H | -(CH₂)₂-CH(OCH₃)-(CH₂)₂- | | O | O | C₂H₅ | ¹H-NMR (400 MHz, CDCl₃): 4.33 (q, 2H, CH₂-CF₃), 4.01 (q, 2H, OCH₂), 3.22 (m, 1H, CH-OCH₃) | cis |
| I-1-c-5 | CH₃ | C₂H₅ | H | 4- | H | F | F | H | -(CH₂)₂-CH(OCH₃)-(CH₂)₂- | | O | O | C₂H₅ | ¹H-NMR (400 MHz, CDCl₃): 4.16 (dt, 2H, CH₂-CHF₂), 4.03 (q, 2H, OCH₂), 3.23 (m, 1H, CH-OCH₃) | cis |
| I-1-c-6 | CH₃ | CH₃ | H | 3- | F | F | F | H | -(CH₂)₂-CH(OC₂H₅)-(CH₂)₂- | | O | O | C₂H₅ | ¹H-NMR (400 MHz, d₆-DMSO): 0.99, 1.11 (2t, je 2H, O-CH₂-CH₃), 1.98, 2.05 (2s, je 3H, ArCH₃), 3.29 (zm, 1H, CHOC₂H₅), 3.46-3.52 (q, 2H, CHO-CH₂CH₃), 3.94-3.99 (q, 2H, OCH₂CH₃), 4.61-4.67 (m, 2H, OCH₂CF₃) | cis |
| I-1-c-7 | CH₃ | C₂H₅ | H | 4- | F | F | F | -CH₂-C(-O-CH₂-CH₂-O-)-CH₂ - | | H | O | O | C₂H₅ | ¹H-NMR (400 MHz, CDCl₃): 6.70 und 6.66 (je s, 1H, Ar-H), 4,89 (s, 1H, CH-N), 4.32 (dt, 2H, CH₂-CF₃), 4.17 (q, 2H, OCH₂) | |

### Beispiel II-1:

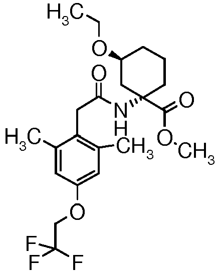

1.5 g (5.7 mmol) der Verbindung gemäß Beispiel (XXXI-1) werden mit 3.4 g (5eq) Thionylchlorid und einem Tropfen DMF versetzt. Man erhitzt bis zum Ende der Gasentwicklung unter Rückfluss zum Sieden, engt anschließend die Reaktionslösung ein und versetzt sie mit 4 ml Dichlormethan (Lösung 1). 1.5 g (1.1 eq) trans-3-Ethoxy-1-amino-cyclohexancarbonsäure-methylester sowie 0.7 g (1.2 eq) Triethylamin werden in 50 ml Dichlormethan gelöst und Lösung 1 innerhalb von 1 h zugetropft. Nach 18 h Rühren wird mit 10 ml Wasser versetzt, die organische Phase abgetrennt, eingeengt und säulenchromatographisch gereinigt. Man erhält 0.56 g = (23% d. Theorie) von Beispiel II-a-1.
1H-NMR (400 MHz, CDCl₃): δ = 4.36 (q, 2H, CH₂-CF₃), 3.71 (s, 3H, OCH₃), 3.40 (m, 2H, CH-OCH₂) ppm.

In Analogie zu Beispiel (II-1) und gemäß den allgemeinen Angaben zur Herstellung erhält man folgende Verbindungen der Formel (II):

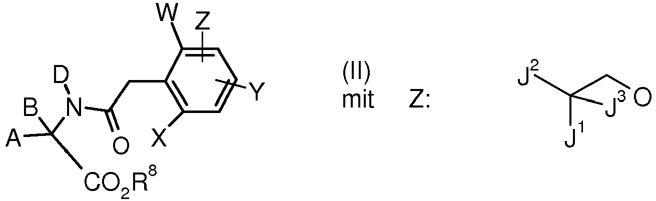

| Bsp-Nr. | W | X | Y | Z | J¹ | J² | J³ | D | A | B | R⁸ | Analytik | Isomer |
|---|---|---|---|---|---|---|---|---|---|---|---|---|---|
| II-2 | CH₃ | CH₃ | H | 4- | F | F | F | H | -CH₂-CH(OC₄H₉)-(CH₂)₃- | | CH₃ | ¹H-NMR (400 MHz, CDCl₃): 4.34 (q, 2H, CH2-CF₃), 3.70 (s, 3H, OCH₃), 3.32 (m, 2H, CH-OCH₂) | trans |
| 11-3 | CH₃ | CH₃ | H | 4- | F | F | F | H | (CH₂)₂-CH(CH₂OCH₃)-(CH₂)₂- | | CH₃ | ¹H-NMR (300 MHz, CDCl₃): 4.34 (q, 2H, CH₂-CF₃), 3.70 (s, 3H, OCH₃), 3.11 (m, 2H, CH₂-OCH₃) | β |
| II-4 | CH₃ | CH₃ | H | 4- | F | F | F | H | -(CH₂)₂-CH(OCH₃)-(CH₂)₂- | | CH₃ | ¹H-NMR (400 MHz, d₆-DMSO): 2.23 (s, 6H, Ar-CH₃), 3.23 (s, 3H, OCH₃), 3.50 (s, 2H, CH₂CO), 3.52 (s, 3H, CO₂CH₃), 4.58-4.65 (q, 2H, O-CH₂CF₃), 6.69 (s, 2H, ArH) | cis |
| II-5 | CH₃ | CH₃ | H | 4- | F | F | F | H | -(CH₂)₂-O-(CH₂)₂- | | CH₃ | ¹H-NMR (400 MHz, d₆-DMSO): 1.84-1.97 (m, 4H, CH₂), 2.23 (s, 6H, Ar-CH₃), 3.51 (s, 2H, CO-CH₂), 3.55 (s, 3H, CO₂CH₃), 4.58-4.65 (q, 2H, ArH) | - |

Folgende Verbindungen der Formel (I-1'-a), die zur Herstellung von Verbindungen der Formel (I-1-a) verwendet wurden, sind neu und lassen sich gemäß Verfahren A herstellen:

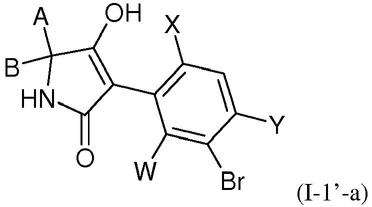

| Bsp.Nr. | W | X | Y | A | B | Fp. | Isomer |
|---|---|---|---|---|---|---|---|
| I-1'-a-1 | C₂H₅ | C₂H₅ | H | -(CH₂)₂-CHOCH₃-(CH₂)₂- | | 225-228 | cis |
| I-1'-a-2 | C₂H₅ | C₂H₅ | H | -(CH₂)₂-O-(CH₂) | | 278 | - |

Die zur Herstellung der Verbindung (I-1'-a) benötigten Phenylessigsäuren der Formel (XXXI') erhält man z.B. durch Bromierung in Eisessig.

### Beispiel (XXXI'-1)

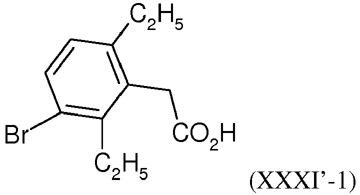

3.85 g (20 mmol) 2,6-Diethyl-phenylessigsäure werden in 40 ml Eisessig vorgelegt. Bei 10°C - 15°C werden 3.2 g (20 mmol) Brom in 12 ml Eisessig in ca. 40 Min. zugetropft. Nach ca. 2 Stunden werden erneut 1.1 g Brom in 4 ml Eisessig zugegeben und über Nacht bei Raumtemperatur nachgerührt. Nach Abdampfen des Eisessigs im Vakuum wird der Rückstand in 40 ml 2 N Natronlauge aufgenommen, mit MTB-Ether gewaschen, die wässrige Phase sauergestellt, mit Dichlormethan extrahiert, getrocknet und im Vakuum eingedampft.
Man erhält 4.3 g (72 % d. Theorie) der Verbindung (XXXI'-1).
¹H-NMR (400 MHz, d₆-DMSO): δ =1.08, 1.12 (2t, je 3H,CH₂-CH₃), 2.54-2.60 (q,2H,CH₂CH₃), 2.76-2.81 (q,2H,CH₂CH₃), 3.70 (s,2H,CH₂CO), 6.98, 7.41 (2d,je 1H,Ar-H) ppm.

### Beispiel (I-2-a-1):

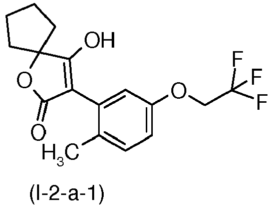

1,36g (34 mmol) Natriumhydrid(60%ig) werden in 30ml THF vorgelegt und 3,10g (31 mmol) Trifluorethanol zugetropft, nach Beendigung der Gasentwicklung werden 6,48g (34 mmol) Kupfer(I)iodid zugesetzt, eine Lösung von 2,00g (6,18 mmol) (I-2-a-1') (generisch bekannt aus WO 98/05638) in 20ml THF gelöst langsam zugetropft und 2,5h am Rückfluß gekocht.

Zur Aufarbeitung wird der abgekühlte Ansatz mit Wasser versetzt, mit verd. HCl angesäuert, mit Ether und Essigester ausgeschüttelt, die org. Phase getrocknet, abfiltriert und eingeengt.
Man erhält 1,57g (70% d.Th.) des Beispiels (I-2-a-1); logP (HCOOH) 2,59
1H-NMR (CD₃CN): δ = 1.80-2.20 (m, 8H), 2,15 (s,3H), 3.35 (m, 2H), 7.15 (m,1H), 7,30 (m, 1H), 7.35 (m, 1H) ppm.

### Beispiel (I-2-a-2)

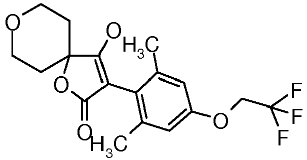

0,294g (2,62 mmol) Kalium-t-butylat werden in 7 ml DMF²⁾ vorgelegt, auf 0°C gekühlt, eine Lösung von 0,732g (1,75 mmol) des Beispiels (III-1) in 3 ml DMF²⁾ bei 0 - 10°C zugetropft und über Nacht bei Raumtemperatur gerührt.

Zur Aufarbeitung wird das DMF²⁾ abrotiert, der Rückstand in Wasser verrührt, die alk. Phase mit Methyl-t-butylether extrahiert, die wäßrige Phase mit Salzsäure angesäuert, mit Dichlormethan extrahiert, getrocknet, abfiltriert und eingeengt. Das Rohprodukt wird mittels Chromatographie an Kieselgel (Eluent Essigester/Cyclohexan) gereinigt.

### ²⁾ Dimethylformamid

0,416g (57% d.Th.) des Beispiels (I-2-a-1), logP (HCOOH) 2,45
1H-NMR (d₆-DMSO): δ = 1.50 (m, 2H), 2.10 (s, 6H), 2.20 (m, 2H), 3.65 (m, 2H), 3.95 (m, 2H), 6.80 (s, 2H) ppm.

In Analogie zu den Beispielen (I-2-a-1) und (I-2-a-2) und gemäß den allgemeinen Angaben zur Herstellung erhält man folgende Verbindungen der Formel (I-2-a)

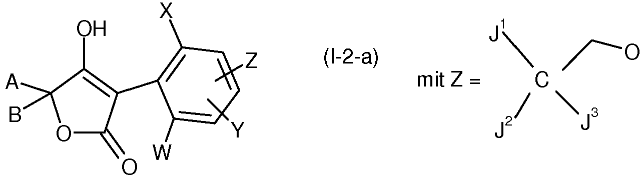

| Bsp. Nr. | W | X | Y | Z | J¹ | J² | J³ | A | B | Analytik | Isomer |
|---|---|---|---|---|---|---|---|---|---|---|---|
| I-2-a-3 | H | CH₃ | H | 3- | F | F | F | -(CH₂)₂-CHOCH₃-(CH₂)₂- | | a) | cis |
| I-2-a-4 | H | CH₃ | H | 3- | F | F | F | -(CH₂)₂-CHOCH₃-(CH₂)₂- | | b) | trans |
| I-2-a-5 | CH₃ | CH₃ | H | 4- | F | F | F | -(CH₂)₂-CHOCH₃-(CH₂)₂- | | c) | cis |
| I-2-a-6 | CH₃ | CH₃ | H | 4- | F | F | F | -(CH₂)₂-CHOCH₃-(CH₂)₂- | | d) | trans |

a)1H-NMR(d₆-DMSO): δ = 1,50 (m,2H), 1,65 (m,2H), 2,05 (m,4H), 2,10 (s,3H), 3,25 (m, 1H), 3,30 (s, 3H), 4,65 (m, 2H), 6,80 (m, 1H), 6,95 (m, 1H), 7,15 (m, 1H) ppm.
b) 1H-NMR(d₆-DMSO): δ = 1,40 (m,2H), 1,70 (m,2H), 1,95 (m,2H), 2,10 (s,3H), 2,15 (m, 2H), 3,30 (s, 3H), 3,50 (m, 1H), 4,65 (m, 2H), 6,80 (m, 1H), 6,95 (m, 1H), 7,15 (m, 1H) ppm.
c) 1H-NMR(d₆-DMSO): δ = 1,45 (m,2H), 1,65 (m,2H), 2,00 (m,4H), 2,05 (s,3H), 3,20 (m, 1H), 3,28 (s, 3H), 4,65 (m, 2H), 6,75 (m, 2H) ppm.
d) 1H-NMR(d₆-DMSO): δ = 1,41 (m,2H), 1,75 (m,2H), 1,95 (m,2H), 2,06 (s,3H), 2,15 (m, 2H), 3,25 (s, 3H), 3,52 (m, 1H), 4,65 (m, 2H), 6,75 (m, 2H) ppm.

### Beispiel (I-2-b-1):

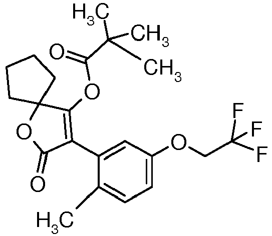

56 mg (0,16 mmol) des Beispiels I-2-a-1 werden in 10 ml Dichlormethan vorgelegt, bei Raumtemperatur werden 18 mg (0,18 mmol) Triethylamin zugegeben, bei 0-10°C werden 21 mg (0,17 mmol) Pivaloylchlorid zugetropft und 1h bei Raumtemperatur verrührt.

Zur Aufarbeitung wird der Ansatz mit verd. Citronensäure und 5%iger NaOH ausgeschüttelt, die org. Phase getrocknet und eingeengt.
Man erhält 61 mg (83% d.Th.) des Beispiels (I-2-b-1), logP (HCOOH) 4,69
1H-NMR (d₆-DMSO): δ = 1.10 (s, 9H), 1.80-2.20 (m, 8H), 2.15 (s,3H), 4,65 (m, 2H), 6,75 (m,1H), 7,00 (m, 1H), 7.20 (m, 1H) ppm.

### Beispiel (III-1):

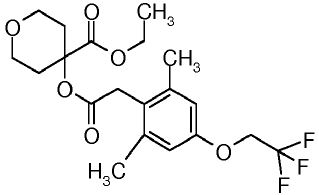

0,621g (3,56 mmol) 1-Hydroxy-tetrahydropyran-carbonsäure-ethylester und 1,00g (3,56 mmol) 2,6-Dimethyl-4-trifluorethoxy-phenylessigsäurechlorid werden in 20ml Toluol 12h unter Rückfluß gekocht.

Zur Aufarbeitung wird das Toluol abrotiert, der Rückstand zwischen Methyl-t-butylether und 5% Natronlauge verteilt, die org.Phase getrocknet und eingeeengt.
Man erhält 0,732g (47% d.Th.) der Verbindung (III-1), logP (HCOOH) 3,78
1H-NMR (d₆-DMSO): δ = 1,15 (m, 3H), 1,80-2.00 (m, 4H), 2,25 (s, 6H), 3.40-3.70 (m, 4H), 4.05 (m, 2H), 4.65 (m, 2H), 6.75 (m, 2H) ppm.

### Beispiel I-6-a-1

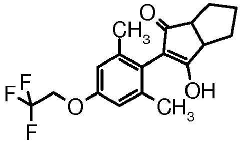

300 mg (0.934 mmol) der Verbindung gemäß Beispiel (I-6'-a-1) wird in 5 ml Collidin gelöst und mit 702 mg (6.26 mmol) Kalium-tert.-butylat versetzt (Lösung 1). In einem separaten Kolben werden 178 mg (0.934 mmol) Kupfer-I-jodid, 841 mg (8.4 mmol) Trifluorethanol und 210 mg (1.87 mmol) Kalium-tert.-butylat in 5 ml Collidin gelöst. Dazu wird Lösung 1 getropft, das Gefäß mit 2 ml DMF nachgespült und die Reaktionsmischung 1 Stunde bei 145 °C in der Mikrowelle gerührt. Das Lösungsmittel wird im Vakuum abgezogen, der Rückstand in Wasser aufgenommen und über Celite filtriert. Zu dem Filtrat werden 10 ml Ammoniumchloridlösung hinzugegeben und mit 2 N Salzsäure angesäuert. Der dabei ausgefallene Feststoff wird abgesaugt und getrocknet.
Ausbeute: 185 mg (58 % d. Theorie)
¹H-NMR, (400MHz, CDCl₃): 1.38-1.48 (m, 1H), 1.70 (mc, 3H), 2.05 und 2.10 (je s, je 3H), 3.15 (mc, 2H), 4.31 (q, 2H), 6.65 (s, 2H) ppm.

Die Verbindung der Formel (I-6'-a-1), die zur Herstellung der Verbindung der Formel (I-6-a-1) verwendet wird ist neu und lässt sich gemäß Verfahren F herstellen:

### Beispiel (I-6'-a-1)

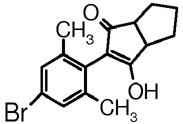

¹H-NMR (400MHz, CDCl₃): 1.43 (mc,1H), 1.68 (mc. 3H), 2.08 und 2.11 (je s, je 3H), 2.98 und 3.28 (je mc, je 1H), 7.18 (s, 2H) ppm.

### Beispiel I-6-c-1

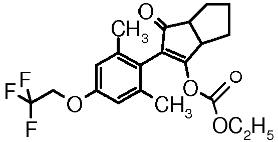

69 mg (0.2 mmol) der Verbindung gemäß Beispiel (I-6-a-1) wird in 5 ml Dichlormethan gelöst und mit 24 mg (0.22 mmol) Chlorameisensäureethylester sowie 62 mg (0.6 mmol) Triethylamin vesetzt. Man lässt 30 Minuten bei Raumtemperatur. Es wird eingeengt und mittels präperativer HPLC (RP-18, Gradient Acetonitril/Wasser (1 % Trifluroessigsäure)) aufgereinigt.
Ausbeute: 57 mg
¹H-NMR (400 MHz CDCl₃): 1.21 (t, 3H), 1.50 (mc, 1H), 1.65 -1.88 (m, 3H), 1.94 (mc, 1H), 2.06 und 2.09 (je s, je 3 H), 3.11 und 3.80 (je mc, je 1H), 4.12 (mc, 2H), 4.30 (q, 2H), 6.62 (s, 2H) ppm.

### Beispiel I-8-a-1

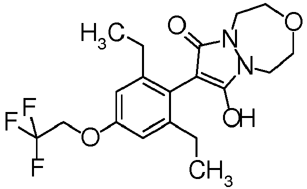

0.3 g 8-(4-Brom-2,6-diethylphenyl)-9-hydroxy-1,2,4,5-tetrahydro-7H-pyrazolo[1,2-d][1,4,5]oxadiazepin-7-on (bekannt aus WO99/047525 Bsp.:1.087) werden unter Stickstoffatmosphäre mit 2eq(0,176g) Kalium-t-butylat in 5ml Collidin gelöst (Lsg.1).

Anschließend werden 150 mg Kupfer(I)iodid, 0.69 g 2,2,2-Trifluorethanol sowie 6,7eq(0,591g) Kalium-t-Butylat in 5ml Collidin unter Stickstoffatmosphäre suspendiert. Man gibt Lsg.1 hinzu und läßt unter Mikrowellenbedigungen 1h bei 145°C rühren.

Das Lösungsmittel wird unter vermindertem Druck entfernt und der vebliebene Rückstand in Wasser aufgenommen. Der verbleibende Rückstand wird abfiltriert und die Muttterlauge mit In Salzsäure auf pH=1 gestellt. Nach Extraktion mit Ethylacetat, trocknen mit Natriumsulfat wird eingeengt und man erhält 0.275 g an erfindungsgemäßer Verbindung I-8-a-1.

In Analogie zu Beispiel (I-8-a-1) und gemäß den allgemeinen Angaben zur Herstellung erhält man folgende Verbindungen der Formel (I-8-a)

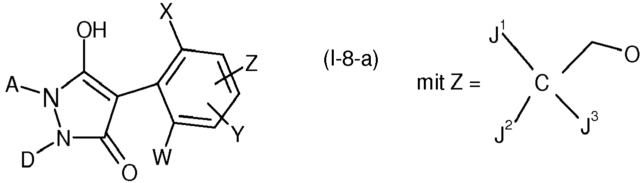

| Bsp.-Nr. | W | X | Y | Z | J¹ | J² | J³ | A | D | Analytik |
|---|---|---|---|---|---|---|---|---|---|---|
| I-8-a-1 | C₂H₅ | C₂H₅ | H | 4- | F | F | F | -(CH₂)₂-O-(CH₂)₂- | | ¹H-NMR (400 MHz CDCl₃): 6.72 (m, 2H, Ar-H) 4.32 (m, 2H, CF₃-CH₂), 3.75 (m, 2H, CH₂-N) |
| I-8-a-2 | C₂H₅ | CH₃ | H | 4- | F | F | F | -(CH₂)₂-O-(CH₂)₂- | | ¹H-NMR (400 MHz CDCl₃): 6.70 (d, 1H, Ar-H) 6.60 (d, 1 H, Ar-H), 4.32 (m, 2H, CF₃-CH₂), 3.80 (m, 2H, CH₂-N) |
| I-8-a-3 | CH₃ | CH₃ | H | 4- | F | F | F | -(CH₂)₂-O-(CH₂)₂- | | ¹H-NMR (400 MHz CDCl₃): 6.72 (d, 1H, Ar-H) 6.62 (d, 1 H, Ar-H), 4.32 (m, 2H, CF₃-CH₂), 3.82 (m, 2H, CH₂-N) |
| I-8-a-4 | C₂H₅ | CH₃ | H | 4- | F | F | F | -(CH₂)₄- | | ¹H-NMR (400 MHz CDCl₃): δ= 3.68 (m,4H,CH₂N), 4.32(m,2H,OCH₂CF₃) |
| I-8-a-5 | C₂H₅ | C₂H₅ | H | 4- | F | F | F | -(CH₂)₄- | | ¹H-NMR (400 MHz CDCl₃): 6.68 (d, 2H, Ar-H), 4.33 (m, 2H, CF₃-CH₂), 3.62 (m, 4H, CH₂-N) |
| I-8-a-6 | CH₃ | CH₃ | H | 4- | F | F | F | -(CH₂)₄- | | ¹H-NMR (400 MHz CDCl₃): 6.68 (d, 2H, Ar-H), 4.33 (m, 2H, CF₃-CH₂), 3.62 (m, 2H, CH₂-N) |

### Beispiel I-8-b-1

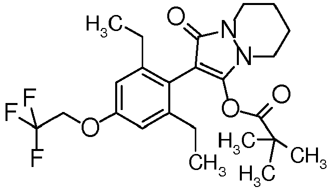

0.05 g der Verbindung gemäß Beispiel I-8-a-4 werden unter Stickstoffatmosphäre in 1 ml Dichlormethan gelöst. Anschließend werden 17 mg 2,2-Dimethylpropanoylchlorid und 17 mg Triethylamin zugegeben und über Nacht bei Raumtemperatur gerührt.

Der Ansatz wird auf 5 ml Wasser gegeben und die Phasen über eine Extraktionskartusche getrennt. Die organische Phase wird eingeengt und anschließend der Rückstand mit reversed Phase HPLC-Chromatographie (Gradient Acetonitril/Wasser 0.05%TFA) aufgereinigt. Man erhält so 0.024 g an erfindungsgemäßer Verbindung I-8-b-1.
¹H-NMR (400MHz, CDCl₃): 6.67 (s, 2H, Ar-H), 4.32 (q, 2H, OCH₂CF₃), 1.92 (m, 4H, CH₂), 1.05 (s, 9H, t-Bu)

In Analogie zu Beispiel (I-8-b-1) und gemäß den allgemeinen Angaben zur Herstellung erhält man folgende Verbindungen der Formel (I-8-b)

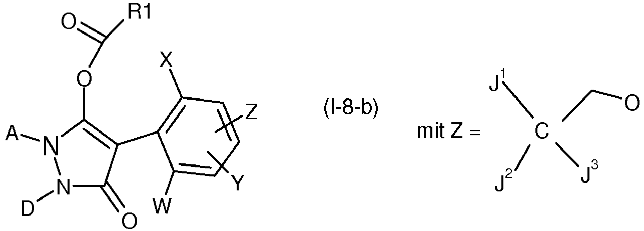

| Bsp.-Nr. | W | X | Y | Z | J¹ | J² | J³ | A | D | R¹ | Analytik |
|---|---|---|---|---|---|---|---|---|---|---|---|
| I-8-b-2 | C₂H₅ | C₂H₅ | H | 4- | F | F | F | -(CH₂)₂-O-(CH₂)₂- | | C(CH₃)₃ | a) |
| I-8-b-3 | C₂H₅ | CH₃ | H | 4- | F | F | F | -(CH₂)₂-O-(CH₂)₂- | | CH(CH₃)₂ | b) |
| I-8-b-4 | C₂H₅ | CH₃ | H | 4- | F | F | F | -(CH₂)₄- | | C(CH₃)₃ | c) |
| I-8-b-5 | CH₃ | CH₃ | H | 4- | F | F | F | -(CH₂)₄- | | CH(CH₃)₂ | d) |
| I-8-b-6 | CH₃ | CH₃ | H | 4- | F | F | F | -(CH₂)₂-O-(CH₂)₂- | | C(CH₃)₃ | e) |
| I-8-b-7 | CH₃ | CH₃ | H | 4- | F | F | F | -(CH₂)₂-O-(CH₂)₂- | | CH(CH₃)₂ | f) |

a) ¹H-NMR (400MHz, CDCl₃): 6.67 (s, 2H, Ar-H), 4.32 (q, 2H, OCH₂CF₃), 3.86 (m, 4H, CH₂-N), 1.03 (s, 9H, t-Bu)
b) ¹H-NMR (400MHz, CDCl₃): 6,67 und 6.62 (je d, 1H, Ar-H), 4.32 (q, 2H, OCH₂CF₃), 2.47 (m, 1H, C**H**(CH₃)₂)
c) ¹H-NMR (400MHz, CDCl₃): 6,67 und 6.62 (je d, 1H, Ar-H), 4.32 (q, 2H, OCH₂CF₃), 1.92 (m, 4H, CH₂), 1.09 (d, 9H, t-Bu)
d) ¹H-NMR (400MHz, CDCl₃): 6.62 (s, 2H, Ar-H), 4.32 (q, 2H, OCH₂CF₃), 1.92 (m, 4H, CH₂), 1.05 (d, 6H, CH(CH₃)₂)
e) ¹H-NMR (400MHz, CDCl₃): 6.62 (s, 2H, Ar-H), 4.32 (q, 2H, OCH₂CF₃), 3.88 (m, 4H, CH₂-N), 1.08 (s, 9H, t-Bu)
f) ¹H-NMR (400MHz, CDCl₃): 6.64 (s, 2H, Ar-H), 4.32 (q, 2H, OCH₂CF₃), 3.87 (m, 4H, CH₂-N), 1.02 (d, 9H, CH(CH₃)₂)

### Beispiel I-8-c-1:

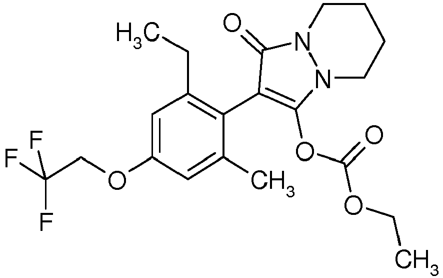

0.58 g an erfindungsgemäßer Verbindung I-8-a-4 werden in 5 ml Dichlormethan gelöst und mit 0.187 g Chlorameisensäureethylester sowie 0.206 g Triethylamin versetzt. Man läßt 18 h bei Raumtemperautr rühren und gibt 10 ml Wasser hinzu. Nach Abtrennung der wässrigen Phase über eine Extraktionskartusche, wird eingeengt und mittels präparativer HPLC (RP-18, Gradient Acetonitril/ Wasser (1% Trifluroessigsäure) aufgereinigt. Man erhält so 0.03 g an erfindungsgemäßer Verbindung I-8-c-1.

In Analogie zu Beispiel (I-8-c-1) und gemäß den allgemeinen Angaben zur Herstellung erhält man folgende Verbindungen der Formel (I-8-c)

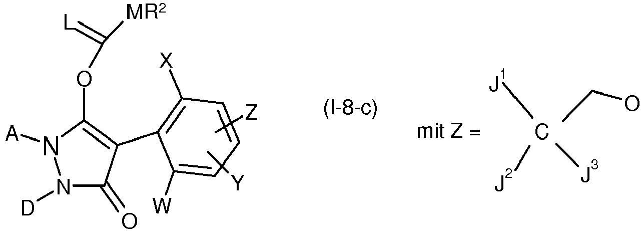

| Bsp.-Nr. | W | X | Y | Z | J¹ | J² | J³ | A | D | L | M | R² | Analytik |
|---|---|---|---|---|---|---|---|---|---|---|---|---|---|
| I-8-c-1 | C₂H₅ | CH₃ | H | 4- | F | F | F | -(CH₂)₄- | | O | O | C₂H₅ | a) |
| I-8-c-2 | C₂H₅ | CH₃ | H | 4- | F | F | F | -(CH₂)₂-O-(CH₂)₂- | | O | O | C₂H₅ | b) |
| I-8-c-3 | C₂H₅ | C₂H₅ | H | 4- | F | F | F | -(CH₂)₂-O-(CH₂)₂- | | O | O | C₂H₅ | c) |
| I-8-c-4 | C₂H₅ | C₂H₅ | H | 4- | F | F | F | -(CH₂)₄- | | O | O | C₂H₅ | d) |
| I-8-c-5 | CH₃ | CH₃ | H | 4- | F | F | F | -(CH₂)₂-O-(CH₂)₂- | | O | O | C₂H₅ | e) |
| I-8-c-6 | CH₃ | CH₃ | H | 4- | F | F | F | -(CH₂)₄- | | O | O | C₂H₅ | f) |

a) ¹H-NMR (400 MHz CDCl₃): 6.70 (s, 1H, Ar-H), 6.67 (s, 1H, Ar-H), 4.32 (m, 2H, CF₃-CH₂), 4.14 (m, 2H, CH₂-OC(=O))
b) ¹H-NMR (400 MHz CDCl₃): 6.70 (d, 1H, Ar-H) 6.65 (d, 1H, Ar-H), 4.32 (m, 2H, CF₃-CH₂), 4.16 (m, 2H, CH₂-OC(=O))
c) ¹H-NMR (400 MHz CDCl₃): 6.68 (d, 2H, Ar-H), 4.32 (m, 2H, CF₃-CH₂), 4.14 (m, 2H, CH₂-OC(=O))
d) ¹H-NMR (400 MHz CDCl₃): 6.68 (d, 2H, Ar-H), 4.34 (m, 2H, CF₃-CH₂), 4.16 (m, 2H, CH₂-OC(=O))
e) ¹H-NMR (400 MHz CDCl₃): 6.68 (d, 2H, Ar-H), 4.30 (m, 2H, CF₃-CH₂), 4.17 (m, 2H, CH₂-OC(=O))
f) ¹H-NMR (400 MHz CDCl₃): 6.65 (d, 2H, Ar-H), 4.32 (m, 2H, CF₃-CH₂), 4.16 (m, 2H, CH₂-OC(=O))

### 2,6-Dimethyl-4-trifluorethoxy-phenylessigsäure (Beispiel XXXI-1)

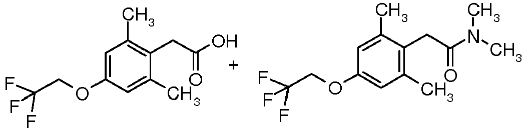

18,05g (451 mmol) Natriumhydrid werden in 500ml DMF vorgelegt, 41,05g (410 mmol) Trifluorethanol zugetropft, nach Beendigung der Gasentwicklung werden 15,63g (82 mmol) Kupfer(I)iodid zugesetzt, eine Lösung von 21,10g (82 mmol) des 2,6-Dimethyl-4-brom-phenylessigsäuremethylesters in 100ml DMF langsam zugetropft und 2,5h am Rückfluß gekocht.

Zur Aufarbeitung wird der Ansatz eingeengt, der Rückstand mit Wasser versetzt und mehrfach mit Diethylether ausgeschüttelt, die Etherphase wird getrocknet, abfiltriert und eingeengt:
12,73g 2,6-Dimethyl-4-trifluorethoxy-phenylessigsäure-N,N-dimethylamid.

Die wässrige Phase wird mit Salzsäure angesäuert und mehrfach mit Dichlormethan extrahiert, die org.Phase wird getrocknet und einrotiert:
7,66g Arylessigsäure.

Die 12,73g 2,6-Dimethyl-4-trifluorethoxy-phenylessigsäure-N,N-dimethylamid werden 36h in einer Lösung aus 58g Kaliumhydroxid in 165ml Methanol und 43 ml Wasser gekocht. Zur Aufarbeitung wird das Methanol abrotiert, der Rückstand zwischen Wasser und Dichlormethan verteilt, die wässrige Phase mit Salzsäure angesäuert und die ausgefallenen Kristalle abgesaugt und getrocknet. Auf diese Weise erhält man weitere 11,6 g der Phenylessigsäure (XXXI-1).
Gesamtausbeute: 19,3 g (90% d.Th.) 2,6-Dimethyl-4-trifluorethoxy-phenylessigsäure (XXXI-1)
1H-NMR (d₆-DMSO): δ = 2,25 (s, 6H), 3,50 (s,2H), 4,60 (m,2H), 6,75 (s,2H) ppm.
logP (HCOOH) 2,51.

Die Bestimmung der in den voran stehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 95 % Acetonitril.

Die Bestimmung mit der LC-MS im sauren Bereich erfolgt bei pH 2,7 mit 0,1 % wässriger Ameisensäure und Acetonitril (enthält 0,1 % Ameisensäure) als Eluenten; linearer Gradient von 10 % Acetonitril bis 95 % Acetonitril.

Die Bestimmung mit der LC-MS im neutralen Bereich erfolgt bei pH 7,8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigtem Alkan-2-onen (mit 3 bis 16 Kohlenstoffatomen), deren logP-Werte bekannte sind (Bestimmung der logP-Werte anhand der Retentionszeiten durch lineare Interpolation zwischen zwei aufeinander folgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel 1

**Myzus-Test (MYZUPE Spritzbehandlung)**

| | | |
|---|---|---|
| Lösungsmittel: | 78 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von ≥ 80 % bei einer Aufwandmenge von 500 g/ha :
Bsp Nr : I-1-a-1, I-1-a-3, I-1-a-4, I-1-a-5, I-1-a-6, I-1-a-7, I-1-a-8, I-1-a-10, I-1-a-12, I-1-a-13, I-1-a-14, I-1-a-15, I-1-a-17, I-1-a-18, I-1-a-19, I-1-a-23, I-1-a-24, I-1-a-25, I-1-a-26, I-1-a-27, I-1-a-28, I-1-a-29, I-1-a-30, I-1-a-31, I-1-a-32, I-1-a-33, I-1-a-34, I-1-a-35, I-1-a-36, I-1-a-37, I-1-a-38, I-1-a-39, I-1-a-40, I-1-a-41, I-1-a-42, I-1-a-43, I-1-a-44, I-1-a-45, I-1-a-46, I-1-a-48, I-1-b-1, I-1-c-1, I-1-c-2, I-1-c-3, I-1-c-4, I-1-c-5, I-1-c-6, I-2-a-1, I-2-a-2, I-2-a-3, I-2-a-4, I-2-a-5, I-2-a-6, I-8-a-2, I-8-b-3, I-8-b-5

Bei diesem Test zeigen z. B. die folgenden Verbindungen der Herstellungsbeispiele Wirkung von ≥ 80 % bei einer Aufwandmenge von 100 g/ha :
Bsp Nr: I-1-a-20

### Beispiel 2

**Phaedon-Test (PHAECO Spritzbehandlung)**

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von ≥ 80 % bei einer Aufwandmenge von 500 g/ha :
Bsp Nr.: I-1-a-1, I-1-a-3, I-1-a-8, I-1-a-11, I-1-a-13, I-1-a-14, I-1-a-15, I-1-a-16, I-1-a-18, I-1-a-19, I-1-a-21, I-1-a-23, I-1-a-27, I-1-a-28, I-1-a-30, I-1-a-31, I-1-a-32, I-1-a-33, I-1-c-6, I-2-a-5

### Beispiel 3

**Tetranychus-Test, OP-resistent (TETRUR Spritzbehandlung)**

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele Wirkung von ≥ 80 % bei einer Aufwandmenge von 100 g/ha :
BspNr.: I-1-a-4, I-1-a-5, I-1-a-6, I-1-a-7, I-1-a-8, I-1-a-10, I-1-a-14, I-1-a-17, I-1-a-2, I-1-a-25, I-1-a-26, I-1-a-28, I-1-a-3, I-1-a-30, I-1-a-31, I-1-a-32, I-1-b-1, I-1-c-1, I-1-c-2, I-1-c-3, I-1-c-4, I-1-c-5, I-1-c-6, I-2-a-5, I-2-a-6, I-2-b-1, I-8-c-1, I-8-a-2, I-8-b-3, I-8-b-5

Bei diesem Test zeigen z. B. die folgenden Verbindungen der Herstellungsbeispiele Wirkung von ≥ 80 % bei einer Aufwandmenge von 20 g/ha :
Bsp Nr.: I-1-a-16, I-1-a-19

### Beispiel 4

**Spodoptera frugiperda-Test (SPODFR Spritzbehandlung)**

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von ≥ 80 % bei einer Aufwandmenge von 500 g/ha :
Bsp Nr .: I-1-a-27, I-1-a-28, I-1-a-32, I-1-a-36, I-1-a-39, I-1-c-1

### Beispiel 5

**Meloidogyne incognita-Test (MELGIN)**

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele Wirkung von ≥ 80 % bei einer Aufwandmenge von 20 ppm :
Bsp Nr.: I-1-a-25, I-1-c-6

### Beispiel 6

### Lucilia cuprina-Test (LUCICU)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele Wirkung von ≥ 80 % bei einer Aufwandmenge von 100 ppm :
BspNr. : I-1-a-25, I-1-a-3, I-2-a-5, I-2-a-6

### Beispiel 7

### Boophilus microplus-Test (BOOPMI Injektion)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele Wirkung von ≥ 80 % bei einer Aufwandmenge von 20 ppm :
Bsp Nr.: I-1-a-25, I-1-a-3, I-1-a-7, I-1-c-4, I-1-c-5, I-2-a-5, I-2-a-6

### Beispiel 8

### Wirkungssteigerung durch Ammonium- / Phosphoniumsalze in Kombination mit Penetrationsförderern

**Myzus persicae -Test**

| | | |
|---|---|---|
| Lösungsmittel: | 7 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Für die Anwendung mit Ammonium- oder Phosphoniumsalzen und Penetrationsförderer (Rapsölmethylester 500 EW) werden diese jeweils in einerKonzen-tration von 1000 ppm der Spritzbrühe hinzugegeben.

Paprikapflanzen (*Capsicum annuum*), die stark von der Grünen Pfirsichblattlaus (*Myzus persicae*) befallen sind, werden durch Tropfnassspritzung mit der Wirkstoffzubereitung in der gewünschten Konzentration behandelt. Nach der gewünschten Zeit wird die Abtötung in % bestimmt. Dabei bedeutet 100 %, dass alle Tiere abgetötet wurden; 0 % bedeutet, dass keine Tiere abgetötet wurden.

**Tabelle**

| Wirkstoff | Konzentration / ppm | Abtötungsgrad / % nach 6 Tagen | | | |
|---|---|---|---|---|---|
| | | | + AS (1000 ppm) | + RME (1000 ppm) | + RME + AS (je 1000 ppm) |
| I-1-a-25 | 0.8 | 0 | 0 | 40 | 55 |

### Beispiel 9

**Aphis gossypii -Test**

| | | |
|---|---|---|
| Lösungsmittel: | 7 | Gewichtsteile Dimethylformamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Ammonium- oder Phosphoniumsalzen und Penetrationsförderern (Rapsölmethylestern 500 EW) werden diese jeweils in einerKonzentration von 1000 ppm der Spritzbrühe hinzugegeben.

Baumwollpflanzen (*Gossypium hirsutum*), die stark von der Baumwollblattlaus (*Aphis gossypii*) befallen sind, werden durch Tropfnassspritzung mit der Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

**Tabelle**

| Wirkstoff | Konzentration /ppm | Abtötungsgrad / % nach 6 Tagen | | | |
|---|---|---|---|---|---|
| | | | + AS (1000 ppm) | + RME (1000 ppm) | + RME + AS (je 1000 ppm) |
| I-1-a-25 | 0.8 | 0 | 35 | 20 | 85 |
| I-1-a-29 | 4 | 10 | 15 | 80 | 95 |
| I-1-a-31 | 4 | 0 | 10 | 5 | 35 |

### Beispiel 10

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von ca. 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Alopecurus myosuroides, Echinocloa crus-galli, Lolium multiflorum und Setaria viridis eine Wirkung von ≥ 80 %: I-1-a-6, I-1-a-7, I-1-a-9, I-1-a-12, I-1-a-13, I-1-a-14, I-1-a-16, I-1-a-19, I-1-a-22, I-1-a-33, I-1-a-34, I-1-a-35, I-1-a-37, I-1-a-38, I-1-a-39, I-1-a-40, I-1-a-41, I-1-b-2, I-1-c-2, I-1-c-3, I-1-c-6, I-2-a-5.

Folgende Verbindungen zeigen neben den zuvor genannten Verbindungen im Vorauflauf mit 80 g/ha a.i. gegen Alopecurus myosuroides, Echinocloa crus-galli, Lolium multiflorum und Setaria viridis eine Wirkung von ≥ 80 %: I-1-a-1, I-1-a-2, I-1-a-3, I-1-a-4, I-1-a-5, I-1-a-8, I-1-a-17, I-1-a-18, I-1-a-21, I-1-a-25, I-1-b-1, I-1-c-1, I-1-c-4, I-1-c-5.

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Neben den zuvor genannten Verbindungen zeigen folgende Verbindungen im Nachauflauf mit 80 g/ha gegen Alopecurus myosuroides, Avena fatua, Echinocloa crus-galli, Lolium multiflorum, Setaria viridis und Veronica persica eine Wirkung von ≥ 80%: I-1-a-4, I-1-a-5, I-1-a-6, I-1-a-8, I-1-a-17, I-1-a-18, I-1-a-19, I-1-a-25, I-1-a-26, I-1-a-34, I-1-a-39, I-1-b-1, I-1-b-2, I-1-c-4, I-1-c-5, I-2-a-5.

Neben den zuvor genannten Verbindungen zeigen folgende Verbindungen im Nachauflauf mit 80 g/ha gegen Alopecurus myosuroides, Avena fatua, Echinocloa crus-galli, Lolium multiflorum und Setaria viridis eine Wirkung von ≥ 80%: I-1-a-1, I-1-a-3, I-1-a-7, I-1-a-9, I-1-a-14, I-1-a-35, I-1-a-37, I-1-a-38, I-1-a-40, I-1-a-41, I-1-c-1, I-8-a-1, I-8-c-1, I-8-c-3, I-8-c-5.

### Verwendung von Safenern:

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

### Gefäßversuche mit Getreide im Gewächshaus

### Mefenpyr 1 Tag vor Herbizidapplikation

| | | 28 Tage nach Applikation | 28 Tage nach Applikation |
|---|---|---|---|
| | Aufwandmenge g a.i./ha | Sommergerste beobachtet (%) | Sommerweizen beobachtet (%) |
| Bsp. (I-1-a-5) | | | |
| | 25 | 95 | |
| | 12,5 | 65 | 65 |
| Bsp. (I-1-a-5) | 25 + 50 | 75 | |
| +Mefenpyr | 12,5 + 50 | 35 | 25 |

| | | 28 Tage nach Applikation |
|---|---|---|
| | Aufwandmenge g a.i./ha | Sommerweizen beobachtet (%) |
| Bsp. (I-1-a-6) | | |
| | 25 | 85 |
| | 12,5 | 70 |
| Bsp. (I-1-a-6) | 25 + 50 | 60 |
| +Mefenpyr | 12,5 + 50 | 50 |

| | | 28 Tage nach Applikation |
|---|---|---|
| | Aufwandmenge g a.i./ha | Sommerweizen beobachtet (%) |
| Bsp. (I-1-a-7) | | |
| | 100 | 70 |
| | 50 | 70 |
| | 25 | 50 |
| | 12,5 | 50 |
| Bsp. (I-1-a-7) | 100 + 50 | 30 |
| +Mefenpyr | 50 + 50 | 20 |
| | 25 + 50 | 20 |
| | 12,5 + 50 | 10 |

| | | 10 Tag nach Applikation | 28 Tage nach Applikation |
|---|---|---|---|
| | Aufwandmenge g a.i./ha | Sommerweizen beobachtet (%) | Sommerweizen beobachtet (%) |
| Bsp. (I-1-a-8) | 12,5 | 65 | 98 |
| Bsp. (I-1-a-8) +Mefenpyr | 12,5 + 50 | 50 | 50 |

| | | 28 Tage nach Applikation |
|---|---|---|
| | Aufwandmenge g a.i./ha | Sommerweizen beobachtet (%) |
| Bsp. (I-1-a-14) | | |
| | 50 | 100 |
| | 25 | 99 |
| | 12,5 | 93 |
| Bsp. (I-1-a-14) | 50 + 50 | 85 |
| +Mefenpyr | 25 + 50 | 85 |
| | 12,5 + 50 | 40 |

| | | 28 Tage nach Applikation | 28 Tage nach Applikation |
|---|---|---|---|
| | Aufwandmenge g a.i./ha | Sommergerste beobachtet (%) | Sommerweizen beobachtet (%) |
| Bsp. (I-1-a-15) | | | |
| | 100 | | 60 |
| | 50 | 97 | 40 |
| | 25 | 85 | 30 |
| | 12,5 | 70 | 10 |
| Bsp. (I-1-a-15) | 100 + 50 | | 20 |
| +Mefenpyr | 50 + 50 | 80 | 10 |
| | 25 + 50 | 80 | 5 |
| | 12,5 + 50 | 20 | 0 |

| | | 28 Tage nach Applikation |
|---|---|---|
| | Aufwandmenge g a.i./ha | Sommergerste beobachtet (%) |
| Bsp. (I-1-a-17) | | |
| | 200 | 95 |
| | 100 | 80 |
| | 50 | 40 |
| Bsp. (I-1-a-17) | 200 + 50 | 60 |
| +Mefenpyr | 100 + 50 | 60 |
| | 50 + 50 | 30 |

| | | 28 Tage nach Applikation | 28 Tage nach Applikation |
|---|---|---|---|
| | Aufwandmenge g a.i./ha | Sommergerste beobachtet (%) | Sommerweizen beobachtet (%) |
| Bsp. (I-1-a-19) | | | |
| | 100 | | 85 |
| | 50 | | 70 |
| | 25 | 70 | 20 |
| | 12,5 | | 10 |
| Bsp. (I-1-a-19) | 100 + 50 | | 70 |
| +Mefenpyr | 50 + 50 | | 50 |
| | 25 + 50 | 40 | 20 |
| | 12,5 + 50 | | 0 |

| | | 10 Tage nach Applikation | 28 Tage nach Applikation |
|---|---|---|---|
| | Aufwandmenge g a.i./ha | Sommerweizen beobachtet (%) | Sommerweizen beobachtet (%) |
| Bsp. (I-1-a-25) | 12,5 | 40 | 70 |
| Bsp. (I-1-a-25) +Mefenpyr | 12,5 + 50 | 25 | 30 |

| | 10 Tage nach Applikation | |
|---|---|---|
| | Aufwandmenge g a.i./ha | Sommergerste beobachtet (%) |
| Bsp. (I-1-b-2) | 12,5 | 50 |
| | | |
| Bsp. (I-1-b-2) +Mefenpyr | 12,5 + 50 | 20 |

| | 28 Tage nach Applikation | |
|---|---|---|
| | Aufwandmenge g a.i./ha | Sommerweizen beobachtet (%) |
| Bsp. (I-1-a-37) | 12,5 | 70 |
| | | |
| Bsp. (I-1-a-37) +Mefenpyr | 12,5 + 50 | 15 |

### Gefäßversuche mit Mais-Soja-Baumwolle im Gewächshaus

### Cyprosulfamide 1 Tag vor Herbizidapplikation

| | | 28 Tage nach Applikation |
|---|---|---|
| | Aufwandmenge g a.i./ha | Mais (Aventura) beobachtet (%) |
| Bsp. (I-1-a-8) | | |
| | 50 | 70 |
| | 25 | 60 |
| | 12,5 | 35 |
| Bsp. (I-1-a-8) | 50 + 200 | 40 |
| +Cyprosulfamide | 25 + 200 | 35 |
| | 12,5 + 200 | 15 |

| | | 28 Tage nach Applikation | 28 Tage nach Applikation |
|---|---|---|---|
| | Aufwandmenge g a.i./ha | Mais (Arsenal) beobachtet (%) | Mais (Cecilia) beobachtet (%) |
| Bsp. (I-1-a-25) | | | |
| | 100 | 100 | 35 |
| | 50 | 75 | 20 |
| | 25 | 15 | 10 |
| | 12,5 | 5 | 0 |
| Bsp. (I-1-a-25) | 100 + 100 | 70 | 10 |
| +Cyprosulfamide | 50 + 100 | 50 | 0 |
| | 25 + 100 | 0 | 0 |
| | 12,5 + 100 | 0 | 0 |

| | | 28 Tage nach Applikation | 28 Tage nach Applikation |
|---|---|---|---|
| | Aufwandmenge g a.i./ha | Mais (Arsenal) beobachtet (%) | Mais (Cecilia) beobachtet (%) |
| Bsp. (I-1-c-2) | | | |
| | 100 | 25 | 75 |
| | 50 | 20 | |
| | 25 | 20 | |
| Bsp. (I-1-c-2) | 100 + 200 | 15 | 25 |
| +Cyprosulfamide | 50 + 200 | 0 | |
| | 25 + 200 | 0 | |

## Patentansprüche

1. Verbindungen der Formel (I) 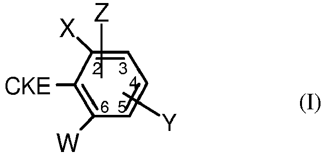 in welcher
W für Wasserstoff, Alkyl, Halogen, Halogenalkyl, Alkoxy oder Halogenalkoxy steht,
X für Alkyl, Alkenyl, Alkinyl, Halogen, Alkoxy, Halogenalkyl, Halogenalkoxy oder Cyano steht,
Y für Wasserstoff, Alkyl, Alkoxy oder Halogen steht
Z für eine Gruppe 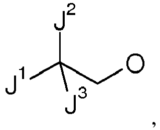
in welcher J¹ und J² jeweils unabhängig voneinander für Wasserstoff oder Halogen stehen und J³ für Halogen oder eine Halogenalkylgruppe steht
CKE für eine der Gruppen 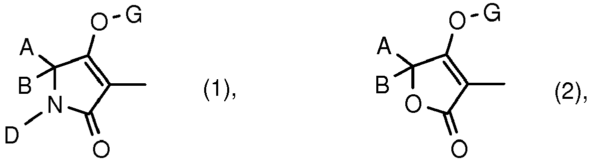 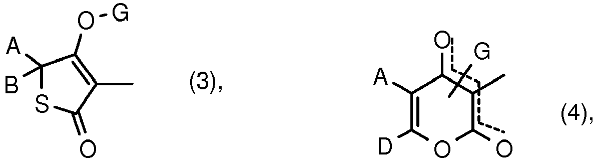 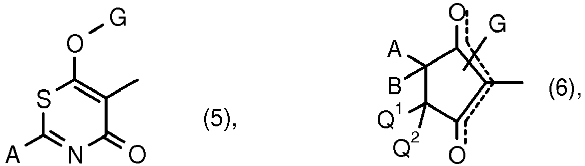 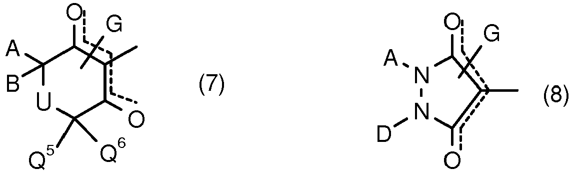 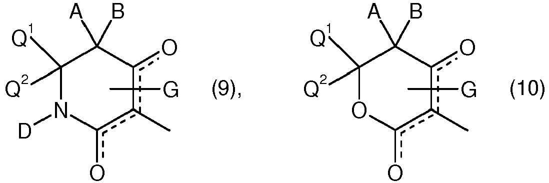
oder 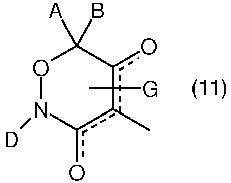 steht, worin
U für -S-, -S(O)-, -S(O)₂-, -O-, 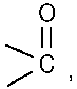 eine S=N-, S(O)=N- oder 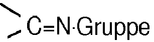 oder für gegebenenfalls durch Q³ und Q⁴ substituiertes C₁-C₄-Alkylen steht, welches gegebenenfalls durch Sauerstoff unterbrochen sein kann,
A für Wasserstoff, für jeweils gegebenenfalls durch Halogen substituiertes Alkyl, Alkenyl, Alkoxyalkyl, Alkylthioalkyl, für gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff, Alkyl oder Alkoxyalkyl steht, oder
A und B gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
D für Wasserstoff oder für einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome ersetzt sind, für jeweils gegebenenfalls substituiertes Arylalkyl, Aryl, Hetarylalkyl oder Hetaryl steht oder
A und D gemeinsam mit den Atomen an die sie gebunden sind für einen gesättigten oder ungesättigten und gegebenenfalls mindestens ein (im Falle CKE=8 und 11 ein weiteres) Heteroatom enthaltenden, im A,D-Teil unsubstituierten oder substituierten Cyclus stehen, oder
A und Q¹ gemeinsam für jeweils gegebenenfalls substituiertes Alkandiyl oder Alkendiyl stehen, welche gegebenenfalls durch mindestens ein Heteroatom, eine 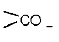 oder substituierte 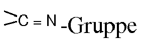 unterbrochen sein können oder
B und Q² gemeinsam mit den Atomen, an die sie gebunden sind, für einen gesättigten oder ungesättigten und gegebenenfalls mindestens ein Heteroatom enthaltenden, im B, Q²-Teil unsubstituierten oder substituierten Cyclus stehen, oder
D und Q¹ gemeinsam mit den Atomen, an die sie gebunden sind, für einen gesättigten oder ungesättigten und gegebenenfalls mindestens ein Heteroatom enthaltenden, im D, Q¹-Teil unsubstituierten oder substituierten Cyclus stehen,
Q¹ für Wasserstoff, Alkyl, Alkoxyalkyl, für gegebenenfalls substituiertes Cycloalkyl, worin gegebenenfalls eine Methylengruppe durch Sauerstoff oder Schwefel ersetzt ist oder für gegebenenfalls substituiertes Phenyl steht,
Q², Q⁴, Q⁵ und Q⁶ unabhängig voneinander für Wasserstoff oder Alkyl stehen,
Q³ für Wasserstoff, für jeweils gegebenenfalls substituiertes Alkyl, Alkoxy, Alkylthio, Alkoxyalkyl, Alkylthioalkyl, für gegebenenfalls substituiertes Cycloalkyl, worin gegebenenfalls eine oder zwei Methylengruppen durch Sauerstoff oder Schwefel ersetzt sind oder für gegebenenfalls substituiertes Phenyl steht, oder
Q¹ und Q² gemeinsam mit dem Kohlenstoffatom, an das sie gebunden sind, für einen gegebenenfalls ein Heteroatom enthaltenden unsubstituierten oder substituierten Cyclus stehen, oder
Q³ und Q⁴ gemeinsam mit dem Kohlenstoffatom, an das sie gebunden sind, für einen gesättigten oder ungesättigten, gegebenenfalls mindestens ein Heteroatom enthaltenden unsubstituierten oder substituierten Cyclus stehen, oder
A und Q³ gemeinsam mit dem Kohlenstoffatom, an das sie gebunden sind, für einen gesättigten oder ungesättigten, gegebenenfalls mindestens ein Heteroatom enthaltenden unsubstituierten oder substituierten Cyclus stehen, oder
A und Q⁵ gemeinsam mit dem Kohlenstoffatom, an das sie gebunden sind, für einen gesättigten oder ungesättigten, gegebenenfalls mindestens ein Heteroatom enthaltenden unsubstituierten oder substituierten Cyclus stehen,
G für Wasserstoff (a) oder für eine der Gruppen 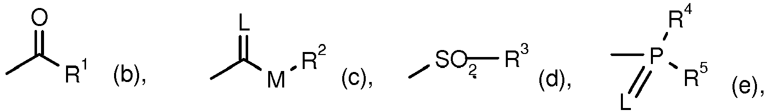
E (f) oder 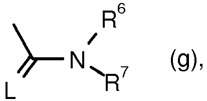 steht,
worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder für gegebenenfalls durch Halogen, Alkyl oder Alkoxy substituiertes Cycloalkyl, das durch mindestens ein Heteroatom unterbrochen sein kann, für jeweils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Benzyl, Phenoxy oder Phenylthio stehen,
R⁶ und R⁷ unabhängig voneinander für Wasserstoff, für jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Cyclus stehen.

2. Verbindungen der Formel (I) gemäß Anspruch 1, in welcher
W für Wasserstoff, C₁-C₆-Alkyl, Halogen, C₁-C₆-Alkoxy, C₁-C₄-Halogenalkyl oder C₁-C₄-Halogenalkoxy steht,
X für Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy oder Cyano steht,
Y für Wasserstoff, Halogen, C₁-C₆-Alkyl, oder C₁-C₆-Alkoxy steht,
Z für eine Gruppe 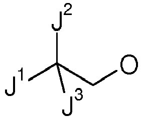
steht, in welcher J¹ und J² jeweils unabhängig voneinander für Wasserstoff, Fluor oder Chlor stehen und J³ für Halogen oder C₁-C₄-Halogenalkyl steht,
CKE für eine der Gruppen 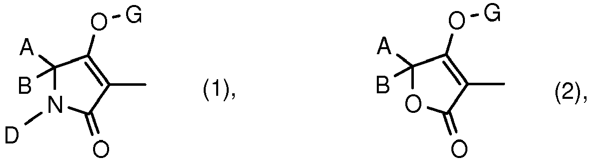 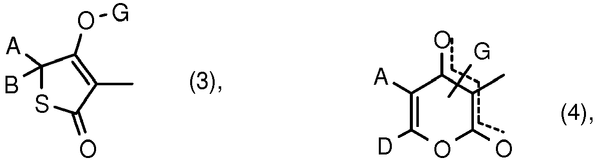 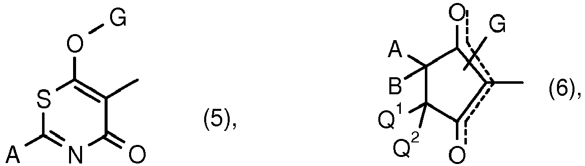 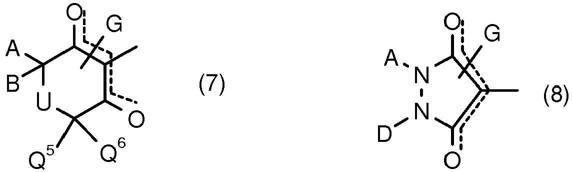 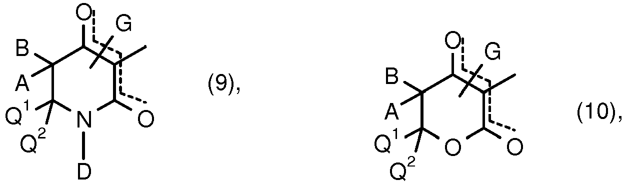
oder 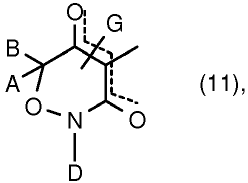 steht,
U für -S-, -S(O)-, -S(O)₂-, -O-, 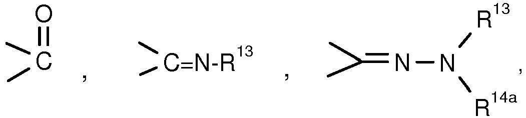
S=N-R¹³, S(O)=N-R¹³ oder 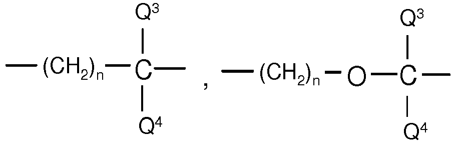 steht,
in welcher n für die Zahl 0, 1 oder 2 steht,
A für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Hetaryl mit 5 bis 6 Ringatomen, Phenyl-C₁-C₆-alkyl oder Naphthyl-C₁-C₆-alkyl steht,
B für Wasserstoff, C₁-C₁₂-Alkyl oder C₁-C₈-Alkoxy-C₁-C₆-alkyl steht oder
A, B und das Kohlenstoffatom an das sie gebunden sind, für gesättigtes C₃-C₁₀-Cycloalkyl oder ungesättigtes C₅-C₁₀-Cycloalkyl, worin gegebenenfalls ein Ringglied durch Stickstoff, Sauerstoff oder Schwefel ersetzt ist und welche gegebenenfalls einfach oder zweifach durch C₁-C₈-Alkyl, C₁-C₈-Alkoxy, C₃-C₈-Alkenyloxy, C₁-C₆-Alkoxy-C₁-C₆-Alkyl, C₃-C₆-Cycloalkyl-C₁-C₂-alkoxy, C₃-C₁₀-Cycloalkyl, C₁-C₈-Halogenalkyl, C₂-C₆-Halogenalkoxy, C₁-C₆-Alkoxy-C₁-C₄-alkoxy substituiert sind, wobei die zuvor genannten Reste auch als N-Substituenten in Frage kommen, oder
A, B und das Kohlenstoffatom, an das sie gebunden sind, für C₃-C₆-Cycloalkyl, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl- oder durch eine Alkylendioxy- oder durch eine Alkylendithioyl-Gruppe substituiert ist, die mit dem Kohlenstoffatom, an das sie gebunden ist, einen weiteren fünfbis achtgliedrigen Ring bildet oder
A, B und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
D für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₁-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen, Phenyl-C₁-C₆-alkyl oder Hetaryl-C₁-C₆-alkyl mit 5 oder 6 Ringatomen steht oder
A und D gemeinsam für jeweils gegebenenfalls substituiertes C₃-C₆-Alkandiyl oder C₃-C₆-Alkendiyl stehen, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder Schwefel enthalten kann, oder worin gegebenenfalls eine der folgenden Gruppen 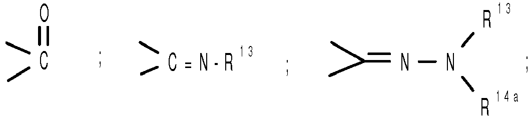 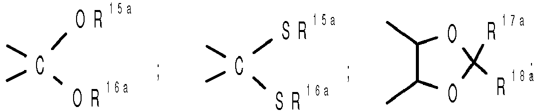 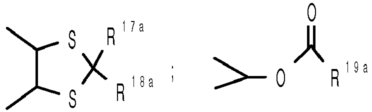
oder 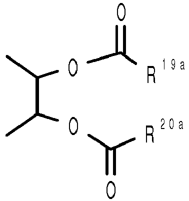 enthalten ist, oder
A und Q¹ gemeinsam mit den Kohlenstoffatomen, an die sie gebunden sind, für jeweils gegebenenfalls einfach oder zweifach, gleich oder verschieden durch Halogen, Hydroxy, durch jeweils gegebenenfalls einfach bis dreifach, gleich oder verschieden durch Halogen substituiertes C₁-C₁₀-Alkyl, C₁-C₈-Alkenyl, C₁-C₆-Alkoxy, C₁-C₆-Alkylthio, C₃-C₇-Cycloalkyl oder durch jeweils gegebenenfalls einfach bis dreifach, gleich oder verschieden durch Halogen, C₁-C₆-Alkyl oder C₁-C₆-Alkoxy substituiertes Benzyloxy oder Phenyl substituiertes C₃-C₆-Alkandiyl oder C₄-C₆-Alkendiyl stehen, welches außerdem gegebenenfalls eine der nachstehenden Gruppen 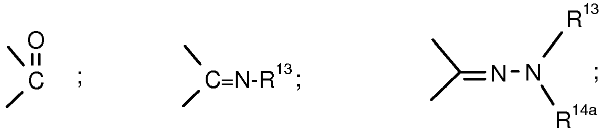 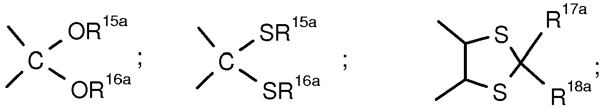 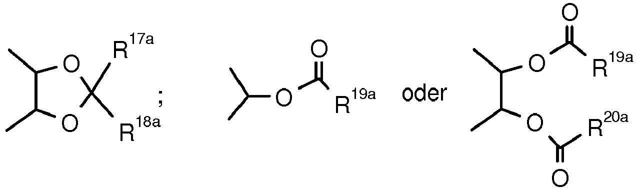 enthält oder durch eine C₁-C₂-Alkandiylgruppe oder durch ein Sauerstoffatom überbrückt ist oder
B und Q² gemeinsam für gegebenenfalls durch C₁-C₂-Alkyl substituiertes C₁-C₃-Alkandiyl stehen, welches gegebenenfalls durch Sauerstoff unterbrochen sein kann, oder
D und Q¹ gemeinsam für gegebenenfalls einfach oder zweifach, gleich oder verschieden durch C₁-C₄-Alkyl, C₁-C₄-Alkoxy substituiertes C₃-C₆-Alkandiyl stehen, oder
Q¹ für Wasserstoff, C₁-C₆-Alkyl, C₁-C₆-Alkoxy-C₁-C₂-alkyl, gegebenenfalls durch Fluor, Chlor, C₁-C₄-Alkyl, C₁-C₂-Halogenalkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl steht,
Q², Q⁴, Q⁵ und Q⁶ unabhängig voneinander für Wasserstoff oder C₁-C₄-Alkyl stehen,
Q³ für Wasserstoff, C₁-C₆-Alkyl, C₁-C₆-Alkoxy, C₁-C₆-Alkylthio, C₁-C₆-Alkoxy-C₁-C₂-alkyl, C₁-C₆-Alkylthio-C₁-C₂-alkyl, gegebenenfalls durch C₁-C₄-Alkyl oder C₁-C₄-Alkoxy substituiertes C₃-C₈-Cycloalkyl, worin gegebenenfalls eine oder zwei Methylengruppen durch Sauerstoff oder Schwefel ersetzt sind oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl steht, oder
Q¹ und Q² mit dem Kohlenstoffatom, an das sie gebunden sind, für gegebenenfalls durch C₁-C₆-Alkyl, C₁-C₆-Alkoxy oder C₁-C₂-Halogenalkyl substituierten C₃-C₇-Ring stehen, in welchem gegebenenfalls ein Ringglied durch Sauerstoff oder Schwefel ersetzt ist,
Q³ und Q⁴ gemeinsam mit dem Kohlenstoffatom, an das sie gebunden sind, für einen gegebenenfalls durch C₁-C₄-Alkyl, C₁-C₄-Alkoxy oder C₁-C₂-Halogenalkyl substituierten gesättigten oder ungesättigten C₃-C₇-Ring stehen, in welchem gegebenenfalls ein oder zwei Ringglieder durch Sauerstoff oder Schwefel ersetzt sind,
A und Q³ gemeinsam mit den Kohlenstoffatomen, an die sie gebunden sind, für einen gesättigten oder ungesättigten, gegebenenfalls durch C₁-C₄-Alkyl, C₁-C₄-Alkoxy oder
C₁-C₂-Halogenalkyl substituierten C₃-C₇-Ring stehen, in welchem gegebenenfalls ein oder zwei Ringglieder durch Sauerstoff oder Schwefel ersetzt sind,
A und Q⁵ gemeinsam mit den Kohlenstoffatomen, an die sie gebunden sind, für einen gesättigten oder ungesättigten, gegebenenfalls durch C₁-C₄-Alkyl, C₁-C₄-Alkoxy oder C₁-C₂-Halogenalkyl substituierten C₃-C₇-Ring stehen, in welchem gegebenenfalls ein Ringglied durch Sauerstoff oder Schwefel ersetzt ist,
G für Wasserstoff (a) oder für eine der Gruppen 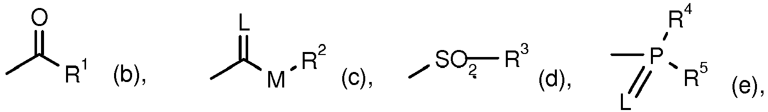
E (f) oder 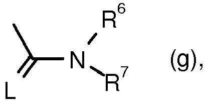 steht, in welchen
E für ein Metallionäquivalent oder ein Ammoniumion steht,
L für Sauerstoff oder Schwefel steht und
M für Sauerstoff oder Schwefel steht.
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest stehen, in welchem gegebenenfalls ein Kohlenstoffatom durch Sauerstoff oder Schwefel ersetzt ist,
R¹³ für Wasserstoff, für jeweils gegebenenfalls durch Halogen substituiertes C₁-C₈-Alkyl oder C₁-C₈-Alkoxy (nur im Fall der C=N-R¹³-Gruppe), für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl, Hetaryl-C₁-C₄-alkyl, oder nur im Fall der C=N-R¹³-Gruppe für Phenyl-C₁-C₄-alkoxy oder Hetaryl-C₁-C₄-alkoxy steht,
R^{14a} für Wasserstoff oder C₁-C₈-Alkyl steht oder
R¹³ und R^{14a} gemeinsam für gegebenenfalls durch C₁-C₄-Alkyl substituiertes C₄-C₆-Alkandiyl stehen, welches gegebenenfalls durch Sauerstoff oder Schwefel unterbrochen sein kann,
R^{15a} und R^{16a} gleich oder verschieden sind und für C₁-C₆-Alkyl stehen oder
R^{15a} und R^{16a} gemeinsam für einen C₂-C₄-Alkandiylrest oder C₄-Alkandiylrest stehen, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist,
R^{17a} und R^{18a} unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl stehen oder
R^{17a} und R^{18a} gemeinsam mit dem Kohlenstoffatom, an das sie gebunden sind, für eine Carbonylgruppe oder für gegebenenfalls durch Halogen, C₁-C₄-Alkyl oder C₁-C₄-Alkoxy substituiertes C₅-C₇-Cycloalkyl stehen, in dem gegebenenfalls eine Methylengruppe durch Sauerstoff oder Schwefel ersetzt ist,
R^{19a} und R^{20a} unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino stehen.

3. Verbindungen der Formel (I) gemäß Anspruch 1, in welcher
W für Wasserstoff, Fluor, Chlor, Brom, C₁-C₄-Alkyl, C₁-C₄-Alkoxy, C₁-C₂-Halogenalkyl oder C₁-C₂-Halogenalkoxy steht,
X für Chlor, Brom, Iod, C₁-C₄-Alkyl, C₂-C₄-Alkenyl, C₂-C₄-Alkinyl, C₁-C₄-Alkoxy, C₁-C₂-Halogenalkyl, C₁-C₂-Halogenalkoxy oder Cyano steht,
Y für Wasserstoff, Methyl, Ethyl, Fluor, Chlor, Brom, Iod, Methoxy oder Ethoxy steht,
Z für die Gruppe 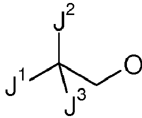
steht, in welcher J¹ und J² jeweils unabhängig voneinander für Wasserstoff, Fluor oder Chlor stehen und J³ für Fluor, Chlor, Trichlormethyl, Difluormethyl, Difluorchlormethyl, Dichlorfluormethyl oder Trifluormethyl steht,
CKE für eine der Gruppen 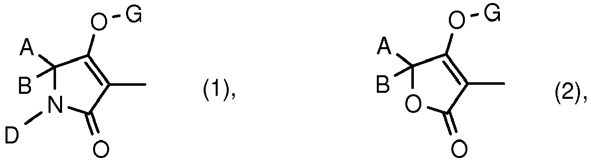 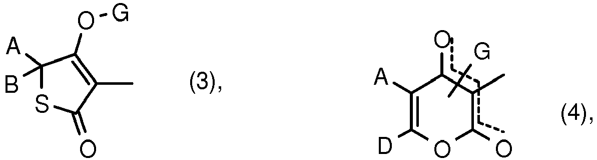 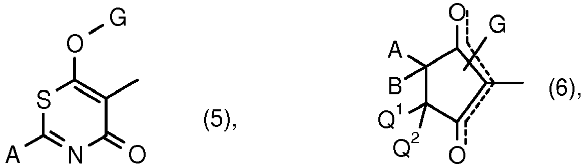 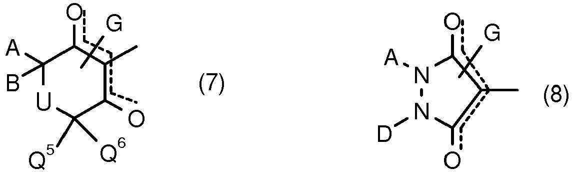 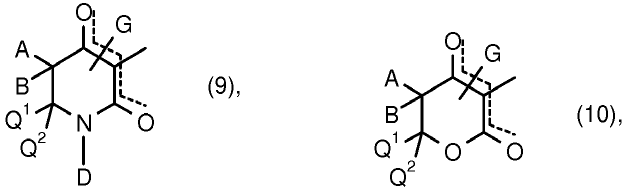 oder 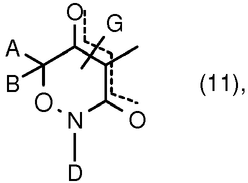 steht,
U für -CH₂-, -CH₂-CH₂-, -O- oder 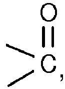 steht,
A für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes und gegebenenfalls durch ein Sauerstoffatom unterbrochenes C₃-C₆-Cycloalkyl oder (jedoch nicht im Fall der Verbindungen der Formeln (I-3), (I-4), (I-6), (I-7), (I-9), (I-10) und (I-11)) jeweils gegebenenfalls einfach bis zweifach durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl, Pyridyl oder Benzyl steht,
B für Wasserstoff, C₁-C₄-Alkyl oder C₁-C₂-Alkoxy-C₁-C₂-alkyl steht oder
A, B und das Kohlenstoffatom an das sie gebunden sind, für gesättigtes oder ungesättigtes C₃-C₇-Cycloalkyl, worin gegebenenfalls ein Ringglied durch Stickstoff, Sauerstoff oder Schwefel ersetzt ist und welches gegebenenfalls einfach bis zweifach durch C₁-C₆-Alkyl, C₁-C₄-Alkoxy-C₁-C₂-alkyl, Trifluormethyl, C₁-C₆-Alkoxy, C₃-C₆-Alkenyloxy, Trifluorethoxy, C₁-C₃-Alkoxy-C₁-C₃-alkoxy oder C₃-C₆-Cycloalkylmethoxy substituiert ist, wobei die zuvor genannten Reste auch als N-Substituenten in Frage kommen, mit der Maßgabe, dass dann Q³ für Wasserstoff oder Methyl steht oder
A, B und das Kohlenstoffatom, an das sie gebunden sind, für C₅-C₆-Cycloalkyl, welches durch eine gegebenenfalls ein oder zwei nicht direkt benachbarte Sauerstoff- oder Schwefelatome enthaltende gegebenenfalls durch Methyl oder Ethyl substituierte Alkylendiyl- oder durch eine Alkylendioxy- oder durch eine Alkylendithiol-Gruppe substituiert ist, die mit dem Kohlenstoffatom, an das sie gebunden ist, einen weiteren fünf- oder sechsgliedrigen Ring bildet mit der Maßgabe, dass dann Q³ für Wasserstoff oder Methyl steht, oder
A, B und das Kohlenstoffatom, an das sie gebunden sind, für C₃-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen, mit der Maßgabe, dass dann Q³ für Wasserstoff oder Methyl steht,
D für Wasserstoff, für jeweils gegebenenfalls einfach bis dreifach durch Fluor substituiertes C₁-C₆-Alkyl, C₃-C₆-Alkenyl, C₁-C₄-Alkoxy-C₁-C₃-alkyl, für gegebenenfalls einfach bis zweifach durch C₁-C₄-Alkyl, C₁-C₄-Alkoxy oder C₁-C₂-Halogenalkyl substituiertes C₃-C₆-Cycloalkyl, in welchem gegebenenfalls eine Methylengruppe durch Sauerstoff ersetzt ist oder (nur im Fall der Verbindungen der Formel (I-4)) für jeweils gegebenenfalls einfach bis zweifach durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl oder Pyridyl steht, oder
A und D gemeinsam für gegebenenfalls einfach bis zweifach substituiertes C₃-C₅-Alkandiyl stehen, in welchem eine Methylengruppe durch eine Carbonylgruppe (nicht jedoch im Fall der Verbindungen der Formel (I-11)), Sauerstoff oder Schwefel ersetzt sein kann, wobei als Substituenten C₁-C₂-Alkyl oder C₁-C₂-Alkoxy in Frage kommen oder
A und D (im Fall der Verbindungen der Formel (I-1)) gemeinsam mit den Atomen, an die sie gebunden sind, für eine der Gruppen AD-1 bis AD-10 stehen: 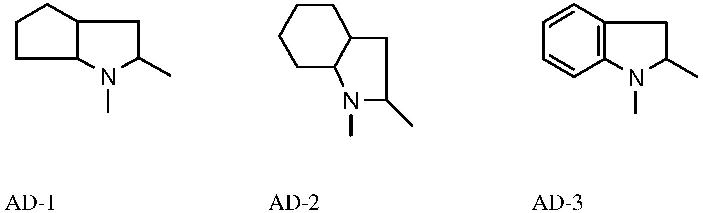 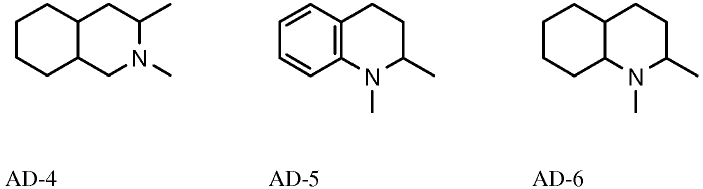 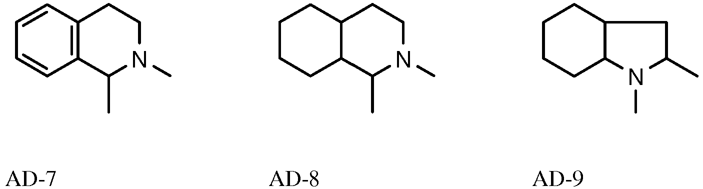 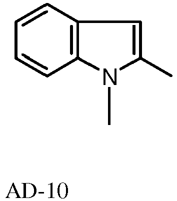
oder
A und D gemeinsam für C₃-C₅-Alkandiyl stehen, welches gegebenenfalls durch eine zwei nicht direkt benachbarten Sauerstoffatomen enthaltende gegebenenfalls einfach bis vierfach durch C₁-C₄-Alkyl oder C₁-C₃-Alkoxy-C₁-C₂-alkyl substituierte Alkylendioxy-Gruppe substituiert ist, wobei ein weiterer 5- oder 6-Ring gebildet wird, oder
A und Q¹ gemeinsam für gegebenenfalls einfach oder zweifach, gleich oder verschieden durch C₁-C₂-Alkyl oder C₁-C₂-Alkoxy substituiertes C₃-C₄-Alkandiyl stehen, welches gegebenenfalls die nachfolgende Gruppe enthält: 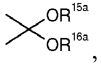
in welcher
R^{15a} und R^{16a} gleich oder verschieden für Methyl oder Ethyl stehen, oder
R^{15a} und R^{16a} gemeinsam für einen C₂-C₄-Alkandiyl- oder C₄-Alkendiylrest stehen, der gegebenenfalls durch Methyl oder Ethyl substituiert ist, oder
B und Q² gemeinsam für -CH₂-, -CH₂-CH₂- -CH₂-CH₂-CH₂-, oder -CH₂-O-CH₂- stehen oder
D und Q¹ gemeinsam für C₃-C₄-Alkandiyl stehen, oder
Q¹ für Wasserstoff, C₁-C₄-Alkyl, C₁-C₄-Alkoxy-C₁-C₂-alkyl, oder gegebenenfalls durch Methyl oder Methoxy substituiertes C₃-C₆-Cycloalkyl steht, worin gegebenenfalls eine Methylengruppe durch Sauerstoff ersetzt ist,
Q² für Wasserstoff, Methyl oder Ethyl steht,
Q⁴ Q⁵ und Q⁶ unabhängig voneinander für Wasserstoff oder C₁-C₃-Alkyl stehen,
Q³ für Wasserstoff, C₁-C₄-Alkyl, C₁-C₄-Alkoxy, oder für gegebenenfalls einfach bis zweifach durch Methyl oder Methoxy substituiertes gegebenenfalls durch ein Sauerstoffatom unterbrochenes C₃-C₆-Cycloalkyl steht, oder
Q¹ und Q² mit dem Kohlenstoff, an das sie gebunden sind, für gegebenenfalls durch Methyl oder Methoxy substituiertes C₃-C₆-Cycloalkyl stehen, worin gegebenenfalls eine Methylengruppe durch Sauerstoff ersetzt ist, mit der Maßgabe, dass dann A und B unabhängig voneinander für Wasserstoff oder Methyl stehen, oder
Q³ und Q⁴ gemeinsam mit dem Kohlenstoff, an das sie gebunden sind, für einen gegebenenfalls durch C₁-C₂-Alkyl oder C₁-C₂-Alkoxy substituierten gesättigten C₅-C₆-Ring stehen, in welchem gegebenenfalls ein oder zwei Ringglieder durch Sauerstoff oder Schwefel ersetzt sind, mit der Maßgabe, dass dann A für Wasserstoff oder Methyl steht, oder
A und Q³ gemeinsam mit dem Kohlenstoff, an das sie gebunden sind, für einen gegebenenfalls durch C₁-C₂-Alkyl oder C₁-C₂-Alkoxy substituierten gesättigten C₅-C₆-Ring stehen, in welchem gegebenenfalls ein Ringglied durch Sauerstoff oder Schwefel ersetzt ist, mit der Maßgabe, dass dann B, Q⁴, Q⁵ und Q⁶ unabhängig voneinander für Wasserstoff oder Methyl stehen, oder
A und Q⁵ gemeinsam mit den Kohlenstoffatomen, an die sie gebunden sind, für einen gegebenenfalls durch C₁-C₂-Alkyl oder C₁-C₂-Alkoxy substituierten gesättigten oder ungesättigten C₅-C₆-Ring stehen, mit der Maßgabe, dass dann B, Q³, Q⁴ und Q⁶ unabhängig voneinenander für Wasserstoff oder Methyl stehen,
G für Wasserstoff (a) oder für eine der Gruppen 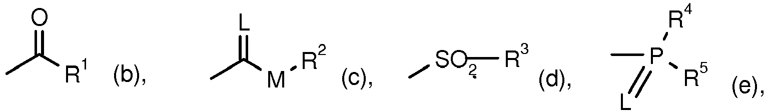
E (f) oder 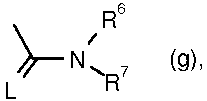 steht, in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₈-Alkyl, C₂-C₈-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenenfalls ein oder zwei nicht direkt benachbarte Ringglieder durch Sauerstoff ersetzt sind,
für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl steht,
R² für jeweils gegebenenfalls einfach bis dreifach durch Fluor substituiertes C₁-C₈-Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl steht,
R³ für gegebenenfalls einfach bis dreifach durch Fluor substituiertes C₁-C₆-Alkyl oder für gegebenenfalls einfach durch Fluor, Chlor, Brom, C₁-C₄-Alkyl, C₁-C₄-Alkoxy, Trifluormethyl, Trifluormethoxy, Cyano oder Nitro substituiertes Phenyl steht,
R⁴ für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder Trifluormethyl substituiertes Phenyl, Phenoxy oder Phenylthio steht,
R⁵ für C₁-C₆-Alkoxy oder C₁-C₆-Alkylthio steht,
R⁶ für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phenyl, für gegebenenfalls einfach durch Fluor, Chlor, Brom, C₁-C₄-Alkyl, Trifluormethyl oder C₁-C₄-Alkoxy substituiertes Benzyl steht,
R⁷ für C₁-C₆-Alkyl, C₃-C₆-Alkenyl oder C₁-C₆-Alkoxy-C₁-C₄-alkyl steht, R⁶ und R⁷ zusammen für einen gegebenenfalls durch Methyl oder Ethyl substituierten C₄-C₅-Alkylenrest stehen, in welchem gegebenenfalls eine Methylengruppe durch Sauerstoff oder Schwefel ersetzt ist.

4. Verbindungen der Formel (I) gemäß Anspruch 1, in welcher
W für Wasserstoff, Chlor, Methyl oder Ethyl steht,
X für Chlor, Methyl, Ethyl, Methoxy oder Ethoxy steht,
Y für Wasserstoff, Methyl oder Chlor steht,
Z für die Gruppe 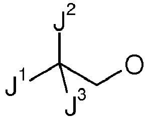
steht, in welcher J¹ und J² jeweils unabhängig für Wasserstoff oder Fluor stehen und J³ für Fluor, Chlor oder Trifluormethyl steht,
CKE für eine der Gruppen 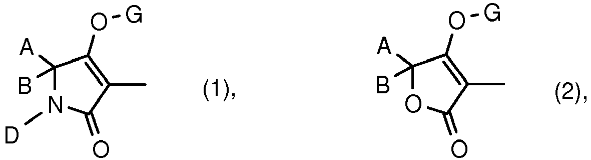 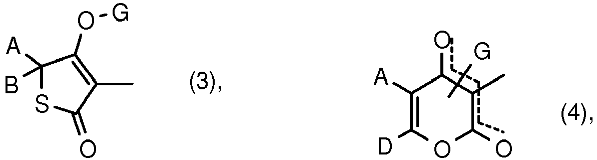 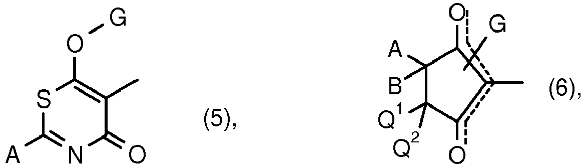 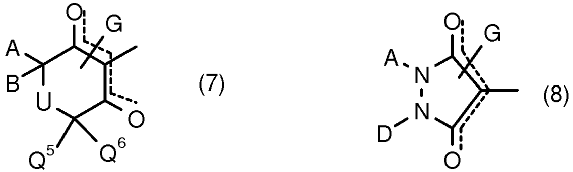 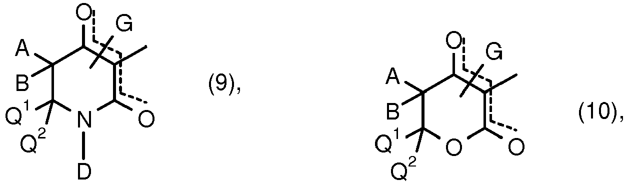 oder 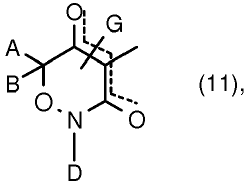 steht, U für -CH₂-, -CH₂-CH₂-, -O- oder 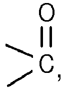 steht,
A für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl und im Fall der Verbindungen der Formel (I-5) für gegebenenfalls einfach bis zweifach durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl steht,
B für Wasserstoff, Methyl oder Ethyl steht oder
A, B und das Kohlenstoffatom an das sie gebunden sind, für gesättigtes C₅-C₆-Cycloalkyl, in welchem gegebenenfalls ein Ringglied durch Stickstoff, Sauerstoff oder Schwefel ersetzt ist und welches gegebenenfalls einfach oder zweifach durch Methyl, Ethyl, Methoxymethyl, Ethoxymethyl, Methoxyethyl, Ethoxyethyl, Trifluormethyl, Methoxy, Ethoxy, Propoxy, Butoxy, Methoxyethoxy, Ethoxyethoxy, Allyloxy, Trifluorethoxy oder Cyclopropylmethoxy substituiert ist, wobei die zuvor genannten Reste auch als N-Substituenten in Frage kommen, mit der Maßgabe, dass dann Q³ für Wasserstoff steht oder
A, B und das Kohlenstoffatom, an das sie gebunden sind für C₆-Cycloalkyl, welches gegebenenfalls durch eine gegebenenfalls durch ein Sauerstoffatom unterbrochene Alkylidendiyl-Gruppe oder durch eine mit zwei nicht direkt benachbarten Sauerstoffatomen enthaltende Alkylendioxy-Gruppe substituiert ist, wobei ein weiter 5- oder 6-Ring gebildet wird (der einfach oder zweifach durch Methyl substituiert sein kann) mit der Maßgabe, dass dann Q³ für Wasserstoff steht oder
A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlenstoffatomen, an die sie gebunden sind, für C₂-C₄-Alkandiyl oder C₂-C₄-Alkendiyl oder Butadiendiyl stehen, mit der Maßgabe, dass dann Q³ für Wasserstoff steht,
D für Wasserstoff, für jeweils gegebenenfalls einfach bis dreifach durch Fluor substituiertes C₁-C₄-Alkyl, C₃-C₄-Alkenyl, C₁-C₄-Alkoxy-C₁-C₃-alkyl, für Cyclopropyl, Cyclopentyl oder Cyclohexyl oder (im Fall der Verbindungen der Formel (I-4)) für jeweils gegebenenfalls einfach durch Fluor, Chlor, Methyl, Ethyl, n-Propyl, iso-Propyl, Methoxy, Ethoxy oder Trifluormethyl substituiertes Phenyl oder Pyridyl steht, oder
A und D gemeinsam für gegebenenfalls einfach durch Methyl oder Methoxy substituiertes C₃-C₅-Alkandiyl stehen, worin gegebenenfalls ein Kohlenstoffatom durch eine Carbonylgruppe (nicht jedoch im Falle der Verbindungen der Formel (I-11)), Sauerstoff oder Schwefel ersetzt ist oder für die Gruppe AD-1 steht, oder
A und D gemeinsam für C₃-C₅-Alkandiyl stehen, welches gegebenenfalls durch eine mit zwei nicht direkt benachbarten Sauerstoffatomen enthaltende gegebenenfalls einfach bis zweifach durch C₁-C₂-Alkyl substituierte Alkylendioxy-Gruppe substituiert ist, wobei ein weiterer 5- Ring gebildet wird, oder
A und Q¹ gemeinsam für gegebenenfalls einfach oder zweifach durch Methyl oder Methoxy substituiertes C₃-C₄-Alkandiyl stehen, welches gegebenenfalls die nachfolgende Gruppe enthält: 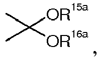
in welcher R^{15a} und R^{16a} gemeinsam für einen C₂-C₄-Alkandiyl- oder C₄-Alkendiylrest stehen, oder
B und Q² gemeinsam für -CH₂-CH₂-CH₂-, oder -CH₂-O-CH₂- stehen oder
D und Q¹ gemeinsam für C₃-C₄-Alkandiyl stehen oder
Q¹ für Wasserstoff, Methyl, Ethyl, Propyl, iso-Propyl, Cyclopropyl, Cyclopentyl oder Cyclohexyl steht,
Q² für Wasserstoff, Methyl oder Ethyl steht,
Q⁴ Q⁵ und Q⁶ unabhängig voneinander für Wasserstoff oder Methyl stehen,
Q³ für Wasserstoff, Methyl, Ethyl, Propyl, Methoxy oder Ethoxy, oder für gegebenenfalls einfach durch Methyl oder Methoxy substituiertes gegebenenfalls durch ein Sauerstoffatom unterbrochenes C₃-C₆-Cycloalkyl steht, oder
Q¹ und Q² mit dem Kohlenstoffatom, an das sie gebunden sind, für gegebenenfalls durch Methyl oder Methoxy substituiertes C₅-C₆-Cycloalkyl stehen, worin gegebenenfalls eine Methylengruppe durch Sauerstoff ersetzt ist, mit der Maßgabe, dass A und B für Wasserstoff stehen, oder
Q³ und Q⁴ gemeinsam mit dem Kohlenstoff, an den sie gebunden sind, für einen gegebenenfalls einfach durch Methyl oder Methoxy substituierten gegebenenfalls durch ein Sauerstoffatom unterbrochenen, gesättigten C₅-C₆-Ring stehen, mit der Maßgabe, dass dann A, B, Q⁵ und Q⁶ für Wasserstoff stehen,
G für Wasserstoff (a) oder für eine der Gruppen 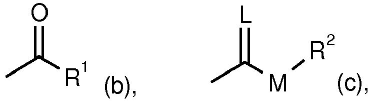
-SO₂-R³ (d) oder E (f) steht,
in welchen
L für Sauerstoff oder Schwefel steht,
M für Sauerstoff oder Schwefel steht und
E für ein Metallionäquivalent oder ein Ammoniumion steht,
R¹ für jeweils gegebenenfalls einfach durch Chlor substituiertes C₁-C₆-Alkyl, C₂-C₆-Alkenyl, C₁-C₂-Alkoxy-C₁-alkyl, C₁-C₂-Alkylthio-C₁-alkyl oder jeweils gegebenenfalls einfach durch Fluor, Chlor, Methyl oder Methoxy substituiertes Cyclopropyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl steht,
R² für jeweils gegebenenfalls einfach durch Fluor substituiertes C₁-C₈-Alkyl, C₂-C₆-Alkenyl oder C₁-C₄-Alkoxy-C₂-C₃-alkyl, Phenyl oder Benzyl steht,
R³ für C₁-C₈-Alkyl steht.

5. Verbindungen der Formel (I) gemäß Anspruch 1, in welcher
W für Wasserstoff, Methyl oder Ethyl steht,
X für Chlor, Methyl oder Ethyl steht,
Y für Wasserstoff steht,
Z für OCH₂-CF₃ in der 3-Position steht, oder
Z für OCH₂-CF₃ in der 4-Position steht, oder
Z für OCH₂-CF₃ in der 5-Position steht,
CKE für eine der Gruppen 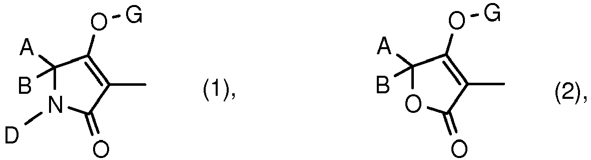 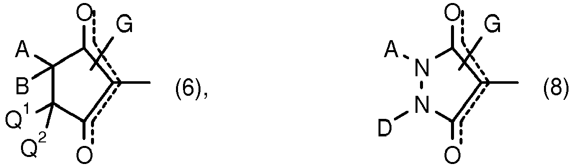
steht,
A für Methyl oder Ethyl steht,
B für Wasserstoff oder Methyl steht,
A, B und das Kohlenstoffatom an das sie gebunden sind, für gesättigtes C₅-C₆-Cycloalkyl, in welchem gegebenenfalls ein Ringglied durch Sauerstoff ersetzt ist und welches gegebenenfalls einfach oder zweifach durch Methyl, Ethyl, Methoxymethyl, Methoxy, Ethoxy, Propoxy, Butoxy, Trifluorethoxy substituiert ist, oder
A, B und das Kohlenstoffatom, an das sie gebunden sind, für C₆-Cycloalkyl stehen,
welches gegebenenfalls durch eine mit zwei nicht direkt benachbarten Sauerstoffatomen enthaltende Alkylendioxy-Gruppe substituiert ist, wobei ein weiterer 5- oder 6-Ring gebildet wird, der einfach oder zweifach durch Methyl substituiert sein kann,
D für Wasserstoff steht, oder
A und D gemeinsam für C₃-C₅-Alkandiyl stehen, worin gegebenenfalls ein Kohlenstoffatom durch Sauerstoff ersetzt ist, oder
A und D gemeinsam für C₃-C₅-Alkandiyl stehen, welches gegebenenfalls durch eine mit zwei nicht direkt benachbarten Sauerstoffatomen enthaltende gegebenenfalls einfach bis zweifach durch Methyl substituierte Alkylendioxy-Gruppe substituiert ist, wobei ein weiterer 5- Ring gebildet wird, oder
A und Q¹ gemeinsam für C₃-C₄-Alkandiyl stehen,
Q² für Wasserstoff steht,
G für Wasserstoff (a) oder für eine der Gruppen 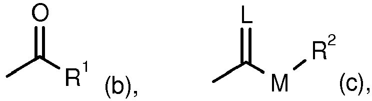
steht, in welchen
L für Sauerstoff steht,
M für Sauerstoff steht,
R¹ für C₁-C₆-Alkyl steht,
R² für C₁-C₆-Alkyl steht.

6. Verfahren zur Herstellung von Verbindungen der Formel (I) gemäß Anspruch 1, **dadurch gekennzeichnet, dass** man zum Erhalt von
(A) Verbindungen der Formel (I-1-a) 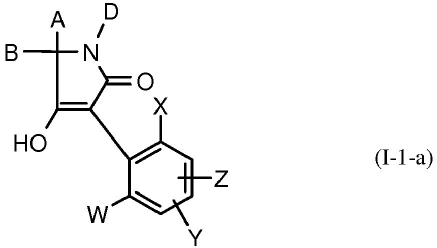 in welcher
A, B, D, W, X, Y und Z die oben angegebenen Bedeutungen haben,
Verbindungen der Formel (II) 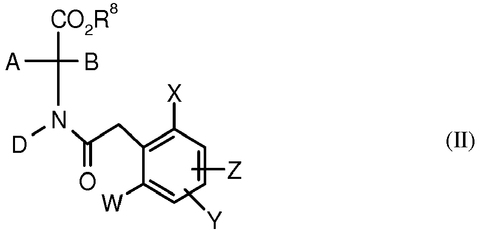 in welcher
A, B, D, W, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 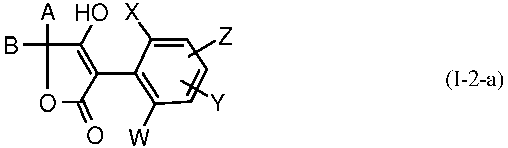 in welcher
A, B, W, X, Y und Z die oben angegebenen Bedeutungen haben,
Verbindungen der Formel (III) 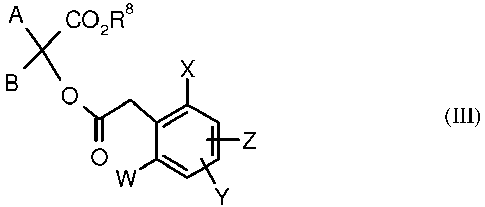 in welcher
A, B, W, X, Y, Z und R⁸ die oben angegebenen Bedeutungen haben,
in Gegenwart eines Verdünnungsmittels und in Gegenwart einer Base intramolekular kondensiert,
(C) Verbindungen der Formel (I-3-a) 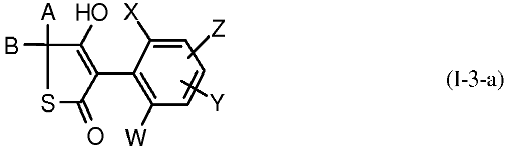 in welcher
A, B, W, X, Y und Z die oben angegebenen Bedeutungen haben,
Verbindungen der Formel (IV) 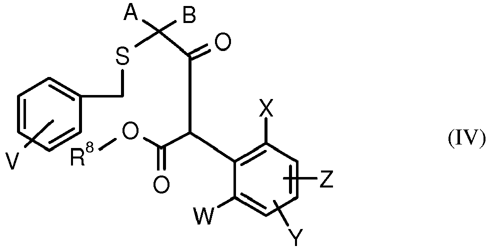 in welcher
A, B, W, X, Y, Z und R⁸ die oben angegebenen Bedeutungen haben und
V für Wasserstoff, Halogen, Alkyl oder Alkoxy steht,
gegebenenfalls in Gegenwart eines Verdünnungsmittels und in Gegenwart einer Säure intramolekular cyclisiert,
(D) Verbindungen der Formel (I-4-a) 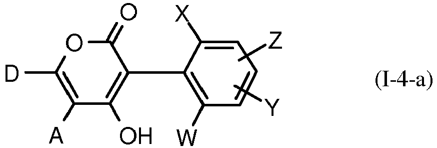 in welcher
A, D, W, X, Y und Z die oben angegebenen Bedeutungen haben,
Verbindungen der Formel (V) 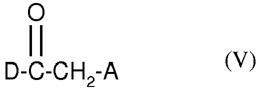 in welcher
A und D die oben angegebenen Bedeutungen haben,
oder deren Silylenolether der Formel (Va) 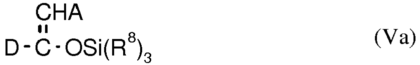 in welcher
A, D und R⁸ die oben angegebene Bedeutung haben,
mit Verbindungen der Formel (VI) 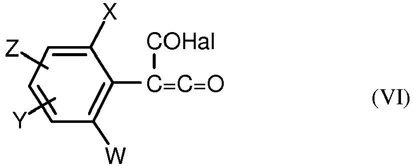 in welcher
W, X, Y und Z die oben angegebenen Bedeutungen haben und
Hal für Halogen steht,
gegebenenfalls in Gegenwart eines Verdünnungsmittels und gegebenenfalls in Gegenwart eines Säureakzeptors umsetzt,
(E) Verbindungen der Formel (I-5-a) 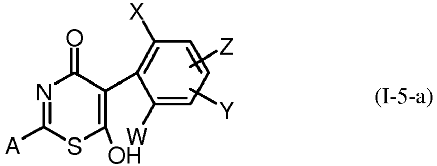 in welcher
A,W, X, Y und Z die oben angegebene Bedeutung haben,
Verbindungen der Formel (VII) 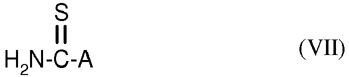 in welcher
A die oben angegebene Bedeutung hat,
mit Verbindungen der Formel (VI) 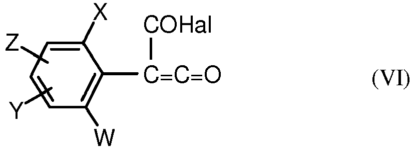 in welcher
Hal, W, X, Y und Z die oben angegebenen Bedeutungen haben,
gegebenenfalls in Gegenwart eines Verdünnungsmittels und gegebenenfalls in Gegenwart eines Säureakzeptors umsetzt,
(F) Verbindungen der Formel (I-6-a) 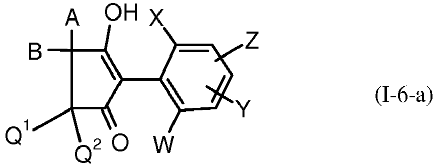 in welcher
A, B, Q¹, Q², W, X, Y und Z die oben angegebene Bedeutung haben,
Verbindungen der Formel (VIII) 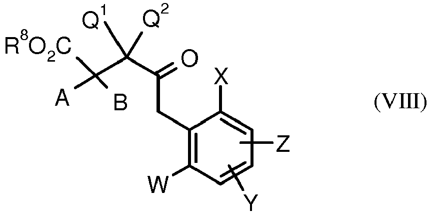 in welcher
A, B, Q¹, Q², W, X, Y und Z die oben angegebene Bedeutung haben, und
R⁸ für Alkyl steht,
gegebenenfalls in Gegenwart eines Verdünnungsmittels und in Gegenwart einer Base intramolekular cyclisiert,
(G) Verbindungen der Formel (I-7-a) 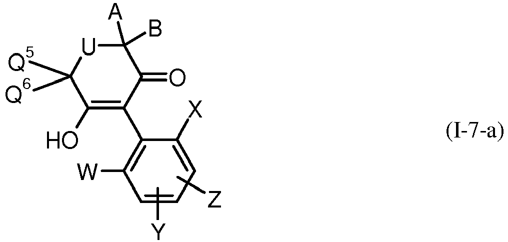 in welcher
A, B, Q⁵, Q⁶, U, W, X, Y und Z die oben angegebene Bedeutung haben,
Verbindungen der Formel (IX) 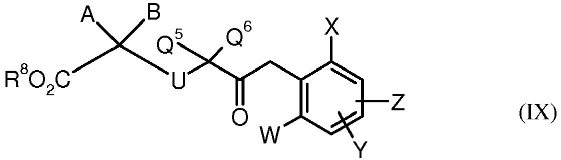 in welcher
A, B, Q⁵, Q⁶, U, W, X, Y und Z die oben angegebene Bedeutung haben und
R⁸ für Alkyl steht,
in Gegenwart eines Verdünnungsmittels und in Gegenwart einer Base intramolekular kondensiert,
(H) Verbindungen der Formel (I-8-a) 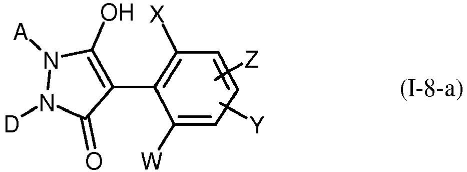 in welcher
A, D, W, X, Y und Z die oben angegebenen Bedeutungen haben,
Verbindungen der Formel (X) 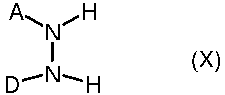 in welcher
A und D die oben angegebene Bedeutung haben,
α) mit Verbindungen der Formel (VI) 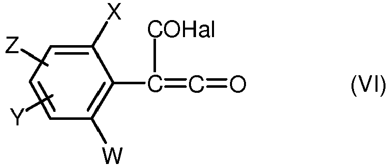 in welcher
Hal, W, X, Y und Z die oben angegebenen Bedeutungen haben,
gegebenenfalls in Gegenwart eines Verdünnungsmittels und gegebenenfalls in Gegenwart eines Säureakzeptors umsetzt, oder
β) mit Verbindungen der Formel (XI) 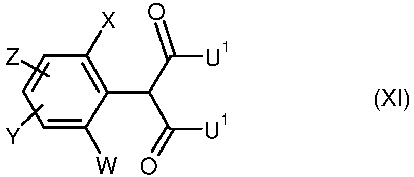 in welcher
W, X, Y und Z die oben angegebene Bedeutung haben,
und U¹ für NH₂ oder O-R⁸ steht, wobei R⁸ die oben genannte Bedeutung hat,
gegebenenfalls in Gegenwart eines Verdünnungsmittels und gegebenenfalls in Gegenwart einer Base umsetzt, oder
γ) mit Verbindungen der Formel (XII) 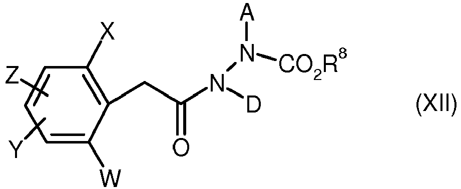 in welcher
A, D, W, X, Y, Z und R⁸ die oben angegebene Bedeutung haben,
gegebenenfalls in Gegenwart eines Verdünnungsmittels und gegebenenfalls in Gegenwart einer Base umsetzt,
(I) Verbindungen der Formel (I-9-a) 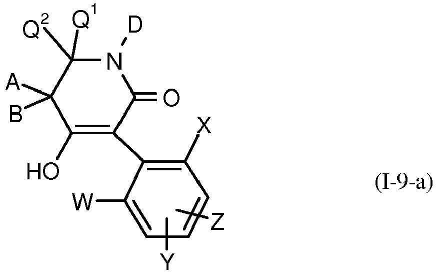 in welcher
A, B, D, Q¹, Q², W, X, Y und Z die oben angegebenen Bedeutungen haben,
Verbindungen der Formel (XIII) 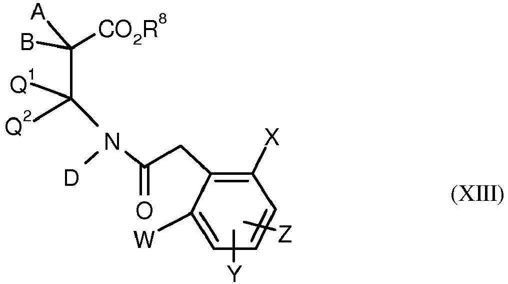 in welcher
A, B, D, Q¹, Q², W, X, Y und Z die oben angegebenen Bedeutungen haben,
und
R⁸ für Alkyl steht,
in Gegenwart eines Verdünnungsmittels und in Gegenwart einer Base intramolekular kondensiert,
(J) Verbindungen der Formel (I-10-a) 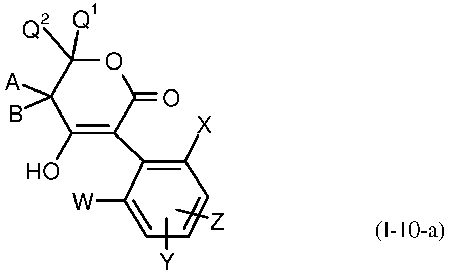 in welcher
A, B, Q¹, Q², W, X, Y und Z die oben angegebene Bedeutungen haben,
Verbindungen der Formel (XIV) 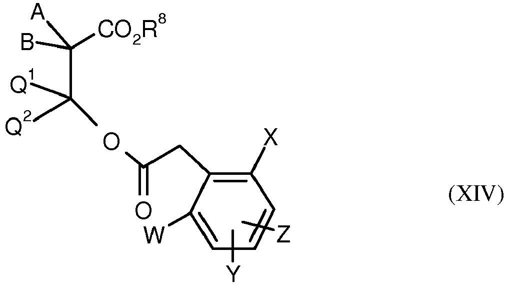 in welcher
A, B, Q¹, Q², W, X, Y und Z die oben angegebenen Bedeutungen haben,
und
R⁸ für Alkyl steht,
gegebenenfalls in Gegenwart eines Verdünnungsmittels und gegebenenfalls in Gegenwart einer Base umsetzt,
(K) Verbindungen der Formel (I-11-a) 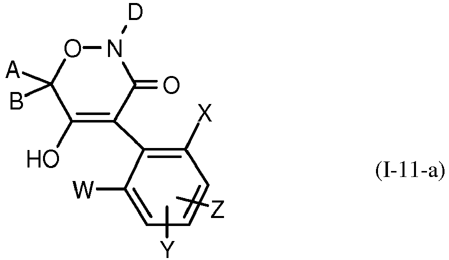 in welcher
A, B, D, W, X, Y und Z die oben angegebenen Bedeutungen haben,
Verbindungen der Formel (XV) 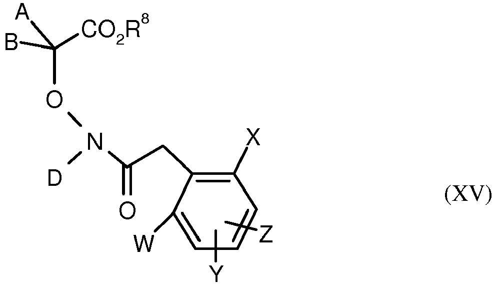 in welcher
A, B, D, W, X, Y und Z die oben angegebenen Bedeutungen haben,
und
R⁸ für Alkyl steht,
in Gegenwart eines Verdünnungsmittels und in Gegenwart einer Base intramolekular kondensiert,
(L) Verbindungen der oben gezeigten Formeln (I-1-b) bis (I-11-b), in welchen A, B, D, Q¹, Q², Q⁵, Q⁶ R¹, U, W, X, Y und Z die oben angegebenen Bedeutungen haben, Verbindungen der oben gezeigten Formeln (1-1-a) bis (I-11-a), in welchen A, B, D, Q¹, Q², Q⁵, Q⁶, U, W, X, Y und Z die oben angegebenen Bedeutungen haben, jeweils
(α) mit Säurehalogeniden der Formel (XVI) 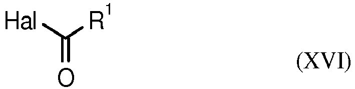 in welcher
R¹ die oben angegebene Bedeutung hat und
Hal für Halogen steht
oder
(β) mit Carbonsäureanhydriden der Formel (XVII)
R¹-CO-O-CO-R¹ (XVII)
in welcher
R¹ die oben angegebene Bedeutung hat,
gegebenenfalls in Gegenwart eines Verdünnungsmittels und gegebenenfalls in Gegenwart eines Säurebindemittels umsetzt;
(M) Verbindungen der oben gezeigten Formeln (I-1-c) bis (I-11-c), in welchen A, B, D, Q¹, Q², Q⁵, Q⁶, R², M, U, W, X, Y und Z die oben angegebenen Bedeutungen haben und L für Sauerstoff steht, Verbindungen der oben gezeigten Formeln (I-1-a) bis (I-11-a), in welchen A, B, D, Q¹, Q², Q⁵, Q⁶, U, W, X, Y und Z die oben angegebenen Bedeutungen haben, jeweils
mit Chlorameisensäureestern oder Chlorameisensäurethiolestern der Formel (XVIII)
R²-M-CO-Cl (XVIII)
in welcher R² und M die oben angegebenen Bedeutungen haben,
gegebenenfalls in Gegenwart eines Verdünnungsmittels und gegebenenfalls in Gegenwart eines Säurebindemittels umsetzt;
(N) Verbindungen der oben gezeigten Formeln (I-1-c) bis (I-11-c), in welchen A, B, D, Q¹, Q², Q⁵, Q⁶, R², M, U, W, X, Y und Z die oben angegebenen Bedeutungen haben und L für Schwefel steht, Verbindungen der oben gezeigten Formeln (I-1-a) bis (I-11-a), in welchen A, B, D, Q¹, Q⁴, Q⁵, Q⁶, U, W, X, Y und Z die oben angegebenen Bedeutungen haben, jeweils
mit Chlormonothioameisensäureestern oder Chlordithioameisensäureestern der Formel (XIX) 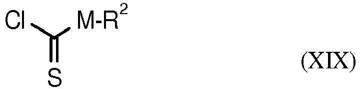 in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(O) Verbindungen der oben gezeigten Formeln (I-1-d) bis (I-11-d), in welchen A, B, D, Q¹, Q², Q⁵, Q⁶, R³, U, W, X, Y und Z die oben angegebenen Bedeutungen haben, Verbindungen der oben gezeigten Formeln (I-1-a) bis (I-11-a), in welchen A, B, D, Q¹, Q², Q⁵, Q⁶, U, W, X, Y und Z die oben angegebenen Bedeutungen haben, jeweils
mit Sulfonsäurechloriden der Formel (XX)
R³-SO₂-Cl (XX)
in welcher
R³ die oben angegebene Bedeutung hat,
gegebenenfalls in Gegenwart eines Verdünnungsmittels und gegebenenfalls in Gegenwart eines Säurebindemittels umsetzt,
(P) Verbindungen der oben gezeigten Formeln (I-1-e) bis (I-11-e), in welchen A, B, D, L, Q¹, Q², Q⁵, Q⁶, R⁴, R⁵, U, W, X, Y und Z die oben angegebenen Bedeutungen haben, Verbindungen der oben gezeigten Formeln (1-1-a) bis (I-11-a), in welchen A, B, D, Q¹, Q², Q⁵, Q⁶, U, W, X, Y und Z die oben angegebenen Bedeutungen haben, jeweils
mit Phosphorverbindungen der Formel (XXI) 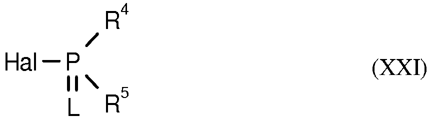 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(Q) Verbindungen der oben gezeigten Formeln (I-1-f) bis (I-11-f), in welchen A, B, D, E, Q¹, Q², Q⁵, Q⁶, U, W, X, Y und Z die oben angegebenen Bedeutungen haben, Verbindungen der Formeln (I-1-a) bis (I-11-a), in welchen A, B, D, Q¹, Q², Q⁵, Q⁶, U, W, X, Y und Z die oben angegebenen Bedeutungen haben, jeweils
mit Metallverbindungen oder Aminen der Formeln (XXII) oder (XXIII) Me(OR¹⁰)ₜ (XXII) 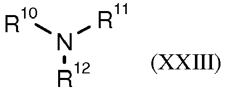 in welchen
Me für ein ein- oder zweiwertiges Metall oder für ein Ammoniumion 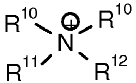
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(R) Verbindungen der oben gezeigten Formeln (I-1-g) bis (I-11-g), in welchen A, B, D, L, Q¹, Q², Q⁵, Q⁶, R⁶, R⁷, U, W, X, Y und Z die oben angegebenen Bedeutungen haben, Verbindungen der oben gezeigten Formeln (I-1-a) bis (I-11-a), in welchen A, B, D, Q¹, Q², Q⁵, Q⁶, U, W, X, Y und Z die oben angegebenen Bedeutungen haben, jeweils
(α) mit Isocyanaten oder Isothiocyanaten der Formel (XXIV)
R⁶-N=C=L (XXIV)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XXV) 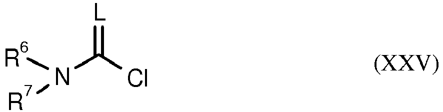 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt,
(S) Verbindungen der oben gezeigten Formeln (I-1-a) bis (I-11-g), in welchen A, B, D, G, Q¹, Q², Q⁵, Q⁶, U, W, X, Y und Z die oben angegebene Bedeutung haben, in Verbindungen der Formeln (I-1') bis (I-11'), in welchen A, B, D, G, Q¹, Q², Q¹, Q⁶, U, W, X, Y und Z die oben genannte Bedeutung haben und Z' bevorzugt für Brom oder Iod steht 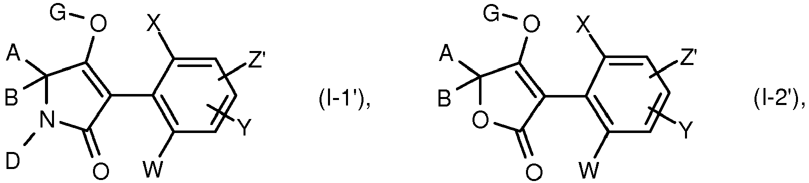 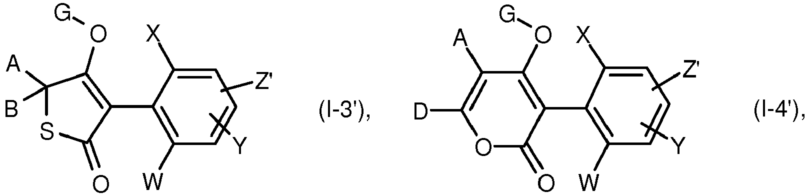 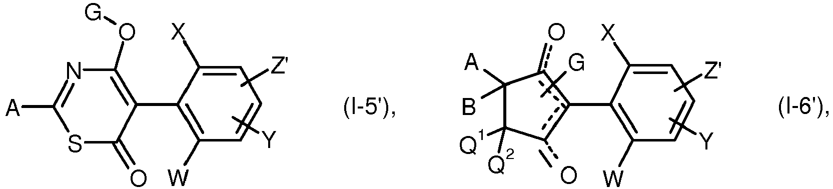 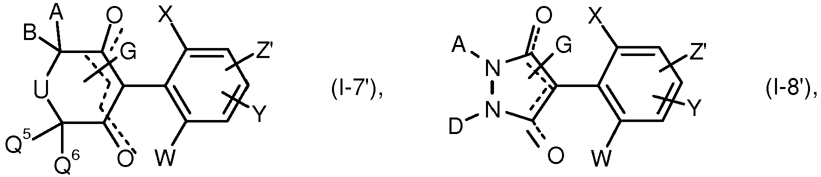 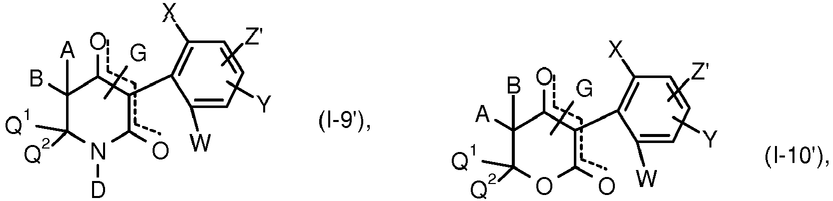 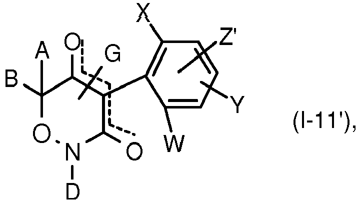 mit halogenierten Alkoholen, z.B. Trifluorethanol (XXVI)
Z-OH (XXVI)
in Gegenwart eines Lösungsmittels in Gegenwart eines Kupfersalzes und in Gegenwart einer Base das Brom- oder Jodatom austauscht.

7. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

8. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs und/oder ihren Lebensraum einwirken lässt, ausgenommen sind Verfahren zur chirurgischen oder therapeutischen Behandlung des menschlichen oder tierischen Körpers.

9. Verwendung von Verbindungen der Formel (I) gemäß Anspruch 1 zur Bekämpfung von tierischen Schädlingen und/oder unerwünschtem Pflanzenbewuchs, ausgenommen ist die Verwendung in Verfahren zur chirurgischen oder therapeutischen Behandlung des menschlichen oder tierischen Körpers.

10. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

11. Verwendung von Verbindungen der Formel (I) gemäß Anspruch 1 zur Herstellung von Mitteln zur Bekämpfung von Schädlingen und/oder unerwünschtem Pflanzenwuchs.

12. Mittel enthaltend einen wirksamen Gehalt an einer Wirkstoffkombination umfassend als Komponenten
(a') mindestens eine Verbindung der Formel (I) gemäß Anspruch 1 , in welcher W, X, Y, Z und CKE die oben angegebene Bedeutung haben
und
(b') zumindest eine die Kulturpflanzen-Verträglichkeit verbessernde Verbindung aus der folgenden Gruppe von Verbindungen: S1, S2, S3, S4, S5, S6, S7, S8, S9, S10, S11, S12, S13, S14, S15, wobei
S1) Verbindungen der Formel (S1), 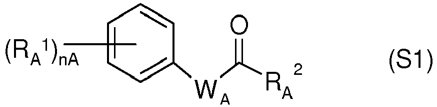 wobei die Symbole und Indizes folgende Bedeutungen haben:
n_{A} ist eine natürliche Zahl von 0 bis 5,
R_{A}¹ ist Halogen, (C₁-C₄)Alkyl, (C₁-C₄)Alkoxy, Nitro oder (C₁-C₄)Haloalkyl;
W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist,
m_{A} ist 0 oder 1;
R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist,
R_{A}³ ist Wasserstoff oder ein unsubstituierter oder substituierter aliphatischer Kohlenwasserstoffrest,
R_{A}⁴ ist Wasserstoff, (C₁-C₆)Alkyl, (C₁-C₆)Alkoxy oder substituiertes oder unsubstituiertes Phenyl;
R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
R_{A}⁶, R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
S2) Chinolinderivate der Formel (S2), 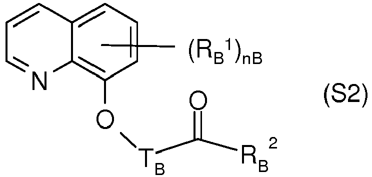 wobei die Symbole und Indizes folgende Bedeutungen haben:
R_{B}¹ ist Halogen, (C₁-C₄)Alkyl, (C₁-C₄)Alkoxy, Nitro oder (C₁-C₄)Haloalkyl;
n_{B} ist eine natürliche Zahl von 0 bis 5,
R_{B}² ist OR_{B}³, SR_{B}³ oder NR_{B}³R_{B}⁴ oder ein gesättigter
oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist,
R_{B}³ ist Wasserstoff oder ein unsubstituierter oder substituierter aliphatischer Kohlenwasserstoffrest,
R_{B}⁴ ist Wasserstoff, (C₁-C₆)Alkyl, (C₁-C₆)Alkoxy oder substituiertes oder unsubstituiertes Phenyl;
T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
S3) Verbindungen der Formel (S3) 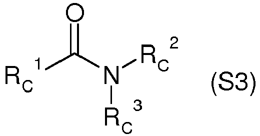 wobei die Symbole und Indizes folgende Bedeutungen haben:
R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl,
R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring,
S4) N-Acylsulfonamide der Formel (S4) und ihre Salze, 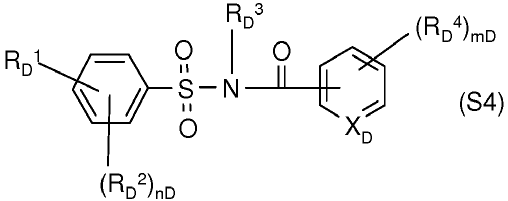 worin die Symbole und Indizes folgende Bedeutungen haben:
X_{D} ist CH oder N;
R_{D}¹ ist CO-NR_{D}⁵R_{D}⁶ oder NHCO-R_{D}⁷;
R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
R_{D}³ ist Wasserstoff, (C₁-C₄)Alkyl, (C₂-C₄)Alkenyl oder (C₂-C₄)Alkinyl;
R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
R_{D}⁶ ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
R_{D}⁵ und R_{D}⁶ gemeinsam mit dem sie tragenden Stickstoffatom einen Pyrrolidinyl- oder Piperidinyl-Rest bilden;
R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
n_{D} ist 0, 1 oder 2;
m_{D} ist 1 oder 2;
v_{D} ist 0, 1, 2 oder 3;
S5) Wirkstoffe aus der Klasse der Hydroxyaromaten und der aromatisch-aliphatischen Carbonsäurederivate (S5),
S6) Wirkstoffe aus der Klasse der 1,2-Dihydrochinoxalin-2-one (S6),
S7) Verbindungen der Formel (S7), 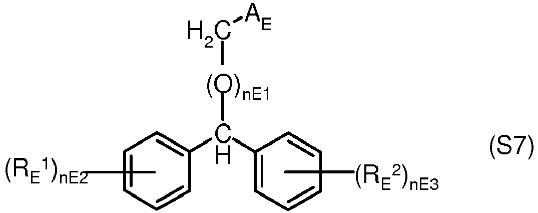 worin die Symbole und Indizes folgende Bedeutungen haben:
R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
A_{E} ist COOR_{E}³ oder COSR_{E}⁴
R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
n_{E}¹ ist 0 oder 1
n_{E}², n_{E}³ sind unabhängig voneinander 0, 1 oder 2,
S8) Verbindungen der Formel (S8),wie sie in der WO-A-98/27049 beschrieben sind 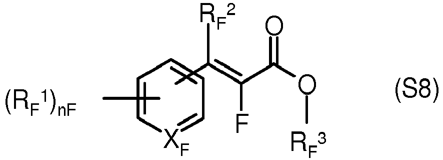 worin
X_{F} CH oder N,
n_{F} für den Fall, dass X_{F}=N ist, eine ganze Zahl von 0 bis 4 und für den Fall, dass X_{F}=CH ist, eine ganze Zahl von 0 bis 5 ,
R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
R_{F}² Wasserstoff oder (C₁-C₄)Alkyl
R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, gleiche oder verschiedene Reste aus der Gruppe, bestehend aus Halogen und Alkoxy substituiert ist;bedeuten, oder deren Salze,
S9) Wirkstoffe aus der Klasse der 3-(5-Tetrazolylcarbonyl)-2-chinolone (S9),
S10) Verbindungen der Formeln (S10^{a}) oder (S10^{b}) 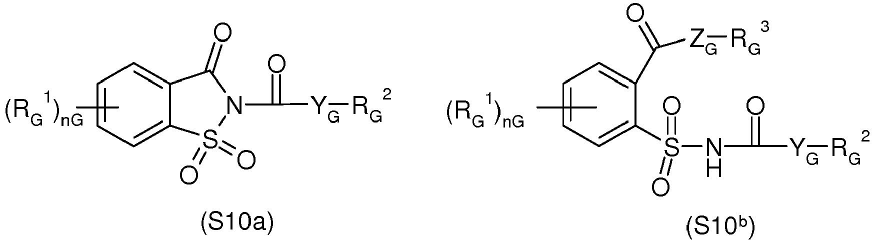 worin
R_{G}¹ Halogen, (C₁-C₄)Alkyl, Methoxy, Nitro, Cyano, CF₃, OCF₃
Y_{G}, Z_{G} unabhängig voneinander O oder S,
n_{G} eine ganze Zahl von 0 bis 4,
R_{G}² (C₁-C₁₆)Alkyl, (C₂-C₆)Alkenyl, (C₃-C₆)Cycloalkyl, Aryl; Benzyl, Halogenbenzyl,
R_{G}³ Wasserstoff oder (C₁-C₆)Alkyl bedeutet.
S11) Wirkstoffe vom Typ der Oxyimino-Verbindungen (S11), die als Saatbeizmittel bekannt sind,
S12) Wirkstoffe aus der Klasse der Isothiochromanone (S12),
S13) Eine oder mehrere Verbindungen aus Gruppe (S13):
"Naphthalic anhydrid" (1,8-Naphthalindicarbonsäureanhydrid) (S13-1),
"Fenclorim" (4,6-Dichlor-2-phenylpyrimidin) (S13-2),
"Flurazole" (Benzyl-2-chlor-4-trifluormethyl-1,3-thiazol-5-carboxylat) (S13-3),
"CL 304415" ((4-Carboxy-3,4-dihydro-2H-1-benzopyran-4-essigsäure) (S13-4) "MG 191" (2-Dichlormethyl-2-methyl-1,3-dioxolan) (S13-5)
"MG-838" (2-propenyl 1-oxa-4-azaspiro[4.5]decane-4-carbodithioate) (S13-6)
"Disulfoton" (O,O-Diethyl S-2-ethylthioethyl phosphordithioat) (S13-7),
"Dietholate" (O,O-Diethyl-O-phenylphosphorotioat) (S13-8),
"Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen,
S15) Wirkstoffe, die vorrangig als Herbizide eingesetzt werden, jedoch auch Safenerwirkung auf Kulturpflanzen aufweisen.

13. Mittel gemäß Anspruch 12, bei denen die Kulturpflanzen-Verträglichkeit verbessernde Verbindung Cyprosulfamide ist.

14. Mittel gemäß Anspruch 12, bei denen die Kulturpflanzen-Verträglichkeit verbessernde Verbindung Mefenpyr-diethyl ist.

15. Verfahren zum Bekämpfen von unerwünschtem Pflanzenwuchs, **dadurch gekennzeichnet, dass** man ein Mittel gemäß Anspruch 12 auf die Pflanzen oder ihre Umgebung einwirken lässt.

16. Verwendung eines Mittels gemäß Anspruch 12 zum Bekämpfen von unerwünschten Pflanzenwuchs.

17. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 12 in zeitlich naher Abfolge getrennt auf die Pflanzen oder ihre Umgebung einwirken lässt.

18. Verbindungen der Formel (II) 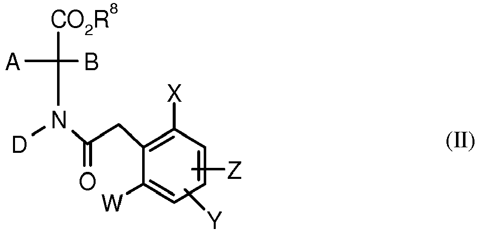 in welcher A, B, D, W, X, Y und Z die oben angegebenen Bedeutungen haben,
und R⁸ für Alkyl steht.

19. Verbindungen der Formel (III) 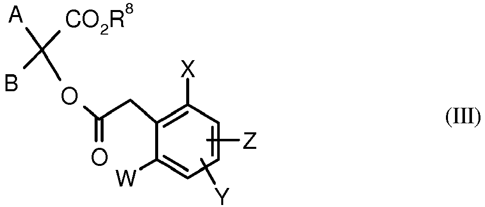 in welcher A, B, W, X, Y, Z und R⁸ die oben angegebenen Bedeutungen haben.

20. Verbindungen der Formel (VIII) 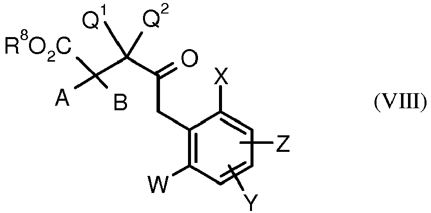 in welcher A, B, Q¹, Q², W, X, Y, Z und R⁸ die oben angegebene Bedeutung haben.

21. Verbindungen der Formel (XII) 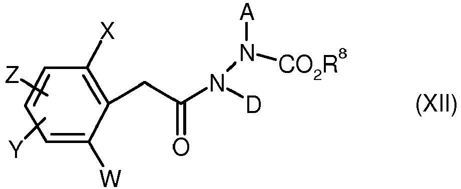 in welcher
A, D, W, X, Y, Z und R⁸ die oben angegebene Bedeutung haben.

22. Verbindungen der Formel (XXXI) 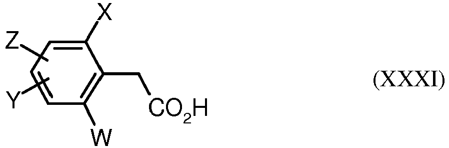 in welcher W, X, Y und Z die oben angegebene Bedeutung haben, ausgenommen die Verbindung der Formel

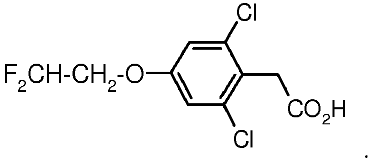

23. Verbindungen der Formel (XXVIII) 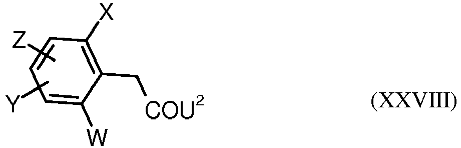 in welcher
W, X, Y, Z die oben angegebenen Bedeutungen haben und U² für eine durch Carbonsäureaktivierungsreagenzien, Carbonyldiimide, Phosphrylierungsreagenzien, Halogenierungsmittel, Phosgen oder Chlorameisensäureester eingeführte Abgangsgruppe steht.

24. Verbindungen der Formel (XXXV) 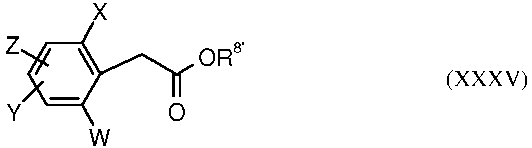 in welcher
W, X, Y, Z die oben angegebene Bedeutung haben und R^{8'} für Alkyl stehen.

25. Zusammensetzung umfassend
- mindestens eine Verbindung der Formel (I) gemäß Anspruch 1 oder ein Mittel gemäß Anspruch 12 und
- mindestens ein Salz der Formel (III') 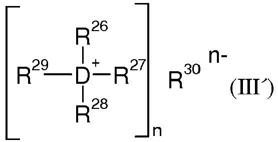
in welcher
D für Stickstoff oder Phosphor steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach, ungesättigtes, gegebenenfalls substituiertes C₁-C₈-Alkylen stehen, wobei die Substituenten aus Halogen, Nitro und Cyano ausgewählt sein können,
n für 1, 2, 3 oder 4 steht,
R³⁰ für ein anorganisches oder organisches Anion steht.

26. Zusammensetzung gemäß Anspruch 25, **dadurch gekennzeichnet, dass** sie mindestens einen Penetrationsförderer enthält.

27. Verfahren zur Steigerung der Wirkung von Schädlingsbekämpfungsmitteln und/oder Herbiziden enthaltend einen Wirkstoff der Formel (I) gemäß Anspruch 1 oder ein Mittel gemäß Anspruch 12, **dadurch gekennzeichnet, dass** das anwendungsfertige Mittel (Spritzbrühe) unter Einsatz eines Salzes der Formel (III') gemäß Anspruch 25 zubereitet wird.

28. Verfahren gemäß Anspruch 27, **dadurch gekennzeichnet, dass** die Spritzbrühe unter Einsatz eines Penetrationsförderers zubereitet wird.

## Claims

1. Compounds of the formula (I) 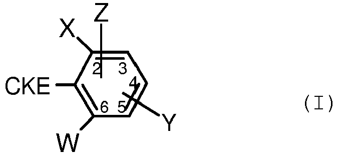 in which
W is hydrogen, alkyl, halogen, haloalkyl, alkoxy or haloalkoxy,
X is alkyl, alkenyl, alkynyl, halogen, alkoxy, haloalkyl, haloalkoxy or cyano,
Y is hydrogen, alkyl, alkoxy or halogen,
Z is a group 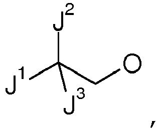
in which J¹ and J² are each independently hydrogen or halogen and J³ is halogen or a haloalkyl group,
CKE is one of the groups 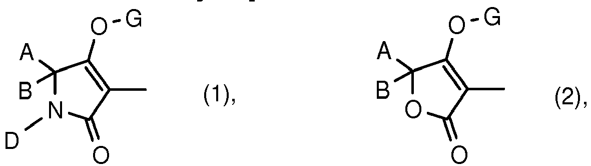 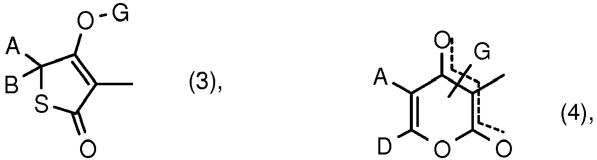 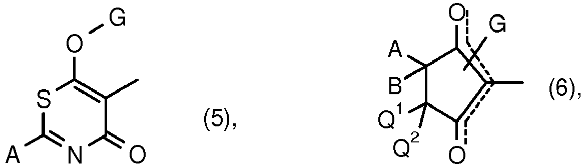 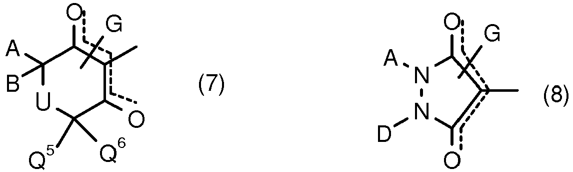 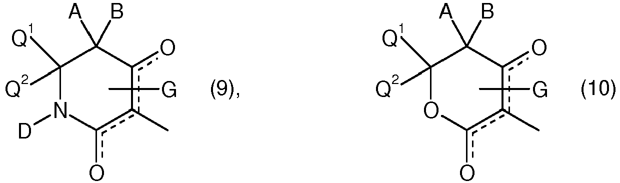 or 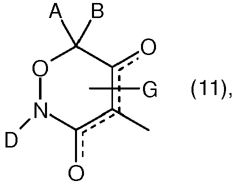 in which
U is -S-, -S(O)-, -S(O)₂-, -O-, 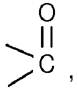 an S=N-, S(O)=N- or 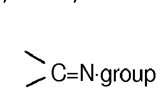 or is optionally Q³- and Q⁴-substituted C₁-C₄-alkylene which may optionally be interrupted by oxygen,
A is hydrogen, in each case optionally halogen-substituted alkyl, alkenyl, alkoxyalkyl, alkylthioalkyl, or is saturated or unsaturated, optionally substituted cycloalkyl in which at least one ring atom is optionally replaced by a heteroatom, or in each case optionally halogen-, alkyl-, haloalkyl-, alkoxy-, haloalkoxy-, cyano-or nitro-substituted aryl, arylalkyl or hetaryl,
B is hydrogen, alkyl or alkoxyalkyl, or
A and B together with the carbon atom to which they are bonded are a saturated or unsaturated, unsubstituted or substituted cycle optionally containing at least one heteroatom,
D is hydrogen or an optionally substituted radical from the group of alkyl, alkenyl, alkynyl, alkoxyalkyl, saturated or unsaturated cycloalkyl in which one or more ring members are optionally replaced by heteroatoms, or in each case optionally substituted arylalkyl, aryl, hetarylalkyl or hetaryl, or
A and D together with the atoms to which they are bonded are a saturated or unsaturated cycle which is unsubstituted or substituted in the A, D moiety and optionally contains at least one (in the case of CKE=8 and 11 one further) heteroatom, or
A and Q¹ together are in each case optionally substituted alkanediyl or alkenediyl, which may optionally be interrupted by at least one heteroatom, a 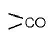 or substituted 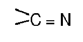 group, or
B and Q² together with the atoms to which they are bonded are a saturated or unsaturated cycle which is unsubstituted or substituted in the B, Q² moiety and optionally contains at least one heteroatom, or
D and Q¹ together with the atoms to which they are bonded are a saturated or unsaturated cycle which is unsubstituted or substituted in the D, Q¹ moiety and optionally contains at least one heteroatom,
Q¹ is hydrogen, alkyl, alkoxyalkyl, optionally substituted cycloalkyl in which one methylene group is optionally replaced by oxygen or sulphur, or is optionally substituted phenyl,
Q², Q⁴, Q⁵ and Q⁶ are each independently hydrogen or
Q³ alkyl, is hydrogen, in each case optionally substituted alkyl, alkoxy, alkylthio, alkoxyalkyl, alkylthioalkyl, or is optionally substituted cycloalkyl in which one or two methylene groups are optionally replaced by oxygen or sulphur, or is optionally substituted phenyl, or
Q¹ and Q² together with the carbon atom to which they are bonded are an unsubstituted or substituted cycle optionally containing one heteroatom, or
Q³ and Q⁴ together with the carbon atom to which they are bonded are a saturated or unsaturated, unsubstituted or substituted cycle optionally containing at least one heteroatom, or
A and Q³ together with the carbon atom to which they are bonded are a saturated or unsaturated, unsubstituted or substituted cycle optionally containing at least one heteroatom, or
A and Q⁵ together with the carbon atom to which they are bonded are a saturated or unsaturated, unsubstituted or substituted cycle optionally containing at least one heteroatom,
G is hydrogen (a) or one of the groups 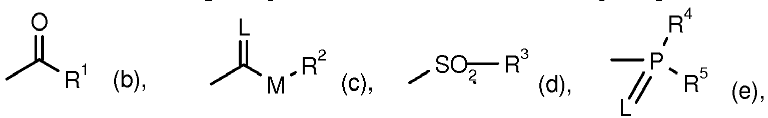 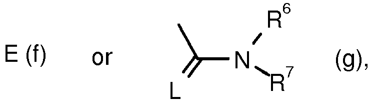
in which
E is one metal ion equivalent or one ammonium ion,
L is oxygen or sulphur,
M is oxygen or sulphur,
R¹ is in each case optionally halogen-substituted alkyl, alkenyl, alkoxyalkyl, alkylthioalkyl, polyalkoxyalkyl, or is optionally halogen- alkyl- or alkoxy-substituted cycloalkyl which may be interrupted by at least one heteroatom, or is in each case optionally substituted phenyl, phenylalkyl, hetaryl, phenoxyalkyl or hetaryloxyalkyl,
R² is in each case optionally halogen-substituted alkyl, alkenyl, alkoxyalkyl, polyalkoxyalkyl, or in each case optionally substituted cycloalkyl, phenyl or benzyl,
R³, R⁴ and R⁵ are each independently in each case optionally halogen-substituted alkyl, alkoxy, alkylamino, dialkylamino, alkylthio, alkenylthio, cycloalkylthio, or in each case optionally substituted phenyl, benzyl, phenoxy or phenylthio,
R⁶ and R⁷ are each independently hydrogen, in each case optionally halogen-substituted alkyl, cycloalkyl, alkenyl, alkoxy, alkoxyalkyl, optionally substituted phenyl, optionally substituted benzyl, or, together with the nitrogen atom to which they are bonded, a cycle optionally interrupted by oxygen or sulphur.

2. Compounds of the formula (I) according to Claim 1, in which
W is hydrogen, C₁-C₆-alkyl, halogen, C₁-C₆-alkoxy, C₁-C₄-haloalkyl or C₁-C₄-haloalkoxy,
X is halogen, C₁-C₆-alkyl, C₂-C₆-alkenyl, C₂-C₆-alkynyl, C₁-C₆-alkoxy, C₁-C₄-haloalkyl, C₁-C₄-haloalkoxy or cyano,
Y is hydrogen, halogen, C₁-C₆-alkyl or C₁-C₆-alkoxy,
Z is a group 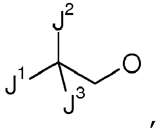
in which J¹ and J² are each independently hydrogen, fluorine or chlorine, and J³ is halogen or C₁-C₄-haloalkyl,
CKE is one of the groups 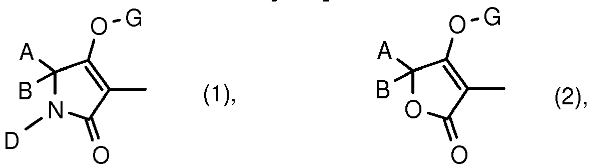 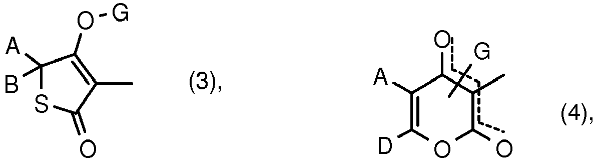 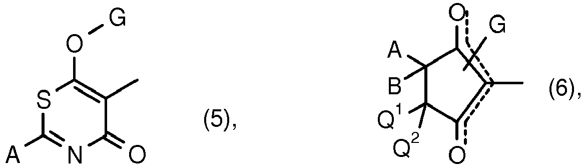 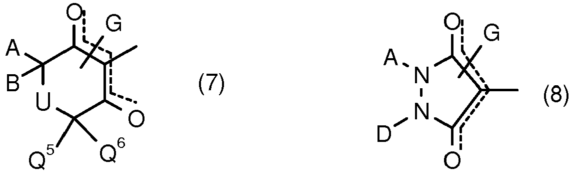 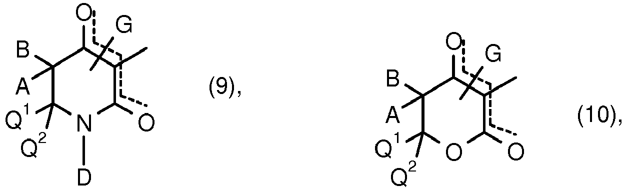 or 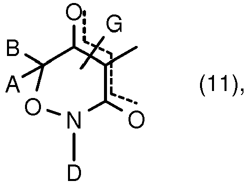 U is -S-, -S(O)-, -S(O)₂-, -O-, 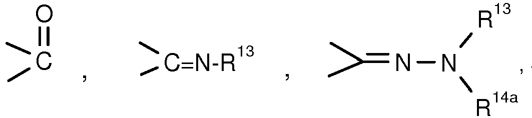 S=N-R¹³, S(O)=N-R¹³ or 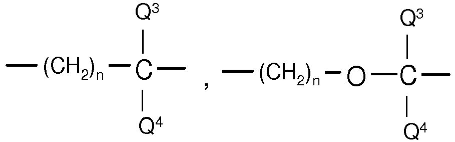 in which n is the number 0, 1 or 2,
A is hydrogen or in each case optionally halogen-substituted C₁-C₁₂-alkyl, C₃-C₈-alkenyl, C₁-C₁₀-alkoxy-C₁-C₈-alkyl, C₁-C₁₀-alkylthio-C₁-C₆-alkyl, optionally halogen-, C₁-C₆-alkyl- or C₁-C₆-alkoxy-substituted C₃-C₈-cycloalkyl in which one or two ring members not directly adjacent are optionally replaced by oxygen and/or sulphur, or is in each case optionally halogen-C₁-C₆-alkyl-, C₁-C₆-haloalkyl-, C₁-C₆-alkoxy-, C₁-C₆-haloalkoxy-, cyano- or nitro-substituted phenyl, naphthyl, hetaryl having 5 to 6 ring atoms, phenyl-C₁-C₆-alkyl or naphthyl-C₁-C₆-alkyl,
B is hydrogen, C₁-C₁₂-alkyl or C₁-C₈-alkoxy-C₁-C₆-alkyl or
A, B and the carbon atom to which they are bonded are saturated C₃-C₁₀-cycloalkyl or unsaturated C₅-C₁₀-cycloalkyl, in which one ring member is optionally replaced by nitrogen, oxygen or sulphur and which is optionally mono- or disubstituted by C₁-C₈-alkyl, C₁-C₈-alkoxy, C₃-C₈-alkenyloxy, C₁-C₆-alkoxy-C₁-C₆-alkyl, C₃-C₆-cycloalkyl-C₁-C₂-alkoxy, C₃-C₁₀-cycloalkyl, C₁-C₈-haloalkyl, C₂-C₆-haloalkoxy, C₁-C₆-alkoxy-C₁-C₄-alkoxy, where the aforementioned radicals are also possible nitrogen substituents, or
A, B and the carbon atom to which they are bonded are C₃-C₆-cycloalkyl which is substituted by an optionally C₁-C₄-alkyl-substituted alkylenediyl group optionally containing one or or two oxygen and/or sulphur atoms which are not directly adjacent, or by an alkylenedioxy or by an alkylenedithioyl group, which group forms a further five- to eight-membered ring with the carbon atom to which it is bonded, or
A, B and the carbon atom to which they are bonded are C₃-C₈-cycloalkyl or C₅-C₈-cycloalkenyl, in which two substituents together with the carbon atoms to which they are bonded are in each case optionally C₁-C₆-alkyl-, C₁-C₆-alkoxy- or halogen-substituted C₂-C₆-alkanediyl, C₂-C₆-alkenediyl or C₄-C₆-alkanedienediyl in which one methylene group is optionally replaced by oxygen or sulphur,
D is hydrogen, in each case optionally halogen-substituted C₁-C₁₂-alkyl, C₃-C₈-alkenyl, C₃-C₈-alkynyl, C₁-C₁₀-alkoxy-C₁-C₈-alkyl, optionally halogen-, C₁-C₄-alkyl-, C₁-C₄-alkoxy- or C₁-C₄-haloalkyl-substituted C₃-C₈-cycloalkyl, in which one ring member is optionally replaced by oxygen or sulphur, or in each case optionally halogen-, C₁-C₆-alkyl-, C₁-C₆-haloalkyl-, C₁-C₆-alkoxy-, C₁-C₆-haloalkoxy-, cyano- or nitro-substituted phenyl, hetaryl having 5 or 6 ring atoms, phenyl-C₁-C₆-alkyl or hetaryl-C₁-C₆-alkyl having 5 or 6 ring atoms, or
A and D together are in each case optionally substituted C₃-C₆-alkanediyl or C₃-C₆-alkenediyl, in which one methylene group is optionally replaced by a carbonyl group, oxygen or sulphur, and
where possible substituents in each case are:
halogen, hydroxyl, mercapto or in each case optionally halogen-substituted C₁-C₁₀-alkyl, C₁-C₆-alkoxy, C₁-C₆-alkylthio, C₃-C₇-cycloalkyl, phenyl or benzyloxy, or a further C₃-C₆-alkanediyl moiety, C₃-C₆-alkenediyl moiety or a butadienyl moiety, which is optionally substituted by C₁-C₆-alkyl or in which two adjacent substituents with the carbon atoms to which they are bonded optionally form a further saturated or unsaturated cycle having 5 or 6 ring atoms (in the case of the compound (I-1), A and D together with the atoms to which they are bonded are then, for example, the AD-1 to AD-10 groups further down), which may contain oxygen or sulphur, or in which one of the following groups 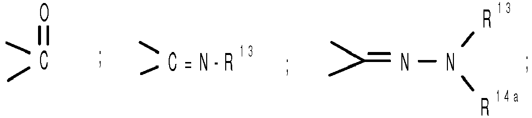 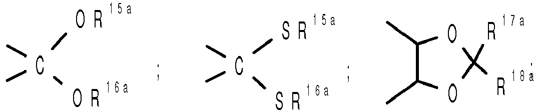 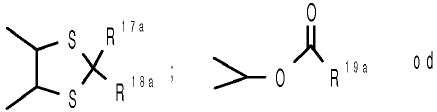
or 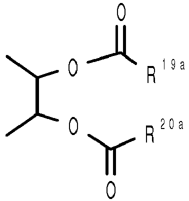 is optionally present, or
A and Q¹ together with the carbon atoms to which they are bonded are in each case C₃-C₆-alkanediyl or C₄-C₆-alkenediyl each optionally mono- or disubstituted identically or differently by halogen, hydroxyl, by C₁-C₁₀-alkyl, C₁-C₈-alkenyl, C₁-C₆-alkoxy, C₁-C₆-alkylthio,
C₃-C₇-cycloalkyl each optionally mono- to trisubstituted identically or differently by halogen, or by benzyloxy or phenyl each optionally mono- to trisubstituted identically or differently by halogen, C₁-C₆-alkyl or
C₁-C₆-alkoxy, and which also optionally contains one of the following groups: 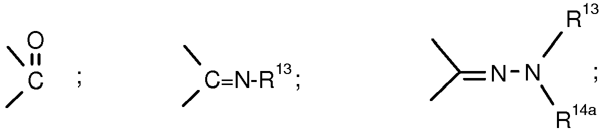 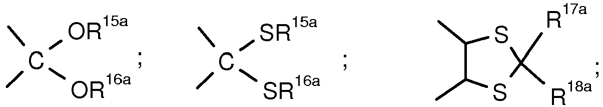 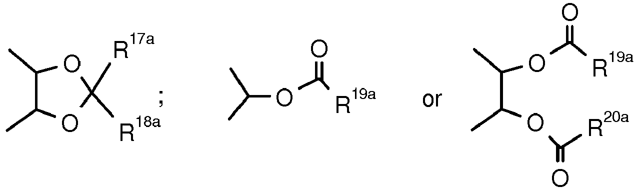
or is bridged by a C₁-C₂-alkanediyl group or by one oxygen atom, or
B and Q² together are optionally C₁-C₂-alkyl-substituted C₁-C₃-alkanediyl which may optionally be interrupted by oxygen, or
D and Q¹ together are C₃-C₆-alkanediyl optionally mono- or disubstituted identically or differently by C₁-C₄-alkyl, C₁-C₄-alkoxy, or
Q¹ is hydrogen, C₁-C₆-alkyl, C₁-C₆-alkoxy-C₁-C₂-alkyl, optionally fluorine-, chlorine-, C₁-C₄-alkyl-, C₁-C₂-haloalkyl- or C₁-C₄-alkoxy-substituted C₃-C₈-cycloalkyl in which one methylene group is optionally replaced by oxygen or sulphur, or optionally halogen-, C₁-C₄-alkyl-, C₁-C₄-alkoxy-, C₁-C₂-haloalkyl-, C₁-C₂-haloalkoxy-, cyano- or nitro-substituted phenyl,
Q², Q⁴, Q⁵ and Q⁶ are each independently hydrogen or C₁-C₄-alkyl,
Q³ is hydrogen, C₁-C₆-alkyl, C₁-C₆-alkoxy, C₁-C₆-alkylthio, C₁-C₆-alkoxy-C₁-C₂-alkyl, C₁-C₆-alkylthio-C₁-C₂-alkyl, optionally C₁-C₄-alkyl- or C₁-C₄-alkoxy-substituted C₃-C₈-cycloalkyl in which one or two methylene groups are optionally replaced by oxygen or sulphur, or optionally halogen-, C₁-C₄-alkyl-, C₁-C₄-alkoxy-, C₁-C₂-haloalkyl-, C₁-C₂-haloalkoxy-, cyano- or nitro-substituted phenyl, or
Q¹ and Q² with the carbon atom to which they are bonded are optionally a C₁-C₆-alkyl-, C₁-C₆-alkoxy- or C₁-C₂-haloalkyl-substituted C₃-C₇ ring in which one ring member is optionally replaced by oxygen or sulphur,
Q³ and Q⁴ together with the carbon atom to which they are bonded are an optionally C₁-C₄-alkyl-, C₁-C₄-alkoxy- or C₁-C₂-haloalkyl-substituted, saturated or unsaturated C₃-C₇ ring in which one or two ring members are optionally replaced by oxygen or sulphur,
A and Q³ together with the carbon atoms to which they are bonded are a saturated or unsaturated, optionally C₁-C₄-alkyl-, C₁-C₄-alkoxy- or C₁-C₂-haloalkyl-substituted C₃-C₇ ring in which one or two ring members are optionally replaced by oxygen or sulphur,
A and Q⁵ together with the carbon atoms to which they are bonded are a saturated or unsaturated, optionally C₁-C₄-alkyl-, C₁-C₄-alkoxy- or C₁-C₂-haloalkyl-substituted C₃-C₇ ring in which one ring member is optionally replaced by oxygen or sulphur,
G is hydrogen (a) or one of the groups 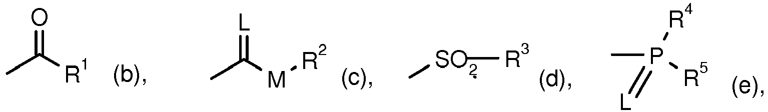
E (f) or 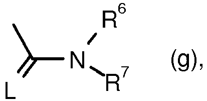 in which
E is one metal ion equivalent or one ammonium ion,
L is oxygen or sulphur and
M is oxygen or sulphur,
R¹ is in each case optionally halogen-substituted C₁-C₂₀-alkyl, C₂-C₂₀-alkenyl, C₁-C₈-alkoxy-C₁-C₈-alkyl, C₁-C₈-alkylthio-C₁-C₈-alkyl, poly-C₁-C₈-alkoxy-C₁-C₈-alkyl or optionally halogen-, C₁-C₆-alkyl- or C₁-C₆-alkoxy-substituted C₃-C₈-cycloalkyl in which one or more ring members not directly adjacent are optionally replaced by oxygen and/or sulphur,
is optionally halogen-, cyano-, nitro-, C₁-C₆-alkyl-, C₁-C₆-alkoxy-, C₁-C₆-haloalkyl-, C₁-C₆-haloalkoxy-, C₁-C₆-alkylthio- or C₁-C₆-alkylsulphonyl-substituted phenyl, is optionally halogen-, nitro-, cyano-, C₁-C₆-alkyl-, C₁-C₆-alkoxy-, C₁-C₆-haloalkyl- or C₁-C₆-haloalkoxy-substituted phenyl-C₁-C₆-alkyl,
is optionally halogen- or C₁-C₆-alkyl-substituted 5- or 6-membered hetaryl,
is optionally halogen- or C₁-C₆-alkyl-substituted phenoxy-C₁-C₆-alkyl, or
is optionally halogen-, amino- or C₁-C₆-alkyl-substituted 5- or 6-membered hetaryloxy-C₁-C₆-alkyl,
R² is in each case optionally halogen-substituted C₁-C₂₀-alkyl, C₂-C₂₀-alkenyl, C₁-C₈-alkoxy-C₂-C₈-alkyl, poly-C₁-C₈-alkoxy-C₂-C₈-alkyl,
is optionally halogen-, C₁-C₆-alkyl- or C₁-C₆-alkoxy-substituted C₃-C₈-cycloalkyl or
is in each case optionally halogen-, cyano-, nitro-, C₁-C₆-alkyl-, C₁-C₆-alkoxy-, C₁-C₆-haloalkyl- or C₁-C₆-haloalkoxy-substituted phenyl or benzyl,
R³ is optionally halogen-substituted C₁-C₈-alkyl or in each case optionally halogen-, C₁-C₆-alkyl-, C₁-C₆-alkoxy-, C₁-C₄-haloalkyl-, C₁-C₄-haloalkoxy-, cyano- or nitro-substituted phenyl or benzyl,
R⁴ and R⁵ are each independently in each case optionally halogen-substituted C₁-C₈-alkyl, C₁-C₈-alkoxy, C₁-C₈-alkylamino, di-(C₁-C₈-alkyl)amino, C₁-C₈-alkylthio, C₂-C₈-alkenylthio, C₃-C₇-cycloalkylthio or in each case optionally halogen-, nitro-, cyano-, C₁-C₄-alkoxy-, C₁-C₄-haloalkoxy-, C₁-C₄-alkylthio-, C₁-C₄-haloalkylthio-, C₁-C₄-alkyl- or C₁-C₄-haloalkyl-substituted phenyl, phenoxy or phenylthio,
R⁶ and R⁷ are each independently hydrogen, in each case optionally halogen-substituted C₁-C₈-alkyl, C₃-C₈-cyclo-alkyl, C₁-C₈-alkoxy, C₃-C₈-alkenyl, C₁-C₈-alkoxy-C₁-C₈-alkyl, optionally halogen-, C₁-C₈-haloalkyl-, C₁-C₈-alkyl- or C₁-C₈-alkoxy-substituted phenyl, optionally halogen-, C₁-C₈-alkyl-, C₁-C₈-haloalkyl- or C₁-C₈-alkoxy-substituted benzyl, or together are an optionally C₁-C₄-alkyl-substituted C₃-C₆-alkylene radical in which one carbon atom is optionally replaced by oxygen or sulphur,
R¹³ is hydrogen, in each case optionally halogen-substituted C₁-C₈-alkyl or C₁-C₈-alkoxy (only in the case of the C=N-R¹³ group), optionally halogen-, C₁-C₄-alkyl- or C₁-C₄-alkoxy-substituted C₃-C₈-cycloalkyl in which one methylene group is optionally replaced by oxygen or sulphur, or is in each case optionally halogen-, C₁-C₆-alkyl-, C₁-C₆-alkoxy-, C₁-C₄-haloalkyl-, C₁-C₄-haloalkoxy-, nitro- or cyano-substituted phenyl, phenyl-C₁-C₄-alkyl, hetaryl-C₁-C₄-alkyl, or, only in the case of the C=N-R¹³ group, phenyl-C₁-C₄-alkoxy or hetaryl-C₁-C₄-alkoxy,
R^{14a} is hydrogen or C₁-C₈-alkyl or
R¹³ and R^{14a} together are optionally C₁-C₄-alkyl-substituted C₄-C₆-alkanediyl which may optionally be interrupted by oxygen or sulphur,
R^{15a} and R^{16a} are the same or different and are C₁-C₆-alkyl or
R^{15a} and R^{16a} together are a C₂-C₄-alkanediyl radical or a C₄-alkanediyl radical which is optionally substituted by C₁-C₆-alkyl, C₁-C₆-haloalkyl or by optionally halogen-, C₁-C₆-alkyl-, C₁ₗ-C₄-haloalkyl-, C₁-C₆-alkoxy-, C₁-C₄-haloalkoxy-, nitro- or cyano-substituted phenyl, R^{17a} and R¹⁸ are each independently hydrogen, optionally halogen-substituted C₁-C₈-alkyl or optionally halogen-, C₁-C₆-alkyl-, C₁-C₆-alkoxy-, C₁-C₄-haloalkyl-, C₁-C₄-haloalkoxy-, nitro- or cyano-substituted phenyl or
R^{17a} and R^{18a} together with the carbon atom to which they are bonded are a carbonyl group or optionally halogen-, C₁-C₄-alkyl- or C₁-C₄-alkoxy-substituted C₅-C₇-cycloalkyl in which one methylene group is optionally replaced by oxygen or sulphur,
R^{19a} and R^{20a} are each independently C₁-C₁₀-alkyl, C₂-C₁₀-alkenyl, C₁-C₁₀-alkoxy, C₁-C₁₀-alkylamino, C₃-C₁₀-alkenylamino, di-(C₁-C₁₀-alkyl) amino or di-(C₃-C₁₀-alkenyl)amino.

3. Compounds of the formula (I) according to Claim 1, in which
W is hydrogen, fluorine, chlorine, bromine, C₁-C₄-alkyl, C₁-C₄-alkoxy, C₁-C₂-haloalkyl or C₁-C₂-haloalkoxy,
X is chlorine, bromine, iodine, C₁-C₄-alkyl, C₂-C₄-alkenyl, C₂-C₄-alkynyl, C₁-C₄-alkoxy, C₁-C₂-haloalkyl, C₁-C₂-haloalkoxy or cyano,
Y is hydrogen, methyl, ethyl, fluorine, chlorine, bromine, iodine, methoxy or ethoxy,
Z is the group 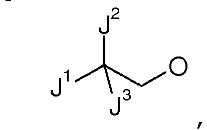
in which J¹ and J² are each independently hydrogen, fluorine or chlorine, and J³ is fluorine, chlorine, trichloromethyl, difluoromethyl, difluorochloromethyl, dichlorofluoromethyl or trifluoromethyl,
CKE is one of the groups 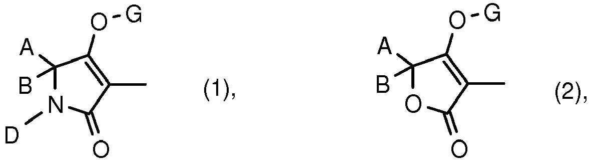 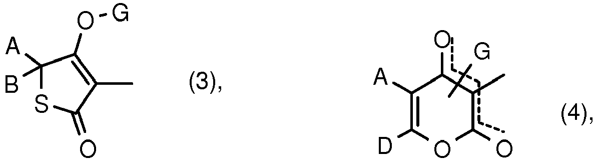 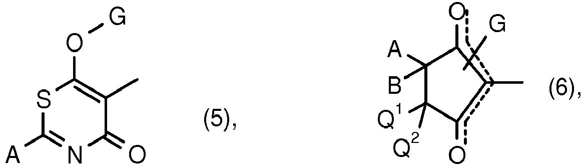 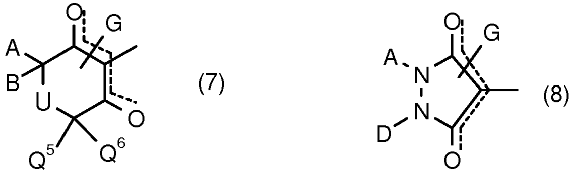 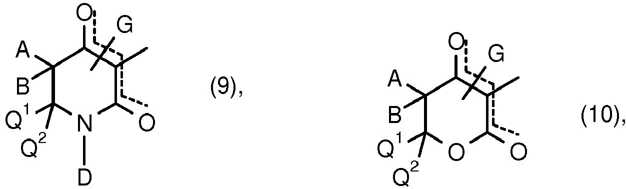 or 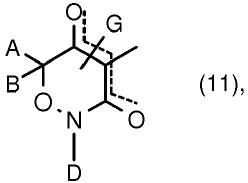
U is -CH₂-, -CH₂-CH₂-, -O- or 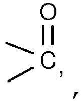
A is hydrogen, in each case optionally mono- to tri-fluorine- or -chlorine-substituted C₁-C₆-alkyl, C₁-C₄-alkoxy-C₁-C₂-alkyl, optionally mono- to di-C₁-C₂-alkyl-or -C₁-C₂-alkoxy-substituted C₃-C₆-cycloalkyl optionally interrupted by one oxygen atom or (but not in the case of the compounds of the formulae (I-3), (I-4), (I-6), (I-7), (I-9), (I-10) and (I-11)) in each case optionally mono- to di-fluorine-, -chlorine-, -bromine-, -C₁-C₄-alkyl-, -C₁-C₂-haloalkyl-, -C₁-C₄-alkoxy-, -C₁-C₂-haloalkoxy-, -cyano- or -nitro-substituted phenyl, pyridyl or benzyl,
B is hydrogen, C₁-C₄-alkyl or C₁-C₂-alkoxy-C₁-C₂-alkyl or
A, B and the carbon atom to which they are bonded are saturated or unsaturated C₃-C₇-cycloalkyl in which one ring member is optionally replaced by nitrogen, oxygen or sulphur and which is optionally mono- to di-C₁-C₆-alkyl-, -C₁-C₄-alkoxy-C₁-C₂-alkyl-, -trifluoromethyl-,-C₁-C₆-alkoxy-, -C₃-C₆-alkenyloxy-, -trifluoroethoxy-,-C₁-C₃-alkoxy-C₁-C₃-alkoxy- or -C₃-C₆-cycloalkylmethoxy-substituted, where the aforementioned radicals are also possible nitrogen substituents, with the proviso that Q³ in that case is hydrogen or methyl, or
A, B and the carbon atom to which they are bonded are C₅-C₆-cycloalkyl which is substituted by an optionally methyl-or ethyl-substituted alkylenediyl group optionally containing one or two oxygen or sulphur atoms not directly adjacent or by an alkylenedioxy group or by an alkylenedithiol group, which group forms, with the carbon atom to which it is bonded, a further five- or six-membered ring, with the proviso that Q³ in that case is hydrogen or methyl, or
A, B and the carbon atom to which they are bonded are C₃-C₆-cycloalkyl or C₅-C₆-cycloalkenyl in which two substituents together with the carbon atoms to which they are bonded are in each case optionally C₁-C₂-alkyl-or C₁-C₂-alkoxy-substituted C₂-C₄-alkanediyl, C₂-C₄-alkenediyl or butadienediyl, with the proviso that Q³ in that case is hydrogen or methyl,
D is hydrogen, in each case optionally mono- to tri-fluorine-substituted C₁-C₆-alkyl, C₃-C₆-alkenyl, C₁-C₄-alkoxy-C₁-C₃-alkyl, in each case optionally mono- to di-C₁-C₄-alkyl-, -C₁-C₄-alkoxy- or -C₁-C₂-haloalkyl-substituted C₃-C₆-cycloalkyl in which one methylene group is optionally replaced by oxygen or (only in the case of the compounds of the formula (I-4)) is in each case optionally mono- to di-fluorine-, -chlorine-,-bromine-, -C₁-C₄-alkyl-, -C₁-C₄-haloalkyl-, -C₁-C₄-alkoxy- or -C₁-C₄-haloalkoxy-substituted phenyl or pyridyl, or
A and D together are optionally mono- to disubstituted C₃-C₅-alkanediyl in which one methylene group may be replaced by a carbonyl group (but not in the case of the compounds of the formula (1-11)), oxygen or sulphur, where possible substituents are C₁-C₂-alkyl or C₁-C₂-alkoxy, or
A and D (in the case of the compounds of the formula (I-1)) together with the atoms to which they are bonded are one of the groups AD-1 to AD-10: 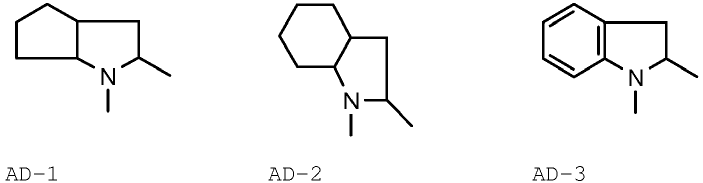 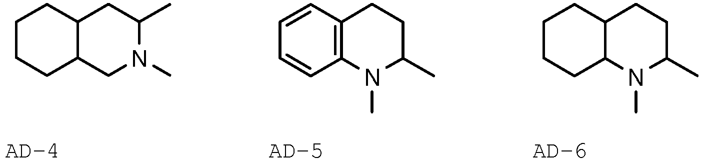 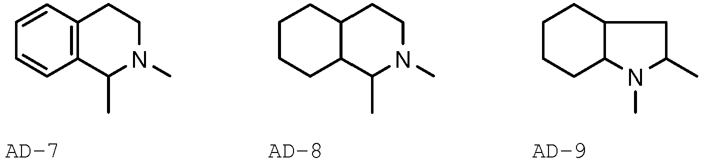 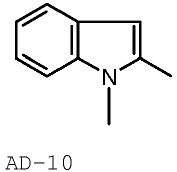
or
A and D together are C₃-C₅-alkanediyl which is optionally substituted by an optionally mono- to tetra-C₁-C₄-alkyl- or -C₁-C₃-alkoxy-C₁-C₂-alkyl-substituted alkylenedioxy group containing two oxygen atoms not directly adjacent, to form a further 5- or 6-membered ring, or
A and Q¹ together are C₃-C₄-alkanediyl which is optionally mono- or disubstituted identically or differently by C₁-C₂-alkyl or C₁-C₂-alkoxy, and which optionally contains the following group: 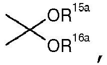
in which
R^{15a} and R^{16a} are the same or different and are each methyl or ethyl, or
R^{15a} and R^{16a} together are a C₂-C₄-alkanediyl or C₄-alkenediyl radical which is optionally substituted by methyl or ethyl, or
B and Q² together are -CH₂-, -CH₂-CH₂-, -CH₂-CH₂-CH₂-, or -CH₂-O-CH₂-, or
D and Q¹ together are C₃-C₄-alkanediyl, or
Q¹ is hydrogen, C₁-C₄-alkyl, C₁-C₄-alkoxy-C₁-C₂-alkyl, or optionally methyl- or methoxy-substituted C₃-C₆-cycloalkyl in which one methylene group is optionally replaced by oxygen,
Q² is hydrogen, methyl or ethyl,
Q⁴, Q⁵ and Q⁶ are each independently hydrogen or C₁-C₃-alkyl,
Q³ is hydrogen, C₁-C₄-alkyl, C₁-C₄-alkoxy, or optionally mono- to di-methyl- or -methoxy-substituted C₃-C₆-cycloalkyl optionally interrupted by one oxygen atom, or
Q¹ and Q² with the carbon to which they are bonded are optionally methyl- or methoxy-substituted C₃-C₆-cycloalkyl in which one methylene group is optionally replaced by oxygen, with the proviso that A and B in that case are each independently hydrogen or methyl, or Q³ and Q⁴ together with the carbon to which they are bonded are an optionally C₁-C₂-alkyl- or C₁-C₂-alkoxy-substituted saturated C₅-C₆ ring in which one or two ring members are optionally replaced by oxygen or sulphur, with the proviso that A in that case is hydrogen or methyl, or
A and Q³ together with the carbon to which they are bonded are an optionally C₁-C₂-alkyl- or C₁-C₂-alkoxy-substituted saturated C₅-C₆ ring in which one ring member is optionally replaced by oxygen or sulphur, with the proviso that B, Q⁴, Q⁵ and Q⁶ in that case are each independently hydrogen or methyl, or
A and Q⁵ together with the carbon atoms to which they are bonded are an optionally C₁-C₂-alkyl- or C₁-C₂-alkoxy-substituted saturated or unsaturated C₅-C₆ ring, with the proviso that B, Q³, Q⁴ and Q⁶ in that case are each independently hydrogen or methyl,
G is hydrogen (a) or one of the groups 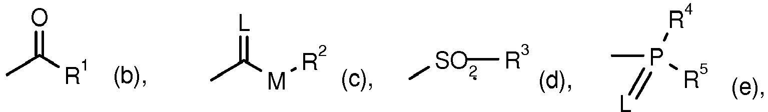
E (f) or 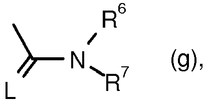 in which
E is one metal ion equivalent or one ammonium ion,
L is oxygen or sulphur and
M is oxygen or sulphur,
R¹ is in each case optionally mono- to tri-fluorine-or -chlorine-substituted C₁-C₈-alkyl, C₂-C₈-alkenyl, C₁-C₄-alkoxy-C₁-C₂-alkyl, C₁-C₄-alkylthio-C₁-C₂-alkyl or optionally mono- to di-fluorine-, -chlorine-, -C₁-C₂-alkyl- or -C₁-C₂-alkoxy-substituted C₁-C₆-cycloalkyl in which one or two ring members not directly adjacent are optionally replaced by oxygen,
is optionally mono- to di-fluorine-, -chlorine-,-bromine-, -cyano-, -nitro-, -C₁-C₄-alkyl-, -C₁-C₄-alkoxy-, -C₁-C₂-haloalkyl- or -C₁-C₂-haloalkoxy-substituted phenyl,
R² is in each case optionally mono- to tri-fluorine-substituted C₁-C₈-alkyl, C₁-C₈-alkenyl or C₁-C₄-alkoxy-C₂-C₄-alkyl,
is optionally mono-C₁-C₂-alkyl- or -C₁-C₂-alkoxy-substituted C₃-C₆-cycloalkyl or
is in each case optionally mono- to di-fluorine-,-chlorine-, -bromine-, -cyano-, -nitro-, -C₁-C₄-alkyl-,-C₁-C₃-alkoxy-, -trifluoromethyl- or -trifluoromethoxy-substituted phenyl or benzyl,
R³ is optionally mono- to tri-fluorine-substituted C₁-C₆-alkyl or optionally mono-fluorine-, -chlorine-,-bromine-, -C₁-C₄-alkyl-, -C₁-C₄-alkoxy-,-trifluoromethyl-, -trifluoromethoxy-, -cyano- or-nitro-substituted phenyl,
R⁴ is C₁-C₆-alkyl, C₁-C₆-alkoxy, C₁-C₆-alkylamino, di-(C₁-C₆-alkyl)amino, C₁-C₆-alkylthio, C₃-C₄-alkenylthio, C₁-C₆-cycloalkylthio, or in each case optionally mono-fluorine-, -chlorine-, -bromine-, -nitro-, -cyano-,-C₁-C₃-alkoxy-, -C₁-C₃-haloalkoxy-, -C₁-C₃-alkylthio-,-C₁-C₃-haloalkylthio-, -C₁-C₃-alkyl- or -trifluoromethyl-substituted phenyl, phenoxy or phenylthio,
R⁵ is C₁-C₆-alkoxy or C₁-C₆-alkylthio,
R⁶ is hydrogen, C₁-C₆-alkyl, C₃-C₆-cycloalkyl, C₁-C₆-alkoxy, C₃-C₆-alkenyl, C₁-C₆-alkoxy-C₁-C₄-alkyl, optionally mono-fluorine-, -chlorine-, -bromine-,-trifluoromethyl-, -C₁-C₄-alkyl- or -C₁-C₄-alkoxy-substituted phenyl, optionally mono-fluorine-,-chlorine-, -bromine-, -C₁-C₄-alkyl-, -trifluoromethyl-or -C₁-C₄-alkoxy-substituted benzyl,
R⁷ is C₁-C₆-alkyl, C₃-C₆-alkenyl or C₁-C₆-alkoxy-C₁-C₄-alkyl,
R⁶ and R⁷ together are an optionally methyl- or ethyl-substituted C₄-C₅-alkylene radical in which one methylene group is optionally replaced by oxygen or sulphur.

4. Compounds of the formula (I) according to Claim 1, in which
W is hydrogen, chlorine, methyl or ethyl,
X is chlorine, methyl, ethyl, methoxy or ethoxy,
Y is hydrogen, methyl or chlorine,
Z is the group 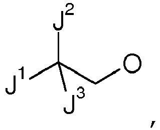
in which J¹ and J² are each independently hydrogen or fluorine and J³ is fluorine, chlorine or trifluoromethyl,
CKE is one of the groups 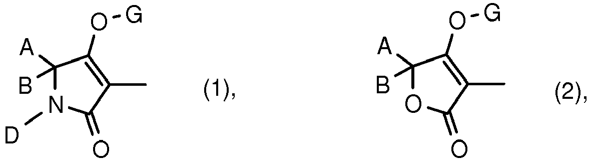 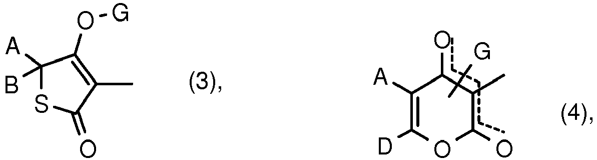 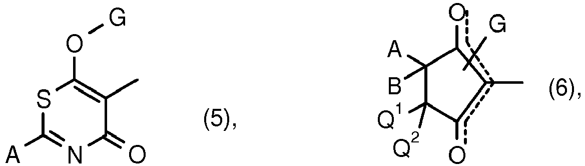 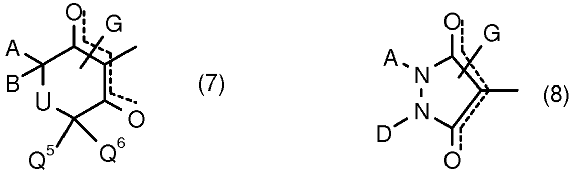 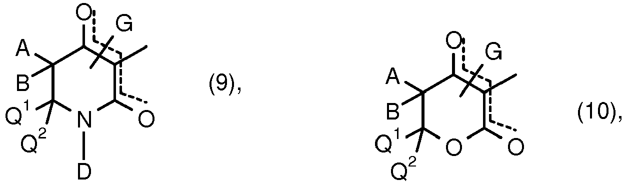 or 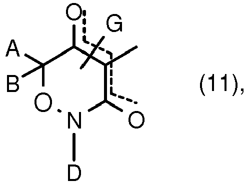
U is -CH₂-, -CH₂-CH₂-, -O- or 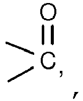
A is hydrogen, in each case optionally mono- to tri-fluorine-substituted C₁-C₄-alkyl or C₁-C₂-alkoxy-C₁-C₂-alkyl, or is cyclopropyl, cyclopentyl or cyclohexyl, and in the case of the compounds of the formula (1-5) is optionally mono- to di-fluorine-, -chlorine-, -bromine-, -methyl-, -ethyl-, -n-propyl-, -isopropyl-, -methoxy-, -ethoxy-, -trifluoromethyl-, - trifluoromethoxy-, -cyano- or -nitro-substituted phenyl,
B is hydrogen, methyl or ethyl, or
A, B and the carbon atom to which they are bonded are saturated C₅-C₆-cycloalkyl in which one ring member is optionally replaced by nitrogen, oxygen or sulphur and which is optionally mono- or di-methyl-, -ethyl-,-methoxymethyl-, -ethoxymethyl-, -methoxyethyl-,-ethoxyethyl-, -trifluoromethyl-, -methoxy-, -ethoxy-,-propoxy-, -butoxy-, -methoxyethoxy-, -ethoxyethoxy-,-allyloxy-, -trifluoroethoxy- or -cyclopropylmethoxy-substituted, where the aforementioned radicals are also possible nitrogen substitutents, with the proviso that Q³ in that case is hydrogen, or
A, B and the carbon atom to which they are bonded are C₆-cycloalkyl which is optionally substituted by an alkylidenediyl group optionally interrupted by one oxygen atom or by an alkylenedioxy group optionally containing two oxygen atoms not directly adjacent, to form a further 5- or 6-membered ring (which may be mono- or di-methyl-substituted), with the proviso that Q³ in that case is hydrogen, or
A, B and the carbon atom to which they are bonded are C₅-C₆-cycloalkyl or C₅-C₆-cycloalkenyl, in which two substituents together with the carbon atoms to which they are bonded are C₂-C₄-alkanediyl or C₂-C₄-alkenediyl or butadienediyl, with the proviso that Q³ in that case is hydrogen,
D is hydrogen, in each case optionally mono- to tri-fluorine-substituted C₁-C₄-alkyl, C₃-C₄-alkenyl, C₁-C₄-alkoxy-C₁-C₃-alkyl, or is cyclopropyl, cyclopentyl or cyclohexyl, or (in the case of the compounds of the formula (I-4)) is in each case optionally mono-fluorine-, -chlorine-, -methyl-, -ethyl-, -n-propyl-,-isopropyl-, -methoxy-, -ethoxy- or -trifluoromethyl-substituted phenyl or pyridyl, or
A and D together are optionally mono-methyl- or-methoxy-substituted C₃-C₅-alkanediyl in which one carbon atom is optionally replaced by a carbonyl group (but not in the case of the compounds of the formula (I-11)), oxygen or sulphur, or is the AD-1 group, or
A and D together are C₃-C₅-alkanediyl which is optionally substituted by an optionally mono- to di-C₁-C₂-alkyl-substituted alkylenedioxy group containing two oxygen atoms not directly adjacent, to form a further 5-membered ring, or
A and Q¹ together are optionally mono- or di-methyl- or -methoxy-substituted C₃-C₄-alkanediyl which optionally contains the following group: 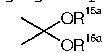
in which R^{15a} and R^{16a} together are a C₂-C₄-alkanediyl or C₄-alkenediyl radical, or
B and Q² together are -CH₂-CH₂-CH₂-, or -CH₂-O-CH₂-, or
D and Q¹ together are C₃-C₄-alkanediyl, or
Q¹ is hydrogen, methyl, ethyl, propyl, isopropyl, cyclopropyl, cyclopentyl or cyclohexyl,
Q² is hydrogen, methyl or ethyl,
Q⁴, Q⁵ and Q⁶ are each independently hydrogen or methyl,
Q³ is hydrogen, methyl, ethyl, propyl, methoxy or ethoxy, or optionally mono-methyl- or -methoxy-substituted C₃-C₆-cycloalkyl optionally interrupted by one oxygen atom, or
Q¹ and Q² with the carbon atom to which they are bonded are optionally methyl- or methoxy-substituted C₅-C₆-cycloalkyl in which one methylene group is optionally replaced by oxygen, with the proviso that A and B are each hydrogen, or
Q³ and Q⁴ together with the carbon to which they are bonded are an optionally mono-methyl- or -methoxy-substituted, saturated C₅-C₆ ring optionally interrupted by one oxygen atom, with the proviso that A, B, Q⁵ and Q⁶ in that case are each hydrogen,
G is hydrogen (a) or one of the groups 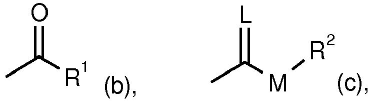
-SO₂-R³ (d) or E (f), in which
L is oxygen or sulphur,
M is oxygen or sulphur and
E is one metal ion equivalent or one ammonium ion,
R¹ is in each case optionally mono-chlorine-substituted C₁-C₆-alkyl, C₂-C₆-alkenyl, C₁-C₂-alkoxy-C₁-alkyl, C₁-C₂-alkylthio-C₁-alkyl, or in each case optionally mono-fluorine-, -chlorine-, -methyl- or-methoxy-substituted cyclopropyl or cyclohexyl, optionally mono-fluorine-, -chlorine-, -bromine-,-cyano-, -nitro-, -methyl-, -methoxy-, -trifluoromethyl-or -trifluoromethoxy-substituted phenyl,
R² is in each case optionally mono-fluorine-substituted C₁-C₈-alkyl, C₂-C₆-alkenyl or C₁-C₄-alkoxy-C₂-C₃-alkyl, phenyl or benzyl,
R³ is C₁-C₈-alkyl.

5. Compounds of the formula (I) according to Claim 1, in which
W is hydrogen, methyl or ethyl,
X is chlorine, methyl or ethyl,
Y is hydrogen,
Z is OCH₂-CF₃ in the 3 position, or
Z is OCH₂-CF₃ in the 4 position, or
Z is OCH₂-CF₃ in the 5 position,
CKE is one of the groups 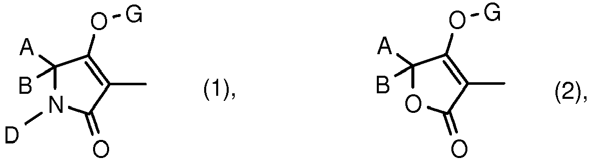 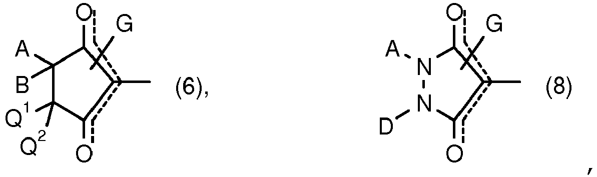
A is methyl or ethyl,
B is hydrogen or methyl,
A, B and the carbon atom to which they are bonded are saturated C₅-C₆-cycloalkyl in which one ring member is optionally replaced by oxygen and which is optionally mono- or di-methyl-, -ethyl-, -methoxymethyl-, - methoxy-, -ethoxy-, -propoxy-, -butoxy-, -trifluoroethoxy-substituted, or
A, B and the carbon atom to which they are bonded are C₆-cycloalkyl which is optionally substituted by an alkylenedioxy group containing two oxygen atoms not directly adjacent, to form a further 5- or 6-membered ring which may be mono- or di-methyl-substituted,
D is hydrogen, or
A and D together are C₃-C₅-alkanediyl in which one carbon atom is optionally replaced by oxygen, or
A and D together are C₃-C₅-alkanediyl which is optionally substituted by an optionally mono- to di-methyl-substituted alkylenedioxy group optionally containing two oxygen atoms not directly adjacent, to form a further 5-membered ring, or
A and Q¹ together are C₃-C₄-alkanediyl,
Q² is hydrogen,
G is hydrogen (a) or one of the groups 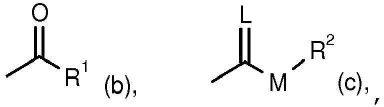
in which
L is oxygen,
M is oxygen,
R¹ is C₁-C₆-alkyl,
R² is C₁-C₆-alkyl.

6. Process for preparing compounds of the formula (I) according to Claim 1, **characterized in that**
(A) Compounds of the formula (I-1-a) 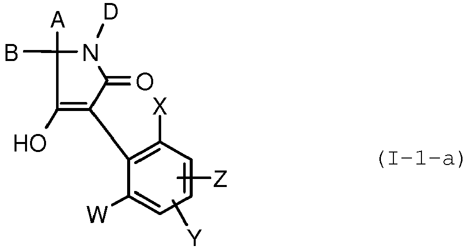 in which
A, B, D, W, X, Y and Z are each as defined above,
are obtained by intramolecularly condensing compounds of the formula (II) 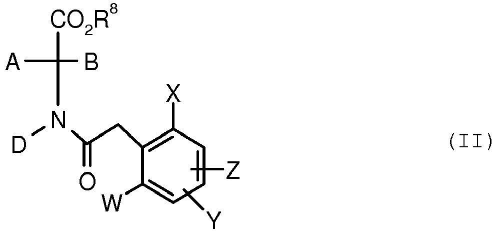 in which
A, B, D, W, X, Y and Z are each as defined above, and
R⁸ is alkyl,
in the presence of a diluent and in the presence of a base,
(B) Compounds of the formula (I-2-a) 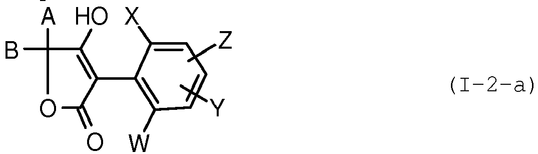 in which
A, B, W, X, Y and Z are each as defined above,
are obtained by intramolecularly condensing compounds of the formula (III) 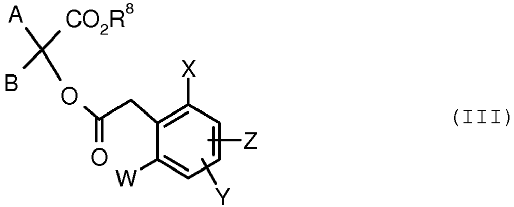 in which
A, B, W, X, Y, Z and R⁸ are each as defined above,
in the presence of a diluent and in the presence of a base,
(C) Compounds of the formula (I-3-a) 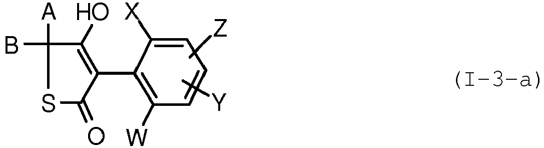 in which
A, B, W, X, Y and Z are each as defined above,
are obtained by intramolecularly cyclizing compounds of the formula (IV) 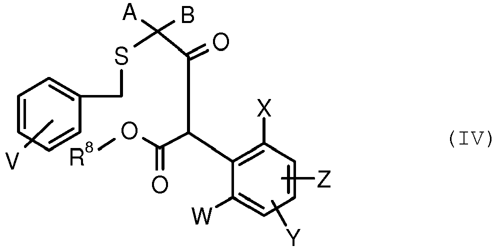 in which
A, B, W, X, Y, Z and R⁸ are each as defined above and
V is hydrogen, halogen, alkyl or alkoxy,
optionally in the presence of a diluent and in the presence of an acid,
(D) Compounds of the formula (I-4-a) 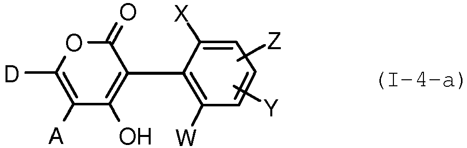 in which
A, D, W, X, Y and Z are each as defined above,
are obtained by reacting compounds of the formula (V) 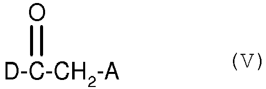 in which
A and D are each as defined above,
or the silyl enol ethers thereof, of the formula (Va) 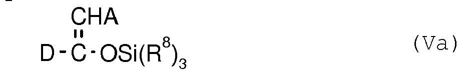 in which
A, D and R⁸ are each as defined above, with compounds of the formula (VI) 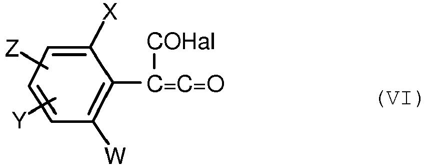 in which
W, X, Y and Z are each as defined above and
Hal is halogen,
optionally in the presence of a diluent and optionally in the presence of an acid acceptor,
(E) Compounds of the formula (I-5-a) 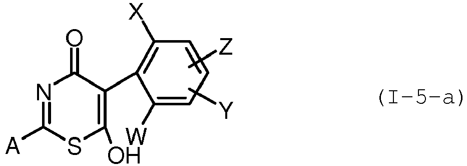 in which
A,W, X, Y and Z are each as defined above,
are obtained by reacting compounds of the formula (VII) 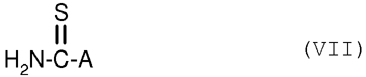 in which
A is as defined above,
with compounds of the formula (VI) 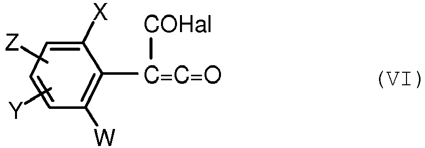 in which
Hal, W, X, Y and Z are each as defined above, optionally in the presence of a diluent and optionally in the presence of an acid acceptor,
(F) Compounds of the formula (I-6-a) 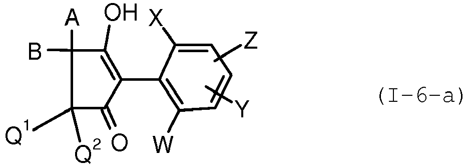 in which
A, B, Q¹, Q², W, X, Y and Z are each as defined above,
are obtained by intramolecularly cyclizing compounds of the formula (VIII) 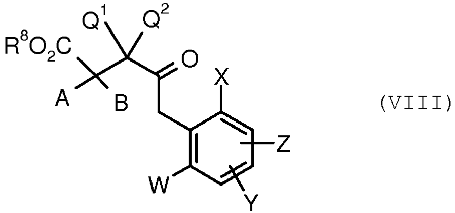 in which
A, B, Q¹, Q², W, X, Y and Z are each as defined above, and
R⁸ is alkyl,
optionally in the presence of a diluent and in the presence of a base,
(G) Compounds of the formula (I-7-a) 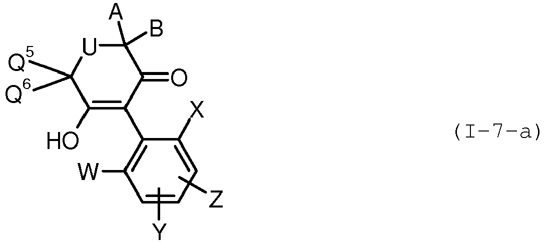 in which
A, B, Q⁵, Q⁶, U, W, X, Y and Z are each as defined above,
are obtained by intramolecularly condensing compounds of the formula (IX) 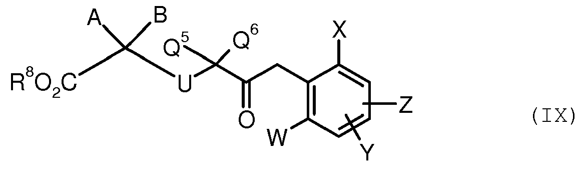 in which
A, B, Q⁵, Q⁶, U, W, X, Y and Z are each as defined above
and
R⁸ is alkyl,
in the presence of a diluent and in the present of a base,
(H) Compounds of the formula (I-8-a) 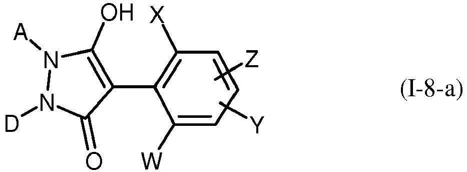 in which
A, D, W, X, Y and Z are each as defined above,
are obtained by reacting compounds of the formula (X) 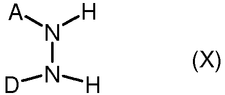 in which
A and D are each as defined above,
α) with compounds of the formula (VI) 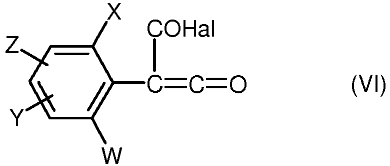 in which
Hal, W, X, Y and Z are each as defined above, optionally in the presence of a diluent and optionally in the presence of an acid acceptor, or
ß) with compounds of the formula (XI) 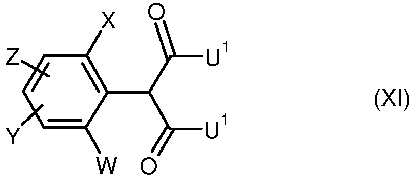 in which
W, X, Y and Z are each as defined above,
and U¹ is NH₂ or O-R⁸ where R⁸ is as defined above, optionally in the presence of a diluent and optionally in the presence of a base, or
γ) with compounds of the formula (XII) 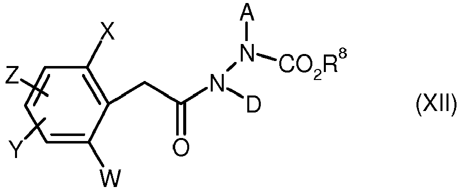 in which
A, D, W, X, Y, Z and R⁸ are each as defined above, optionally in the presence of a diluent and optionally in the presence of a base,
(I) Compounds of the formula (I-9-a) 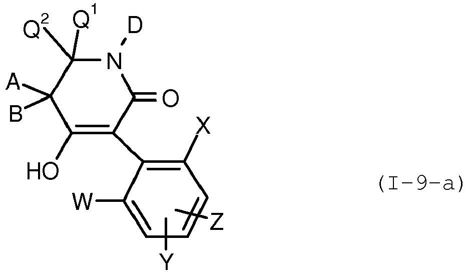 in which
A, B, D, Q¹, Q², W, X, Y and Z are each as defined above,
are obtained by intramolecularly condensing compounds of the formula (XIII) 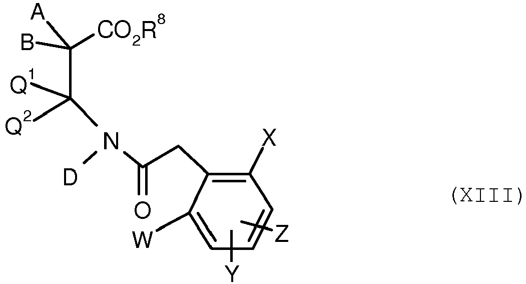 in which
A, B, D, Q¹, Q², W, X, Y and Z are each as defined above,
and
R⁸ is alkyl,
in the presence of a diluent and in the presence of a base,
(J) Compounds of the formula (I-10-a) 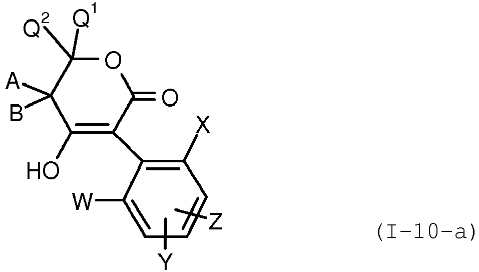 in which
A, B, Q¹, Q², W, X, Y and Z are each as defined above,
are obtained by converting compounds of the formula (XIV) 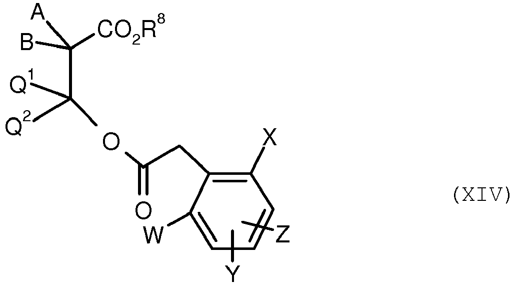 in which
A, B, Q¹, Q², W, X, Y and Z are each as defined above, and
R⁸ is alkyl,
optionally in the presence of a diluent and optionally in the presence of a base,
(K) Compounds of the formula (I-11-a) 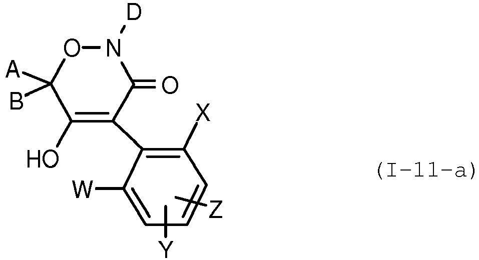 in which
A, B, D, W, X, Y and Z are each as defined above,
are obtained by intramolecularly condensing compounds of the formula (XV) 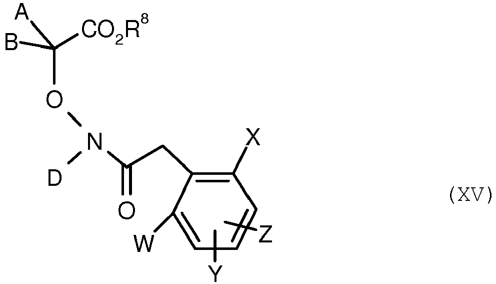 in which
A, B, D, W, X, Y and Z are each as defined above,
and
R⁸ is alkyl,
in the presence of a diluent and in the presence of a base,
(L) Compounds of the formulae (I-1-b) to (I-11-b) shown above, in which A, B, D, Q¹, Q², Q⁵, Q⁶, R¹, U, W, X, Y and Z are each as defined above are obtained by reacting compounds of the formulae (I-1-a) to (I-11-a) shown above, in which A, B, D, Q¹, Q², Q⁵, Q⁶, U, W, X, Y and Z are each as defined above in each case
(α) with acid halides of the formula (XVI) 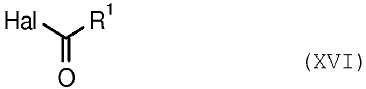 in which
R¹ is as defined above and
Hal is halogen
or
(ß) with carboxylic anhydrides of the formula (XVII)
R¹-CO-O-CO-R¹ (XVII)
in which
R¹ is as defined above,
optionally in the presence of a diluent and optionally in the presence of an acid binder;
(M) Compounds of the formulae (I-1-c) to (I-11-c) shown above, in which A, B, D, Q¹, Q², Q⁵, Q⁶, R², M, U, W, X, Y and Z are each as defined above and L is oxygen are obtained by reacting compounds of the formulae (I-1-a) to (I-11-a) shown above, in which A, B, D, Q¹, Q², Q⁵, Q⁶, U, W, X, Y and Z are each as defined above in each case
with chloroformic esters or chloroformic thiol esters of the formula (XVIII)
R²-M-CO-Cl (XVIII)
in which
R² and M are each as defined above,
optionally in the presence of a diluent and optionally in the presence of an acid binder;
(N) Compounds of the formulae (I-1-c) to (I-11-c) shown above, in which A, B, D, Q¹, Q², Q⁵, Q⁶, R², M, U, W, X, Y and Z are each as defined above and L is sulphur are obtained by reacting compounds of the formulae (I-1-a) to (I-11-a) shown above, in which A, B, D, Q¹, Q⁴, Q⁵, Q⁶, U, W, X, Y and Z are each as defined above in each case
with chloromonothioformic esters or chlorodithioformic esters of the formula (XIX) 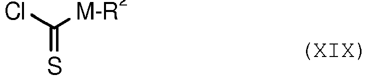 in which
M and R² are each as defined above,
optionally in the presence of a diluent and optionally in the presence of an acid binder,
and
(O) Compounds of the formulae (I-1-d) to (I-11-d) shown above, in which A, B, D, Q¹, Q², Q⁵, Q⁶, R³, U, W, X, Y and Z are each as defined above are obtained by reacting compounds of the formulae (I-1-a) to (I-11-a) shown above, in which A, B, D, Q¹, Q², Q⁵, Q⁶, U, W, X, Y and Z are each as defined above in each case with sulphonyl chlorides of the formula (XX)
R³-SO₂-Cl (XX)
in which
R³ is as defined above, optionally in the presence of a diluent and optionally in the presence of an acid binder,
(P) Compounds of the formulae (I-1-e) to (I-11-e) shown above, in which A, B, D, L, Q¹, Q², Q⁵, Q⁶, R⁴, R⁵, U, W, X, Y and Z are each as defined above are obtained by reacting compounds of the formulae (I-1-a) to (I-11-a) shown above, in which A, B, D, Q¹, Q², Q⁵, Q⁶, U, W, X, Y and Z are each as defined above in each case
with phosphorus compounds of the formula (XXI) 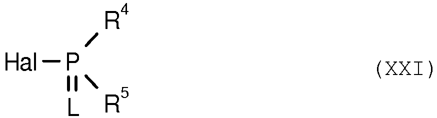 in which
L, R⁴ and R⁵ are each as defined above and
Hal is halogen,
optionally in the presence of a diluent and optionally in the presence of an acid binder,
(Q) Compounds of the formulae (I-1-f) to (I-11-f) shown above, in which A, B, D, E, Q¹, Q², Q⁵, Q⁶, U, W, X, Y and Z are each as defined above are obtained by reacting compounds of the formulae (I-1-a) to (I-11-a) in which A, B, D, Q¹, Q², Q⁵, Q⁶, U, W, X, Y and Z are each as defined above in each case
with metal compounds or amines of the formaule (XXII) or (XXIII)
Me(OR¹⁰)ₜ (XXII) 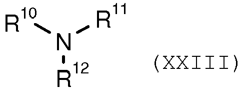
in which
Me is a mono- or divalent metal or an ammonium ion 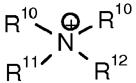
t is the number 1 or 2 and
R¹⁰, R¹¹, R¹² are each independently hydrogen or alkyl, optionally in the presence of a diluent,
(R) Compounds of the formulae (I-1-g) to (I-11-g) shown above, in which A, B, D, L, Q¹, Q², Q⁵, Q⁶, R⁶, R⁷, U, W, X, Y and Z are each as defined above are obtained by reacting compounds of the formulae (I-1-a) to (I-11-a) shown above, in which A, B, D, Q¹, Q², Q⁵, Q⁶, U, W, X, Y and Z are each as defined above in each case
(α) with isocyanates or isothiocyanates of the formula (XXIV)
R⁶-N=C=L (XXIV)
in which R⁶ and L are each as defined above,
optionally in the presence of a diluent and optionally in the presence of a catalyst, or
(ß) with carbamyl chlorides or thiocarbamyl chlorides of the formula (XXV) 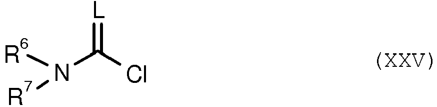 in which
L, R⁶ and R⁷ are each as defined above,
optionally in the presence of a diluent and optionally in the presence of an acid binder,
(S) Compounds of the formulae (I-1-a) to (I-11-g) shown above, in which A, B, D, G, Q¹, Q², Q⁵, Q⁶, U, W, X, Y and Z are each as defined above are obtained by exchanging the bromine or iodine atom in compounds of the formulae (I-1') to (I-11') in which A, B, D, G, Q¹, Q², Q⁵, Q⁶, U, W, X, Y and Z are each as defined above and Z' is preferably bromine or iodine 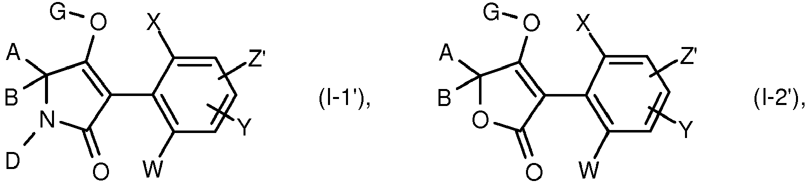 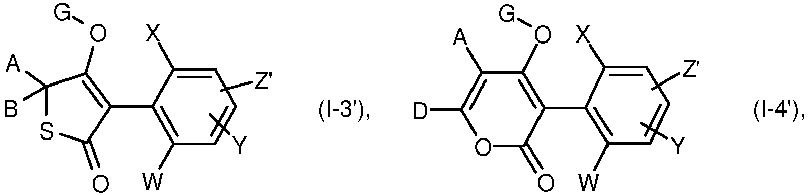 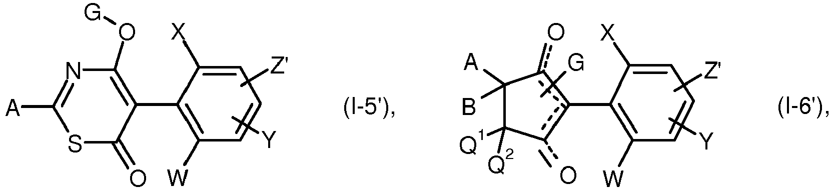 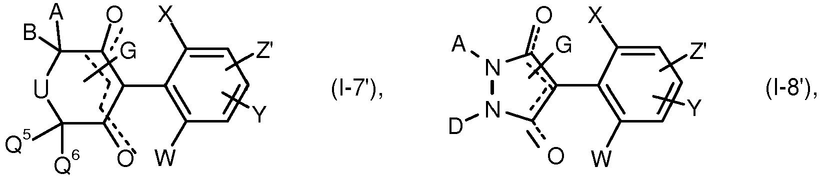 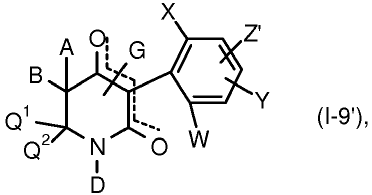 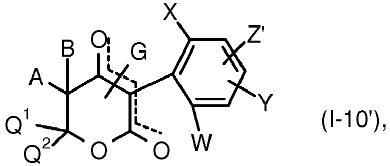 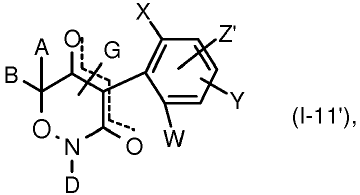 with halogenated alcohols, for example trifluoroethanol of the formula (XXVI)
Z-OH (XXVI)
in the presence of a solvent, in the presence of a copper salt and in the presence of a base.

7. Composition for controlling pests and/or undesired plant growth, **characterized by** a content of at least one compound of the formula (I) according to Claim 1.

8. Method for controlling animal pests and/or undesired plant growth, **characterized in that** compounds of the formula (I) according to Claim 1 are allowed to act on pests, undesired plant growth and/or the habitat thereof, excluding methods of surgical or therapeutic treatment of the human or animal body.

9. Use of compounds of the formula (I) according to Claim 1 for controlling animal pests and/or undesired plant growth, excluding use in methods of surgical or therapeutic treatment of the human or animal body.

10. Method for producing compositions for controlling pests and/or undesired plant growth, **characterized in that** compounds of the formula (I) according to Claim 1 are mixed with extenders and/or surfactants.

11. Use of compounds of the formula (I) according to Claim 1 for producing compositions for controlling pests and/or undesired plant growth.

12. Composition comprising an effective content of an active ingredient combination comprising, as components,
(a') at least one compound of the formula (I) according to Claim 1 in which W, X, Y, Z and CKE are each as defined above and
(b') at least one compound which improves crop plant compatibility from the following group of compounds: S1, S2, S3, S4, S5, S6, S7, S8, S9, S10, S11, S12, S13, S14, S15, wherein
S1) compounds of the formula (S1) 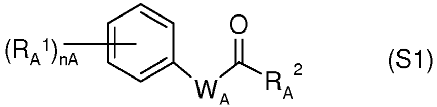 where the symbols and indices are each defined as follows:
n_{A} is a natural number from 0 to 5,
R_{A}¹ is halogen, (C₁-C₄)-alkyl, (C₁-C₄)-alkoxy, nitro or (C₁-C₄)-haloalkyl;
W_{A} is an unsubstituted or substituted divalent heterocyclic radical from the group of partially unsaturated or aromatic five-membered heterocycles having 1 to 3 hetero ring atoms from the group of N and O, where at least one nitrogen atom and at most one oxygen atom is present in the ring,
m_{A} is 0 or 1;
R_{A}² is OR_{A}³, SR_{A}³ or NR_{A}³R_{A}⁴ or a saturated or unsaturated 3- to 7-membered heterocycle having at least one nitrogen atom and up to 3 heteroatoms, preferably from the group of O and S, which is attached via the nitrogen atom to the carbonyl group in (S1) and which is unsubstituted or substituted by radicals from the group of (C₁-C₄)-alkyl, (C₁-C₄)-alkoxy or optionally substituted phenyl,
R_{A}³ is hydrogen or an unsubstituted or substituted aliphatic hydrocarbon radical,
R_{A}⁴ is hydrogen, (C₁-C₆)-alkyl, (C₁-C₆)-alkoxy or substituted or unsubstituted phenyl;
R_{A}⁵ is H, (C₁-C₈)-alkyl, (C₁-C₈)-haloalkyl, (C₁-C₄)-alkoxy-(C₁-C₈)-alkyl, cyano or COOR_{A}⁹ where R_{A}⁹ is hydrogen, (C₁-C₈)-alkyl, (C₁-C₈)-haloalkyl, (C₁-C₄)-alkoxy-(C₁-C₄)-alkyl, (C₁-C₆)-hydroxyalkyl, (C₃-C₁₂)-cycloalkyl or tri-(C₁-C₄)-alkylsilyl;
R_{A}⁶, R_{A}⁷, R_{A}⁸ are identical or different and are hydrogen, (C₁-C₈)-alkyl, (C₁-C₈)-haloalkyl, (C₃-C₁₂)-cycloalkyl or substituted or unsubstituted phenyl;
S2) Quinoline derivatives of the formula (S2) 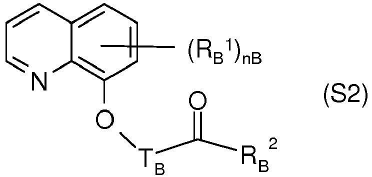 where the symbols and indices are each defined as follows:
R_{B}¹ is halogen, (C₁-C₄)-alkyl, (C₁-C₄)-alkoxy, nitro or (C₁-C₄)-haloalkyl;
n_{B} is a natural number from 0 to 5,
R_{B}² is OR_{B}³, SR_{B}³ or NR_{B}³R_{B}⁴ or a saturated
or unsaturated 3- to 7-membered heterocycle having at least one nitrogen atom and up to 3 heteroatoms, preferably from the group of O and S, which is attached via the nitrogen atom to the carbonyl group in (S2) and which is unsubstituted or substituted by radicals from the group of (C₁-C₄)-alkyl, (C₁-C₄)-alkoxy or optionally substituted phenyl,
R_{B}³ is hydrogen or an unsubstituted or substituted aliphatic hydrocarbon radical,
R_{B}⁴ is hydrogen, (C₁-C₆)-alkyl, (C₁-C₆)-alkoxy or substituted or unsubstituted phenyl;
T_{B} is a (C₁- or C₂)-alkanediyl chain which is unsubstituted or substituted by one or two (C₁-C₄)-alkyl radicals or by (C₁-C₃)-alkoxy]carbonyl;
S3) Compounds of the formula (S3) 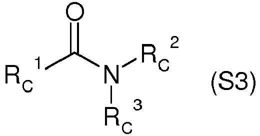 where the symbols and indices are each defined as follows:
R_{C}¹ is (C₁-C₄)-alkyl, (C₁-C₄)-haloalkyl, (C₂-C₄)-alkenyl, (C₂-C₄)-haloalkenyl, (C₃-C₇)-cycloalkyl,
R_{C}², R_{C}³ are identical or different and are hydrogen, (C₁-C₄)-alkyl, (C₂-C₄)-alkenyl, (C₂-C₄)-alkynyl, (C₁-C₄)-haloalkyl, (C₂-C₄)-haloalkenyl, (C₁-C₄)-alkylcarbamoyl-(C₁-C₄)-alkyl, (C₂-C₄)-alkenylcarbamoyl-(C₁-C₄)-alkyl, (C₁-C₄)-alkoxy-(C₁-C₄)-alkyl, dioxolanyl-(C₁-C₄)-alkyl, thiazolyl, furyl, furylalkyl, thienyl, piperidyl, substituted or unsubstituted phenyl, or R_{C}² and R_{C}³ together form a substituted or unsubstituted heterocyclic ring,
S4) N-Acylsulphonamides of the formula (S4) and salts thereof, 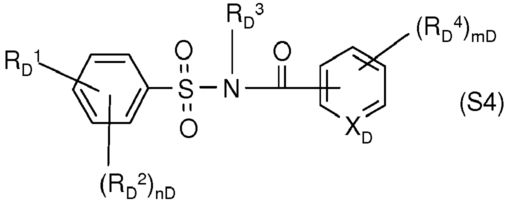 where the symbols and indices are each defined as follows:
X_{D} is CH or N;
R_{D}¹ is CO-NR_{D}⁵R_{D}⁶ or NHCO-R_{D}⁷;
R_{D}² is halogen, (C₁-C₄)-haloalkyl, (C₁-C₄)-haloalkoxy, nitro, (C₁-C₄)-alkyl, (C₁-C₄)-alkoxy, (C₁-C₄)-alkylsulphonyl, (C₁-C₄)-alkoxycarbonyl or (C₁-C₄)-alkylcarbonyl;
R_{D}³ is hydrogen, (C₁-C₄)-alkyl, (C₂-C₄)-alkenyl or (C₂-C₄)-alkynyl;
R_{D}⁴ is halogen, nitro, (C₁-C₄)-alkyl, (C₁-C₄)-haloalkyl, (C₁-C₄)-haloalkoxy, (C₃-C₆)-cycloalkyl, phenyl, (C₁-C₄)-alkoxy, cyano, (C₁-C₄)-alkylthio, (C₁-C₄)-alkylsulphinyl, (C₁-C₄)-alkylsulphonyl, (C₁-C₄)-alkoxycarbonyl or (C₁-C₄)-alkylcarbonyl;
R_{D}⁵ is hydrogen, (C₁-C₆)-alkyl, (C₃-C₆)-cycloalkyl, (C₂-C₆)-alkenyl, (C₂-C₆)-alkynyl, (C₅-C₆)-cycloalkenyl, phenyl or 3- to 6-membered heterocyclyl which contains v_{D} heteroatoms from the group of nitrogen, oxygen and sulphur, where the last seven radicals are substituted by v_{D} substituents from the group of halogen, (C₁-C₆)-alkoxy, (C₁-C₆)-haloalkoxy, (C₁-C₂)-alkylsulphinyl, (C₁-C₂)-alkylsulphonyl, (C₃-C₆)-cycloalkyl, (C₁-C₄)-alkoxycarbonyl, (C₁-C₄)-alkylcarbonyl and phenyl and, in the case of cyclic radicals, also (C₁-C₄)-alkyl and (C₁-C₄)-haloalkyl;
R_{D}⁶ is hydrogen, (C₁-C₆)-alkyl, (C₂-C₆)-alkenyl or (C₂-C₆)-alkynyl, where the last three radicals are substituted by v_{D} radicals from the group of halogen, hydroxy, (C₁-C₄)-alkyl, (C₁-C₄)-alkoxy and (C₁-C₄)-alkylthio, or
R_{D}⁵ and R_{D}⁶ together with the nitrogen atom bearing them form a pyrrolidinyl or piperidinyl radical;
R_{D}⁷ is hydrogen, (C₁-C₄)-alkylamino, di-(C₁-C₄)-alkylamino, (C₁-C₆)-alkyl, (C₃-C₆)-cycloalkyl, where the last 2 radicals are substituted by v_{D} substituents from the group of halogen, (C₁-C₄)-alkoxy, halo- (C₁-C₆)-alkoxy and (C₁-C₄)-alkylthio and, in the case of cyclic radicals, also (C₁-C₄)-alkyl and (C₁-C₄)-haloalkyl;
n_{D} is 0, 1 or 2;
m_{D} is 1 or 2;
v_{D} is 0, 1, 2 or 3;
S5) Active ingredients from the class of the hydroxyaromatics and aromatic-aliphatic carboxylic acid derivatives (S5),
S6) Active ingredients from the class of the 1,2-dihydroquinoxalin-2-ones (S6),
S7) Compounds of the formula (S7), 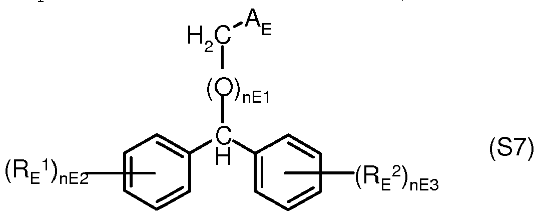 where the symbols and indices are each defined as follows:
R_{E}¹, R_{E}² are each independently halogen, (C₁-C₄)-alkyl, (C₁-C₄)-alkoxy, (C₁-C₄)-haloalkyl, (C₁-C₄)-alkylamino, di-(C₁-C₄)-alkylamino, nitro;
A_{E} is COOR_{E}³ or COSR_{E}⁴
R_{E}³, R_{E}⁴ are each independently hydrogen, (C₁-C₄)-alkyl, (C₂-C₆)-alkenyl, (C₂-C₄)-alkynyl, cyanoalkyl, (C₁-C₄)-haloalkyl, phenyl, nitrophenyl, benzyl, halobenzyl, pyridinylalkyl or alkylammonium,
n_{E}¹ is 0 or 1
n_{E}², n_{E}³ are each independently 0, 1 or 2,
S8) Compounds of the formula (S8), as described in WO-A-98/27049, 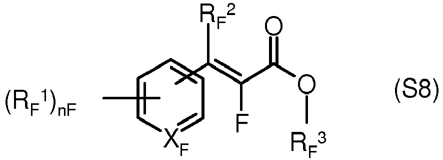 in which
X_{F} is CH or N,
n_{F} is, if X_{F}=N, an integer from 0 to 4 and is, if X_{F}=CH, an integer from 0 to 5,
R_{F}¹ is halogen, (C₁-C₄)-alkyl, (C₁-C₄)-haloalkyl, (C₁-C₄)-alkoxy, (C₁-C₄)-haloalkoxy, nitro, (C₁-C₄)-alkylthio, (C₁-C₄)-alkylsulphonyl, (C₁-C₄)-alkoxycarbonyl, optionally substituted phenyl, optionally substituted phenoxy,
R_{F}² is hydrogen or (C₁-C₄)-alkyl,
R_{F}³ is hydrogen, (C₁-C₈)-alkyl, (C₂-C₄)-alkenyl, (C₂-C₄)-alkynyl or aryl, where each of the carbon-containing radicals mentioned above is unsubstituted or substituted by one or more, identical or different radicals from the group consisting of halogen and alkoxy; or salts thereof,
S9) Active ingredients from the class of the 3-(5-tetrazolylcarbonyl)-2-quinolones (S9),
S10) Compounds of the formula (S10^{a}) or (S10^{b}) 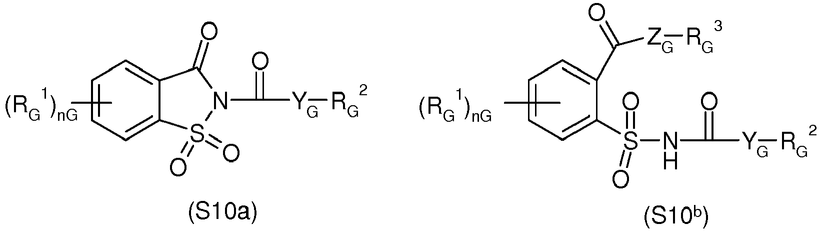 in which
R_{G}¹ is halogen, (C₁-C₄)-alkyl, methoxy, nitro, cyano, CF₃, OCF₃
Y_{G}, Z_{G} are each independently O or S,
n_{G} is an integer from 0 to 4,
R_{G}² is (C₁-C₁₆)-alkyl, (C₂-C₆)-alkenyl, (C₃-C₆)-cycloalkyl, aryl; benzyl, halobenzyl,
R_{G}³ is hydrogen or (C₁-C₆)-alkyl.
S11) Active ingredients of the oxyimino compound type (S11), which are known as seed dressings,
S12) Active ingredients from the class of the isothiochromanones (S12),
S13) One or more compounds from group (S13):
"naphthalic anhydride" (1,8-naphthalenedicarboxylic anhydride) (S13-1),
"fenclorim" (4,6-dichloro-2-phenylpyrimidine) (S13-2),
"flurazole" (benzyl 2-chloro-4-trifluoromethyl-1,3-thiazole-5-carboxylate) (S13-3),
"CL-304415" ((4-carboxy-3,4-dihydro-2H-1-benzopyran-4-acetic acid) (S13-4) "MG-191" (2-dichloromethyl-2-methyl-1,3-dioxolane) (S13-5)
"MG-838" (2-propenyl 1-oxa-4-azaspiro[4.5]decane-4-carbodithioate) (S13-6)
"disulfoton" (0,0-diethyl S-2-ethylthioethyl phosphorodithioate) (S13-7),
"dietholate" (0,0-diethyl 0-phenyl phosphorothioate) (S13-8),
"mephenate" (4-chlorophenyl methylcarbamate) (S13-9).
S14) Active ingredients which, in addition to a herbicidal effect against harmful plants, also have a safener effect on crop plants such as rice,
S15) Active ingredients which are primarily used as herbicides, but also have safener effect on crop plants.

13. Composition according to Claim 12, in which the compound which improves crop plant compatibility is cyprosulfamide.

14. Composition according to Claim 12, in which the compound which improves crop plant compatibility is mefenpyr-diethyl.

15. Method for controlling undesired plant growth, **characterized in that** a composition according to Claim 12 is allowed to act on the plants or the environment thereof.

16. Use of a composition according to Claim 12 for controlling undesired plant growth.

17. Method for controlling undesired plant growth, **characterized in that** a compound of the formula (I) according to Claim 1 and the compound which improves crop plant compatibility according to Claim 12 are allowed to act separately, in close succession, on the plants or the environment thereof.

18. Compounds of the formula (II) 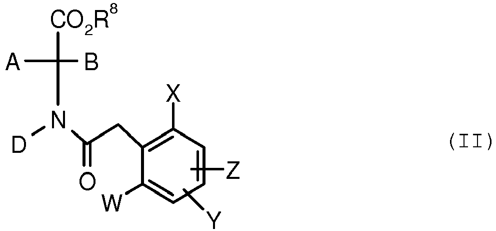
in which A, B, D, W, X, Y and Z are each as defined above,
and R⁸ is alkyl.

19. Compounds of the formula (III) 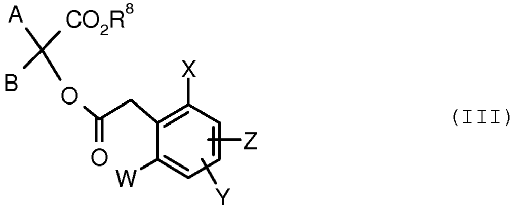 in which A, B, W, X, Y, Z and R⁸ are each as defined above.

20. Compounds of the formula (VIII) 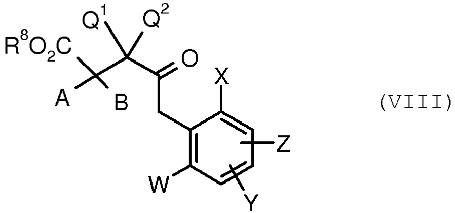 in which A, B, Q¹, Q², W, X, Y, Z and R⁸ are each as defined above.

21. Compounds of the formula (XII) 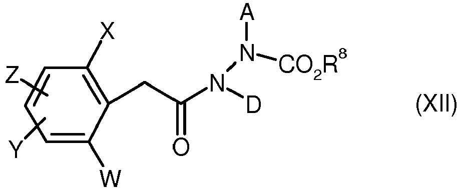 in which
A, D, W, X, Y, Z and R⁸ are each as defined above.

22. Compounds of the formula (XXXI) 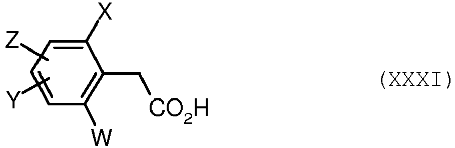 in which W, X, Y and Z are each as defined above, excluding the compound of the formula

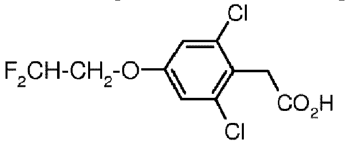

23. Compounds of the formula (XXVIII) 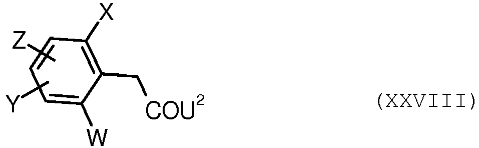 in which
W, X, Y, Z are each as defined above and U² is a leaving group introduced by carboxylic acid activating reagents, carbonyldiimides, phosphorylating reagents, halogenating agents, phosgene or chloroformic esters.

24. Compounds of the formula (XXXV) 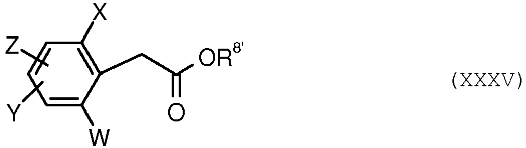 in which W, X, Y, Z are each as defined above and R^{8'} is alkyl.

25. Composition comprising
- at least one compound of the formula (I) according to Claim 1 or a composition according to Claim 12 and
- at least one salt of the formula (III') 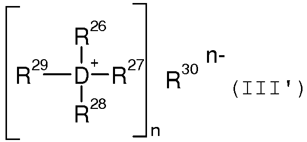 in which
D is nitrogen or phosphorus,
R²⁶, R²⁷, R²⁸ and R²⁹ are each independently hydrogen or in each case optionally substituted C₁-C₈-alkyl or mono or polyunsaturated, optionally substituted C₁-C₈-alkylene, where the substituents may be selected from halogen, nitro and cyano,
n is 1, 2, 3 or 4,
R³⁰ is an inorganic or organic anion.

26. Composition according to Claim 25, **characterized in that** it comprises at least one penetration enhancer.

27. Method for enhancing the action of pesticides and/or herbicides comprising an active ingredient of the formula (I) according to Claim 1 or a composition according to Claim 12, **characterized in that** the ready-to-use composition (spray liquor) is prepared using a salt of the formula (III') according to Claim 25.

28. Method according to Claim 27, **characterized in that** the spray liquor is prepared using a penetration enhancer.

## Revendications

1. Composés de formule (I) 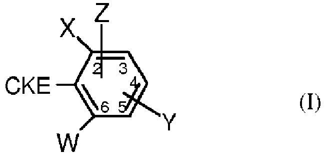 dans laquelle
W représente hydrogène, alkyle, halogène, halogénoalkyle, alcoxy ou halogénoalcoxy,
X représente alkyle, alcényle, alcynyle, halogène, alcoxy, halogénoalkyle, halogénoalcoxy ou cyano,
Y représente hydrogène, alkyle, alcoxy ou halogène,
Z représente un groupe 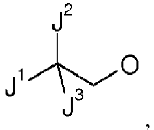
dans lequel J¹ et J² représentent, à chaque fois indépendamment l'un de l'autre, hydrogène ou halogène et J³ représente halogène ou un groupe halogénoalkyle,
CKE représente un des groupes 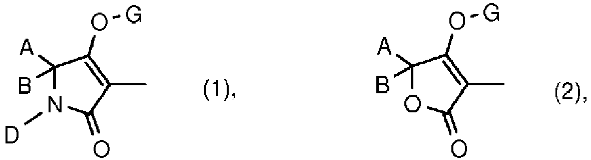 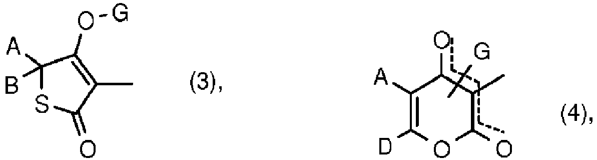 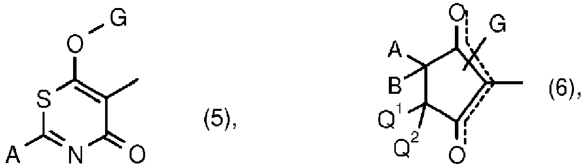 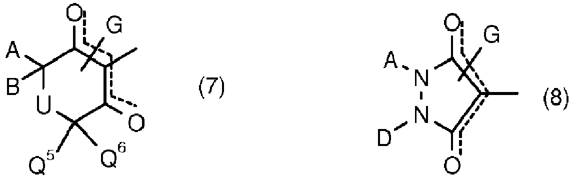 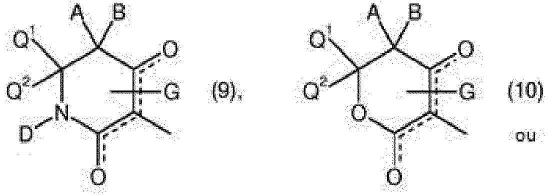 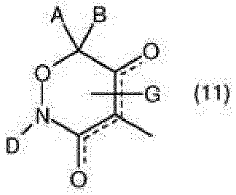 dans lesquels
U représente -S-, -S(O)-, -S(O)₂-, -O-, 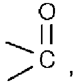 un groupe S=N, S(O)=N ou 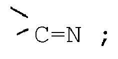 ou C₁-C₄-alkylène le cas échéant substitué par Q³ et Q⁴, qui peut le cas échéant être interrompu par oxygène,
A représente hydrogène ; alkyle, alcényle, alcoxyalkyle, alkylthioalkyle à chaque fois le cas échéant substitué par halogène ; cycloalkyle saturé ou insaturé, le cas échéant substitué, dans lequel le cas échéant au moins un atome de cycle est remplacé par un hétéroatome ; ou aryle, arylalkyle ou hétéroaryle à chaque fois le cas échéant substitué par halogène, alkyle, halogénoalkyle, alcoxy, halogénoalcoxy, cyano ou nitro,
B représente hydrogène, alkyle ou alcoxyalkyle, ou
A et B représentent, ensemble avec l'atome de carbone auquel ils sont liés, un cycle saturé ou insaturé, non substitué ou substitué, contenant le cas échéant au moins un hétéroatome,
D représente hydrogène ou un radical le cas échéant substitué de la série alkyle, alcényle, alcynyle, alcoxyalkyle ; cycloalkyle saturé ou insaturé, dans lequel le cas échéant un ou plusieurs chaînons de cycle sont remplacés par des hétéroatomes ; arylalkyle, aryle, hétéroarylalkyle ou hétéroaryle à chaque fois le cas échéant substitué ou
A et D représentent, ensemble avec les atomes auxquels ils sont liés, un cycle saturé ou insaturé et contenant le cas échéant au moins un hétéroatome (dans le cas de CKE=8 et 11 un autre), non substitué ou substitué dans la partie A,D, ou
A et Q¹ représentent ensemble alcanediyle ou alcènediyle, à chaque fois le cas échéant substitué, qui peuvent le cas échéant être interrompus par au moins un hétéroatome ou un groupe 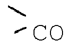 ou 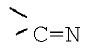 substitué ou
B et Q² représentent, ensemble avec les atomes auxquels ils sont liés, un cycle saturé ou insaturé et contenant le cas échéant au moins un hétéroatome, non substitué ou substitué dans la partie B, Q², ou
D et Q¹ représentent, ensemble avec les atomes auxquels ils sont liés, un cycle saturé ou insaturé et contenant le cas échéant au moins un hétéroatome, non substitué ou substitué dans la partie D, Q¹,
Q¹ représente hydrogène, alkyle, alcoxyalkyle ; cycloalkyle le cas échéant substitué, dans lequel le cas échéant un groupe méthylène est remplacé par oxygène ou soufre ; ou phényle le cas échéant substitué, ou
Q², Q⁴, Q⁵ et Q⁶ représentent, indépendamment les uns des autres, hydrogène ou alkyle,
Q³ représente hydrogène ; alkyle, alcoxy, alkylthio, alcoxyalkyle, alkylthioalkyle à chaque fois le cas échéant substitué ; cycloalkyle le cas échéant substitué, dans lequel le cas échéant un ou deux groupes méthylène sont remplacés par oxygène ou soufre ; ou phényle le cas échéant substitué, ou
Q¹ et Q² représentent, ensemble avec l'atome de carbone auquel ils sont liés, un cycle non substitué ou substitué, contenant le cas échéant un hétéroatome, ou
Q³ et Q⁴ représentent, ensemble avec l'atome de carbone auquel ils sont liés, un cycle saturé ou insaturé, non substitué ou substitué, contenant le cas échéant au moins un hétéroatome, ou
A et Q³ représentent, ensemble avec l'atome de carbone auquel ils sont liés, un cycle saturé ou insaturé, non substitué ou substitué, contenant le cas échéant au moins un hétéroatome, ou
A et Q⁵ représentent, ensemble avec l'atome de carbone auquel ils sont liés, un cycle saturé ou insaturé, non substitué ou substitué, contenant le cas échéant au moins un hétéroatome,
G représente hydrogène (a) ou un des groupes 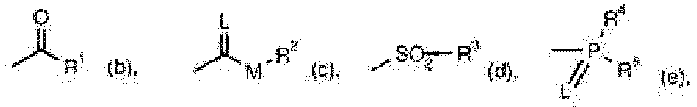
E(f) ou 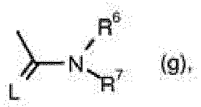 dans lesquels
E représente un équivalent d'ion métallique ou un ion d'ammonium,
L représente oxygène ou soufre,
M représente oxygène ou soufre,
R¹ représente alkyle, alcényle, alcoxyalkyle, alkylthioalkyle, polyalcoxyalkyle à chaque fois le cas échéant substitué par halogène ; ou cycloalkyle le cas échéant substitué par halogène, alkyle ou alcoxy, qui peut être interrompu par au moins un hétéroatome ; phényle, phénylalkyle, hétéroaryle, phénoxyalkyle ou hétéroaryloxyalkyle à chaque fois le cas échéant substitué,
R² représente alkyle, alcényle, alcoxyalkyle, polyalcoxyalkyle à chaque fois le cas échéant substitué par halogène ; ou cycloalkyle, phényle ou benzyle à chaque fois le cas échéant substitué,
R³, R⁴ et R⁵ représentent, indépendamment les uns des autres, alkyle, alcoxy, alkylamino, dialkylamino, alkylthio, alcénylthio, cycloalkylthio à chaque fois le cas échéant substitué par halogène ; ou phényle, benzyle, phénoxy ou phénylthio à chaque fois le cas échéant substitué,
R⁶ et R⁷ représentent, indépendamment l'un de l'autre, hydrogène ; alkyle, cycloalkyle, alcényle, alcoxy, alcoxyalkyle à chaque fois le cas échéant substitué par halogène ; phényle le cas échéant substitué ; benzyle le cas échéant substitué ; ou ensemble avec l'atome de N auquel ils sont liés, un cycle le cas échéant interrompu par oxygène ou soufre.

2. Composés de formule (I) selon la revendication 1, dans laquelle
W représente hydrogène, C₁-C₆-alkyle, halogène, C₁-C₆-alcoxy, C₁-C₄-halogénoalkyle ou C₁-C₄-halogénoalcoxy,
X représente halogène, C₁-C₆-alkyle, C₂-C₆-alcényle, C₂-C₆-alcynyle, C₁-C₆-alcoxy, C₁-C₄-halogénoalkyle, C₁-C₄-halogénoalcoxy ou cyano,
Y représente hydrogène, halogène, C₁-C₆-alkyle ou C₁-C₆-alcoxy,
Z représente un groupe 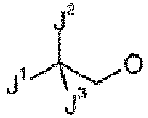
dans lequel J¹ et J² représentent, à chaque fois indépendamment l'un de l'autre, hydrogène, fluor ou chlore et J³ représente halogène ou C₁-C₄-halogénoalkyle,
CKE représente un des groupes 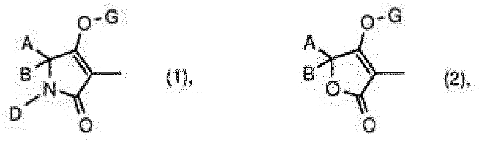 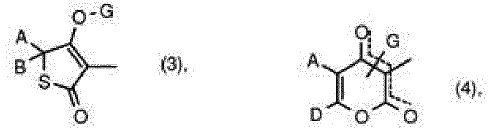 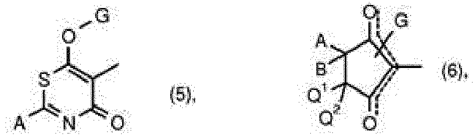 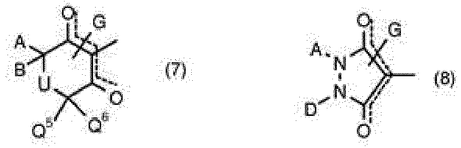 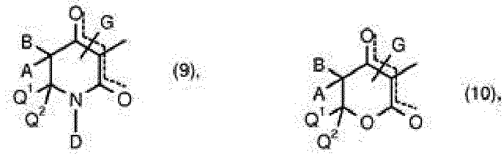 ou 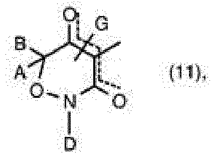 dans lesquels
U représente -S-, -S(O)-, -S(O)₂-, -O-, 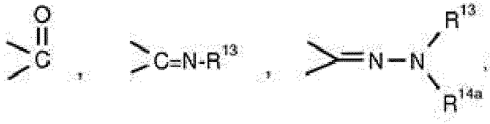
S=N-R¹³, S(O)=N-R¹³ ou 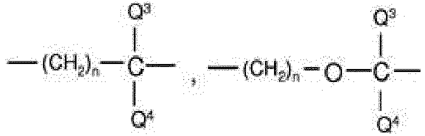 où n représente le nombre 0, 1 ou 2,
A représente hydrogène ; ou C₁-C₁₂-alkyle, C₃-C₈-alcényle, C₁-C₁₀-alcoxy-C₁-C₈-alkyle, C₁-C₁₀-alkylthio-C₁-C₆-alkyle à chaque fois le cas échéant substitué par halogène ; C₃-C₈-cycloalkyle le cas échéant substitué par halogène, C₁-C₆-alkyle ou C₁-C₆-alcoxy, dans lequel le cas échéant un ou deux chaînons de cycle non directement adjacents sont remplacés par oxygène et/ou soufre ; ou phényle, naphtyle, hétéroaryle comprenant 5 à 6 atomes de cycle, phényl-C₁-C₆-alkyle ou naphtyl-C₁-C₆-alkyle à chaque fois le cas échéant substitué par halogène, C₁-C₆-alkyle, C₁-C₆-halogénoalkyle, C₁-C₆-alcoxy, C₁-C₆-halogénoalcoxy, cyano ou nitro,
B représente hydrogène, C₁-C₁₂-alkyle ou C₁-C₈-alcoxy-C₁-C₆-alkyle ou
A, B et l'atome de carbone auquel ils sont liés représentent C₃-C₁₀-cycloalkyle saturé ou C₅-C₁₀-cycloalkyle insaturé, dans lesquels le cas échéant un chaînon de cycle est remplacé par azote, oxygène ou soufre et qui sont le cas échéant monosubstitués ou disubstitués par C₁-C₈-alkyle, C₁-C₈-alcoxy, C₃-C₈-alcényloxy, C₁-C₆-alcoxy-C₁-C₆-alkyle, C₃-C₆-cycloalkyl-C₁-C₂-alcoxy, C₃-C₁₀-cycloalkyle, C₁-C₈-halogénoalkyle, C₂-C₆-halogénoalcoxy, C₁-C₆-alcoxy-C₁-C₄-alcoxy, les radicaux susmentionnés entrant également en ligne de compte comme substituants de N, ou
A, B et l'atome de carbone auquel ils sont liés représentent C₃-C₆-cycloalkyle, qui est substitué par un groupe alkylènediyle contenant le cas échéant un ou deux atomes d'oxygène et/ou de soufre non directement adjacents, le cas échéant substitué par C₁-C₄-alkyle ou par un groupe alkylènedioxy ou par un groupe alkylènedithioyle, qui forme avec l'atome de carbone auquel il est lié un autre cycle de cinq à huit chaînons, ou
A, B et l'atome de carbone auquel ils sont liés représentent C₃-C₈-cycloalkyle ou C₅-C₈-cycloalcényle, dans lesquels deux substituants, ensemble avec les atomes de carbone auxquels ils sont liés, représentent C₂-C₆-alcanediyle, C₂-C₆-alcènediyle ou C₄-C₆-alcanediènediyle à chaque fois le cas échéant substitué par C₁-C₆-alkyle, C₁-C₆-alcoxy ou halogène, dans lesquels le cas échéant un groupe méthylène est remplacé par oxygène ou soufre,
D représente hydrogène ; C₁-C₁₂-alkyle, C₃-C₈-alcényle, C₃-C₈-alcynyle, C₁-C₁₀-alcoxy-C₁-C₈-alkyle à chaque fois le cas échéant substitué par halogène ; C₃-C₈-cycloalkyle le cas échéant substitué par halogène, C₁-C₄-alkyle, C₁-C₄-alcoxy ou C₁-C₄-halogénoalkyle, dans lequel le cas échéant un chaînon de cycle est remplacé par oxygène ou soufre ; ou phényle, hétéroaryle comprenant 5 ou 6 atomes de cycle, phényl-C₁-C₆-alkyle ou hétéroaryl-C₁-C₆-alkyle comprenant 5 ou 6 atomes de cycle, à chaque fois le cas échéant substitué par halogène, C₁-C₆-alkyle, C₁-C₆-halogénoalkyle, C₁-C₆-alcoxy, C₁-C₆-halogénoalcoxy, cyano ou nitro, ou
A et D représentent ensemble C₃-C₆-alcanediyle ou C₃-C₆-alcènediyle à chaque fois le cas échéant substitué, dans lesquels le cas échéant un groupe méthylène est remplacé par un groupe carbonyle, oxygène ou soufre et les substituants qui entrent à chaque fois en considération étant : halogène, hydroxy, mercapto ; ou C₁-C₁₀-alkyle, C₁-C₆-alcoxy, C₁-C₆-alkylthio, C₃-C₇-cycloalkyle, phényle ou benzyloxy à chaque fois le cas échéant substitué par halogène ; ou un autre groupe C₃-C₆-alcanediyle, C₃-C₆-alcènediyle ; ou un groupe butadiényle, qui est le cas échéant substitué par C₁-C₆-alkyle ou dans lequel le cas échéant deux substituants adjacents, avec les atomes de carbone auxquels ils sont liés, forment un autre cycle saturé ou insaturé, comprenant 5 ou 6 atomes de cycle (dans le cas du composé de formule (I-1), A et D représentent alors ensemble avec les atomes auxquels ils sont liés par exemple les autres groupes mentionnés ci-dessous AD-1 à AD-10), qui peut contenir oxygène ou soufre, ou dans lequel le cas échéant un des groupes suivants est contenu 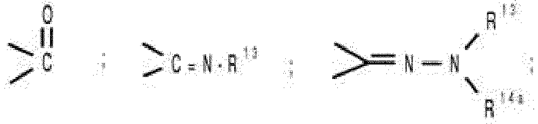 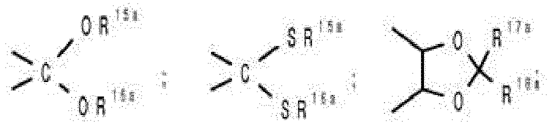 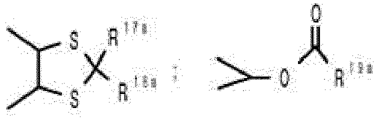
ou 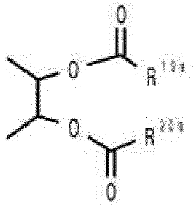 ou
A et Q¹ représentent ensemble avec les atomes de carbone auxquels ils sont liés, C₃-C₆-alcanediyle ou C₄-C₆-alcènediyle, à chaque fois le cas échéant monosubstitué ou disubstitué, de manière identique ou différente par halogène, hydroxy ; par C₁-C₁₀-alkyle, C₁-C₈-alcényle, C₁-C₆-alcoxy, C₁-C₆-alkylthio, C₃-C₇-cycloalkyle à chaque fois le cas échéant monosubstitué à trisubstitué, de manière identique ou différente par halogène ; ou par benzyloxy ou phényle à chaque fois monosubstitué ou trisubstitué, de manière identique ou différente par halogène, C₁-C₆-alkyle ou C₁-C₆-alcoxy, qui contient en outre le cas échéant un des groupes suivants 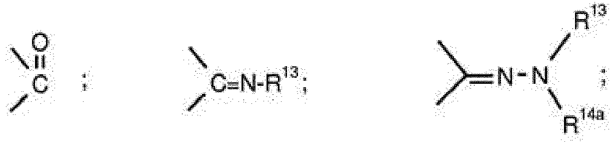 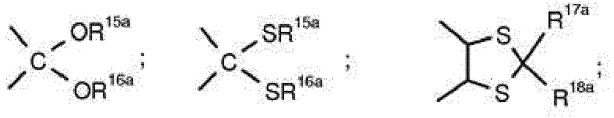 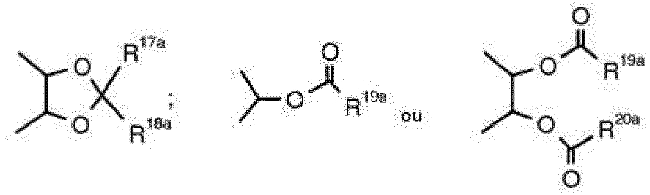
ou qui est ponté par un groupe C₁-C₂-alcanediyle ou par un atome d'oxygène ou
B et Q² représentent ensemble C₁-C₃-alcanediyle le cas échéant substitué par C₁-C₂-alkyle, qui peut le cas échéant être interrompu par oxygène, ou
D et Q¹ représentent, ensemble, C₃-C₆-alcanediyle le cas échéant monosubstitué ou disubstitué, de manière identique ou différente, par C₁-C₄-alkyle, C₁-C₄-alcoxy, ou
Q¹ représente hydrogène, C₁-C₆-alkyle, C₁-C₆-alcoxy-C₁-C₂-alkyle ; C₃-C₈-cycloalkyle le cas échéant substitué par fluor, chlore, C₁-C₄-alkyle, C₁-C₂-halogénoalkyle ou C₁-C₄-alcoxy, dans lequel le cas échéant un groupe méthylène est remplacé par oxygène ou soufre ; ou phényle le cas échéant substitué par halogène, C₁-C₄-alkyle, C₁-C₄-alcoxy, C₁-C₂-halogénoalkyle, C₁-C₂-halogénoalcoxy, cyano ou nitro,
Q², Q⁴, Q⁵ et Q⁶ représentent, indépendamment les uns des autres, hydrogène ou C₁-C₄-alkyle,
Q³ représente hydrogène, C₁-C₆-alkyle, C₁-C₆-alcoxy, C₁-C₆-alkylthio, C₁-C₆-alcoxy-C₁-C₂-alkyle, C₁-C₆-alkylthio-C₁-C₂-alkyle ; C₃-C₈-cycloalkyle le cas échéant substitué par C₁-C₄-alkyle ou C₁-C₄-alcoxy, dans lequel le cas échéant un ou deux groupes méthylène sont remplacés par oxygène ou soufre ; ou phényle le cas échéant substitué par halogène, C₁-C₄-alkyle, C₁-C₄-alcoxy, C₁-C₂-halogénoalkyle, C₁-C₂-halogénoalcoxy, cyano ou nitro, ou
Q¹ et Q² représentent avec l'atome de carbone auquel ils sont liés, un cycle en C₃-C₇ le cas échéant substitué par C₁-C₆-alkyle, C₁-C₆-alcoxy ou C₁-C₂-halogénoalkyle, dans lequel le cas échéant un chaînon de cycle est remplacé par oxygène ou soufre,
Q³ et Q⁴ représentent, ensemble avec l'atome de carbone auquel ils sont liés, un cycle en C₃-C₇ saturé ou insaturé, le cas échéant substitué par C₁-C₄-alkyle, C₁-C₄-alcoxy ou C₁-C₂-halogénoalkyle, dans lequel le cas échéant un ou deux chaînons de cycle sont remplacés par oxygène ou soufre,
A et Q³ représentent, ensemble avec les atomes de carbone auxquels ils sont liés, un cycle en C₃-C₇ saturé ou insaturé, le cas échéant substitué par C₁-C₄-alkyle, C₁-C₄-alcoxy ou C₁-C₂-halogénoalkyle, dans lequel le cas échéant un ou deux chaînons de cycle sont remplacés par oxygène ou soufre,
A et Q⁵ représentent, ensemble avec les atomes de carbone auxquels ils sont liés, un cycle en C₃-C₇ saturé ou insaturé, le cas échéant substitué par C₁-C₄-alkyle, C₁-C₄-alcoxy ou C₁-C₂-halogénoalkyle, dans lequel le cas échéant un chaînon de cycle est remplacé par oxygène ou soufre,
G représente hydrogène (a) ou un des groupes 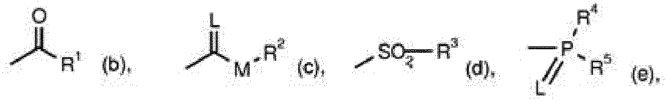
E(f) ou 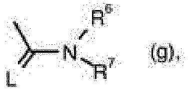 dans lesquels
E représente un équivalent d'ion métallique ou un ion d'ammonium,
L représente oxygène ou soufre et
M représente oxygène ou soufre,
R¹ représente C₁-C₂₀-alkyle, C₂-C₂₀-alcényle, C₁-C₈-alcoxy-C₁-C₈-alkyle, C₁-C₈-alkylthio-C₁-C₈-alkyle, poly-C₁-C₈-alcoxy-C₁-C₈-alkyle à chaque fois le cas échéant substitué par halogène ; ou C₃-C₈-cycloalkyle le cas échéant substitué par halogène, C₁-C₆-alkyle ou C₁-C₆-alcoxy, dans lequel le cas échéant un ou plusieurs chaînons de cycle non directement adjacents sont remplacés par oxygène et/ou soufre ; phényle, le cas échéant substitué par halogène, cyano, nitro, C₁-C₆-alkyle, C₁-C₆-alcoxy, C₁-C₆-halogénoalkyle, C₁-C₆-halogénoalcoxy, C₁-C₆-alkylthio ou C₁-C₆-alkylsulfonyle ; phényl-C₁-C₆-alkyle le cas échéant substitué par halogène, nitro, cyano, C₁-C₆-alkyle, C₁-C₆-alcoxy, C₁-C₆-halogénoalkyle ou C₁-C₆-halogénoalcoxy ; hétéroaryle de 5 ou 6 chaînons, le cas échéant substitué par halogène ou C₁-C₆-alkyle ; phénoxy-C₁-C₆-alkyle le cas échéant substitué par halogène ou C₁-C₆-alkyle ; ou hétéroaryloxy-C₁-C₆-alkyle de 5 ou 6 chaînons, le cas échéant substitué par halogène, amino ou C₁-C₆-alkyle,
R² représente C₁-C₂₀-alkyle, C₂-C₂₀-alcényle, C₁-C₈-alcoxy-C₂-C₈-alkyle, poly-C₁-C₈-alcoxy-C₂-C₈-alkyle à chaque fois le cas échéant substitué par halogène ; C₃-C₈-cycloalkyle le cas échéant substitué par halogène, C₁-C₆-alkyle ou C₁-C₆-alcoxy ; ou phényle ou benzyle à chaque fois le cas échéant substitué par halogène, cyano, nitro, C₁-C₆-alkyle, C₁-C₆-alcoxy, C₁-C₆-halogénoalkyle ou C₁-C₆-halogénoalcoxy,
R³ représente C₁-C₈-alkyle le cas échéant substitué par halogène ; ou phényle ou benzyle à chaque fois le cas échéant substitué par halogène, C₁-C₆-alkyle, C₁-C₆-alcoxy, C₁-C₄-halogénoalkyle, C₁-C₄-halogénoalcoxy, cyano ou nitro,
R⁴ et R⁵ représentent, indépendamment l'un de l'autre, C₁-C₈-alkyle, C₁-C₈-alcoxy, C₁-C₈-alkylamino, di- (C₁-C₈-alkyl) amino, C₁-C₈-alkylthio, C₂-C₈-alcénylthio, C₃-C₇-cycloalkylthio le cas échéant à chaque fois substitué par halogène ; ou phényle, phénoxy ou phénylthio le cas échéant à chaque fois substitué par halogène, nitro, cyano, C₁-C₄-alcoxy, C₁-C₄-halogénoalcoxy, C₁-C₄-alkylthio, C₁-C₄-halogénoalkylthio, C₁-C₄-alkyle ou C₁-C₄-halogénoalkyle,
R⁶ et R⁷ représentent, indépendamment l'un de l'autre, hydrogène, C₁-C₈-alkyle, C₃-C₈-cycloalkyle, C₁-C₈-alcoxy, C₃-C₈-alcényle, C₁-C₈-alcoxy-C₁-C₈-alkyle à chaque fois le cas échéant substitué par halogène ; phényle le cas échéant substitué par halogène, C₁-C₈-halogénoalkyle, C₁-C₈-alkyle ou C₁-C₈-alcoxy ; benzyle le cas échéant substitué par halogène, C₁-C₈-alkyle, C₁-C₈-halogénoalkyle ou C₁-C₈-alcoxy ou ensemble un radical C₃-C₆-alkylène le cas échéant substitué par C₁-C₄-alkyle, dans lequel le cas échéant un atome de carbone est remplacé par oxygène ou soufre,
R¹³ représente hydrogène ; C₁-C₈-alkyle ou C₁-C₈-alcoxy à chaque fois le cas échéant substitué par halogène (uniquement dans le cas du groupe C=N-R¹³) ; C₃-C₈-cycloalkyle le cas échéant substitué par halogène, C₁-C₄-alkyle ou C₁-C₄-alcoxy, dans lequel le cas échéant un groupe méthylène est remplacé par oxygène ou soufre ; ou phényle, phényl-C₁-C₄-alkyle, hétéroaryl-C₁-C₄-alkyle à chaque fois le cas échéant substitué par halogène, C₁-C₆-alkyle, C₁-C₆-alcoxy, C₁-C₄-halogénoalkyle, C₁-C₄-halogénoalcoxy, nitro ou cyano ; ou, uniquement dans le cas du groupe C=N-R¹³, phényl-C₁-C₄-alcoxy ou hétéroaryl-C₁-C₄-alcoxy,
R^{14a} représente hydrogène ou C₁-C₈-alkyle, ou
R¹³ et R^{14a} représentent ensemble C₄-C₆-alcanediyle le cas échéant substitué par C₁-C₄-alkyle, qui peut le cas échéant être interrompu par oxygène ou soufre,
R^{15a} et R^{16a} sont identiques ou différents et représentent C₁-C₆-alkyle, ou
R^{15a} et R^{16a} représentent ensemble un radical C₂-C₄-alcanediyle ou un radical C₄-alcanediyle, qui est le cas échéant substitué par C₁-C₆-alkyle, C₁-C₆-halogénoalkyle ou par phényle, le cas échéant substitué par halogène, C₁-C₆-alkyle, C₁-C₄-halogénoalkyle, C₁-C₆-alcoxy, C₁-C₄-halogénoalcoxy, nitro ou cyano,
R^{17a} et R^{18a} représentent, indépendamment l'un de l'autre, hydrogène ; C₁-C₈-alkyle le cas échéant substitué par halogène ; ou phényle le cas échéant substitué par halogène, C₁-C₆-alkyle, C₁-C₆-alcoxy, C₁-C₄-halogénoalkyle, C₁-C₄-halogénoalcoxy, nitro ou cyano, ou
R^{17a} et R^{18a} représentent, ensemble avec l'atome de carbone auquel ils sont liés, un groupe carbonyle ; ou C₅-C₇-cycloalkyle le cas échéant substitué par halogène, C₁-C₄-alkyle ou C₁-C₄-alcoxy, dans lequel le cas échéant un groupe méthylène est remplacé par oxygène ou soufre,
R^{19a} et R^{20a} représentent, indépendamment l'un de l'autre, C₁-C₁₀-alkyle, C₂-C₁₀-alcényle, C₁-C₁₀-alcoxy, C₁-C₁₀-alkylamino, C₃-C₁₀-alcénylamino, di-(C₁-C₁₀-alkyl) amino ou di-(C₃-C₁₀-alcényl) amino.

3. Composés de formule (I) selon la revendication 1, dans laquelle
W représente hydrogène, fluor, chlore, brome, C₁-C₄-alkyle, C₁-C₄-alcoxy, C₁-C₂-halogénoalkyle ou C₁-C₂-halogénoalcoxy,
X représente chlore, brome, iode, C₁-C₄-alkyle, C₂-C₄-alcényle, C₂-C₄-alcynyle, C₁-C₄-alcoxy, C₁-C₂-halogénoalkyle, C₁-C₂-halogénoalcoxy ou cyano,
Y représente hydrogène, méthyle, éthyle, fluor, chlore, brome, iode, méthoxy ou éthoxy,
Z représente le groupe 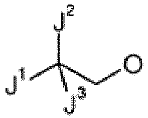
dans lequel J¹ et J² représentent, à chaque fois indépendamment l'un de l'autre, hydrogène, fluor ou chlore et J³ représente fluor, chlore, trichlorométhyle, difluorométhyle, difluorochlorométhyle, dichlorofluorométhyle ou trifluorométhyle,
CKE représente un des groupes 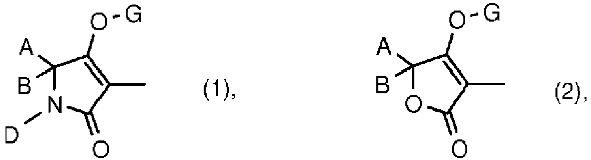 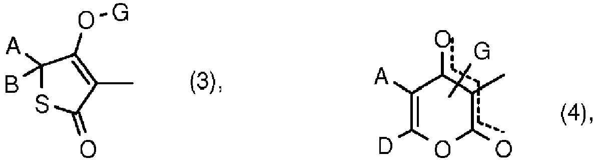 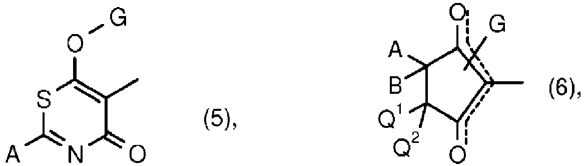 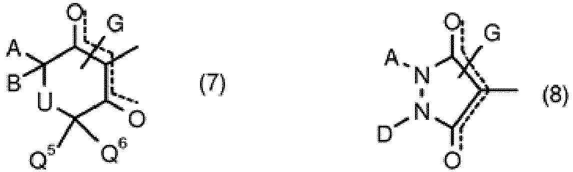 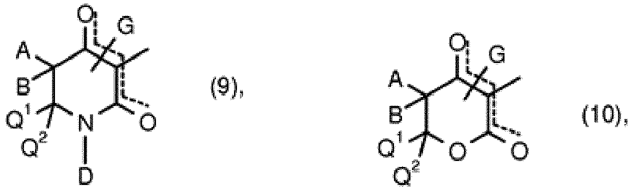
ou 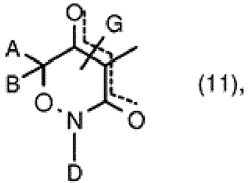
U représente -CH₂-, -CH₂-CH₂-, -O- ou 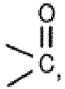
A représente hydrogène ; C₁-C₆-alkyle, C₁-C₄-alcoxy-C₁-C₂-alkyle à chaque fois le cas échéant monosubstitué à trisubstitué par fluor ou chlore ; C₃-C₆-cycloalkyle le cas échéant monosubstitué à disubstitué par C₁-C₂-alkyle ou C₁-C₂-alcoxy et le cas échéant interrompu par un atome d'oxygène ; ou (mais pas dans le cas des composés des formules (I-3), (1-4), (1-6), (1-7), (1-9), (1-10) et (I-11)) phényle, pyridyle ou benzyle à chaque fois le cas échéant monosubstitué à disubstitué par fluor, chlore, brome, C₁-C₄-alkyle, C₁-C₂-halogénoalkyle, C₁-C₄-alcoxy, C₁-C₂-halogénoalcoxy, cyano ou nitro,
B représente hydrogène, C₁-C₄-alkyle ou C₁-C₂-alcoxy-C₁-C₂-alkyle ou
A, B et l'atome de carbone auquel ils sont liés représentent C₃-C₇-cycloalkyle saturé ou insaturé, dans lequel le cas échéant un chaînon de cycle est remplacé par azote, oxygène ou soufre et qui est le cas échéant monosubstitué à disubstitué par C₁-C₆-alkyle, C₁-C₄-alcoxy-C₁-C₂-alkyle, trifluorométhyle, C₁-C₆-alcoxy, C₃-C₆-alcényloxy, trifluoroéthoxy, C₁-C₃-alcoxy-C₁-C₃-alkyle ou C₃-C₆-cycloalkylméthoxy, les radicaux susmentionnés entrant également en ligne de compte comme substituants de N, à condition que Q³ représente alors hydrogène ou méthyle ou
A, B et l'atome de carbone, auquel ils sont liés représentent C₅-C₆-cycloalkyle, qui est substitué par un groupe alkylènediyle, contenant le cas échéant un ou deux atomes d'oxygène ou de soufre non directement adjacents, le cas échéant substitué par méthyle ou éthyle ; ou par un groupe alkylènedioxy ou alkylènedithiol, qui forme, avec l'atome de carbone auquel il est lié, un autre cycle de cinq ou six chaînons, à condition que Q³ représente alors hydrogène ou méthyle ou
A, B et l'atome de carbone auquel ils sont liés représentent C₃-C₆-cycloalkyle ou C₅-C₆-cycloalcényle, dans lesquels deux substituants, ensemble avec les atomes de carbone auxquels ils sont liés, représentent C₂-C₄-alcanediyle, C₂-C₄-alcènediyle ou butadiènediyle à chaque fois le cas échéant substitué par C₁-C₂-alkyle ou C₁-C₂-alcoxy, à condition que Q³ représente alors hydrogène ou méthyle,
D représente hydrogène ; C₁-C₆-alkyle, C₃-C₆-alcényle, C₁-C₄-alcoxy-C₁-C₃-alkyle à chaque fois le cas échéant monosubstitué à trisubstitué par fluor ; C₃-C₆-cycloalkyle, le cas échéant monosubstitué à disubstitué par C₁-C₄-alkyle, C₁-C₄-alcoxy ou C₁-C₂-halogénoalkyle, dans lequel le cas échéant un groupe méthylène est remplacé par oxygène ; ou (uniquement dans le cas des composés de formule (I-4)) phényle ou pyridyle à chaque fois le cas échéant monosubstitué à disubstitué par fluor, chlore, brome, C₁-C₄-alkyle, C₁-C₄-halogénoalkyle, C₁-C₄-alcoxy ou C₁-C₄-halogénoalcoxy, ou
A et D représentent ensemble C₃-C₅-alcanediyle le cas échéant monosubstitué à disubstitué, dans lequel un groupe méthylène peut être remplacé par un groupe carbonyle (mais pas dans le cas des composés de formule (I-11)), oxygène ou soufre et où les substituants qui entrent en considération sont C₁-C₂-alkyle ou C₁-C₂-alcoxy, ou
A et D (dans le cas des composés de formule (I-1)) ensemble avec les atomes, auxquels ils sont liés, représentent un des groupes AD-1 à AD-10 : 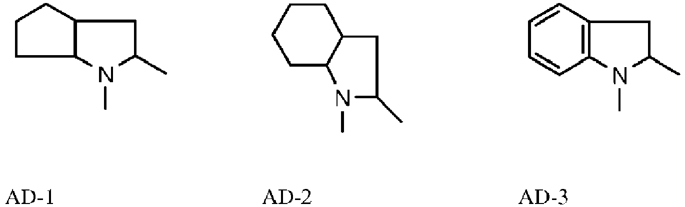 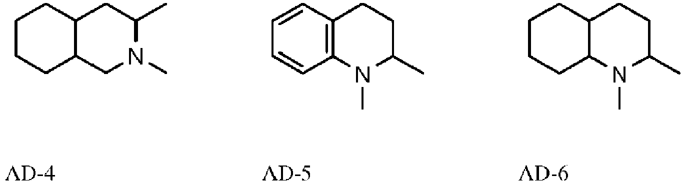 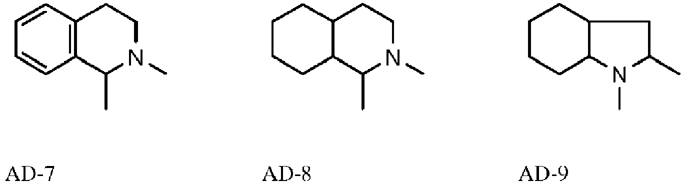 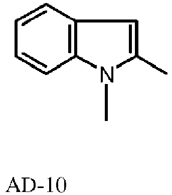
ou
A et D représentent ensemble C₃-C₅-alcanediyle, qui est le cas échéant substitué par un groupe alkylènedioxy contenant deux atomes d'oxygène non directement adjacents, le cas échéant monosubstitué à tétrasubstitué par C₁-C₄-alkyle ou C₁-C₃-alcoxy-C₁-C₂-alkyle, un autre cycle de 5 ou 6 chaînons étant formé, ou
A et Q¹ représentent, ensemble, C₃-C₄-alcanediyle le cas échéant monosubstitué ou disubstitué, de manière identique ou différente, par C₁-C₂-alkyle ou C₁-C₄-alcoxy, qui contient le cas échéant le groupe suivant : 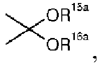
dans lequel
R^{15a} et R^{16a} représentent, en étant identiques ou différents, méthyle ou éthyle, ou
R^{15a} et R^{16a} représentent, ensemble, un radical C₂-C₄-alcanediyle ou un radical C₄-alcènediyle qui est le cas échéant substitué par méthyle ou éthyle, ou
B et Q² représentent ensemble -CH₂-, -CH₂-CH₂-, -CH₂-CH₂-CH₂- ou -CH₂-O-CH₂- ou
D et Q¹ représentent ensemble C₃-C₄-alcanediyle, ou
Q¹ représente hydrogène, C₁-C₄-alkyle, C₁-C₄-alcoxy-C₁-C₂-alkyle ; ou C₃-C₆-cycloalkyle le cas échéant substitué par méthyle ou méthoxy, dans lequel le cas échéant un groupe méthylène est remplacé par oxygène,
Q² représente hydrogène, méthyle ou éthyle,
Q⁴, Q⁵ et Q⁶ représentent, indépendamment les uns des autres, hydrogène ou C₁-C₃-alkyle,
Q³ représente hydrogène, C₁-C₄-alkyle, C₁-C₄-alcoxy ; ou C₃-C₆-cycloalkyle le cas échéant monosubstitué à disubstitué par méthyle ou méthoxy, le cas échéant interrompu par un atome d'oxygène ou
Q¹ et Q² avec le carbone auquel ils sont liés représentent C₃-C₆-cycloalkyle le cas échéant substitué par méthyle ou méthoxy, dans lequel le cas échéant un groupe méthylène est remplacé par oxygène, à condition que A et B représentent alors, indépendamment l'un de l'autre, hydrogène ou méthyle, ou
Q³ et Q⁴ représentent, ensemble avec le carbone auquel ils sont liés, un cycle en C₅-C₆ saturé, le cas échéant substitué par C₁-C₂-alkyle ou C₁-C₂-alcoxy, dans lequel le cas échéant un ou deux chaînons de cycle sont remplacés par oxygène ou soufre, à condition que A représente alors hydrogène ou méthyle, ou
A et Q³ représentent, ensemble avec le carbone auquel ils sont liés, un cycle en C₅-C₆ saturé, le cas échéant substitué par C₁-C₂-alkyle ou C₁-C₂-alcoxy, dans lequel le cas échéant un chaînon de cycle est remplacé par oxygène ou soufre, à condition que B, Q⁴, Q⁵ et Q⁶ représentent alors indépendamment les uns des autres hydrogène ou méthyle, ou
A et Q⁵ représentent, ensemble avec les atomes de carbone auxquels ils sont liés, un cycle en C₅-C₆ saturé ou insaturé, le cas échéant substitué par C₁-C₂-alkyle ou C₁-C₂-alcoxy, à condition que B, Q³, Q⁴ et Q⁶ représentent alors indépendamment les uns des autres hydrogène ou méthyle,
G représente hydrogène (a) ou un des groupes 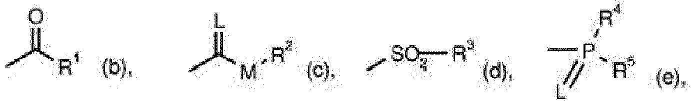
E(f) ou 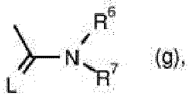 dans lesquels
E représente un équivalent d'ion métallique ou un ion d'ammonium,
L représente oxygène ou soufre et
M représente oxygène ou soufre,
R¹ représente C₁-C₈-alkyle, C₂-C₈-alcényle, C₁-C₄-alcoxy-C₁-C₂-alkyle, C₁-C₄-alkylthio-C₁-C₂-alkyle à chaque fois le cas échéant monosubstitué à trisubstitué par fluor ou chlore ; ou C₃-C₆-cycloalkyle le cas échéant monosubstitué à disubstitué par fluor, chlore, C₁-C₂-alkyle ou C₁-C₂-alcoxy, dans lequel le cas échéant un ou deux chaînons de cycle non directement adjacents sont remplacés par oxygène ; phényle le cas échéant monosubstitué à disubstitué par fluor, chlore, brome, cyano, nitro, C₁-C₄-alkyle, C₁-C₄-alcoxy, C₁-C₂-halogénoalkyle ou C₁-C₂-halogénoalcoxy,
R² représente C₁-C₈-alkyle, C₂-C₈-alcényle ou C₁-C₄-alcoxy-C₂-C₄-alkyle à chaque fois le cas échéant monosubstitué à trisubstitué par fluor ; C₃-C₆-cycloalkyle le cas échéant monosubstitué par C₁-C₂-alkyle ou C₁-C₂-alcoxy ; ou phényle ou benzyle à chaque fois le cas échéant monosubstitué à disubstitué par fluor, chlore, brome, cyano, nitro, C₁-C₄-alkyle, C₁-C₃-alcoxy, trifluorométhyle ou trifluorométhoxy,
R³ représente C₁-C₆-alkyle le cas échéant monosubstitué à trisubstitué par fluor ; ou phényle le cas échéant monosubstitué par fluor, chlore, brome, C₁-C₄-alkyle, C₁-C₄-alcoxy, trifluorométhyle, trifluorométhoxy, cyano ou nitro,
R⁴ représente C₁-C₆-alkyle, C₁-C₆-alcoxy, C₁-C₆-alkylamino, di- (C₁-C₆-alkyl) amino, C₁-C₆-alkylthio, C₃-C₄-alcénylthio, C₃-C₆-cycloalkylthio ou phényle, phénoxy ou phénylthio à chaque fois le cas échéant monosubstitué par fluor, chlore, brome, nitro, cyano, C₁-C₃-alcoxy, C₁-C₃-halogénoalcoxy, C₁-C₃-alkylthio, C₁-C₃-halogénoalkylthio, C₁-C₃-alkyle ou trifluorométhyle,
R⁵ représente C₁-C₆-alcoxy ou C₁-C₆-alkylthio,
R⁶ représente hydrogène ; C₁-C₆-alkyle, C₃-C₆-cycloalkyle, C₁-C₆-alcoxy, C₃-C₆-alcényle, C₁-C₆-alcoxy-C₁-C₄-alkyle ; phényle le cas échéant monosubstitué par fluor, chlore, brome, trifluorométhyle, C₁-C₄-alkyle ou C₁-C₄-alcoxy ; benzyle le cas échéant monosubstitué par fluor, chlore, brome, C₁-C₄-alkyle, trifluorométhyle ou C₁-C₄-alcoxy,
R⁷ représente C₁-C₆-alkyle, C₃-C₆-alcényle ou C₁-C₆-alcoxy-C₁-C₄-alkyle,
R⁶ et R⁷ représentent ensemble un radical C₄-C₅-alkylène le cas échéant substitué par méthyle ou éthyle, dans lequel le cas échéant un groupe méthylène est remplacé par oxygène ou soufre.

4. Composés de formule (I) selon la revendication 1, dans laquelle
W représente hydrogène, chlore, méthyle ou éthyle,
X représente chlore, méthyle, éthyle, méthoxy ou éthoxy,
Y représente hydrogène, méthyle ou chlore,
Z représente le groupe 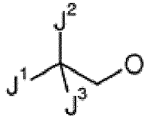
dans lequel J¹ et J² représentent à chaque fois indépendamment hydrogène ou fluor et J³ représente fluor, chlore ou trifluorométhyle, CKE représente un des groupes 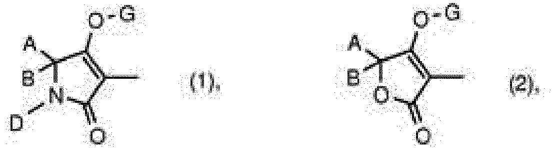 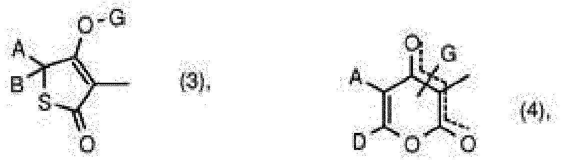 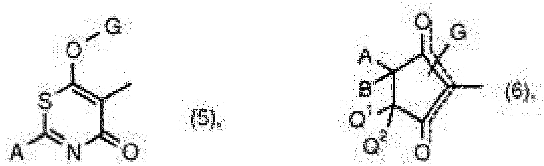 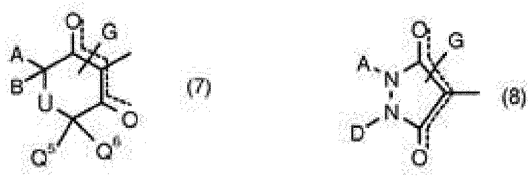 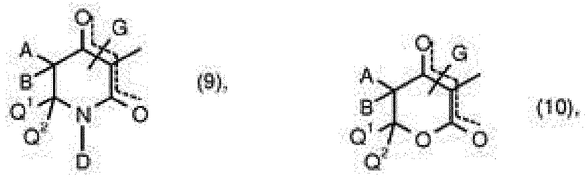 ou 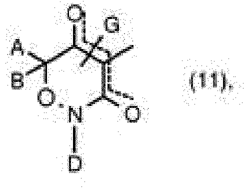
U représente -CH₂-, -CH₂-CH₂-, -O- ou 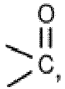
A représente hydrogène ; C₁-C₄-alkyle ou C₁-C₂-alcoxy-C₁-C₂-alkyle à chaque fois le cas échéant monosubstitué à trisubstitué par fluor ; cyclopropyle, cyclopentyle ou cyclohexyle ; et dans le cas des composés de formule (1-5), phényle le cas échéant monosubstitué à disubstitué par fluor, chlore, brome, méthyle, éthyle, n-propyle, iso-propyle, méthoxy, éthoxy, trifluorométhyle, trifluorométhoxy, cyano ou nitro,
B représente hydrogène, méthyle ou éthyle ou
A, B et l'atome de carbone auquel ils sont liés représentent C₅-C₆-cycloalkyle saturé, dans lequel le cas échéant un chaînon de cycle est remplacé par azote, oxygène ou soufre et qui est le cas échéant monosubstitué ou disubstitué par méthyle, éthyle, méthoxyméthyle, éthoxyméthyle, méthoxyéthyle, éthoxyéthyle, trifluorométhyle, méthoxy, éthoxy, propoxy, butoxy, méthoxyéthoxy, éthoxyéthoxy, allyloxy, trifluoréthoxy ou cyclopropylméthoxy, les radicaux susmentionnés entrant également en ligne de compte comme substituants de N, à condition que Q³ représente alors hydrogène ou méthyle ou
A, B et l'atome de carbone auquel ils sont liés représentent C₆-cycloalkyle, qui est le cas échéant substitué par un groupe alkylidènediyle le cas échéant interrompu par un atome d'oxygène ou substitué par un groupe alkylènedioxy contenant deux atomes d'oxygène non directement adjacents, un autre cycle de 5 ou 6 chaînons étant formé (qui peut être monosubstitué ou disubstitué par méthyle) à condition que Q³ représente alors hydrogène ou
A, B et l'atome de carbone auquel ils sont liés représentent C₅-C₆-cycloalkyle ou C₅-C₆-cycloalcényle, dans lesquels deux substituants, ensemble avec les atomes de carbone auxquels ils sont liés, représentent C₂-C₄-alcanediyle, C₂-C₄-alcènediyle ou butadiènediyle, à condition que Q³ représente alors hydrogène,
D représente hydrogène ; C₁-C₄-alkyle, C₃-C₄-alcényle, C₁-C₄-alcoxy-C₁-C₃-alkyle à chaque fois le cas échéant monosubstitué à trisubstitué par fluor ; cyclopropyle, cyclopentyle ou cyclohexyle ou (dans le cas des composés de formule (1-4)), phényle ou pyridyle à chaque fois le cas échéant monosubstitué par fluor, chlore, méthyle, éthyle, n-propyle, iso-propyle, méthoxy, éthoxy ou trifluorométhyle,
A et D représentent ensemble C₃-C₅-alcanediyle le cas échéant monosubstitué par méthyle ou méthoxy, dans lequel le cas échéant un atome de carbone est remplacé par un groupe carbonyle (mais pas dans le cas des composés de formule (1-11)), oxygène ou soufre ; ou le groupe AD-1, ou
A et D représentent ensemble C₃-C₅-alcanediyle, qui est le cas échéant substitué par un groupe alkylènedioxy contenant deux atomes d'oxygène non directement adjacents, le cas échéant monosubstitué à disubstitué par C₁-C₂-alkyle, un autre cycle de 5 chaînons étant formé, ou
A et Q¹ représentent, ensemble, C₃-C₄-alcanediyle le cas échéant monosubstitué ou disubstitué par méthyle ou méthoxy, qui contient le cas échéant le groupe suivant : 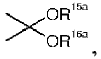
dans lequel R^{15a} et R^{16a} représentent ensemble un radical C₂-C₄-alcanediyle ou C₄-alcènediyle, ou
B et Q² représentent ensemble -CH₂-CH₂-CH₂-ou -CH₂-O-CH₂- ou
D et Q¹ représentent ensemble C₃-C₄-alcanediyle, ou
Q¹ représente hydrogène, méthyle, éthyle, propyle, iso-propyle, cyclopropyle, cyclopentyle ou cyclohexyle,
Q² représente hydrogène, méthyle ou éthyle,
Q⁴, Q⁵ et Q⁶ représentent, indépendamment les uns des autres, hydrogène ou méthyle,
Q³ représente hydrogène, méthyle, éthyle, propyle, méthoxy ou éthoxy ; ou C₃-C₆-cycloalkyle le cas échéant monosubstitué par méthyle ou méthoxy, le cas échéant interrompu par un atome d'oxygène, ou
Q¹ et Q² avec l'atome de carbone auquel ils sont liés représentent C₅-C₆-cycloalkyle le cas échéant substitué par méthyle ou méthoxy, dans lequel le cas échéant un groupe méthylène est remplacé par oxygène, à condition que A et B représentent hydrogène, ou
Q³ et Q⁴ représentent, ensemble avec le carbone auquel ils sont liés, un cycle en C₅-C₆ saturé, le cas échéant monosubstitué par méthyle ou méthoxy, le cas échéant interrompu par un atome d'oxygène, à condition que A, B, Q⁵ et Q⁶ représentent alors hydrogène,
G représente hydrogène (a) ou un des groupes 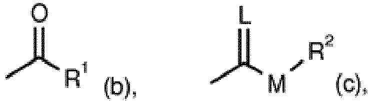
-SO₂-R³ (d) ou E (f) dans lesquels
L représente oxygène ou soufre,
M représente oxygène ou soufre et
E représente un équivalent d'ion métallique ou un ion d'ammonium,
R¹ représente C₁-C₆-alkyle, C₂-C₆-alcényle, C₁-C₂-alcoxy-C₁-alkyle, C₁-C₂-alkylthio-C₁-alkyle à chaque fois le cas échéant monosubstitué par chlore ; ou cyclopropyle ou cyclohexyle à chaque fois le cas échéant monosubstitué par fluor, chlore, méthyle ou méthoxy ; phényle le cas échéant monosubstitué par fluor, chlore, brome, cyano, nitro, méthyle, méthoxy, trifluorométhyle ou trifluorométhoxy,
R² représente C₁-C₈-alkyle, C₂-C₆-alcényle ou C₁-C₄-alcoxy-C₂-C₃-alkyle à chaque fois le cas échéant monosubstitué à trisubstitué par fluor ; phényle ou benzyle,
R³ représente C₁-C₈-alkyle.

5. Composés de formule (I) selon la revendication 1, dans laquelle
W représente hydrogène, méthyle ou éthyle,
X représente chlore, méthyle ou éthyle,
Y représente hydrogène,
Z représente OCH₂-CF₃ en 3ème position, ou
Z représente OCH₂-CF₃ en 4ème position, ou
Z représente OCH₂-CF₃ en 5ème position, CKE représente un des groupes 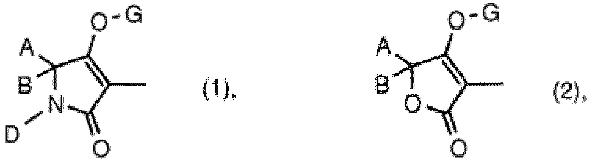 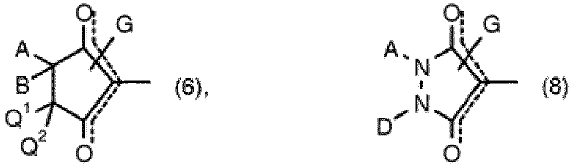
A représente méthyle ou éthyle,
B représente hydrogène ou méthyle,
A, B et l'atome de carbone auquel ils sont liés représentent C₅-C₆-cycloalkyle saturé, dans lequel le cas échéant un chaînon de cycle est remplacé par oxygène et qui est le cas échéant monosubstitué ou disubstitué par méthyle, éthyle, méthoxyméthyle, méthoxy, éthoxy, propoxy, butoxy, trifluoroéthoxy, ou
A, B et l'atome de carbone auquel ils sont liés représentent C₆-cycloalkyle, qui est le cas échéant substitué par un groupe alkylènedioxy contenant deux atomes d'oxygène non directement adjacents, un autre cycle de 5 ou 6 chaînons étant formé, qui peut être monosubstitué ou disubstitué par méthyle,
D représente hydrogène, ou
A et D représentent ensemble C₃-C₅-alcanediyle, dans lequel le cas échéant un atome de carbone est remplacé par oxygène, ou
A et D représentent ensemble C₃-C₅-alcanediyle, qui est le cas échéant substitué par un groupe alkylènedioxy contenant deux atomes d'oxygène non directement adjacents, le cas échéant monosubstitué à disubstitué par méthyle, un autre cycle de 5 chaînons étant formé, ou
A et Q¹ représentent ensemble C₃-C₄-alcanediyle,
Q² représente hydrogène,
G représente hydrogène (a) ou un des groupes 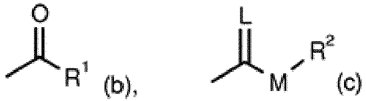
dans lesquels
L représente oxygène,
M représente oxygène,
R¹ représente alkyle en C₁-C₆,
R² représente C₁-C₆-alkyle.

6. Procédé pour la préparation de composés de formule (I) selon la revendication 1, **caractérisé en ce que**, pour obtenir
(A) des composés de formule (I-1-a) 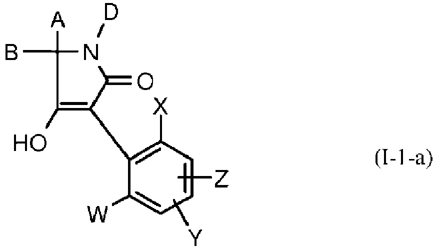 dans laquelle
A, B, D, W, X, Y et Z présentent les significations indiquées ci-dessus, des composés de formule (II) 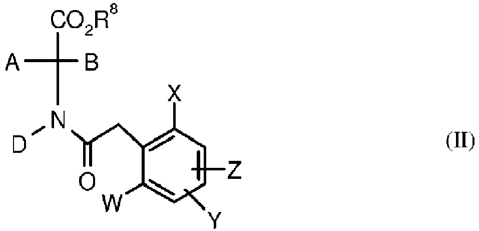
dans laquelle
A, B, D, W, X, Y et Z présentent les significations indiquées ci-dessus, et
R⁸ représente alkyle,
sont condensés de manière intramoléculaire en présence d'un diluant et en présence d'une base,
(B) des composés de formule (I-2-a) 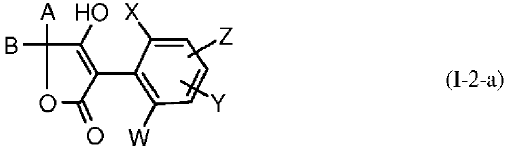 dans laquelle
A, B, W, X, Y et Z présentent les significations indiquées ci-dessus, des composés de formule (III) 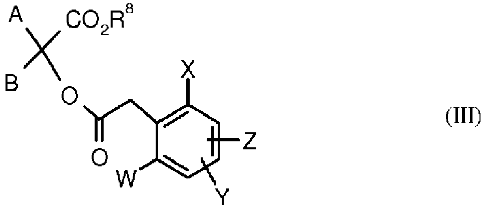
dans laquelle
A, B, W, X, Y, Z et R⁸ présentent les significations indiquées ci-dessus, sont condensés de manière intramoléculaire en présence d'un diluant et en présence d'une base,
(C) des composés de formule (I-3-a) 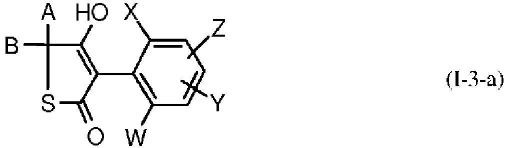 dans laquelle
A, B, W, X, Y et Z présentent les significations indiquées ci-dessus, des composés de formule (IV) 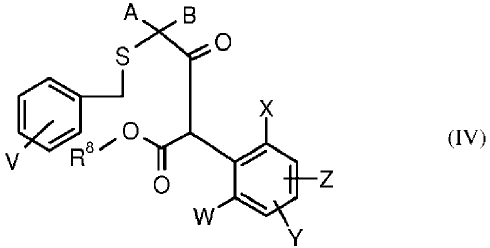
dans laquelle
A, B, W, X, Y, Z et R⁸ présentent les significations indiquées ci-dessus, et
V représente hydrogène, halogène, alkyle ou alcoxy,
sont cyclisés de manière intramoléculaire, le cas échéant en présence d'un diluant et en présence d'un acide,
(D) des composés de formule (I-4-a) 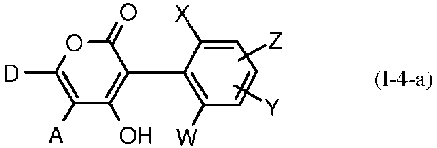 dans laquelle
A, D, W, X, Y et Z présentent les significations indiquées ci-dessus,
des composés de formule (V) 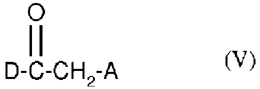 dans laquelle
A et D présentent les significations indiquées ci-dessus,
ou leurs silylénoléthers de formule (Va) 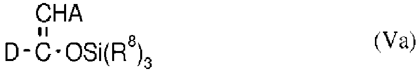 dans laquelle
A, D et R⁸ présentent la signification indiquée ci-dessus,
sont transformés avec des composés de formule (VI) 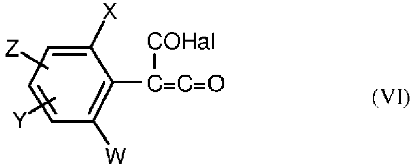 dans laquelle
W, X, Y et Z présentent les significations indiquées ci-dessus, et
Hal représente halogène,
le cas échéant en présence d'un diluant et le cas échéant en présence d'un accepteur d'acide,
(E) des composés de formule (I-5-a) 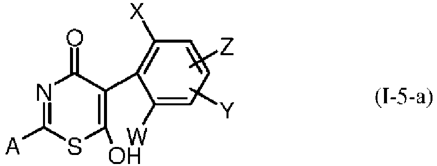 dans laquelle
A, W, X, Y et Z présentent la signification indiquée ci-dessus, des composés de formule (VII) 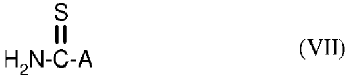
dans laquelle
A présente la signification susmentionnée,
sont transformés avec des composés de formule (VI) 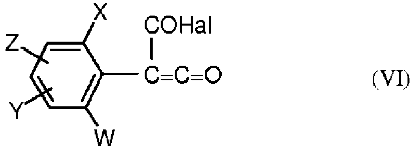 dans laquelle
Hal, W, X, Y et Z présentent les significations indiquées ci-dessus,
le cas échéant en présence d'un diluant et le cas échéant en présence d'un accepteur d'acide,
(F) des composés de formule (I-6-a) 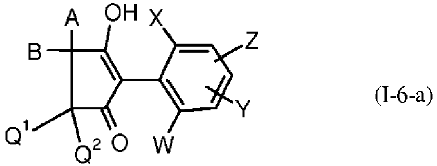 dans laquelle
A, B, Q¹, Q², W, X, Y et Z présentent la signification indiquée ci-dessus, des composés de formule (VIII) 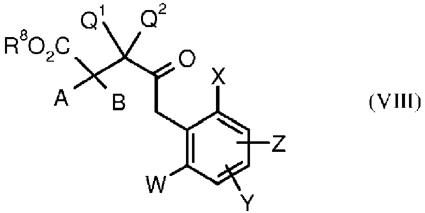
dans laquelle
A, B, Q¹, Q², W, X, Y et Z présentent la signification susmentionnée et
R⁸ représente alkyle,
sont cyclisés de manière intramoléculaire, le cas échéant en présence d'un diluant et en présence d'une base,
(G) des composés de formule (I-7-a) 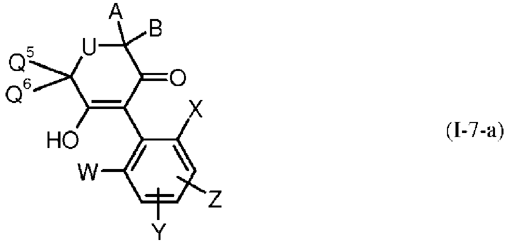 dans laquelle
A, B, Q⁵, Q⁶, U, W, X, Y et Z présentent la signification indiquée ci-dessus, des composés de formule (IX) 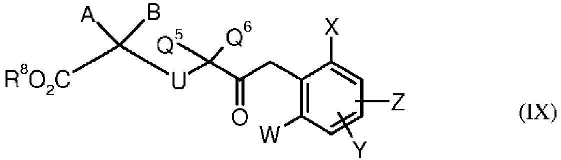
dans laquelle
A, B, Q⁵, Q⁶, U, W, X, Y et Z présentent la signification susmentionnée et
R⁸ représente alkyle,
sont condensés de manière intramoléculaire en présence d'un diluant et en présence d'une base,
(H) des composés de formule (I-8-a) 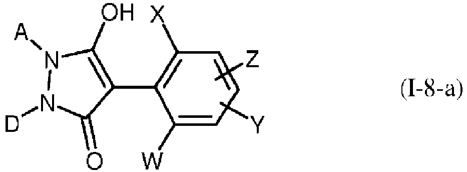 dans laquelle
A, D, W, X, Y et Z présentent les significations indiquées ci-dessus, des composés de formule (X) 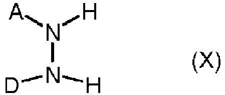
dans laquelle
A et D présentent la signification susmentionnée,
α) sont transformés avec des composés de formule (VI) 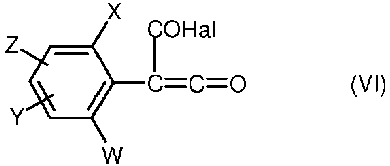 dans laquelle
Hal, W, X, Y et Z présentent les significations indiquées ci-dessus,
le cas échéant en présence d'un diluant et le cas échéant en présence d'un accepteur d'acide, ou
ß) sont transformés avec des composés de formule (XI) 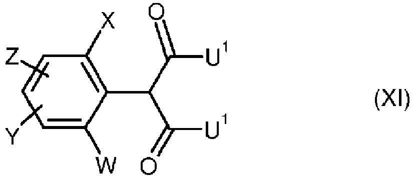 dans laquelle
W, X, Y et Z présentent la signification susmentionnée et
U¹ représente NH₂ ou O-R⁸, R⁸ présentant la signification susmentionnée,
le cas échéant en présence d'un diluant et le cas échéant en présence d'une base, ou
γ) sont transformés avec des composés de formule (XII) 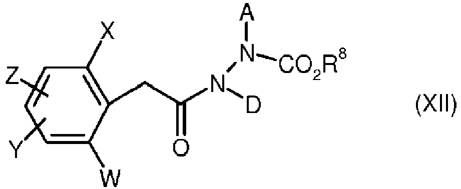
dans laquelle
A, D, W, X, Y, Z et R⁸ présentent la signification susmentionnée,
le cas échéant en présence d'un diluant et le cas échéant en présence d'une base,
(I) des composés de formule (I-9-a) 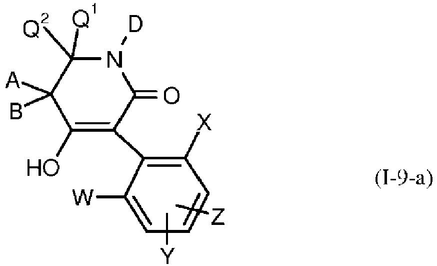 dans laquelle
A, B, D, Q¹, Q², W, X, Y et Z présentent les significations indiquées ci-dessus, des composés de formule (XIII) 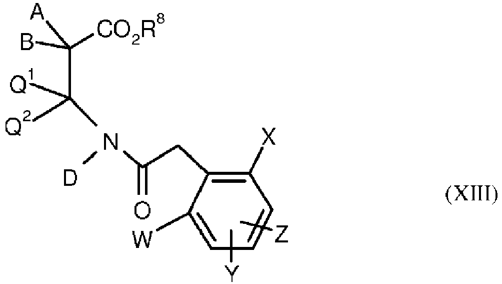
dans laquelle
A, B, D, Q¹, Q², W, X, Y et Z présentent les significations indiquées ci-dessus, et
R⁸ représente alkyle,
sont condensés de manière intramoléculaire en présence d'un diluant et en présence d'une base,
(J) des composés de formule (I-10-a) 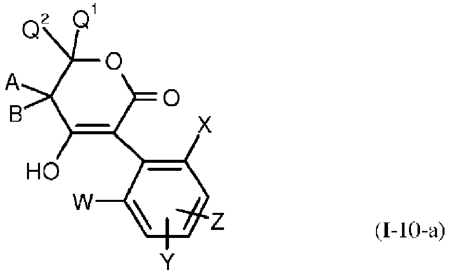 dans laquelle
A, B, Q¹, Q², W, X, Y et Z présentent les significations indiquées ci-dessus, des composés de formule (XIV) 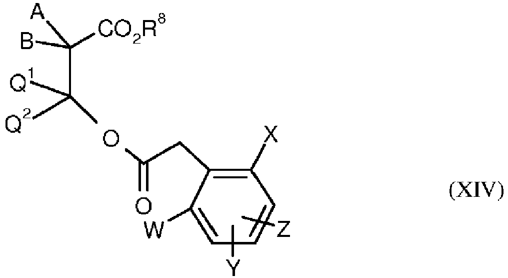
dans laquelle
A, B, Q¹, Q², W, X, Y et Z présentent les significations indiquées ci-dessus, et
R⁸ représente alkyle,
sont transformés le cas échéant en présence d'un diluant et le cas échéant en présence d'une base,
(K) des composés de formule (I-11-a) 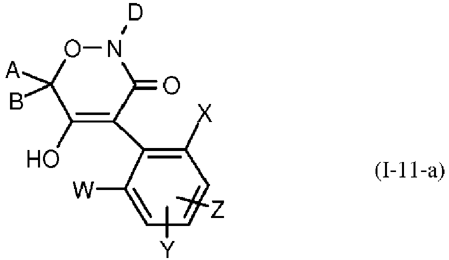 dans laquelle
A, B, D, W, X, Y et Z présentent les significations indiquées ci-dessus, des composés de formule (XV) 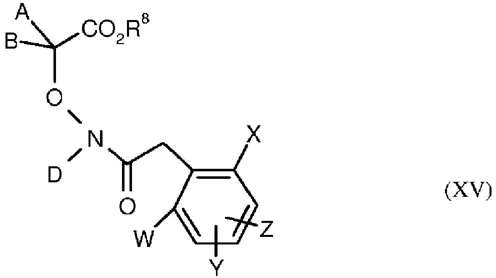
dans laquelle
A, B, D, W, X, Y et Z présentent les significations indiquées ci-dessus, et
R⁸ représente alkyle,
sont condensés de manière intramoléculaire en présence d'un diluant et en présence d'une base,
(L) des composés des formules (I-1-b) à (I-11-b) représentées ci-dessus, dans lesquelles A, B, D, Q¹, Q², Q⁵, Q⁶, R¹, U, W, X, Y et Z présentent les significations indiquées ci-dessus, des composés des formules (I-1-a) à (I-11-a) représentées ci-dessus, dans lesquelles A, B, D, Q¹, Q², Q⁵, Q⁶, U, W, X, Y et Z présentent les significations indiquées ci-dessus, sont transformés à chaque fois
(α) avec des halogénures d'acide de formule (XVI) 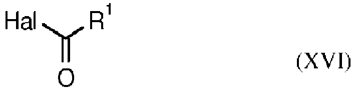 dans laquelle
R¹ présente la signification susmentionnée et
Hal représente halogène
ou
(ß) avec des anhydrides d'acide carboxylique de formule (XVII)
R¹-CO-O-CO-R¹ (XVII)
dans laquelle
R¹ présente la signification susmentionnée,
le cas échéant en présence d'un diluant et le cas échéant en présence d'un liant d'acide ;
(M) des composés des formules (I-1-c) à (I-11-c) représentées ci-dessus, dans lesquelles A, B, D, Q¹, Q², Q⁵, Q⁶, R², M, U, W, X, Y et Z présentent les significations indiquées ci-dessus et L représente oxygène, des composés des formules (I-1-a) à (I-11-a) représentées ci-dessus, dans lesquelles A, B, D, Q¹, Q², Q⁵, Q⁶, U, W, X, Y et Z présentent les significations indiquées ci-dessus, sont transformés à chaque fois
avec des esters de l'acide chloroformique ou des thiolesters de l'acide chloroformique de formule (XVIII)
R²-M-CO-Cl (XVIII)
dans laquelle
R² et M présentent les significations indiquées ci-dessus,
le cas échéant en présence d'un diluant et le cas échéant en présence d'un liant d'acide ;
(N) des composés des formules (I-1-c) à (I-11-c) représentées ci-dessus, dans lesquelles A, B, D, Q¹ Q², Q⁵, Q⁶, R², M, U, W, X, Y et Z présentent les significations indiquées ci-dessus et L représente soufre, des composés des formules (I-1-a) à (I-11-a) représentées ci-dessus, dans lesquelles A, B, D, Q¹, Q⁴, Q⁵, Q⁶, U, W, X, Y et Z présentent les significations indiquées ci-dessus, sont transformés à chaque fois
avec des esters de l'acide chloromonothioformique ou des esters de l'acide chlorodithioformique de formule (XIX) 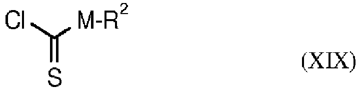 dans laquelle
M et R² présentent les significations indiquées ci-dessus,
le cas échéant en présence d'un diluant et le cas échéant en présence d'un liant d'acide et
(O) des composés des formules (I-1-d) à (I-11-d) représentées ci-dessus, dans lesquelles A, B, D, Q¹, Q², Q⁵, Q⁶, R³, U, W, X, Y et Z présentent les significations indiquées ci-dessus, des composés des formules (I-1-a) à (I-11-a) représentées ci-dessus, dans lesquelles A, B, D, Q¹, Q², Q⁵, Q⁶, U, W, X, Y et Z présentent les significations indiquées ci-dessus, sont transformés à chaque fois avec des chlorures d'acide sulfonique de formule (XX)
R³-SO₂-Cl (XX)
dans laquelle
R³ présente la signification susmentionnée,
le cas échéant en présence d'un diluant et le cas échéant en présence d'un liant d'acide,
(P) des composés des formules (I-1-e) à (I-11-e) représentées ci-dessus, dans lesquelles A, B, D, L, Q¹, Q², Q⁵, Q⁶, R⁴, R⁵, U, W, X, Y et Z présentent les significations indiquées ci-dessus, des composés des formules (I-1-a) à (I-11-a) représentées ci-dessus, dans lesquelles A, B, D, Q¹, Q², Q⁵, Q⁶, U, W, X, Y et Z présentent les significations indiquées ci-dessus, sont transformés à chaque fois
avec des composés phosphorés de formule (XXI) 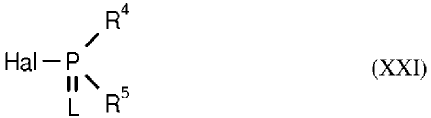 dans laquelle
L, R⁴ et R⁵ présentent les significations indiquées ci-dessus, et
Hal représente halogène,
le cas échéant en présence d'un diluant et le cas échéant en présence d'un liant d'acide,
(Q) des composés des formules (I-1-f) à (I-11-f) représentées ci-dessus, dans lesquelles A, B, D, E, Q¹, Q², Q⁵, Q⁶, U, W, X, Y et Z présentent les significations indiquées ci-dessus, des composés des formules (I-1-a) à (I-11-a) représentées ci-dessus, dans lesquelles A, B, D, Q¹, Q², Q⁵, Q⁶, U, W, X, Y et Z présentent les significations indiquées ci-dessus, sont transformés à chaque fois
avec des composés métalliques ou des amines des formules (XXII ) ou (XXIII)
Me(OR¹⁰)ₜ (XXII) 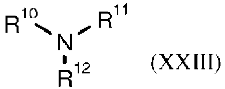
dans lesquelles
Me représente un métal monovalent ou divalent ou un ion d'ammonium 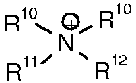
t vaut le nombre 1 ou 2 et
R¹⁰, R¹¹, R¹² représentent, indépendamment les uns des autres, hydrogène ou alkyle,
le cas échéant en présence d'un diluant,
(R) des composés des formules (I-1-g) à (I-11-g) représentées ci-dessus, dans lesquelles A, B, D, L, Q¹, Q², Q⁵, Q⁶, R⁶, R⁷, U, W, X, Y et Z présentent les significations indiquées ci-dessus, des composés des formules (I-1-a) à (I-11-a) représentées ci-dessus, dans lesquelles A, B, D, Q¹, Q², Q⁵, Q⁶, U, W, X, Y et Z présentent les significations indiquées ci-dessus, sont transformés à chaque fois
(α) avec des isocyanates ou des isothiocyanates de formule (XXIV)
R⁶-N=C=L (XXIV)
dans laquelle
R⁶ et L présentent les significations indiquées ci-dessus,
le cas échéant en présence d'un diluant et le cas échéant en présence d'un catalyseur ou
(ß) avec des chlorures d'acide carbamique ou des chlorures d'acide thiocarbamique de formule (XXV) 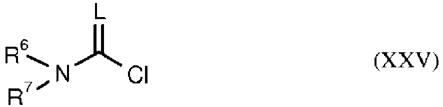
dans laquelle
L, R⁶ et R⁷ présentent les significations indiquées ci-dessus,
le cas échéant en présence d'un diluant et le cas échéant en présence d'un liant d'acide,
(S) des composés des formules (I-1-a) à (I-11-g) représentées ci-dessus, dans lesquelles A, B, D, G, Q¹, Q², Q⁵, Q⁶, U, W, X, Y et Z présentent la signification indiquée ci-dessus, on remplace dans des composés des formules (I-1') à (I-11'), dans lesquelles A, B, D, G, Q¹, Q², Q⁵, Q⁶, U, W, X, Y et Z présentent la signification indiquée ci-dessus et Z' représente de préférence brome ou iode 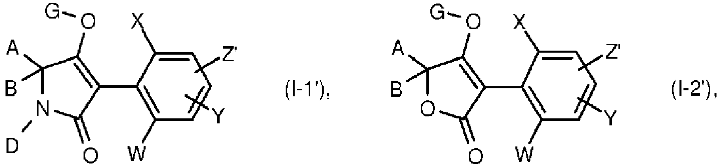 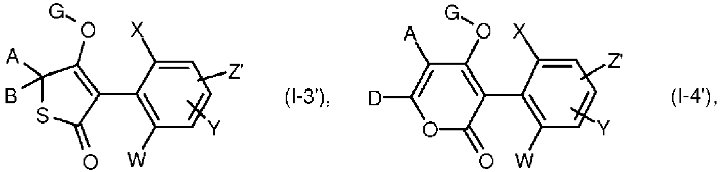 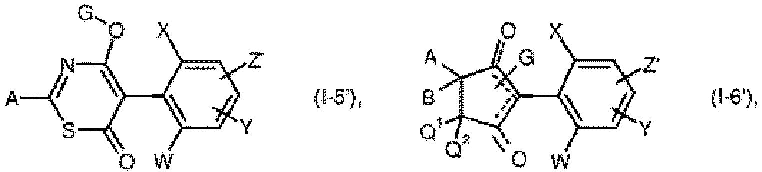 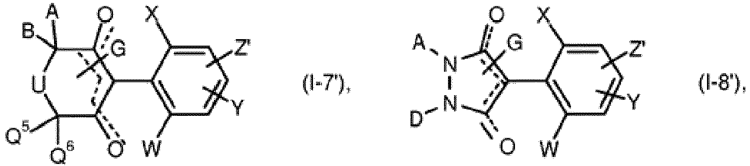 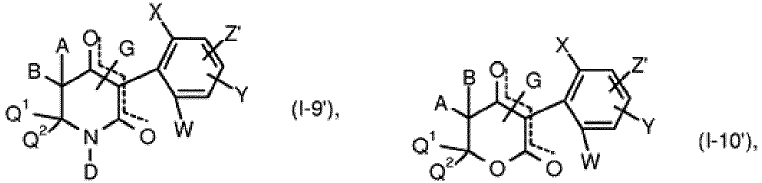 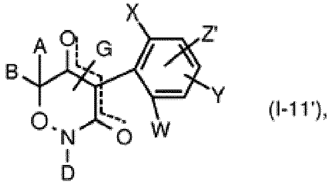 à l'aide d'alcools halogénés, par exemple le trifluoroéthanol de formule (XXVI)
Z-OH (XXVI)
en présence d'un solvant, en présence d'un sel de cuivre et en présence d'une base, l'atome de brome ou d'iode.

7. Agent pour lutter contre des organismes nuisibles et/ou une croissance non souhaitée de plantes, **caractérisé par** une teneur en au moins un composé de formule (I) selon la revendication 1.

8. Procédé pour lutter contre des organismes nuisibles animaux et/ou une couverture végétale non souhaitée, **caractérisé en ce qu'**on laisse agir des composés de formule (I) selon la revendication 1 sur des organismes nuisibles, sur une croissance non souhaitée des plantes et/ou sur leur espace de vie, à l'exception de procédés de traitement chirurgical ou thérapeutique du corps humain et animal.

9. Utilisation de composés de formule (I) selon la revendication 1 pour lutter contre des organismes nuisibles animaux et/ou une couverture végétale non souhaitée, à l'exception de l'utilisation dans des procédés de traitement chirurgical ou thérapeutique du corps humain ou animal.

10. Procédé pour la préparation d'agents destinés à lutter contre des organismes nuisibles et/ou une couverture végétale non souhaitée, **caractérisé en ce qu'**on mélange des composés de formule (I) selon la revendication 1 avec des agents d'allongement et/ou des substances tensioactives.

11. Utilisation de composés de formule (I) selon la revendication 1 pour la préparation d'agents destinés à lutter contre des organismes nuisibles et/ou une croissance non souhaitée des plantes.

12. Agent contenant une teneur active en une combinaison de substances actives comprenant, comme composants
(a') au moins un composé de formule (I) selon la revendication 1, dans laquelle W, X, Y, Z et CKE présentent la signification indiquée ci-dessus et
(b') au moins un composé améliorant la tolérance des plantes de culture du groupe suivant de composés : S1, S2, S3, S4, S5, S6, S7, S8, S9, S10, S11, S12, S13, S14, S15, dans lequel
S1) des composés de formule (S1), 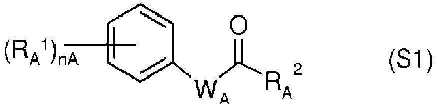 dans laquelle les symboles et les indices présentent les significations suivantes :
n_{A} vaut un nombre naturel de 0 à 5,
R_{A}¹ représente halogène, (C₁-C₄)-alkyle, (C₁-C₄)-alcoxy, nitro ou (C₁-C₄)-halogénoalkyle ;
W_{A} représente un radical hétérocyclique divalent non substitué ou substitué du groupe des hétérocycles à cinq chaînons, partiellement insaturés ou aromatiques, comprenant 1 à 3 hétéroatomes de cycle du groupe N et O, au moins un atome de N et au plus un atome de O étant contenus dans le cycle,
m_{A} vaut 0 ou 1 ;
R_{A}² représente OR_{A}³ SR_{A}³ ou NR_{A}³R_{A}⁴ ou un hétérocycle saturé ou insaturé de 3 à 7 chaînons, présentant au moins un atome de N et jusqu'à 3 hétéroatomes, de préférence du groupe O et S, qui est lié via l'atome de N au groupe carbonyle dans (S1) et qui est non substitué ou substitué par des radicaux du groupe (C₁-C₄)alkyle, (C₁-C₄)alcoxy ou phényle le cas échéant substitué,
R_{A}³ représente hydrogène ou un radical hydrocarboné aliphatique non substitué ou substitué,
R_{A}⁴ représente hydrogène, (C₁-C₆)alkyle, (C₁-C₆)alcoxy ou phényle substitué ou non substitué ;
R_{A}⁵ représente H, (C₁-C₈)-alkyle, (C₁-C₈)-halogénoalkyle, (C₁-C₄)-alcoxy-(C₁-C₈)-alkyle, cyano ou COOR_{A}⁹, où R_{A}⁹ représente hydrogène, (C₁-C₈)-alkyle, (C₁-C₈)-halogénoalkyle, (C₁-C₄)-alcoxy-(C₁-C₄)-alkyle, (C₁-C₆)-hydroxyalkyle, (C₃-C₁₂)-cycloalkyle ou tri-(C₁-C₄)-alkyl-silyle;
R_{A}⁶, R_{A}⁷, R_{A}⁸ sont identiques ou différents et représentent hydrogène, (C₁-C₈)-alkyle, (C₁-C₈)-halogénoalkyle, (C₃-C₁₂)-cycloalkyle ou phényle substitué ou non substitué ;
S2) des dérivés de quinoléine de formule (S2) 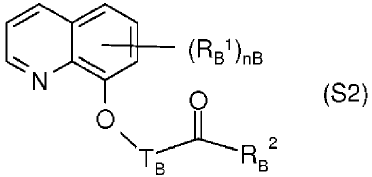 dans laquelle les symboles et les indices présentent les significations suivantes :
R_{B}¹ représente halogène, (C₁-C₄)-alkyle, (C₁-C₄)-alcoxy, nitro ou (C₁-C₄)-halogénoalkyle ;
n_{B} vaut un nombre naturel de 0 à 5,
R_{B}² représente OR_{B}³, SR_{B}³ ou NR_{B}³R_{B}⁴ ou un hétérocycle saturé ou insaturé de 3 à 7 chaînons, présentant au moins un atome de N et jusqu'à 3 hétéroatomes, de préférence du groupe O et S, qui est lié via l'atome de N au groupe carbonyle dans (S2) et qui est non substitué ou substitué par des radicaux du groupe (C₁-C₄)-alkyle, (C₁-C₄)-alcoxy ou phényle le cas échéant substitué,
R_{B}³ représente hydrogène ou un radical hydrocarboné aliphatique non substitué ou substitué,
R_{B}⁴ représente hydrogène, (C₁-C₆)alkyle, (C₁-C₆)alcoxy ou phényle substitué ou non substitué ;
T_{B} représente une chaîne (C₁ ou C₂)-alcanediyle, qui est non substituée ou substituée par un ou deux radicaux (C₁-C₄)alkyle ou par [(C₁-C₃)-alcoxy]-carbonyle ;
S3) des composés de formule (S3) 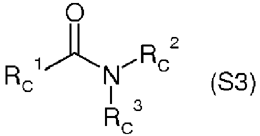 dans laquelle les symboles et les indices présentent les significations suivantes :
R_{c}¹ représente (C₁-C₄)-alkyle, (C₁-C₄)-halogénoalkyle, (C₂-C₄)-alcényle, (C₂-C₄)-halogénoalcényle, (C₃-C₇)-cycloalkyle,
R_{c}², R_{c}³ sont identiques ou différents et représentent hydrogène, (C₁-C₄)-alkyle, (C₂-C₄)-alcényle, (C₂-C₄)-alcynyle, (C₁-C₄)-halogénoalkyle, (C₂-C₄)-halogénoalcényle, (C₁-C₄)-alkylcarbamoyl-(C₁-C₄)-alkyle, (C₂-C₄)-alcénylcarbamoyl-(C₁-C₄)-alkyle, (C₁-C₄)-alcoxy-(C₁-C₄)-alkyle, dioxolanyl-(C₁-C₄)-alkyle, thiazolyle, furyle, furylalkyle, thiényle, pipéridyle, phényle substitué ou non substitué ou R_{c}² et R_{c}³ forment ensemble un cycle hétérocyclique substitué ou non substitué,
S4) des N-acylsulfonamides de formule (S4) et leurs sels, 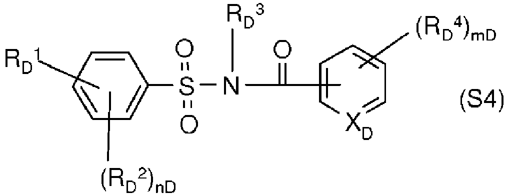 dans laquelle les symboles et les indices présentent les significations suivantes :
X_{D} représente CH ou N ;
R_{D}¹ représente CO-NR_{D}⁵R_{D}⁶ ou NHCO-R_{D}⁷ ;
R_{D}² représente halogène, (C₁-C₄) -halogénoalkyle, (C₁-C₄) -halogénoalcoxy, nitro, (C₁-C₄)-alkyle, (C₁-C₄)-alcoxy, (C₁-C₄)-alkylsulfonyle, (C₁-C₄)-alcoxycarbonyle ou (C₁-C₄)-alkylcarbonyle ;
R_{D}³ représente hydrogène, (C₁-C₄)-alkyle, (C₂-C₄)-alcényle ou (C₂-C₄)-alcynyle ;
R_{D}⁴ représente halogène, nitro, (C₁-C₄)-alkyle, (C₁-C₄)-halogénoalkyle, (C₁-C₄)-halogénoalcoxy, (C₃-C₆)-cycloalkyle, phényle, (C₁-C₄)-alcoxy, cyano, (C₁-C₄) -alkylthio, (C₁-C₄)-alkylsulfinyle, (C₁-C₄) -alkylsulfonyle, (C₁-C₄)-alcoxycarbonyle ou (C₁-C₄)-alkylcarbonyle ;
R_{D}⁵ représente hydrogène, (C₁-C₆)-alkyle, (C₃-C₆)-cycloalkyle, (C₂-C₆) -alcényle, (C₂-C₆)-alcynyle, (C₅-C₆)-cycloalcényle, phényle ou hétérocyclyle de 3 à 6 chaînons, contenant v_{D} hétéroatomes du groupe formé par azote, oxygène et soufre, les sept derniers radicaux mentionnés étant substitués par v_{D} substituants du groupe formé par halogène, (C₁-C₆)-alcoxy, (C₁-C₆)-halogénoalcoxy, (C₁-C₂)-alkylsulfinyle, (C₁-C₂)-alkylsulfonyle, (C₃-C₆)-cycloalkyle, (C₁-C₄)-alcoxycarbonyle, (C₁-C₄) -alkylcarbonyle et phényle et, dans le cas de radicaux cycliques, également par (C₁-C₄)-alkyle et (C₁-C₄)-halogénoalkyle ;
R_{D}⁶ représente hydrogène, (C₁-C₆)-alkyle, (C₂-C₆)-alcényle ou (C₂-C₆)-alcynyle, les trois derniers radicaux mentionnés étant substitués par v_{D} radicaux du groupe formé par halogène, hydroxy, (C₁-C₄)-alkyle, (C₁-C₄)-alcoxy et (C₁-C₄)-alkylthio, ou
R_{D}⁵ et R_{D}⁶ forment, ensemble avec l'atome d'azote qui les porte, un radical pyrrolidinyle ou pipéridinyle ;
R_{D}⁷ représente hydrogène, (C₁-C₄)-alkylamino, di-(C₁-C₄)-alkylamino, (C₁-C₆)-alkyle, (C₃-C₆)-cycloalkyle, les 2 derniers radicaux mentionnés étant substitués par v_{D} substituants du groupe formé par halogène, (C₁-C₄)-alcoxy, (C₁-C₆)-halogénoalcoxy et (C₁-C₄)-alkylthio et, dans le cas de radicaux cycliques, également par (C₁-C₄)-alkyle et (C₁-C₄)-halogénoalkyle ;
n_{D} vaut 0, 1 ou 2 ;
m_{D} vaut 1 ou 2 ;
v_{D} vaut 0, 1, 2 ou 3 ;
S5) des substances actives de la classe des
hydroxyaromatiques et des dérivés d'acide carboxylique aromatique-aliphatique (S5),
S6) des substances actives de la classe des 1,2-dihydroquinoxalin-2-ones (S6),
S7) des composés de formule (S7), 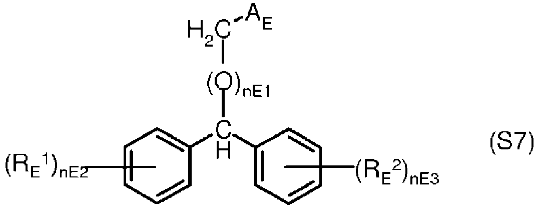 dans laquelle les symboles et les indices présentent les significations suivantes :
R_{E}¹, R_{E}² représentent, indépendamment l'un de l'autre, halogène, (C₁-C₄)-alkyle, (C₁-C₄)-alcoxy, (C₁-C₄)-halogénoalkyle, (C₁-C₄)-alkylamino, di-(C₁-C₄)-alkylamino, nitro ;
A_{E} représente COOR_{E}³ ou COSR_{E}⁴
R_{E}³, R_{E}⁴ représentent, indépendamment l'un de l'autre, hydrogène, (C₁-C₄)-alkyle, (C₂-C₆)-alcényle, (C₂-C₄)-alcynyle, cyanoalkyle, (C₁-C₄)-halogénoalkyle, phényle, nitrophényle, benzyle, halogénobenzyle, pyridinylalkyle et alkylammonium,
n_{E}¹ vaut 0 ou 1,
n_{E}², n_{E}³ valent, indépendamment l'un de l'autre, 0, 1 ou 2,
S8) des composés de formule (S8), tels qu'ils sont décrits dans le document WO-A-98/27049 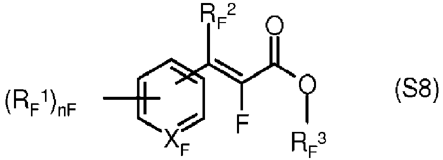 dans laquelle
X_{F} représente CH ou N
n_{F} dans le cas où X_{F} = N, vaut un nombre entier de 0 à 4 et pour le cas où X_{F} = CH, vaut un nombre entier de 0 à 5,
R_{F}¹ halogène, (C₁-C₄)-alkyle, (C₁-C₄)-halogénoalkyle, (C₁-C₄)-alcoxy, (C₁-C₄)-halogénoalcoxy, nitro, (C₁-C₄) -alkylthio, (C₁-C₄)-alkylsulfonyle, (C₁-C₄)-alcoxycarbonyle, phényle le cas échéant substitué, phénoxy le cas échéant substitué,
R_{F}² signifie hydrogène ou (C₁-C₄)-alkyle,
R_{F}³ signifie hydrogène, (C₁-C₈)-alkyle, (C₂-C₄)-alcényle, (C₂-C₄)-alcynyle ou aryle, chacun des radicaux contenant C susmentionnés étant non substitué ou substitué par un ou plusieurs radicaux identiques ou différents du groupe constitué par halogène et alcoxy ; ou leurs sels,
S9) des substances actives de la classe des 3-(5-tétrazolylcarbonyl)-2-quinolones (S9),
S10) des composés des formules (S10^{a}) ou (S10^{b}) 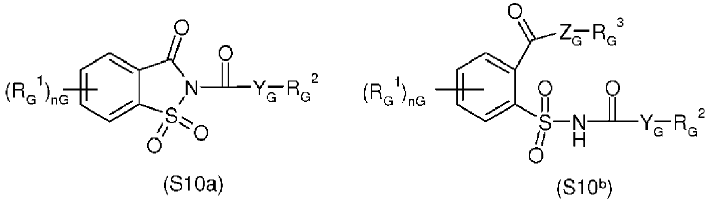 dans lesquelles
R_{G}¹ représente halogène, (C₁-C₄)-alkyle, méthoxy, nitro, cyano, CF₃, OCF₃
Y_{G}, Z_{G} représentent, indépendamment l'un de l'autre, O ou S,
n_{G} vaut un nombre entier de 0 à 4,
R_{G}² représente (C₁-C₁₆)-alkyle, (C₂-C₆) -alcényle, (C₃-C₆)-cycloalkyle, aryle ; benzyle, halogénobenzyle,
R_{G}³ signifie hydrogène ou (C₁-C₆)-alkyle ;
S11) des substances actives du type des composés oxyimino (S11), qui sont connus comme agent de décapage de semences,
S12) des substances actives de la classe des isothiochromanones (S12),
S13) un ou plusieurs des composés du groupe (S13) :
"Naphthalic anhydrid" (anhydride de l'acide 1,8-naphtalènedicarboxylique) (S13-1),
"Fenclorim" (4,6-dichloro-2-phénylpyrimidine) (S13-2),
"Flurazole" (2-chloro-4-trifluorométhyl-1,3-thiazole-5-carboxylate de benzyle) (S13-3),
"CL 304415" (acide 4-carboxy-3,4-dihydro-2H-1-benzopyran-4-acétique) (S13-4) "MG 191" (2-dichlorométhyl-2-méthyl-1,3-dioxolane) (S13-5)
"MG-838" (1-oxa-4-azaspiro[4.5]décane-4-carbodithioate de 2-propényle) (S13-6) "Disulfoton" (phosphorodithioate d'O,O-diéthyle et de S-2-éthylthioéthyle) (S13-7),
"Dietholate" (phosphorotioate d'O,O-diéthyl-O-phényle) (S13-8),
"Mephenate" (4-chlorophényl-méthylcarbamate) (S13-9).
S14) des substances actives qui présentent, outre un effet herbicide contre des plantes nuisibles, également un effet d'antidote sur les plantes de culture, telles que le riz,
S15) des substances actives qui sont utilisées principalement comme herbicides mais qui présentent également un effet d'antidote sur les plantes de culture.

13. Agent selon la revendication 12, dans lequel le composé améliorant la tolérance des plantes de culture est le cyprosulfamide.

14. Agent selon la revendication 12, dans lequel le composé améliorant la tolérance des plantes de culture est le Méfenpyr-diéthyl.

15. Procédé pour lutter contre la croissance non souhaitée de plantes, **caractérisé en ce qu'**on laisse agir un agent selon la revendication 12 sur les végétaux ou leur environnement.

16. Utilisation d'un agent selon la revendication 12 pour lutter contre la croissance non souhaitée de végétaux.

17. Procédé pour lutter contre la croissance non souhaitée de plantes, **caractérisé en ce qu'**on laisse agir un composé de formule (I) selon la revendication 1 et le composé améliorant la tolérance des plantes de culture selon la revendication 12 séparément, dans une succession proche dans le temps, sur les végétaux ou leur environnement.

18. Composés de formule (II) 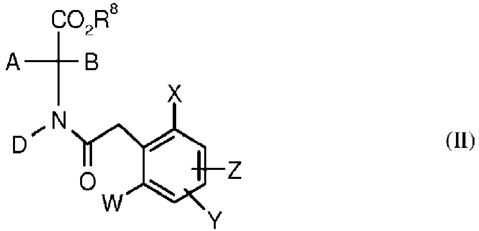 dans laquelle A, B, D, W, X, Y et Z présentent les significations indiquées ci-dessus et R⁸ représente alkyle.

19. Composés de formule (III) 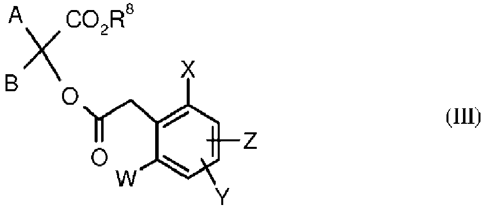 dans laquelle A, B, W, X, Y, Z et R⁸ présentent les significations indiquées ci-dessus.

20. Composés de formule (VIII) 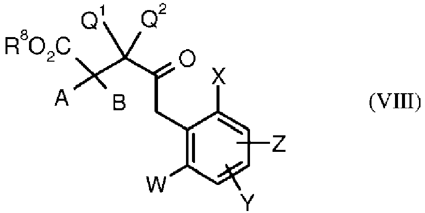 dans laquelle A, B, Q¹, Q², W, X, Y, Z et R⁸ présentent la signification indiquée ci-dessus.

21. Composé de formule (XII) 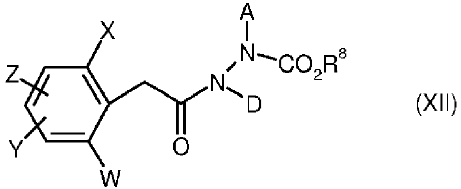 dans laquelle A, D, W, X, Y, Z et R⁸ présentent la signification indiquée ci-dessus.

22. Composés de formule (XXXI) 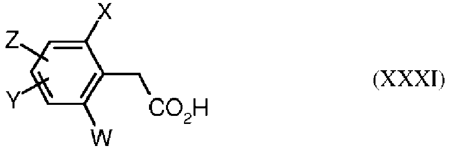 dans laquelle W, X, Y et Z présentent la signification indiquée ci-dessus, à l'exception du composé de formule

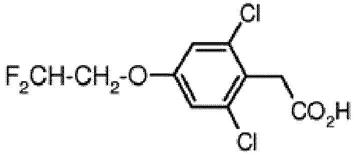

23. Composés de formule (XXVIII) 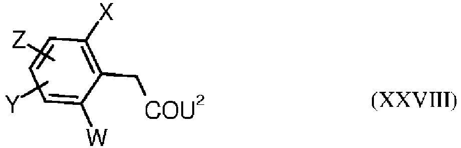 dans laquelle W, X, Y, Z présentent les significations indiquées ci-dessus et U² représente un groupe partant introduit par des réactifs d'activation d'acide carboxylique, des carbonyldiimides, des réactifs de phosphorylation, des agents d'halogénation, le phosgène ou des esters de l'acide chloroformique.

24. Composés de formule (XXXV) 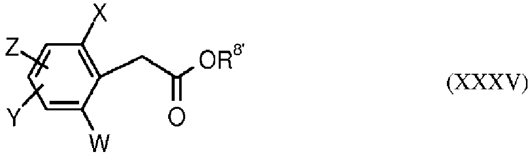 dans laquelle W, X, Y, Z présentent la signification indiquée ci-dessus et R⁸' représente alkyle.

25. Composition comprenant
- au moins un composé de formule (I) selon la revendication 1 ou un agent selon la revendication 12 et
- au moins un sel de formule (III') 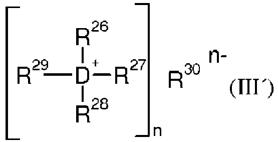 dans laquelle
D représente azote ou phosphore,
R²⁶, R²⁷, R²⁸ et R²⁹ représentent, indépendamment les uns des autres, hydrogène ; ou C₁-C₈-alkyle à chaque fois le cas échéant substitué ou C₁-C₈-alkylène monoinsaturé ou polyinsaturé, le cas échéant substitué, les substituants pouvant être choisis parmi halogène, nitro et cyano,
n vaut 1, 2, 3 ou 4,
R³⁰ représente un anion inorganique ou organique.

26. Composition selon la revendication 25, **caractérisée en ce qu'**elle contient au moins un agent favorisant la pénétration.

27. Procédé pour augmenter l'action d'agents de lutte contre les organismes nuisibles et/ou d'herbicides contenant une substance active de formule (I) selon la revendication 1 ou un agent selon la revendication 12, **caractérisé en ce que** l'agent prêt à l'emploi (bouillon à pulvériser) est préparé avec utilisation d'un sel de formule (III') selon la revendication 25.

28. Procédé selon la revendication 27, **caractérisé en ce que** le bouillon à pulvériser est préparé avec utilisation d'un agent favorisant la pénétration.
